# EUROPEAN PATENT SPECIFICATION

(11) **EP 1 976 849 B1**
(45) Date of publication and mention of the grant of the patent: **09.03.2016**
(21) Application number: 07709799.6
(22) Date of filing: 16.01.2007
(51) Int. Cl.: C07D 401/14, C07D 403/06, A61P 35/00, A61K 31/4166, C07D 471/04, C07D 473/34, C07D 473/00, C07D 491/04, C07D 495/04, C07D 417/14, C07D 413/14, C07D 405/14, C07D 403/14

(54) **COMPOUNDS FOR THE TREATMENT OF INFLAMMATORY DISORDERS**
VERBINDUNGEN ZUR BEHANDLUNG ENTZÜNDLICHER ERKRANKUNGEN
COMPOSES POUR LE TRAITEMENT DE TROUBLES INFLAMMATOIRES

(30) Priority: 17.01.2006 US 333663
(43) Date of publication of application: 08.10.2008
(73) Proprietor: Merck Sharp & Dohme Corp., Rahway, NJ 07065-0907 (US)
(72) Inventor: YU, Wensheng, Edison, NJ 08820 (US); TONG, Ling, Warren, NJ 07059 (US); CHEN, Lei, Roselle Park, NJ 07204 (US); KOZLOWSKI, Joseph, A., Princeton, NJ 08540 (US); LAVEY, Brian, J., New Providence, NJ 07974 (US); SHIH, Neng-Yang, Warren, NJ 07059 (US); MADISON, Vincent, S., Mountain Lakes, NJ 07046 (US); ZHOU, Guowei, Livingston, NJ 07039 (US); ORTH, Peter, New York, NY 10016 (US); GUO, Zhuyan, Scotch Plains, NJ 07076 (US); WONG, Michael, K.C., North Brunswick, NJ 08902 (US); YANG, De-Yi, Morris Plains, NJ 07950 (US); KIM, Seong-Heon, Livingtston, NJ 07039 (US); SHANKAR, Bandarpalle, B., Branchburg, NJ 08853 (US); SIDDIQUI, M., Arshad, Newton, Massachusetts 02464 (US); ROSNER, Kristin, E., Watertown, MA 02472 (US); DAI, Chaoyang, Acton, MA 01720 (US); POPOVICI-MULLER, Janeta, Waltham, MA 02452 (US); GIRIJAVALLABHAN, Vinay, M., Denville,New Jersey 07834 (US); LI, Dansu, Watertown, Massachusetts 02472 (US); RIZVI, Razia, Reading,Massachusetts 01867 (US); MICULA, Aneta, S, South Amboy, NJ 08879 (US); FELTZ, Robert Schering-Plough Corporation, Kenilworth, NJ 07033-0530 (US)
(74) Representative: Horgan, James Michael Frederic
(86) International application number: PCT/US2007/000930
(87) International publication number: WO 2007/084415

(56) References cited:
- WO-A-02/074750
- WO-A-02/096426
- WO-A-2007/084455
- US-A1- 2006 276 506
- DATABASE CA [Online] CHEMICAL ABSTRACTS SERVICE, COLUMBUS, OHIO, US; RN 877316-69-3 2006, YU, WENSHENG ET AL: "Hydantoin derivatives as matrix metalloprotease inhibitors and their preparation, pharmaceutical compositions, and use for the treatment of inflammatory disorders" XP002447652 retrieved from STN Database accession no. 2006:167381 -& WO 2006/019768 A1 (SCHERING CORPORATION, USA) 23 February 2006 (2006-02-23)

## Description

### Field of the Invention

This invention relates generally to novel hydantoin derivatives that can inhibit matrix metalloproteinases (MMPs), a disintegrin and metalloproteases (ADAMs) and/or tumor necrosis factor alpha - converting enzyme (TACE) and in so doing prevent the release of tumor necrosis factor alpha (TNF-α), pharmaceutical compositions comprising such compounds.

### BACKGROUND OF THE INVENTION

Osteo- and rheumatoid arthritis (OA and RA, respectively) are destructive diseases of articular cartilage characterized by localized erosion of the cartilage surface. Findings have shown that articular cartilage from the femoral heads of patients with OA, for example, had a reduced incorporation of radiolabeled sulfate over controls, suggesting that there must be an enhanced rate of cartilage degradation in OA (Mankin et al. J. Bone Joint Surg. 52A (1970) 424-434). There are four classes of protein degradative enzymes in mammalian cells: serine, cysteine, aspartic and metalloproteases. The available evidence supports the belief that it is the metalloproteases that are responsible for the degradation of the extracellular matrix of articullar cartilage in OA and RA. Increased activities of collagenases and stromelysin have been found in OA cartilage and the activity correlates with severity of the lesion (Mankin et al. Arthritis Rheum. 21, 1978, 761-766, Woessner et al. Arthritis Rheum. 26, 1983, 63-68 and Ibid. 27, 1984, 305-312). In addition, aggrecanase (a newly identified metalloprotease) has been identified that provides the specific cleavage product of proteoglycan, found in RA and OA patients (Lohmander L. S. et al. Arthritis Rheum. 36, 1993, 1214-22).

Metalloproteases (MPs) have been implicated as the key enzymes in the destruction of mammalian cartilage and bone. It can be expected that the pathogenesis of such diseases can be modified in a beneficial manner by the administration of MP inhibitors (see Wahl et al. Ann. Rep. Med. Chem. 25, 175-184, AP, San Diego, 1990).

MMPs are a family of over 20 different enzymes that are involved in a variety of biological processes important in the uncontrolled breakdown of connective tissue, including proteoglycan and collagen, leading to resorption of the extracellular matrix. This is a feature of many pathological conditions, such as RA and OA, corneal, epidermal or gastric ulceration; tumor metastasis or invasion; periodontal disease and bone disease. Normally these catabolic enzymes are tightly regulated at the level of their synthesis as well as at their level of extracellular activity through the action of specific inhibitors, such as alpha-2-macroglobulins and TIMPs (tissue inhibitor of MPs), which form inactive complexes with the MMP's.

Tumor necrosis factor alpha (TNF-α) is a cell-associated cytokine that is processed from a 26 kDa precursor form to a 17 kd active form. *See* Black R.A. "Tumor necrosis factor-alpha converting enzyme" Int J Biochem Cell Biol. 2002 Jan; 34(1):1-5 and Moss ML, White JM, Lambert MH, Andrews RC."TACE and other ADAM proteases as targets for drug discovery" Drug Discov Today. 2001 Apr 1;6(8):417-426, each of which is incorporated by reference herein.

TNF-α has been shown to play a pivotal role in immune and inflammatory responses. Inappropriate or over-expression of TNF-α is a hallmark of a number of diseases, including RA, Crohn's disease, multiple sclerosis, psoriasis and sepsis. Inhibition of TNF-α production has been shown to be beneficial in many preclinical models of inflammatory disease, making inhibition of TNF-α production or signaling an appealing target for the development of novel anti-inflammatory drugs.

TNF-α is a primary mediator in humans and animals of inflammation, fever and acute phase responses, similar to those observed during acute infection and shock. Excess TNF-α has been shown to be lethal. Blocking the effects of TNF-α with specific antibodies can be beneficial in a variety of conditions, including autoimmune diseases such as RA (Feldman et al, Lancet, (1994) 344, 1105), non-insulin dependent diabetes mellitus (Lohmander L. S. et al., Arthritis Rheum. 36 (1993) 1214-22) and Crohn's disease (Macdonald T. et al., Clin. Exp. Immunol. 81 (1990) 301).

Compounds that inhibit the production of TNF-α are therefore of therapeutic importance for the treatment of inflammatory disorders. Recently it has been shown that metalloproteases, such as TACE, are capable of converting TNF-α from its inactive to active form (Gearing et al Nature, 1994, 370, 555). Since excessive TNF-α production has been noted in several disease conditions also characterized by MMP-mediated tissue degradation, compounds which inhibit both MMPs and TNF-α production may also have a particular advantage in diseases where both mechanisms are involved.

One approach to inhibiting the harmful effects of TNF-α is to inhibit the enzyme, TACE before it can process TNF-α to its soluble form. TACE is a member of the ADAM family of type I membrane proteins and mediates the ectodomain shedding of various membrane-anchored signaling and adhesion proteins. TACE has become increasingly important in the study of several diseases, including inflammatory disease, because of its role in cleaving TNF-α from its "stalk" sequence and thus releasing the soluble form of the TNF-α protein (Black R.A. Int J Biochem Cell Biol. 2002 34,1-5).

There are numerous patents and publications which disclose hydroxamate, sulphonamide, hydantoin, carboxylate and/or lactam based MMP inhibitors.

US 6,677,355 and US 6,534,491 (B2), describe compounds that are hydroxamic acid derivatives and MMP inhibitors.

US 6,495,565 discloses lactam derivatives that are potential inhibitors of MMPs and/or TNF-α.

PCT Publications WO2002/074750, WO2002/096426, WO20040067996, WO2004012663, WO200274750 and WO2004024721 disclose hydantoin derivatives that are potential inhibitors of MMPs.

PCT Publications WO2004024698 and WO2004024715 disclose sulphonamide derivatives that are potential inhibitors of MMPs.

PCT Publications WO2004056766, WO2003053940 and WO2003053941 also describe potential inhibitors of TACE and MMPs.

WO-A-02074750 (Astra Zeneca AB) discloses metalloproteinase inhibitors derived from hydantoins bearing unspecified functional groups.

WO-A-02096426 (Bristol-Myers Squibb Company) discloses spirocyclic hydantoin derivatives as inhibitors of matrix metalloproteinases.

US-A-20060276506 and WO-A-2006019768 (both Schering-Plough Corporation) are relevant for novelty only and disclose various hydantoin derivatives as inhibitors of matrix metalloproteinases.

There is a need in the art for inhibitors of MMPs, ADAMs, TACE, and TNF-α, which can be useful as anti-inflammatory compounds and cartilage protecting therapeutics. The inhibition of TNF-α, TACE and or other MMPs can prevent the degradation of cartilage by these enzymes, thereby alleviating the pathological conditions of OA and RA as well as many other auto-immune diseases.

### SUMMARY OF THE INVENTION

In its many embodiments, the present invention provides novel compounds as inhibitors of TACE, the production of TNF-α, MMPs, ADAMs or any combination thereof, methods of preparing such compounds, pharmaceutical compositions comprising one or more such compounds, methods of preparing pharmaceutical formulations comprising one or more such compounds, and discloses methods of treatment, prevention, inhibition or amelioration of one or more diseases associated with TACE, TNF-α, MMPs, ADAMs or any combination thereof using such compounds or pharmaceutical compositions.

The present invention provides a compound of formula (I) selected from the group consisting of:

| **Compound ID** | **Structure** |
|---|---|
| 3000 | 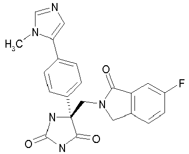 |
| 3001 | 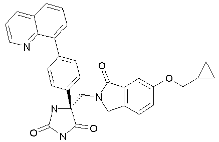 |
| 3002 | 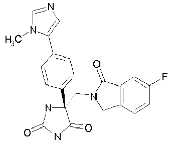 |
| 3003 | 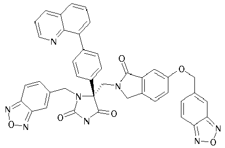 |
| 3004 | 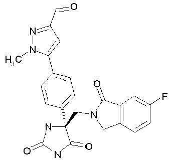 |
| 3005 | 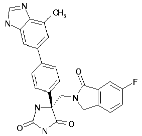 |
| 3006 | 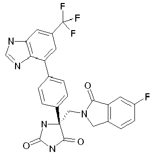 |
| 3007 | 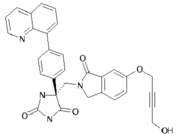 |
| 3008 | 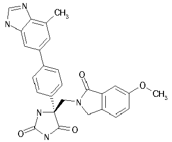 |
| 3009 | 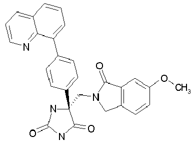 |
| 3010 | 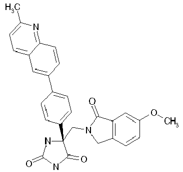 |
| 3011 | 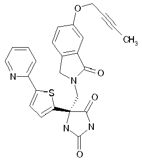 |
| 3012 | 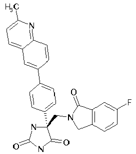 |
| 3013 | 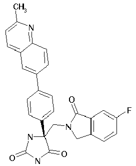 |
| 3014 | 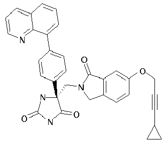 |
| 3015 | 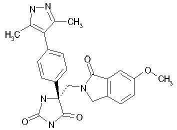 |
| 3016 | 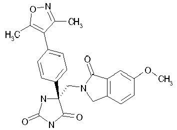 |
| 3017 | 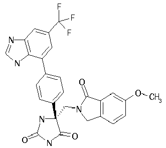 |
| 3018 | 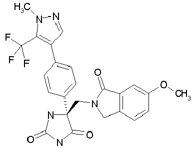 |
| 3019 | 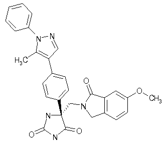 |
| 3020 | 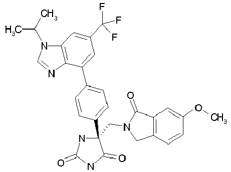 |
| 3021 | 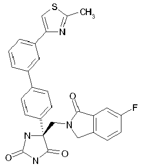 |
| 3022 | 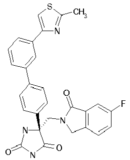 |
| 3024 | 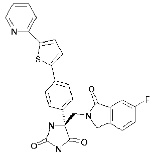 |
| 3025 | 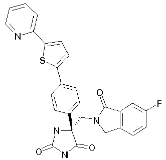 |
| 3026 | 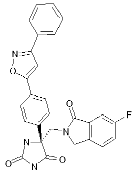 |
| 3027 | 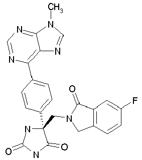 |
| 3028 | 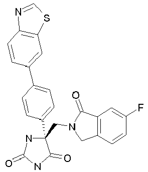 |
| 3029 | 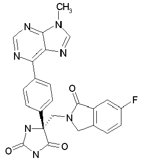 |
| 3030 | 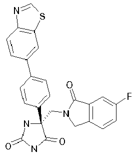 |
| 3031 | 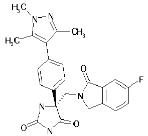 |
| 3032 | 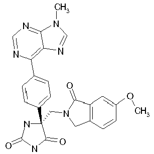 |
| 3033 | 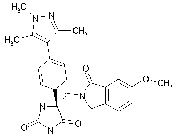 |
| 3034 | 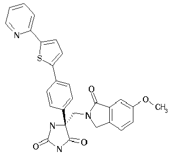 |
| 3035 | 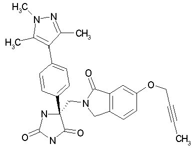 |
| 3003 | 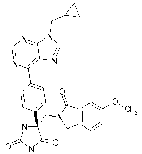 |
| 3037 | 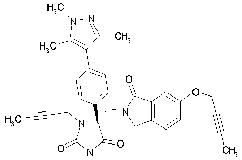 |
| 3038 | 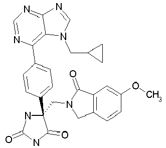 |
| 3039 | 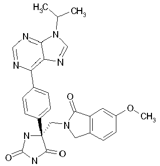 |
| 3040 | 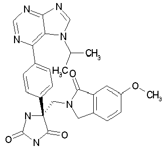 |
| 3041 | 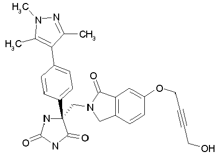 |
| 3042 | 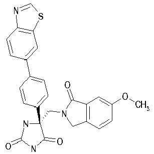 |
| 3043 | 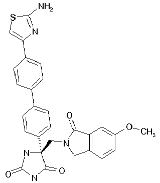 |
| 3044 | 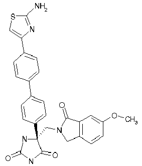 |
| 3045 | 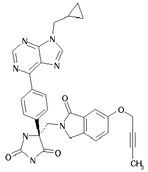 |
| 3046 | 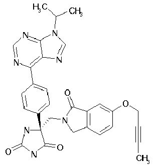 |
| 3047 | 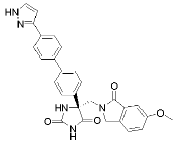 |
| 3048 | 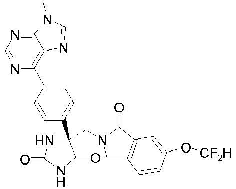 |
| 4001 | 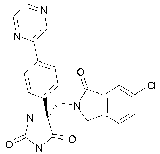 |
| 4002 | 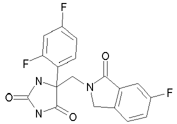 |
| 4003 | 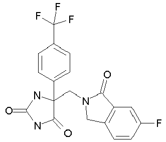 |
| 4004 | 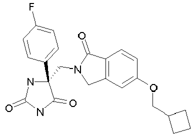 |
| 4005 | 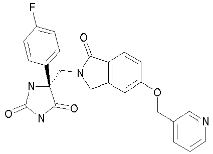 |
| 4006 | 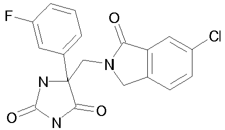 |
| 4007 | 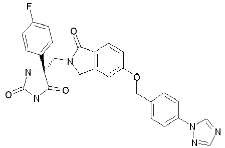 |
| 4008 | 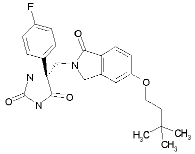 |
| 4009 | 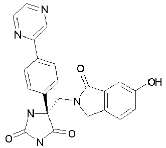 |
| 4010 | 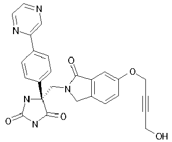 |
| 4011 | 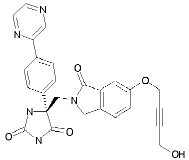 |
| 4012 | 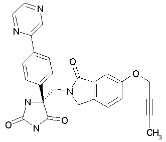 |
| 4013 | 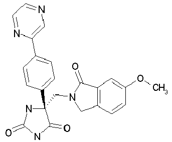 |
| 4014 | 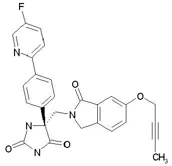 |
| 4015 | 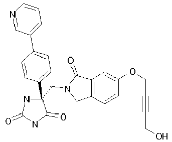 |
| 4016 | 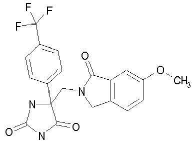 |
| 4017 | 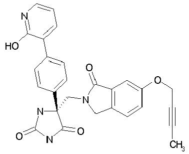 |
| 4018 | 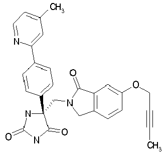 |
| 4019 | 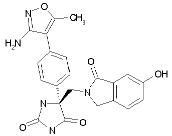 |
| 4020 | 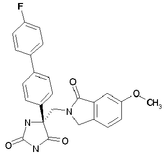 |
| 4021 | 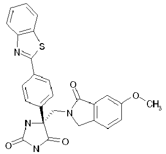 |
| 4022 | 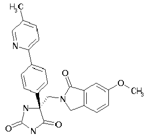 |
| 4023 | 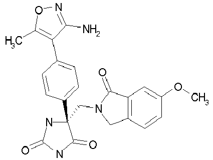 |
| 4024 | 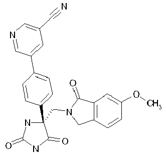 |
| 4025 | 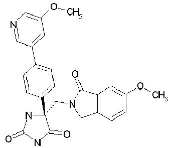 |
| 4026 | 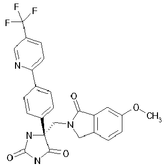 |
| 4027 | 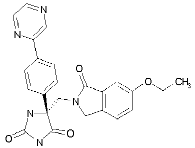 |
| 4028 | 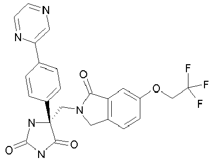 |
| 4029 | 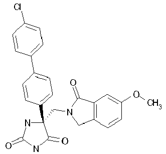 |
| 4030 | 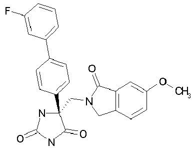 |
| 4031 | 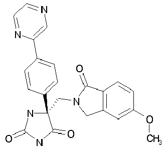 |
| 4032 | 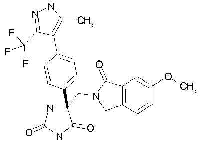 |
| 4033 | 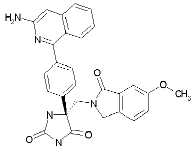 |
| 4034 | 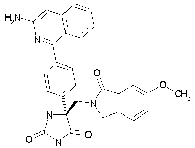 |
| 4035 | 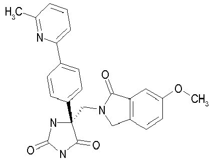 |
| 4036 | 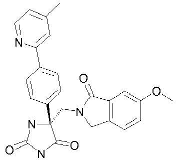 |
| 4037 | 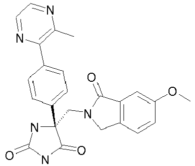 |
| 4038 | 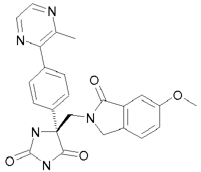 |
| 4039 | 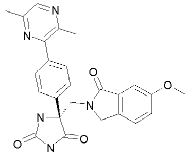 |
| 4040 | 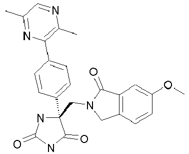 |
| 4041 | 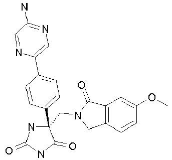 |
| 4042 | 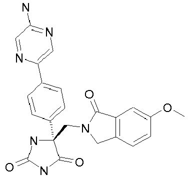 |
| 5000 | 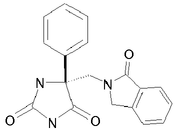 |
| 5001 | 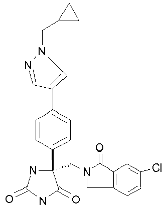 |
| 5003 | 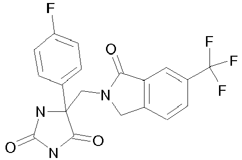 |
| 5005 | 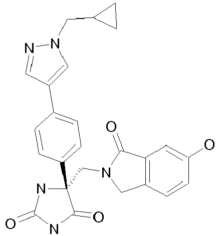 |
| 5006 | 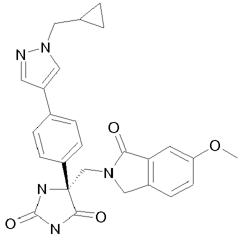 |
| 5007 | 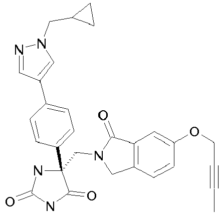 |
| 5009 | 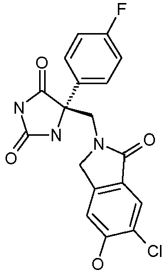 |
| 5010 | 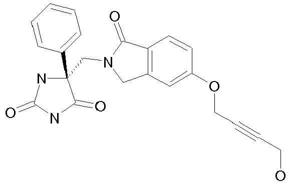 |
| 5016 | 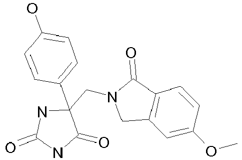 |
| 5017 | 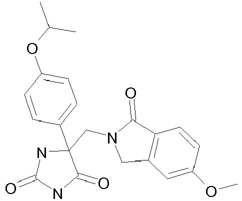 |
| 5018 | 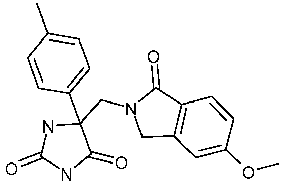 |
| 5019 | 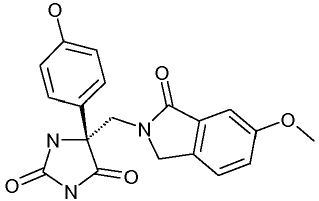 |
| 5020 | 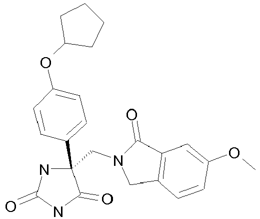 |
| 5021 | 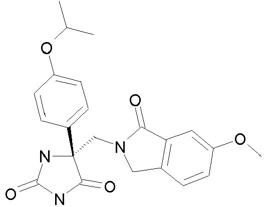 |
| 5022 | 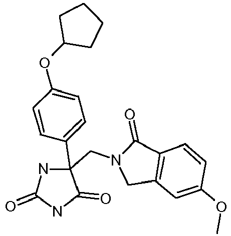 |
| 5023 | 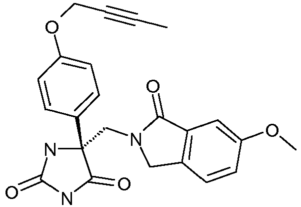 |
| 5024 | 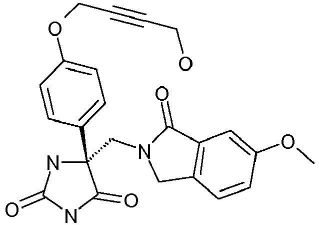 |
| 5025 | 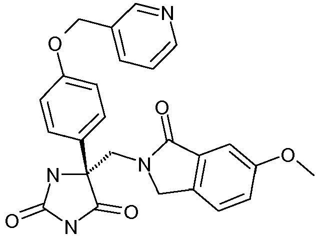 |
| 6000 | 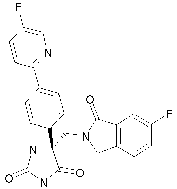 |
| 6001 | 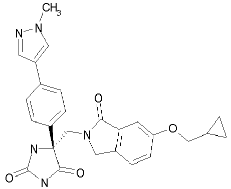 |
| 6002 | 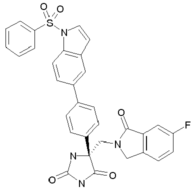 |
| 6003 | 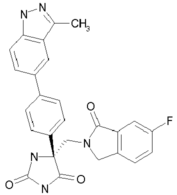 |
| 6004 | 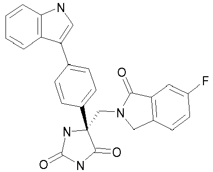 |
| 6005 | 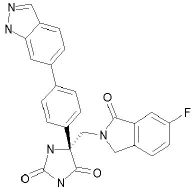 |
| 6006 | 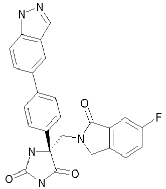 |
| 6007 | 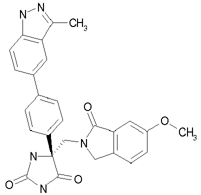 |
| 6008 | 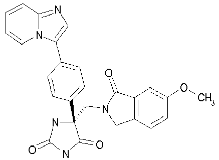 |
| 6009 | 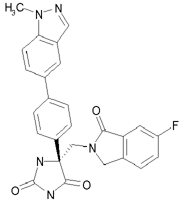 |
| 6010 | 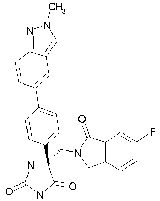 |
| 6011 | 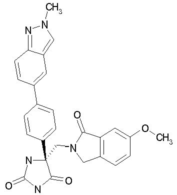 |
| 6012 | 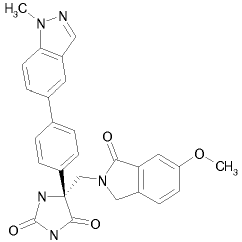 |
| 6013 | 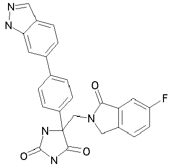 |
| 6014 | 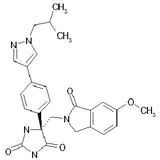 |
| 6015 | 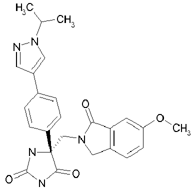 |
| 6016 | 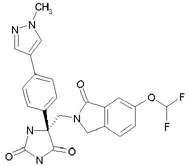 |
| 6017 | 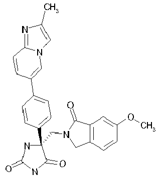 |
| 6018 | 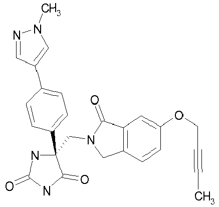 |
| 6019 | 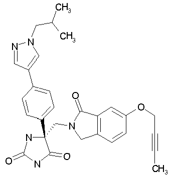 |
| 6020 | 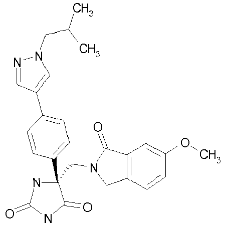 |
| 6021 | 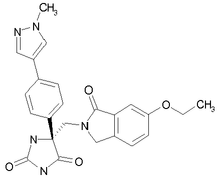 |
| 6022 | 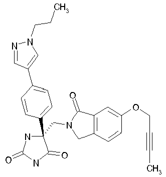 |
| 6023 | 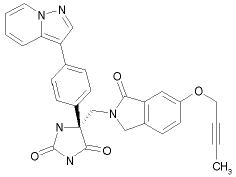 |
| 6024 | 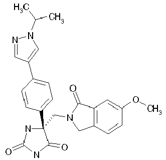 |
| 6025 | 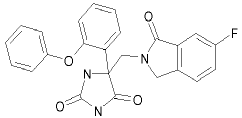 |
| 6026 | 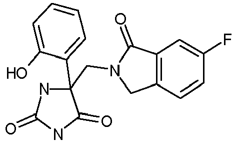 |
| 6027 | 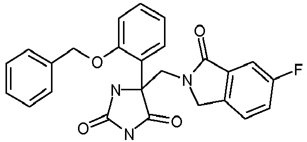 |
| 6028 | 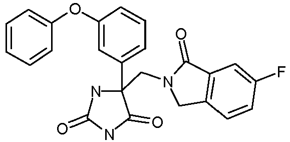 |
| 6029 | 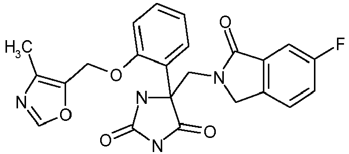 |
| 7000 | 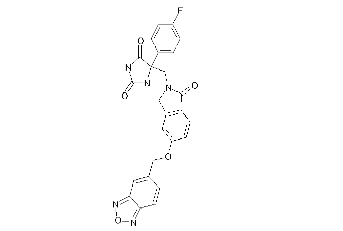 |
| 7001 | 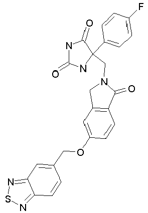 |
| 7002 | 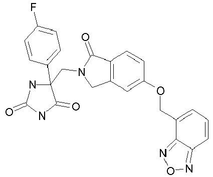 |
| 7003 | 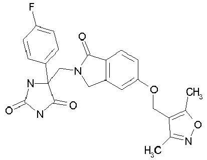 |
| 7004 | 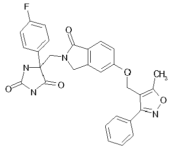 |
| 7005 | 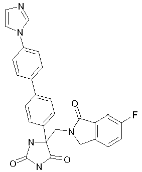 |
| 7006 | 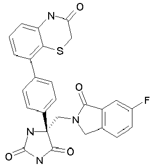 |
| 7007 | 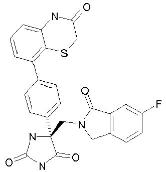 |
| 7008 | 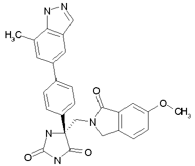 |
| 7009 | 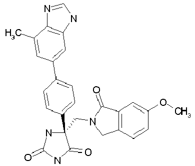 |
| 7010 | 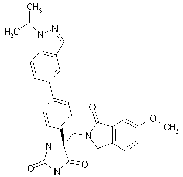 |
| 7011 | 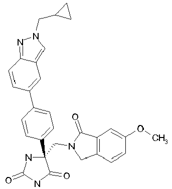 |
| 7012 | 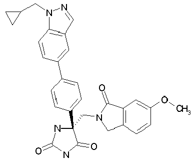 |
| 7013 | 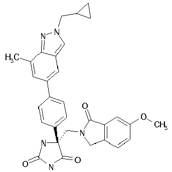 |
| 7014 | 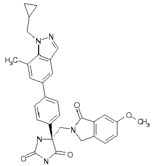 |
| 7015 | 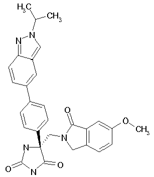 |
| 7016 | 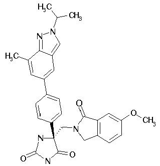 |
| 7017 | 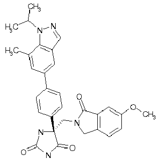 |
| 7018 | 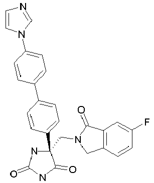 |
| 7019 | 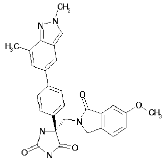 |
| 7020 | 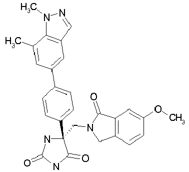 |
| 7021 | 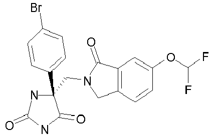 |
| 7022 | 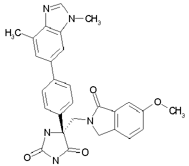 |
| 7023 | 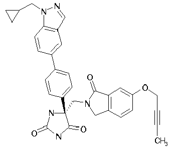 |
| 7024 | 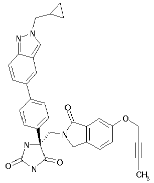 |
| 7025 | 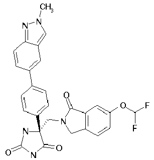 |
| 7026 | 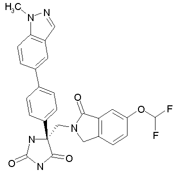 |
| 8001 | 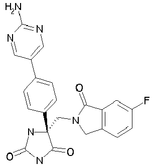 |
| 8002 | 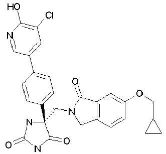 |
| 8003 | 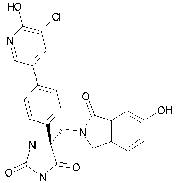 |
| 8004 | 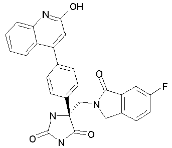 |
| 8005 | 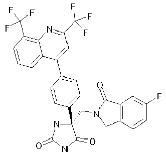 |
| 8006 | 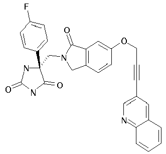 |
| 8007 | 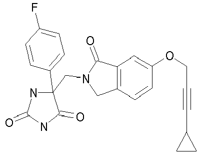 |
| 8008 | 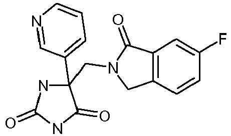 |
| 8009 | 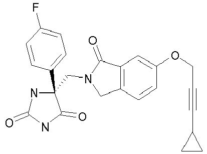 |
| 8010 | 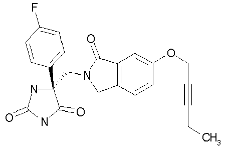 |
| 8011 | 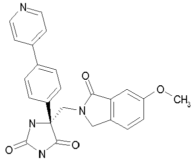 |
| 8012 | 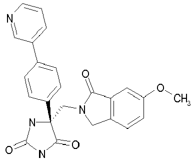 |
| 8013 | 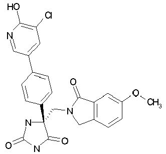 |
| 8014 | 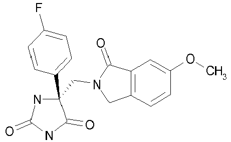 |
| 8015 | 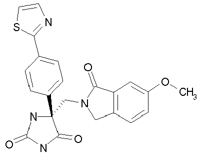 |
| 8016 | 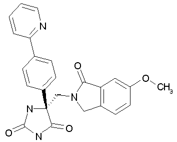 |
| 8017 | 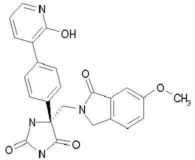 |
| 8018 | 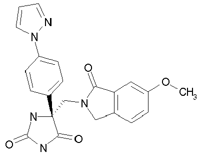 |
| 8019 | 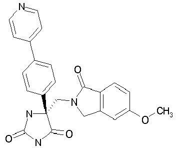 |
| 8020 | 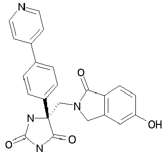 |
| 8021 | 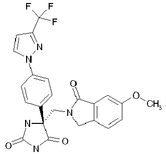 |
| 8022 | 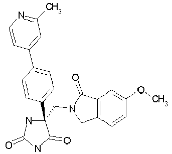 |
| 8023 | 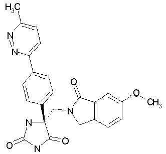 |
| 8024 | 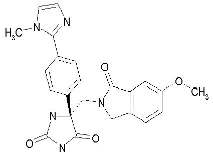 |
| 8025 | 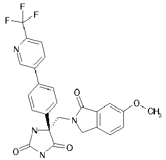 |
| 8027 | 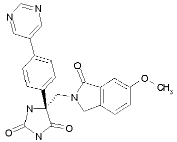 |
| 2021F | 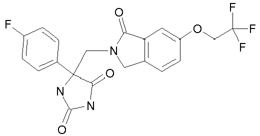 |
| 2023C | 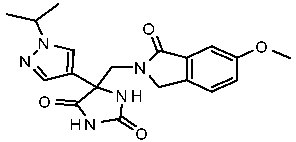 |
| 2024D | 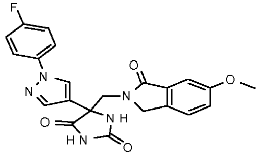 |
| 2022G | 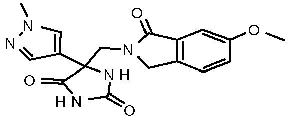 |
| 2025C | 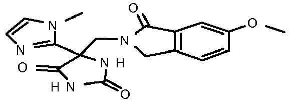 |
| 2028 | 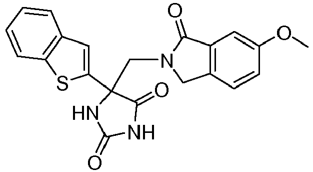 |
| 2026H | 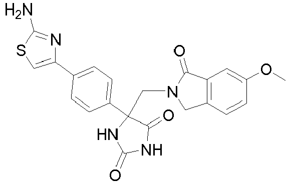 |
| 2030A | 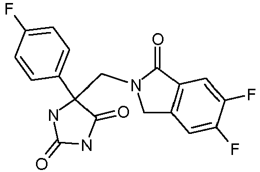 |
| 2030C | 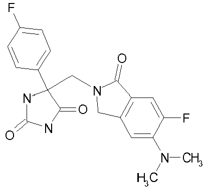 |
| 2030B | 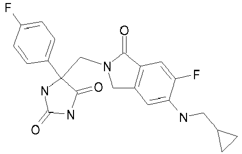 |
| 2031C | 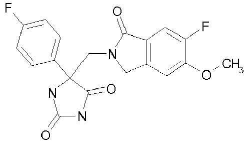 |
| 2031D | 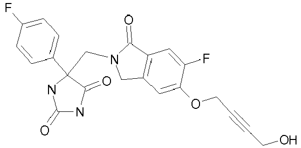 |
| 2030D | 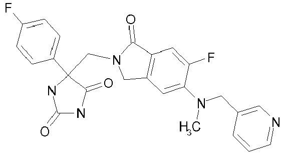 |
| 2031A | 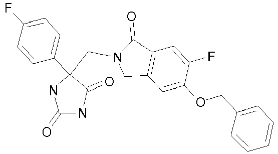 |
| 2031B | 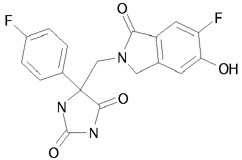 |
| 2032B | 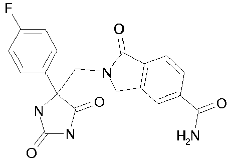 |
| 2031E | 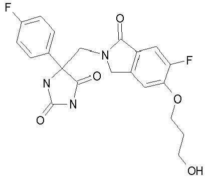 |
| 2031F | 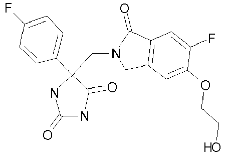 |
| 9200 | 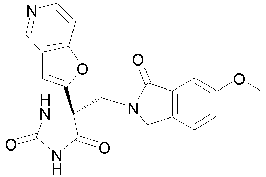 |

or a pharmaceutically acceptable salt or solvate thereof.

As used above, and throughout this disclosure, the following terms, unless otherwise indicated, shall be understood to have the following meanings:
"Patient" includes both human and animals.
"Mammal" means humans and other mammalian animals.

The term "isolated" or "in isolated form" for a compound refers to the physical state of said compound after being isolated from a synthetic process or natural source or combination thereof. The term "purified" or "in purified form" for a compound refers to the physical state of said compound after being obtained from a purification process or processes described herein or well known to the skilled artisan, in sufficient purity to be characterizable by standard analytical techniques described herein or well known to the skilled artisan.

It should also be noted that any carbon as well as heteroatom with unsatisfied valences in the text, schemes, examples and Tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in organic Synthesis (1991), Wiley, New York.

As used herein, the term "composition" is intended to encompass a product comprising the specified ingredients in the specified amounts, as well as any product which results, directly or indirectly, from combination of the specified ingredients in the specified amounts.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Non-limiting examples of suitable solvates include ethanolates, methanolates, and the like. "Hydrate" is a solvate wherein the solvent molecule is H₂O.

"Effective amount" or "therapeutically effective amount" is meant to describe an amount of compound or a composition of the present invention effective in inhibiting TACE, the production of TNF-α, MMPs, ADAMS or any combination thereof and thus producing the desired therapeutic, ameliorative, inhibitory or preventative effect.

The compounds of Formula I can form salts which are also within the scope of this invention. Reference to a compound of Formula I herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula I contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful. Salts of the compounds of the Formula I may be formed, for example, by reacting a compound of Formula I with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Compounds of Formula I, and salts and solvates thereof, may exist in their tautomeric form (for example, as an amide or imino ether). All such tautomeric forms are contemplated herein as part of the present invention.

All stereoisomers (for example, geometric isomers, optical isomers and the like) of the present compounds (including those of the salts and solvates of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention. Individual stereoisomers of the compounds of the invention may, for example, be substantially free of other isomers, or may be admixed, for example, as racemates or with all other, or other selected, stereoisomers. The chiral centers of the present invention can have the S or R configuration as defined by the IUPAC 1974 Recommendations. The use of the terms "salt" and "solvate", is intended to equally apply to the salt and solvate of enantiomers, stereoisomers, rotamers, tautomers or racemates of the inventive compounds.

Polymorphic forms of the compounds of Formula I, and of the salts, solvates and prodrugs of the compounds of Formula I, are intended to be included in the present invention.

The compounds according to the invention have pharmacological properties; in particular, the compounds of Formula I can be inhibitors of TACE, TNF-α and/or MMP activity.

In one aspect, the invention provides a pharmaceutical composition comprising as an active ingredient at least one compound of formula (I).

In another aspect, the invention provides a pharmaceutical composition of formula (I) additionally comprising at least one pharmaceutically acceptable carrier.

Disclosed is a method of treating disorders associated with TACE, TNF-α, MMPs, ADAMs or any combination thereof, said method comprising administering to a patient in need of such treatment a pharmaceutical composition which comprises therapeutically effective amounts of at least one compound of formula (I).

Also disclosed is the use of a compound of formula (I) for the manufacture of a medicament to treat disorders associated with TACE, TNF-α, MMPs, ADAMs or any combination thereof.

The compounds of Formula I can have anti-inflammatory activity and/or immunomodulatory activity and can be useful in the treatment of diseases including but not limited to septic shock, haemodynamic shock, sepsis syndrome, post ischaemic reperfusion injury, malaria, mycobacterial infection, meningitis, psoriasis, congestive heart failure, fibrotic diseases, cachexia, graft rejection, cancers such as cutaneous T-cell lymphoma, diseases involving angiogenesis, autoimmune diseases, skin inflammatory diseases, inflammatory bowel diseases such as Crohn's disease and colitis, OA and RA, ankylosing spondylitis, psoriatic arthritis, adult Still's disease, ureitis, Wegener's granulomatosis, Behcehe disease, Sjogren's syndrome, sarcoidosis, polymyositis, dermatomyositis, multiple sclerosis, sciatica, complex regional pain syndrome, radiation damage, hyperoxic alveolar injury, periodontal disease, HIV, non-insulin dependent diabetes mellitus, systemic lupus erythematosus, glaucoma, sarcoidosis, idiopathic pulmonary fibrosis, bronchopulmonary dysplasia, retinal disease, scleroderma, osteoporosis, renal ischemia, myocardial infarction, cerebral stroke, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, reversible airway obstruction, adult respiratory distress syndrome, asthma, chronic obstructive pulmonary disease (COPD) and/or bronchitis. It is contemplated that a compound of this invention may be useful in treating one or more of the diseases listed.

Also disclosed is a method of preparing a pharmaceutical composition for treating the disorders associated with TACE, TNF-α, MMPs, ADAMs or any combination thereof, said method comprising bringing into intimate contact at least one compound of formula (I) and at least one pharmaceutically acceptable carrier.

In another aspect, the invention provides a compound of formula (I) exhibiting TACE, TNF-α, MMPs, ADAMs or any combination thereof inhibitory activity, including enantiomers, stereoisomers and tautomers of said compound, and pharmaceutically acceptable salts, esters, or solvates of said compound, said compound being selected from the compounds of structures listed in **Table A** set forth above.

In another aspect, the invention provides a pharmaceutical composition for treating disorders associated with TACE, TNF-α, MMP, ADAM or any combination thereof in a subject comprising, administering to the subject in need of such treatment a therapeutically effective amount of at least one compound of formula (I) or a pharmaceutically acceptable salt, solvate, ester or stereoisomer thereof.

In another aspect, the invention provides a compound of formula (I) in purified form.

Also disclosed is a method of treating a condition or disease mediated by TACE, MMPs, TNF-α, aggrecanase, or any combination thereof in a subject comprising: administering to the subject in need of such treatment a therapeutically effective amount of at least one compound of formula (I) or a pharmaceutically acceptable salt, solvate, ester or stereoisomer thereof.

Further disclosed is a method of treating a condition or disease selected from the group consisting of rheumatoid arthritis, osteoarthritis, periodontitis, gingivitis, corneal ulceration, solid tumor growth and tumor invasion by secondary metastases, neovascular glaucoma, inflammatory bowel disease, multiple sclerosis and psoriasis in a subject, comprising: administering to the subject in need of such treatment a therapeutically effective amount of at least one compound of formula (I) or a pharmaceutically acceptable salt, solvate, ester, or stereoisomer thereof.

Also disclosed is a method of treating a condition or disease selected from the group consisting of fever, cardiovascular conditions, hemorrhage, coagulation, cachexia, anorexia, alcoholism, acute phase response, acute infection, shock, graft versus host reaction, autoimmune disease and HIV infection in a subject comprising administering to the subject in need of such treatment a therapeutically effective amount of at least one compound of formula (I) or a pharmaceutically acceptable salt, solvate, ester, or stereoisomer thereof.

Further disclosed is a method of treating a condition or disease selected from the group consisting of septic shock, haemodynamic shock, sepsis syndrome, post ischaemic reperfusion injury, malaria, mycobacterial infection, meningitis, psoriasis, congestive heart failure, fibrotic diseases, cachexia, graft rejection, cancers such as cutaneous T-cell lymphoma, diseases involving angiogenesis, autoimmune diseases, skin inflammatory diseases, inflammatory bowel diseases such as Crohn's disease and colitis, osteo and rheumatoid arthritis, ankylosing spondylitis, psoriatic arthritis, adult Still's disease, ureitis, Wegener's granulomatosis, Behcehe disease, Sjogren's syndrome, sarcoidosis, polymyositis, dermatomyositis, multiple sclerosis, sciatica, complex regional pain syndrome, radiation damage, hyperoxic alveolar injury, periodontal disease, HIV, non-insulin dependent diabetes mellitus, systemic lupus erythematosus, glaucoma, sarcoidosis, idiopathic pulmonary fibrosis, bronchopulmonary dysplasia, retinal disease, scleroderma, osteoporosis, renal ischemia, myocardial infarction, cerebral stroke, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, reversible airway obstruction, adult respiratory distress syndrome, asthma, chronic obstructive pulmonary disease (COPD) and bronchitis in a subject comprising administering to the subject in need of such treatment a therapeutically effective amount of at least one compound of formula (I) or a pharmaceutically acceptable salt, solvate, ester or stereoisomer thereof.

Also disclosed is a method of treating a condition or disease associated with COPD, comprising: administering to the subject in need of such treatment a therapeutically effective amount of at least one compound of formula (I) or a pharmaceutically acceptable salt, solvate, ester, or stereoisomer thereof.

Further disclosed is a method of treating a condition or disease associated with rheumatoid arthritis, comprising: administering to the subject in need of such treatment a therapeutically effective amount of at least one compound of formula (I) or a pharmaceutically acceptable salt, solvate, ester or stereoisomer thereof.

Also disclosed is a method of treating a condition or disease associated with Crohn's disease, comprising: administering to the subject in need of such treatment a therapeutically effective amount of at least one compound of formula (I) or a pharmaceutically acceptable salt, solvate, ester or stereoisomer thereof.

Further disclosed is a method of treating a condition or disease associated with psoriasis, comprising: administering to the subject in need of such treatment a therapeutically effective amount of at least one compound of formula (I) or a pharmaceutically acceptable salt, solvate, ester, or stereoisomer thereof.

Also disclosed is a method of treating a condition or disease associated with ankylosing spondylitis, comprising: administering to the subject in need of such treatment a therapeutically effective amount of at least one compound of formula (I) or a pharmaceutically acceptable salt, solvate, ester, or stereoisomer thereof.

Further disclosed is a method of treating a condition or disease associated with sciatica, comprising: administering to the subject in need of such treatment a therapeutically effective amount of at least one compound of formula (I) or a pharmaceutically acceptable salt, solvate, ester or stereoisomer thereof.

Also disclosed is a method of treating a condition or disease associated with complex regional pain syndrome, comprising: administering to the subject in need of such treatment a therapeutically effective amount of at least one compound of formula (I) or a pharmaceutically acceptable salt, ester, solvate or stereoisomer thereof.

Further disclosed is a method of treating a condition or disease associated with psoriatic arthritis, comprising: administering to the subject in need of such treatment a therapeutically effective amount of at least one compound of formula (I) or a pharmaceutically acceptable salt, solvate, ester or stereoisomer thereof.

Also disclosed is a method of treating a condition or disease associated with multiple sclerosis, comprising: administering to the subject in need of such treatment a therapeutically effective amount of at least one compound of formula (I) or a pharmaceutically acceptable salt, solvate, ester or stereoisomer thereof, in combination with a compound selected from the group consisting of Avonex®, Betaseron, Copaxone or other compounds indicated for the treatment of multiple sclerosis.

Additionally, a compound of the present invention may be co-administered or used in combination with disease-modifying antirheumatic drugs (DMARDS) such as methotrexate, azathioprine, leflunomide, pencillinamine, gold salts, mycophenolate mofetil, cyclophosphamide and other similar drugs. They may also be co-administered with or used in combination with non-steroidal anti-inflammatory drugs (NSAIDs) such as piroxicam, naproxen, indomethacin, ibuprofen and the like; cycloxygenase-2 selective (COX-2) inhibitors such as Vioxx® and Celebrex®; immunosuppressives such as steroids, cyclosporin, Tacrolimus, rapamycin and the like; biological response modifiers (BRMs) such as Enbrel®, Remicade®, IL-1 antagonists, anti-CD40, anti-CD28, IL-10, anti-adhesion molecules and the like; and other anti-inflammatory agents such as p38 kinase inhibitors, PDE4 inhibitors, other chemically different TACE inhibitors, chemokine receptor antagonists, Thalidomide and other small molecule inhibitors of pro-inflammatory cytokine production.

Also, a compound of the present invention may be co-administered or used in combination with an H1 antagonist for the treatment of seasonal allergic rhinitis and/or asthma. Suitable H1 antagonists may be, for example, Claritin®, Clarinex®, Allegra®, or Zyrtec®.

Also disclosed is a method of treating a condition or disease mediated by TACE, MMPs, TNF-α, aggrecanase, or any combination thereof in a subject comprising: administering to the subject in need of such treatment a therapeutically effective amount of at least one compound of formula (I) or a pharmaceutically acceptable salt, solvate, ester or isomer thereof in combination with a therapeutically effective amount of at least one medicament selected from the group consisting of disease modifying antirheumatic drugs (DMARDS), NSAIDs, COX-2 inhibitors, COX-1 inhibitors, immunosuppressives, biological response modifiers (BRMs), anti-inflammatory agents and H1 antagonists.

Further disclosed is a method of treating a condition or disease selected from the group consisting of rheumatoid arthritis, osteoarthritis, periodontitis, gingivitis, corneal ulceration, solid tumor growth and tumor invasion by secondary metastases, neovascular glaucoma, inflammatory bowel disease, multiple sclerosis and psoriasis in a subject, comprising: administering to the subject in need of such treatment a therapeutically effective amount of at least one compound of formula (I) or a pharmaceutically acceptable salt, solvate, ester or isomer thereof in combination with a therapeutically effective amount of at least one medicament selected from the group consisting of DMARDS, NSAIDs, COX-2 inhibitors, COX-1 inhibitors, immunosuppressives, BRMs, anti-inflammatory agents and H1 antagonists.

Also disclosed is a method of treating a condition or disease selected from the group consisting of septic shock, haemodynamic shock, sepsis syndrome, post ischaemic reperfusion injury, malaria, mycobacterial infection, meningitis, psoriasis, congestive heart failure, fibrotic diseases, cachexia, graft rejection, cancers such as cutaneous T-cell lymphoma, diseases involving angiogenesis, autoimmune diseases, skin inflammatory diseases, inflammatory bowel diseases such as Crohn's disease and colitis, osteo and rheumatoid arthritis, ankylosing spondylitis, psoriatic arthritis, adult Still's disease, ureitis, Wegener's granulomatosis, Behcehe disease, Sjogren's syndrome, sarcoidosis, polymyositis, dermatomyositis, multiple sclerosis, sciatica, complex regional pain syndrome, radiation damage, hyperoxic alveolar injury, periodontal disease, HIV, non-insulin dependent diabetes mellitus, systemic lupus erythematosus, glaucoma, sarcoidosis, idiopathic pulmonary fibrosis, bronchopulmonary dysplasia, retinal disease, scleroderma, osteoporosis, renal ischemia, myocardial infarction, cerebral stroke, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, reversible airway obstruction, adult respiratory distress syndrome, asthma, chronic obstructive pulmonary disease (COPD) and bronchitis in a subject comprising administering to the subject in need of such treatment a therapeutically effective amount of at least one compound of formula (I) or a pharmaceutically acceptable salt, solvate, ester or isomer thereof in combination with a therapeutically effective amount of at least one medicament selected from the group consisting of DMARDS, NSAIDs, COX-2 inhibitors, COX-1 inhibitors, immunosuppressives, BRMs, anti-inflammatory agents and H1 antagonists.

Further disclosed is a method for treating RA comprising administering a compound of the formula I in combination with compound selected from the class consisting of a COX-2 inhibitor e.g. Celebrex® or Vioxx®; a COX-1 inhibitor e.g. Feldene®; an immunosuppressive e.g. methotrexate or cyclosporin; a steroid e.g. β-methasone; and anti-TNF-α compound, e.g. Enbrel® or Remicade®; a PDE IV inhibitor, or other classes of compounds indicated for the treatment of RA.

Also disclosed is a method for treating multiple sclerosis comprising administering a compound of the formula I in combination with a compound selected from the group consisting of Avonex®, Betaseron, Copaxone or other compounds indicated for the treatment of multiple sclerosis.

TACE activity is determined by a kinetic assay measuring the rate of increase in fluorescent intensity generated by TACE catalyzed cleavage of an internally quenched peptide substrate (SPDL-3). The purified catalytic domain of recombinant human TACE (rhTACEc, Residue 215 to 477 with two mutation (S266A and N452Q) and a 6xHis tail) is used in the assay. It is purified from the baculovirus/Hi5 cells expression system using affinity chromatography. The substrate SPDL-3 is an internally quenched peptide (MCA-Pro-Leu-Ala-Gln-Ala-Val-Arg-Ser-Ser-Ser-Dpa-Arg-NH2), with its sequence derived from the pro-TNFα cleavage site. MCA is (7-Methoxycoumarin-4-yl)acetyl. Dpa is N-3-(2,4-Dinitrophenyl)-L-2,3-diaminopropionyl.

A 50 µl assay mixture contains 20 mM HEPES, pH 7.3, 5 mM CaCl₂, 100 µM ZnCl₂, 2 % DMSO, 0.04% Methylcellulose, 30 µM SPDL-3, 70 pM rhTACEc and a test compound. RhTACEc is pre-incubated with the testing compound for 90 min. at 25 °C. Reaction is started by addition of the substrate. The fluorescent intensity (excitation at 320 nm, emission at 405 nm) was measured every 45 seconds for 30 min. using a fluorospectrometer (GEMINI XS, Molecular Devices). Rate of enzymatic reaction is shown as Units per second. Effect of a test compound is shown as % of TACE activity in the absence of the compound.

Useful compounds for TACE inhibitory activity can exhibit Kᵢ values of less than about 1000 nm, preferably about 0.01 nm to about 1000 nm, more preferably about 0.1 nm to about 100 nm, and most preferably less than about 15 nm. The TACE inhibitory activity (Ki values) of some representative compounds of the present invention are listed in the "EXAMPLES" section hereinbelow.

The pharmaceutical compositions containing the active ingredient may be in a form suitable for oral use, for example, as tablets, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in the U.S. Pat. Nos. 4,256,108; 4,166,452; and 4,265,874 to form osmotic therapeutic tablets for controlled release.

The term pharmaceutical composition is also intended to encompass both the bulk composition and individual dosage units comprised of more than one (e.g., two) pharmaceutically active agents such as, for example, a compound of the present invention and an additional agent selected from the lists of the additional agents described herein, along with any pharmaceutically inactive excipients. The bulk composition and each individual dosage unit can contain fixed amounts of the afore-said "more than one pharmaceutically active agents". The bulk composition is material that has not yet been formed into individual dosage units. An illustrative dosage unit is an oral dosage unit such as tablets, pills and the like. Similarly, the herein-described method of treating a patient by administering a pharmaceutical composition of the present invention is also intended to encompass the administration of the afore-said bulk composition and individual dosage units.

Formulations for oral use may also be presented as hard gelatin capsules wherein the active ingredients is mixed with an inert solid diluent, for example, calcium carbonate, calcium phosphate or kaolin, or a soft gelatin capsules where in the active ingredient is mixed with water or an oil medium, for example peanut oil, liquid paraffin or olive oil.

Aqueous suspensions contain the active material in admixture with excipients suitable for the manufacture of aqueous suspensions. Such excipients are suspending agents, for example, sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example, ethyl or n-propyl, p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example, arachis oil, olive oil, sesame oil or coconut oil, or in mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, e.g., sweetening, flavoring and coloring agents, may also be present.

The pharmaceutical compositions of the invention may also be in the form of an oil-in-water emulsion. The oily phase may be a vegetable oil, e.g., olive oil or arachis oil, or a mineral oil, e.g., liquid paraffin or mixtures of these. Suitable emulsifying agents may be naturally-occurring phosphatides, e.g., soy beans, lecithin, and esters or partial esters derived from fatty acids and hexitol anhydrides, for example, sorbitan monooleate, and condensation products of the said partial esters with ethylene oxide, e.g., polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain a demulcent, a preservative and flavoring and coloring agents.

The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, e.g., as a solution in 1,3-butane diol. Among the acceptable vehicles and solvents that may be employed are water, Ringer's solution and isotonic sodium chloride solution. In addition, sterile fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. The compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compounds of the invention are employed. (For purposes of this application, topical application shall include mouthwashes and gargles.)

The compounds for the present invention can be administered in the intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The dosage regimen utilizing the compounds of the present invention is selected in accordance with a variety of factors including type, species, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter, arrest or reverse the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug. Preferably, doses of the compound of Formula I useful in the method of the present invention range from 0.01 to 1000 mg per day. More preferably, dosages range from 0.1 to 1000 mg/day. Most preferably, dosages range from 0.1 to 500 mg/day. For oral administration, the compositions are preferably provided in the form of tablets containing 0.01 to 1000 milligrams of the active ingredient, particularly 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. An effective amount of the drug is ordinarily supplied at a dosage level of from about 0.0002 mg/kg to about 50 mg/kg of body weight per day. The range is more particularly from about 0.001 mg/kg to 1 mg/kg of body weight per day.

Advantageously, the active agent of the present invention may be administered in a single daily dose, or the total daily dosage may be administered in dividend doses of two, three or four time daily.

The amount of active ingredient that may be combined with the carrier materials to produce single dosage form will vary depending upon the host treated and the particular mode of administration.

It will be understood, however, that the specific dose level for any particular patient will depend upon a variety of factors including the age, body weight, general health, sex, diet, time of administration, route or administration, rate of excretion, drug combination and the severity of the particular disease undergoing therapy.

The compounds of the invention may be produced by processes known to those skilled in the art and as shown in the following reaction schemes and in the preparations and examples described below.

Any compounds below having activity and not falling within claim 1 are included for reference only.

### EXAMPLES

The following abbreviations may be used in the procedures and schemes:

| | |
|---|---|
| ACN | Acetonitrile |
| AcOH | Acetic acid |
| Aq | Aqueous |
| BOC | tert-Butoxycarbonyl |
| BOC₂O | BOC Anhydride |
| C | degrees Celsius |
| CBZCl | Benzyl chloroformate |
| DBU | 1,8-Diazabicyclo[5.4.0]undec-7-ene |
| DCM | Dichloromethane |
| DEAD | Diethyl azodicarboxylate |
| (DHQ)2PHAL | Hydroquinine 1,4-phthalazinediyl diether |
| DIAD | Diisopropylazodicarboxylate |
| DIPEA | Diisopropylethylamine |
| DMA | N,N-Dimethylacetamide |
| DMAP | 4-Dimethylaminopyridine |
| DME | Dimethoxyethane |
| DMF | Dimethylformamide |
| DMPU | 1,3-Dimethyl-3,4,5,fi-tetrahydro-2(1 h)-pyrimidinone |
| DMSO | Dimethyl sulfoxide |
| EDCl | 1-(3-Dimethylaminopropyl)-3-ethylcarbodiimide hydrochloride |
| EI | Electron ionization |
| Eq | Equivalents |
| EtOAc | Ethyl acetate |
| EtOH | Ethanol |
| g | grams |
| h | hours |
| hr | hours |
| ¹H | proton |
| HATU | N,N,N',N'-Tetramethyl-O-(7-Azabenzotriazol-1-yl)Uronium hexafluorophosphate |
| Hex | hexanes |
| HOBT | 1-Hydroxybenzotriazole |
| HPLC | High pressure liquid chromatography |
| LAH | Lithium aluminum hydride |
| LDA | Lithium diisopropylamide |
| M | Molar |
| mmol | milimolar |
| mCPBA | *meta*-Chloroperoxybenzoic acid |
| Me | Methyl |
| MeCN | Acetonitrile |
| MeOH | Methanol |
| min | Minutes |
| mg | Milligrams |
| MHZ | Megahertz |
| mL | Milliliter |
| MPLC | Medium Pressure Liquid Chromatography |
| NMR | Nuclear Magnetic Resonance |
| MS | Mass Spectroscopy |
| NBS | N-Bromosuccinimide |
| NMM | N-Methylmorpholine |
| NMP | 1-methyl-2-pyrrolidone |
| ON | Overnight |
| PCC | Pyridinium Chlorochromate |
| PTLC | Preparative thin layer chromatography |
| PyBrOP | Bromo-tris-pyrrolidino-phosphonium hexafluorophosphate |
| Pyr | Pyridine |
| RT | Room temperature |
| sgc | Silica gel 60 chromatography |
| tBOC | tert-Butoxycarbonyl |
| TACE | TNF-alpha converting enzyme |
| TEA | Triethylamine |
| TFA | Trifluoroacetic acid |
| THF | Tetrahydrofuran |
| TLC | Thin layer chromatography |

NMR spectra were acquired on the following instruments: 400 MHZ NMR (Bruker), 500 MHZ NMR (Bruker), 400 MHz NMR (Varian), 300 MHZ NMR (Varian) using CD₃OD, CDCl₃ or DMSO-d₆ as solvents. LC-MS data were obtained using a PESciex API 150EX quadropole mass spectrometer using electroscopy ionization.

Purification via reverse phase chromatography (Gilson) was accomplished using a C18 reverse phase column with a gradient of (0.1 % formic acid) 5:95 to 90:10 acetonitrile:water, at a flow rate of 14 mL/min. Samples were collected using UV detection. Alternatively an ISCO Companion with (0.1% formic acid) 5:95 to 95:5 acetonitrile:water, at a flow rate = 10 - 55 mL/min.

Normal phase silica gel chromatography was either accomplished on a Biotage instrument using a 60 Å 12/M, 25/M, or 40/M flash cartridges, or on a Jones Flash Master Personal instrument using Isolute flash SI 5g, 10g, 20g, 50g, or 70 g cartridges.

The compounds of formula (I) may be produced by processes known to those skilled in the art and as shown in the following reaction schemes and in the preparations and examples described below. Alternate mechanistic pathways and analogous structures may be apparent to those skilled in the art. Some of the compounds made by these processes are listed in the tables below.

### SYNTHETIC ROUTES AND EXAMPLES

### Example 1

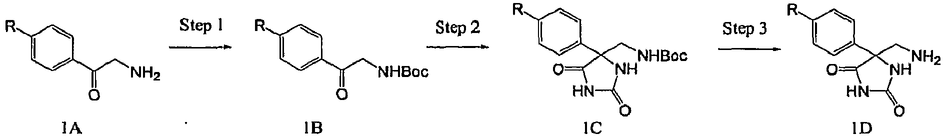

### General procedures for Example 1:

In step 1, Compound **1A** (either commercially available, or prepared by a procedure similar to that described by Abdalla, G. M. and Sowell, J. W. Journal of Heterocyclic Chemistry, 1987, 24(2), 297-301) was treated with one equivalent of Di-tert-butyl dicarbonate in polar solvent, such as DMF, for 30 minutes to 12 hours. The solvent was removed and compound **1B** could be used without further purification or purified by silica gel chromatography.

In step 2, compound **1B** was reacted with potassium cyanide and ammonium carbonate in appropriated alcohol and water solution, at 50 °C to 90 °C, for 5 hours to 48 hours. After cooling down, water was added and compound **1C** could be collected by filtration.

In step 3, compound **1C** was stirred with 2 to 20 equivalents of hydrogen chloride in methanol for 5 to 48 hours. After ethyl ether was added, compound **1D** could be collected by filtration.

### Example 2

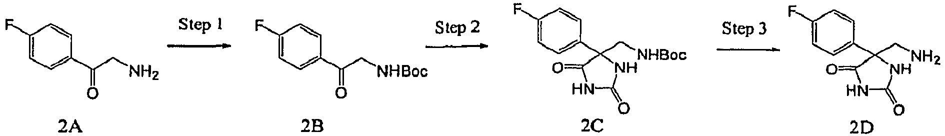

### Step 1

Compound **2A** (Abdalla, G. M. and Sowell, J. W. Journal of Heterocyclic Chemistry, 1987, 24(2), 297-301) (Hydrochloride salt, 8.60g, 45.4 mmol), triethyl amine (19.0 mL, 136 mmol), and di-tert-butyl dicarbonate (11.9g, 54.4 mmol) were stirred in methylene chloride (100 mL) at 25 °C for 16 hours. Saturated aqueous NaHCO₃ (150 mL) was added. The aqueous layer was extracted with CH₂Cl₂ (100 mL) twice. The organic phase was washed with brine (100 mL) and dried over Na₂SO₄. The solvent was removed by rotary evaporator to give compound **2B** which was used without further purification.

### Step 2

Compound **2B** (9.06g, 35.8 mmol), KCN (3.49g, 53.7 mmol), and (NH₄)₂CO₃ (12.0 g, 125.2 mmol) were suspended in a mixture of EtOH (35 mL) and water (35 mL). The solution was stirred at 70 °C for three days. After cooling down, water (35 mL) was added. The solid was filtered and washed with water three times. The solid was dried under vacuum at 40 °C for 16 hours to give compound **2C** (7.9 g, 68%).

### Step 3

Compound **2C** (4.0 g) was suspended in methanol (50 mL) and HCl (4M in dioxane, 20 mL) was added. The solution was stirred at 25 °C for 3 hours. Ethyl ether (50 ml) was added. The solid was filtered, washed with ethyl ether twice, and dried under vacuum for 12 hours to give compound **2D** (2.7 g, 84%).

The following intermediates were prepared as described in **Examples 1 and 2**.

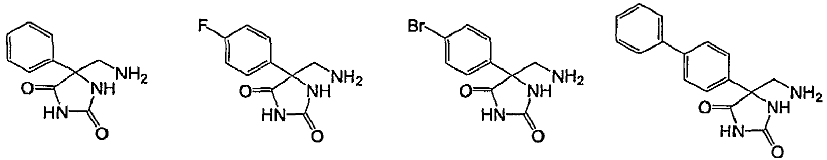

### Example 3

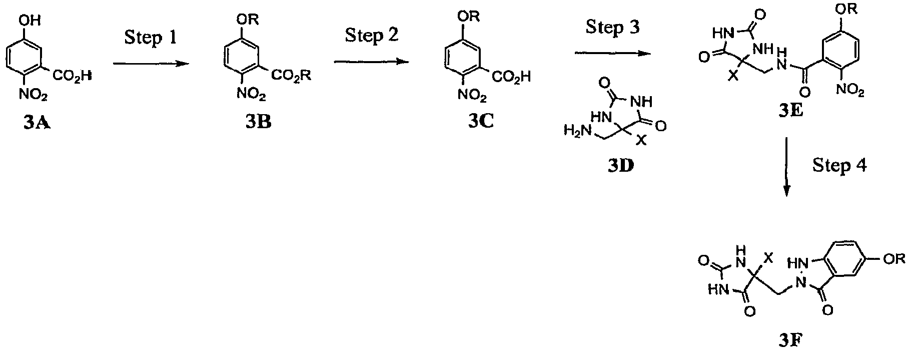

### General procedures for Example 3

In step 1, 5-Hydroxy-2-nitro-benzoic acid (compound **3A**) was dissolved in a suitable solvent, such as DMF, and reacted with an alkyl chloride or alkyl bromide in the presence of cesium carbonate at room temperature for 2 to 16 hours. Water and EtOAc were added. The organic phase was washed by water 1 to 5 times to remove DMF. The organic phase was washed with brine, dried, concentrated to give the crude product (compound **3B**) which was used without further purification.

In step 2, compound **3B** was dissolved in dioxane/water (3:1) and treated with lithium hydroxide at room temperature for 3 to 6 hours. The solution was made acidic by addition of 1 N HCl solution and extracted with EtOAc. The products (compound **3C**) were either used without further purification or purified by chromatography depending on the boiling point of the alcohol side products.

In step 3, compound **3C** was dissolved in a suitable solvent, such as DMF, and coupled with compound **3D** using EDCI and HOBT at room temperature overnight. After an aqueous/EtOAc work up, the product (compound **3E**) was isolated by chromatography.

In step 4, compound **3E** was suspended in MeOH/water (1:1) under N₂ atmosphere. NaOH and Zinc powder were added and the reaction mixture was stirred at 70 °C to 80 °C for 8 to 24 hours. After cooling to room temperature, the solution was adjusted to pH=6∼7 with 1N HCl solution. The product (compound **3F**) was extracted with EtOAc and purified by reverse phase HPLC.

### Example 4

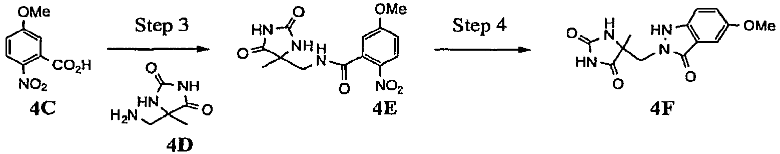

### Step 3

A 25 mL flask was charged with compound **4C** (331 mg, 1.68 mmol), compound **4D** (Stratford, E. S. and Curley, R. W. Jr, *J. Med. Chem.* **1983**, *26*, 1463-1469) (200 mg, 1.4 mmol), EDCI (403 mg, 2.1 mmol), HOBT (227 mg, 1.68 mmol), NMM (0.46 mL, 4.2 mmol), and DMF (7 mL). The solution was stirred at room temperature overnight. Saturated aqueous NaHCO₃ (30 mL) and EtOAc (50 mL) were added. The organic phase was separated and washed with water (20 mL) and brine (20 mL), then dried over Na₂SO₄. The solvent was evaporated and the crude product was isolated by silica gel chromatography (CH₂Cl₂/MeOH/NH₄OH 20:1:0.1 to 10:1:0.1) to give compound **4E** (201 mg, 45%).

### Step 4

To a 10 mL flask was added compound **4E** (50 mg, 0.155 mmol), NaOH (25 mg, 0.62 mmol), Zinc powder (62 mg, 0.47 mmol), MeOH (0.5 mL), and water (0.5 mL). The solution was stirred at 75 °C for 16 hours. After cooling to room temperature, solid was removed by filtration. The filtrate was adjusted to pH=5 by adding 2N HCl. The aqueous phase was extracted by EtOAc (10 mL). The organic solution was dried over Na₂SO₄ and concentrated. The product was isolated by silica gel chromatography (CH₂Cl₂/MeOH/NH₄OH, 40:1:0.1 to 20:1:0.1 to 10:1:0.1) to give compound **4F** 6.5 mg (14%).

### Example 5

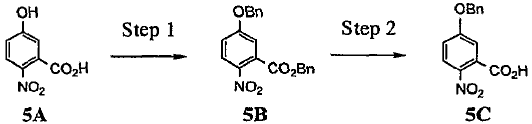

### Step 1

Compound **5A** (1.33 g, 7.26 mmol), benzyl bromide (2.73 g, 16.0 mmol), and Cs₂CO₃ (7.1 g, 22.0 mmol) were mixed in DMF (30 mL) and stirred at room temperature overnight. Saturated aqueous NaHCO₃ (100 mL) was added and the aqueous phase was extracted with EtOAc (100 mL) twice. The combined organic phases were washed with brine (50 mL), dried over Na₂SO₄, filtered, and concentrated by rotary evaporator. The product was isolated by silica gel chromatography (Hexane/EtOAc: 10:1 to 5:1) to give compound **5B** (2.25 g, 89%).

### Step 2

Compound **5B** (2.25 g, 6.44 mmol) was dissolved in dioxane/water (3:1, 35 mL) and LiOH (810 mg, 19.3 mmol) was added. The solution was stirred at room temperature for 3 hours. Water (30 mL) was added followed by addition of 2N HCl (30 mL). The aqueous phase was extracted with EtOAc (50 mL) three times. The organic phase was washed with brine, dried over Na₂SO₄, filtered, and concentrated by rotary evaporator. The crude product was purified by silica gel chromatography (CH₂Cl₂/MeOH/HCO₂H: 40:1:0.1 to 20:1:0.1) to give compound **5C** (1.6 g, 91%).

The following compounds were prepared as described in **Examples 3-5.**

**In each of the tables below, those compounds having a Kᵢ value of less than 10 nM (<10 nM) are designated with letter "A"; those with a Ki value of from 10 to less than 100 nM (10 - <100 nM) are designated with letter "B"; those with a Ki value of from 100 to 1000 nM are designated with letter "C"; and those with a Ki value of more than 1000 nM (>1000 nM) are designated with letter "D".**

**Table 1**

| Compound # | Structure | Exact Mass | Mass Obsvd | Ki (nM) |
|---|---|---|---|---|
| **1** | 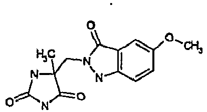 | 290.27 | 291.1 [M+H]⁺ | B |
| **2** | 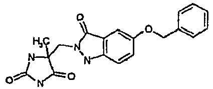 | 366.13 | 367.1 [M+H]⁺ | C |
| **3** | 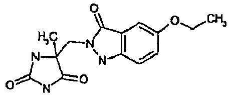 | 304.12 | 305.0 [M+H]⁺ | C |
| **4** | 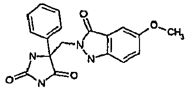 | 352.12 | 353.1 [M+H]⁺ | A |
| **5** | 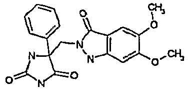 | 382.13 | 383.1 [M+H]⁺ | B |

### Example 6

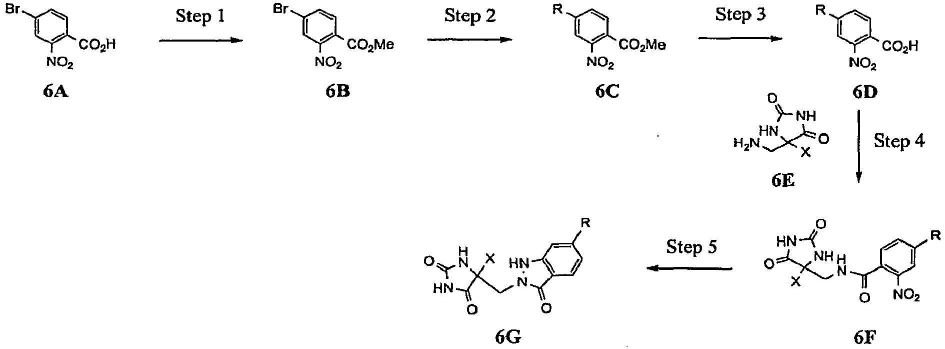

### General procedures for Example 6

In step 1, 4-Bromo-2-nitro-benzoic acid (compound **6A**) was dissolved in a suitable solvent, such as DMF, and reacted with methyl iodide in the presence of cesium carbonate at room temperature for 2-16 hours. Water and EtOAc were added and the organic phase was washed by water 1-5 times to remove DMF. The organic phase was washed with brine, dried, concentrated, and dried to give the crude product (compound **6B**) which used without further purification.

In step 2, the methyl ester (compound **6B**) was mixed with Pd(OAc)₂, Cs₂CO₃, and an appropriate ligand, such as racemic-2-(Di-*t*-butylphosphino)-1,1'-binaphthyl. The mixture was placed under vacuum for 1 to 10 minutes to remove oxygen, and refilled with N₂. An alcohol and toluene were added and the solution was stirred at 50 °C to reflux temperature for 12 to 72 hours. After cooling to room temperature, the solid was removed by filtration and the solvent was removed. The product could be purified by chromatography. During this reaction, the methyl ester may be partially converted to the ester of the alcohol used. This side product was also collected and hydrolyzed in the next step.

In step 3, compound **6C** was dissolved in Dioxane/water (3:1) and treated with lithium hydroxide at room temperature for 3-6 hours. The solution was made acidic by addition of 1 N HCl solution and subjected to aqueous/EtOAc work up. The products (compound **6D1**) were either used without further purification or purified by chromatography depending on the boiling point of the alcohol side products.

In step 4, compound **6D** was dissolved in a suitable solvent, such as DMF, and coupled with compound **6E** under EDCI and HOBT conditions at room temperature overnight. After an aqueous/EtOAc work up, the product (compound **6F**) could be isolated by chromatography.

In step 5, compound **6F** was suspended in MeOH/water (1:1) under N₂ atmosphere. NaOH and zinc powder were added and the reaction mixture was stirred at 70 °C to 80 °C for 8 to 24 hours. After cooling to room temperature, the solution was adjusted to pH=6∼7 with 1N HCl solution. Compound **6G** was extracted with EtOAc and isolated by reverse phase HPLC.

### Example 7

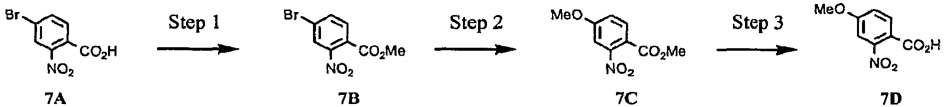

### Step 1

Compound **7A** (10.0 g, 40.7 mmol) was dissolved in DMF (100 mL). Cs₂CO₃ (27.0g, 81.3 mmol) and methyl iodide (7.60 mL, 122.0 mmol) were added. The solution was stirred at room temperature overnight. EtOAc (250 mL) and water (100 mL) were added. The organic phase was separated and washed with water (100 mL) three times and brine (50 mL), then dried over Na₂SO₄, filtered, and concentrated using a rotary evaporator. The product was dried under vacuum to give compound **7B** (10.3 g, 97%).

### Step 2

Pd(OAc)₂ (43 mg, 0.19 mmol), racemic-2-(di-*t*-butylphosphino)-1,1'-binaphthyl (92 mg, 0.23 mmol), and Cs₂CO₃ (1.88 g, 5.76 mmol) were placed in a 50 mL flask. The flask was placed under vacuum for 2 minutes and refilled with N₂. Compound **7B** (1.00 g, 3.84 mmol) and MeOH (0.311 mL, 7.69 mmol) were dissolved in toluene (10 mL). The resulting solution was added to the above flask by pipette. The reaction mixture was stirred at 70 °C oil bath for 48 hours. After cooling to room temperature, the solid was filtered and the solvent was removed using a rotary evaporator. The product was isolated by silica gel chromatography (Hexane/EtOAc 20:1 to 10:1) to give compound **7C** (380 mg, 47%).

### Step 3

Compound **7C** (380 mg, 1.80 mmol) was dissolved in dioxane/water (3:1, 8 mL) and LiOH (378 mg, 9.0 mmol) was added. The solution was stirred at room temperature for 3 hours. Water (5 mL) was added followed by addition of 2N HCl to adjust the pH = 2∼4. The aqueous phase was extracted with EtOAc (10 mL) three times. The organic phase was washed with brine, dried over Na₂SO₄, filtered, and concentrated. The crude product was dried under vacuum to give compound **7D** which was used without further purification.

The following compounds were prepared as described in **Examples 6-7**

**Table 2**

| Compound # | Structure | Exact Mass | Mass Obsvd | Ki (nM) |
|---|---|---|---|---|
| **6** | 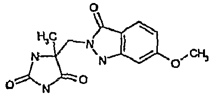 | 289.11 | 291.1 [M+H]⁺ | B |
| **7** | 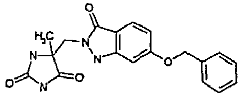 | 366.13 | 367.1 [M+H]⁺ | C |

### Example 8

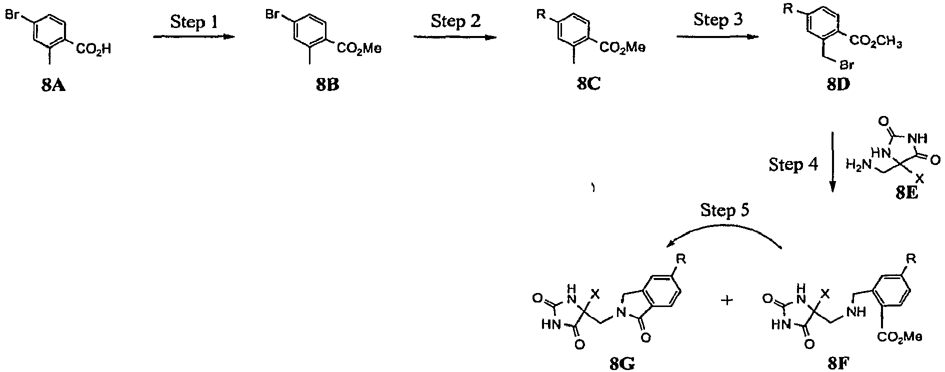

### General procedure for Example 8

In step 1, Compound **8A** was dissolved in a suitable solvent, such as DMF, and reacted with methyl iodide in the presence of cesium carbonate at room temperature for 2-16 hours. Water and EtOAc were added and the organic phase was washed by water 1-5 times to remove DMF. The organic phase was washed with brine, dried, concentrated, and dried to give the crude product (compound **8B**) which was used without further purification.

In step 2, when alcohol was used, the reaction was operated in a similar manner as step 2 in **example 6**. When an aromatic or heterocyclic stannane was used, the reaction was operated in the following manner. The aromatic or heterocyclic stannane was added into a dry flask, followed by addition of the 4-Bromo-2-methyl-benzoic acid methyl ester (compound **8B**), a base, such as Cs₂CO₃, K₃PO₄, and a palladium catalyst, such as Pd(PPh₃)₂Cl₂. The flask was placed under vacuum for 1 to 10 minutes to remove oxygen and was refilled with N₂. An appropriate solvent, such as dry CH₃CN, was added and the solution was stirred at 60 °C to reflux temperature overnight to 3 days. The solid was removed by filtration and the solvent was removed. Compound **8C** was isolated by chromatography.

In step 3, compound **8C** was dissolved in a suitable inert solvent, such as benzene, CCl₄ or α,α,α-Trifluorotoluene. NBS and benzoyl peroxide were added and the solution was stirred at 50 °C to 90 °C for 1 to 24 hours. The solid was filtered and the solvent was removed. The residue was dissolved in ether and washed by water. The ether was removed to afford the compound **8D** which was used without further purification.

In step 4, the benzyl bromide (compound **8D**) was mixed with hydantoin methyl amine **8E**, K₂CO₃, and DMF. The solution was stirred at room temperature for 12 to 24 hours. Then the solid was removed by filtration. The product could be purified by reverse phase HPLC. Compounds **8F** and **8G** could be obtained in a variable ratio.

Step 5 is used when the compound **8F** was isolated in step 4. Compound **8F** was dissolved in an appropriate solvent, such as MeOH, and stirred at 50 °C to reflux temperature for 1 to 12 hours. The product could be obtained by removing the solvent by rotary evaporator or purifying via reverse phase chromatography.

### Example 9

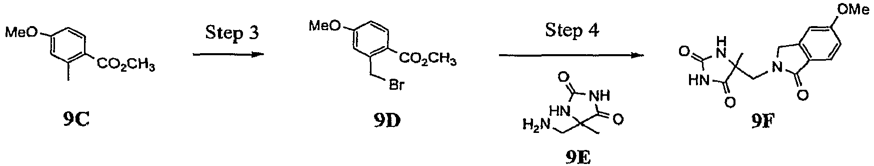

### Step 3

Compound **9C** (prepared according to the procedure described by Wyrick, S. D. et al. *Journal of Medicinal Chemistry*, **1987**, 30(*10*),1798-806) (3.33 g, 18.5 mmol) was dissolved in dry benzene (40 mL). NBS (3.45 g, 19.4 mmol) and benzoyl peroxide (134 mg, 0.55 mmol) were added. The solution was stirred in a 75 °C oil bath for about 2 hours. After cooling down, the solid was filtered and washed with Et₂O (150 mL). The organic solution was then washed with water (50 mL) twice, dried over Na₂SO₄ or MgSO₄, filtered, and concentrated by rotary evaporator. The crude product was dried under vacuum to give compound **9D** which was used without further purification. ¹H-NMR appeared to indicate that approximately 75% of this material was compound **9D**.

### Step 4

Compound **9D** (4.62 mmol), Compound **9E** (824 mg, 4.62 mmol), and K₂CO₃ (1.28 g, 9.24 mmol) were mixed in DMF (30 mL). The solution was stirred at room temperature for 20 hours. DMF (15 mL) was added and the solid was filtered and washed with DMF. All the DMF solution was combined and concentrated to 25 mL. The resulting solution was applied to reverse phase MPLC (CH₃CN/water, 5% to 90%, containing 0.1%HCO₂H) to give compound **9F** (198 mg, 15%).

### Example 10

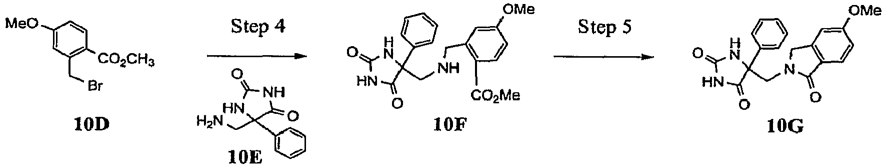

### Step 4

Compound **10D** (prepared in example 9) (902 mg, 2.07 mmol, factor = 0.75), Compound **10E** (prepared as described in **Example 1**, 500 mg, 2.07 mmol), and K₂CO₃ (629 mg, 4.56 mmol) were mixed in DMF (15 mL). The solution was stirred at room temperature for 20 hours. DMF (15 mL) was added and the solid was filtered and washed with DMF. All the DMF solution was combined and concentrated to 20 mL. It was applied to reverse phase MPLC (CH₃CN/water: 5% to 90%, containing 0.1% HCO₂H) to give compound **10F.**

### Step 5

Compound **10F** (prepared in step 4) was dissolved in MeOH (5 mL), stirred at 65 °C for 5 hours, then concentrated to dryness. The compound was suspended in water and dried with lyophilizer to give compound **10G** (68.3 mg, 9.4%).

### Example 11

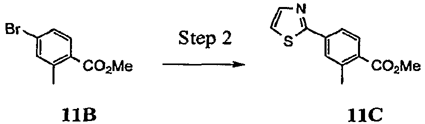

### Step 2

Compound **11B** (500 mg, 2.18 mmol), 2-tributylstannylthiazole (0.97 mL, 2.84 mmol), Pd(PPh₃)₂Cl₂, and dry CH₃CN were stirred under nitrogen at reflux temperature overnight. After cooling to room temperature, the solid was filtered. The product was isolated by silica gel chromatography (Hexane/EtOAc: 20:1 to 10:1 to 5:1) to give compound **11C** (480 mg, 94%).

The following compounds were prepared as described in **Examples 8-11**.

**Table 3**

| Compound # | Structure | Exact Mass | Mass Obsvd | Ki (nM) |
|---|---|---|---|---|
| **8** | 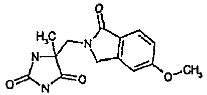 | 289.11 | 290.1 [M+H]⁺ | B |
| **9** | 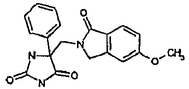 | 351.12 | 352.1 [M+H]⁺ | A |
| **10** | 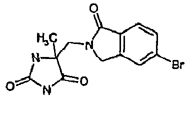 | 337.01 | 338 [M+H]⁺ | C |
| **11** | 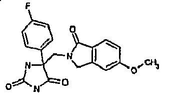 | 369.11 | 370.1 [M+H]⁺ | A |
| **12** | 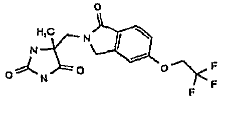 | 357.09 | 358.1 [M+H]⁺ | B |
| **13** | 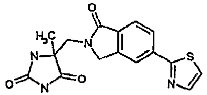 | 342.08 | 343.1 [M+H]⁺ | C |
| **14** | 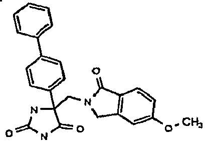 | 427.2 | 428.2 [M+H]⁺ | A |
| **15** | 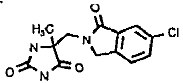 | 293.06 | 294.1 [M+H]⁺ | B |
| **16** | 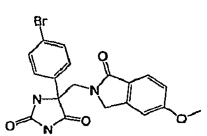 | 431.0 | 432.1 [M+H]⁺ | A |
| **17** | 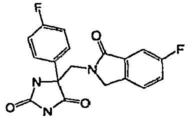 | 357.1 | 358.1 [M+H]⁺ | A |
| **18** | 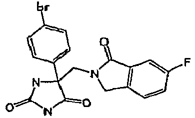 | 417.0 | 418.1 [M+H]⁺ | A |
| **19** | 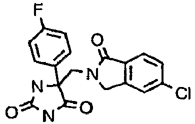 | 373.1 | 374.2 [M+H]⁺ | A |

The following additional compounds were prepared as described in **Examples 8** to **11**.

**Table 4**

| **Compound #** | **Structures** | **Exact Mass** | **Mass Obsvd** | **Ki (nM)** |
|---|---|---|---|---|
| **294** | 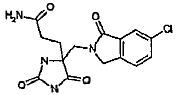 | **350.08** | **351.1[M+H]⁺** | **B** |
| **295** | 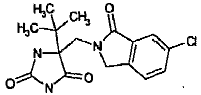 | **335.10** | **336.1[M+H]⁺** | **B** |

### Example 12

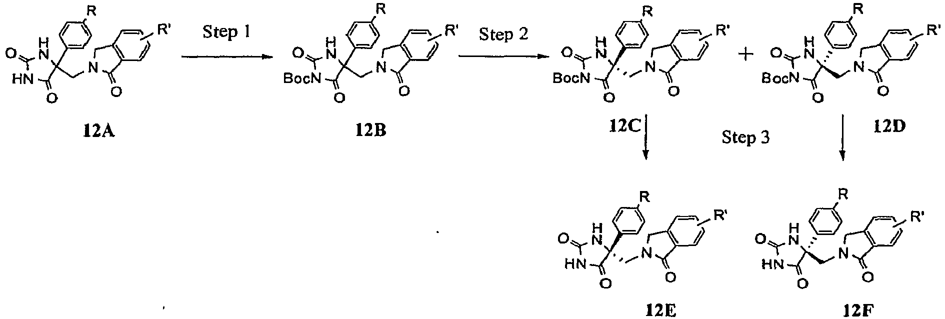

### General procedures for Example 12:

In step 1, racemic compound **12A** was treated with one equivalent of di-tert-butyl dicarbonate and 4-N,N-dimethylaminopyridine in polar solvent, such as DMF, for 30 minutes to 12 hours. The solvent was removed and the product (compound **12B**) was isolated by silica gel (pretreated with 1% triethylamine in Hexane) chromatography.

In step 2, compound **12B** was dissolved in proper solvents allowed by HPLC column, and resolved by HPLC using a preparative Chiralpak AD or Chiralcel OD column to give compound **12C** and **12D**.

In step 3, compound **12C** and **12D** were treated with excess HCl in methanol at 25 °C to 60 °C for one hour to 12 hours. The solvent was concentrated to give compound **12E** and **12F**.

### Example 13

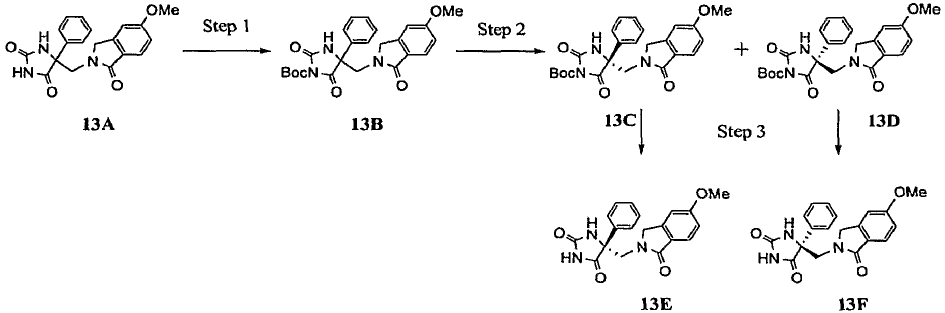

### Step 1

Compound **13A** (810 mg, 2.07 mmol), di-tert-butyl dicarbonate (429 mg, 1.97 mmol), and 4-dimethylaminopyridine (20 mg) were dissolved in a mixture of DMF (10 mL) and THF (20 mL). The solution was stirred at 25 °C overnight. Solvents were removed by rotary evaporator. The product was isolated by C18 chromatography (CH₃CN/water: 5% to 90%) to give product **13B** (650 mg, 70%).

### Step 2

Compound **13B** (600 mg) was dissolved in a mixture of iso-propanol (6 mL) and CHCl₃ (4 mL). 2.5 mL was separated via HPLC with preparative chiralcel OD column (Mobile phase: iso-propanol/Hexane: 1:4). Fractions for each peak were collected and concentrated by rotary evaporator to give compound **13C** (First peak, 197 mg) and compound **13D** (second peak, 178 mg).

### Step 3

Compound **13C** (197 mg) was dissolved in methanol (3 mL). HCl (4M in Dioxane, 0.5 mL) was added. The solution was stirred in a 60 °C oil bath for three hours. Methanol was removed by rotary evaporator to give compound **13E**.

Compound **13F** was prepared in the same way as compound **13D** (178 mg).

The following compounds were prepared as described in **Examples 12-13**

**Table 5**

| Compound # | Structures | Exact Mass | Mass Obsvd | Ki (nM) |
|---|---|---|---|---|
| 20 | 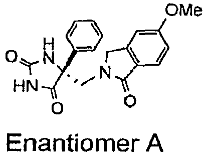 | 351.1 | 352.2 [M+H]⁺ | A |
| 21 | 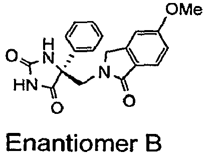 | 351.1 | 352.2 [M+H]⁺ | C |
| 22 | 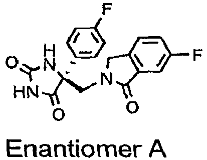 | 357.1 | 358.1 [M+H]⁺ | C |
| 23 | 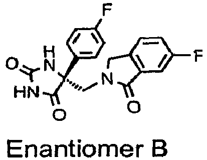 | 357.1 | 358.1 [M+H]⁺ | A |
| 24 | 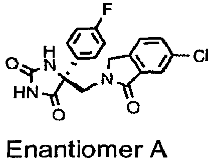 | 373.06 | 374.2 [M+H] | C |
| 25 | 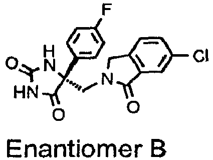 | 373.06 | 374.2 [M+H] | A |

**Proton NMR Spectral Data for Selected Compounds in Table 5.**

**Compound 25.** ¹H NMR (500 Hz, DMSO-d₆) δ4.06 (d, J = 14Hz, 1H), 4.20 (d, J = 14 Hz, 1H), 4.32 (d, J = 18 Hz, 1H), 4.38 (d, J = 18 Hz, 1H), 7.19-7.39 (m, 2H), 7.55-7.80 (m, 5H), 8.93 (s, 1H), 10.96 (s, 1H).

### Example 14

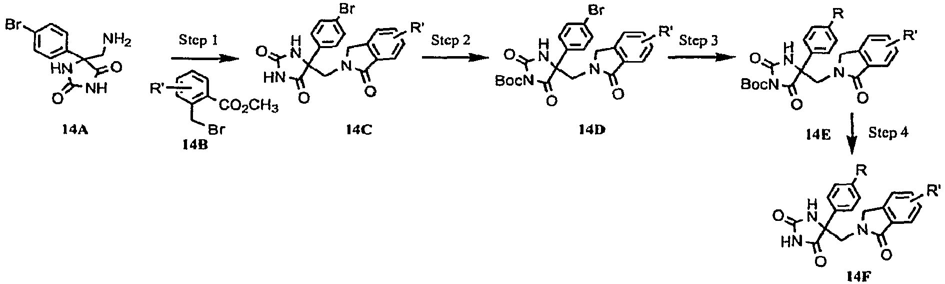

### General procedure for Example 14

In step 1, compound **14A** (prepared as described in **Example 1**) was treated with a benzyl bromide (Compound **14B**) and DIPEA base in DMF at 25 °C to 60 °C for 12 to 24 hours. The reaction solution was purified via C18 reverse phase chromatography to give compound **14C**.

In step 2, compound **14C** was treated with one equivalent di-tert-butyl dicarbonate in polar solvent, such as DMF, for 30 minutes to 12 hours. The solvent was removed and the product (compound **14D**) was isolated by silica gel (pretreated with 1% triethylamine in Hexane) chromatography.

In step 3, compound **14D** was subjected to either a Pd catalyzed reaction with a heterocyclic boronic acid or a heterocyclic stannane, or a copper catalyzed reaction with a heterocyclic amine. The reaction were heated in appropriate solvents, such as DMF and acetonitrile, at 60 °C to 150 °C, for 5 minutes to 12 hours. In some cases, a microwave reactor was used. The product was purified by silica gel chromatography to give compound **14E** or compound **14F**.

In step 4, compound **14E** was dissolved in methanol and was stirred with HCl for 1 hour to 12 hours at 25 °C to 60 °C. The solvent was removed to give compound **14F**.

The following compounds were prepared as described in step 1 of **Example 14** above.

**Table 6**

| **Compound #** | **Structure** | **Exact Mass** | **Mass Obsvd** | **Ki (nM)** |
|---|---|---|---|---|
| **100** | 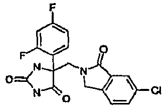 | **391.05** | **392.07 [M+H]⁺** | **n/a** |
| **101** | 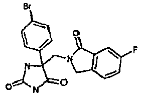 | **417.01** | **418.1 [M+H]⁺** | **A** |
| **102** | 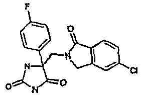 | **373.06** | **374.2 [M+H]⁺** | **A** |
| **103** | 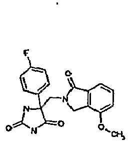 | **369.11** | **370.1 [M+H]⁺** | **D** |
| **104** | 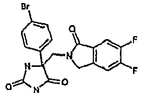 | **435.00** | **436.1 [M+H]⁺** | **B** |
| **105** | 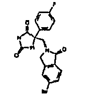 | **417.01** | **418.420 [M+H]⁺** | **B** |
| **106** | 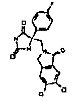 | **407.0** | **408 [M+H]⁺** | **A** |
| **107** | 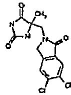 | **327.0** | **328 [M+H]⁺** | **B** |
| **108** | 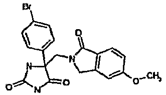 | **429.03** | **430.432 [M+H]⁺** | **B** |
| **109** | 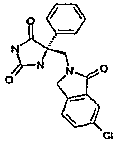 | **355.07** | **378.2 [M+Na]⁺** | **B** |

### Example 15

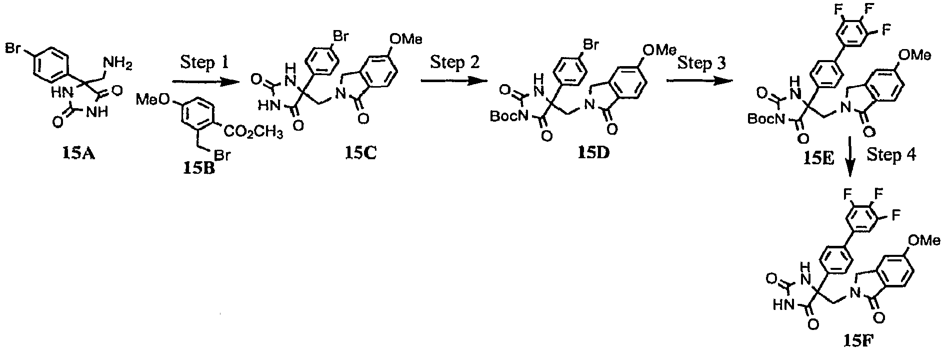

### Step 1

Compound **15A** (prepared as described in **Example 1**, 1.0 g, 3.12 mmol), Compound **15B** (prepared in **Example 9**, 1.06 g, 3.12 mmol, factor = 0.76), and DIPEA base (1.14 mL, 6.55 mmol) were mixed in DMF (22 mL). The solution was stirred at 55 °C for 20 hours. The reaction solution was purified via C18 reverse phase MPLC (130g column, CH₃CN/water/0.1% HCO₂H, 5% to 90%, two separations) to give compound **15C** (900 mg, 67%).

### Step 2

Compound **15C** (2.7 g, 6.28 mmol) was suspended in a mixture of DMF (20 mL) and THF (40 mL). Di-tert-butyl dicarbonate (1.51 g, 6.91 mmol) and 4-dimethyaminopyridine (38 mg, 0.31 mmol) were added. The solution was stirred at 25 °C for 16 hours. The solvents were removed by rotary evaporator. The residue was subjected to silica gel chromatography (Hexane/EtOAc: 2:1 to 1:1) to give compound **15D** (2.36 g, 71%).

### Step 3

Compound **15D** (100 mg, 0.19 mmol), 3,4,5-trifluorophenyl boronic acid (40 mg, 0.23 mmol), 1,1'-bis(triphenylphosphino)ferrocene palladium (II) chloride (15 mg, 0.02 mmol), potassium carbonate (1 M in water, 1 mL) and acetonitrile (1 mL) were added to a microwave reactor tube. The tube was sealed and reacted in the microwave reactor at 150 °C for 10 minutes. After cooling down, the aqueous layer was removed and the organic layer was concentrated. The crude product was purified by silica gel chromatography (CH₂Cl₂/MeOH/NH₃: 40:1:0.1) to give compound **15E**.

### Step 4

Compound **15E** obtained from step 3 was suspended in MeOH. HCl (2M in ethyl ether, 0.5 mL) was added. The reaction mixture was stirred at 50 °C for five hours. The solvent was removed. The product was purified via C18 reverse phase chromatography (CH₃CN/water/0.1% HCO₂H, 5% to 90%) to give compound **15F** (8 mg, 8.8% from compound **15D**).

### Example 16

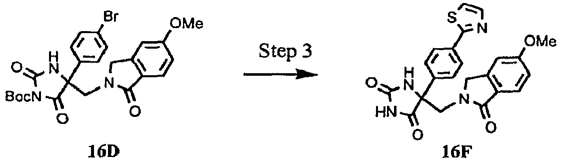

### Step 3

Compound **16D** (50 mg, 0.094 mmol, prepared in example 13), 2-tributylstannylthiazole (53 mg, 0.14 mmol), dichlorobis(triphenylphosphine) palladium (II) (7 mg, 0.01 mmol), and acetonitrile (1 mL) were added to a microwave reactor tube. The tube was sealed and reacted in a microwave reactor at 150 °C for 10 minutes. The solvent was evaporated and the product was purified by silica gel chromatography (CH₂Cl₂/MeOH/NH₃: 40:1:0.1 to 20:1:0.1) to give compound **16F** (15 mg, 37%).

### Example 17

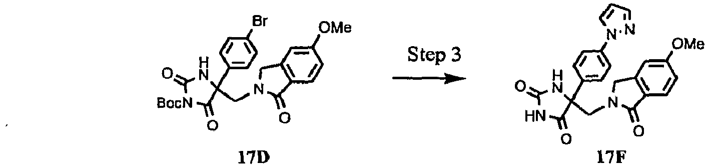

### Step 3

Compound **17D** (100 mg, 0.19 mmol, prepared in example 13), pyrazole (15.4 mg, 0.23 mmol), cesium carbonate (124 mg, 0.38 mmol), copper iodide (7.2 mg, 0.038 mmol), 1,10-phenanthroline (14 mg, 0.076 mmol), and N,N-dimethylacetamide (0.5 mL) were added to a dry reaction tube and filled with nitrogen. The reaction tube was sealed and heated in a 120 °C oil bath for two days. After cooling down, the reaction solution was purified by C18 chromatography (CH₃CN/water/0.1% HCO₂H, 5% to 90%) to give compound **17F** (5 mg, 6.4%).

The following compounds were prepared as described in **Examples 14-17**

**Table 7.**

| Compound # | Structure | Exact Mass | Mass Obsvd | Ki (nM) |
|---|---|---|---|---|
| 26 | 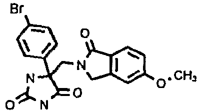 | 429.0 | 430.1 [M+H]⁺ | A |
| 27 | 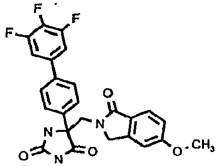 | 481.1 | 482.3 [M+H]⁺ | B |
| 28 | 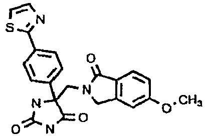 | 434.1 | 435.1 [M+H]⁺ | A |
| 29 | 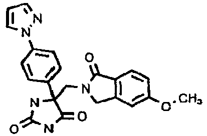 | 417.1 | 418.2 [M+H]⁺ | A |
| 30 | 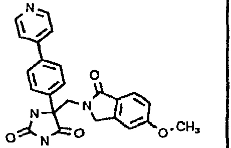 | 428.2 | 429.1 [M+H]⁺ | A |

### Example 18

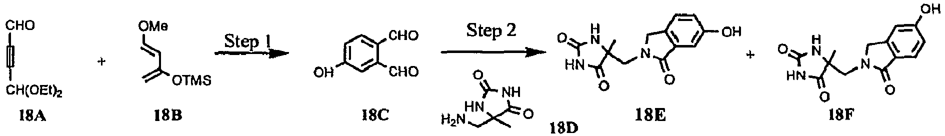

### Step 1:

Compound **18A** (1.0 g, 6.4 mmol) and compound **18B** (1.324 g, 7.68 mmol) were dissolved in toluene (4 mL) and stirred at 80 °C for 24 hours. After cooling to room temperature, the solvent was removed by rotary evaporator. Half of the crude product was dissolved in THF/1N HCl (1:1, 14 mL) and stirred at room temperature for 2 hours. EtOAc (15 mL) and water (5 mL) were added. The organic phase was separated and the aqueous phase was extracted with EtOAc (15 mL) twice. The combined organic phase was dried over Na₂SO₄ and concentrated by rotary evaporator to give compound **18C** which was used without further purification.

### Step 2

Compound **18C** (prepared in step 1) was dissolved in DMF (15 mL) and was cooled to 0 °C in an ice-water bath. Compound **18D** (571 mg, 3.2 mmol) was added in one portion. The solution was allowed to warm up to room temperature over 2 hours, and stirred at room temperature for 3 days. A 2N HCl solution (20 mL) was added and the aqueous phase was extracted with EtOAc (50 mL) three times. The organic phases were combined, dried over Na₂SO₄, and concentrated to dryness. The product was isolated by reverse phase LC (CH₃CN/water/0.1% HCO₂H: 5% to 90%) to give compound **18E** (65 mg, 7.4% from step 1) and Compound **18F** (16 mg, 1.8% from step 1).

The following compounds were prepared as described in **Example 18**

**Table 8**

| Compound # | Structure | Exact Mass | Mass Obsvd | Ki (nM) |
|---|---|---|---|---|
| **31** | 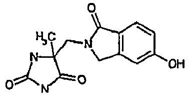 | 275.09 | 276.0 [M+H]⁺ | B |
| **32** | 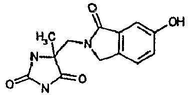 | 275.09 | 276.0 [M+H]⁺ | C |

### Example 19

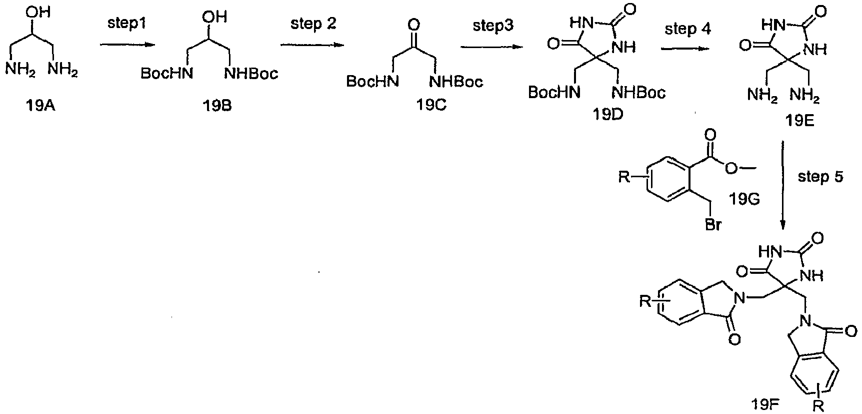

### General procedures for Example 19:

In step1, compound **19A** was treated with two equivalent of Boc₂O in a suitable solvent, such as dichloromethane, for 30 min. to 12 h. The solvent was removed and the compound **19B** could be used without further purification or purified by silica gel chromatography.

In step 2, compound **19B** was treated with PCC and celite in a suitable solvent such as dichloromethane, for 2 hr to 12 hr. Compound **19C** was purified by silica gel chromatography.

In step 3, compound **19C** was reacted with potassium cyanide and ammonium carbonate in appropriated alcohol and water solution, at 50 °C to 90 °C, for 5 hours to 48 hours. After cooling down, water was added and compound **19D** could be collected by filtration.

In step 4, compound **19D** was stirred with 2 to 20 equivalents of hydrogen chloride in methanol for 5 to 48 hours. The solvent was removed and the compound **19E** could be used without further purification.

In step 5, the benzyl bromide (compound **19G**) was mixed with hydantoin methyl amine **19E**, DIPEA, and DMF. The solution was stirred at room temperature for 12 to 24 hours. The product (**19F**) was either removed by filtration or purified by silica gel chromatography.

### Example 20.

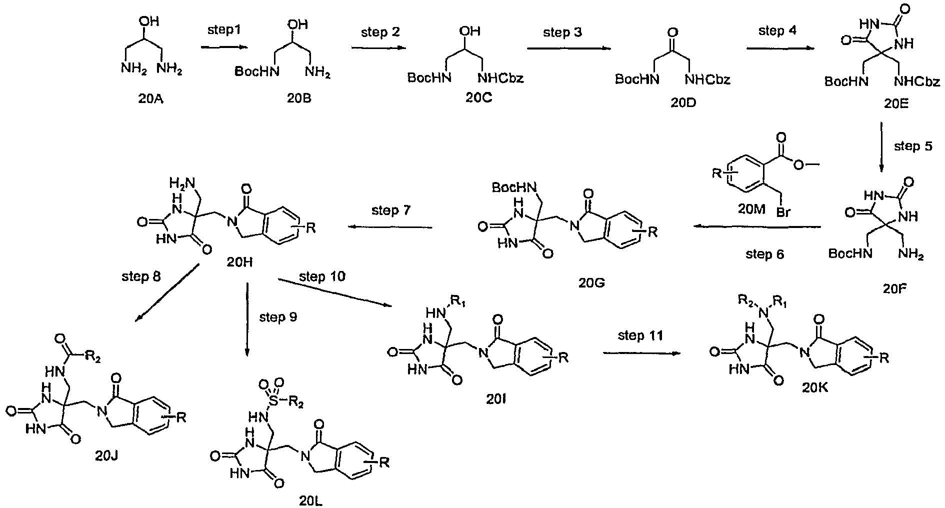

### General procedures for Example 20:

In step 1, Compound **20A** was treated with BOC-ON in a suitable solvent such as dichloromethane, for 2 hr to 12 hr. Compound **20B** was purified by silica gel chromatography.

In step 2, Compound **20B** was treated with CbzCl and a base such as DIPEA, in a suitable solvent, such as dichloromethane, for 2 hr to 12 hr. Compound **20C** was purified by silica gel chromatography.

In step 3, compound **20C** was treated with PCC and celite in a suitable solvent such as dichloromethane, for 2 hr to 12 hr. Compound **20D** was purified by silica gel chromatography.

In step 4, compound **20D** was reacted with potassium cyanide and ammonium carbonate in appropriated alcohol and water solution, at 50 °C to 90 °C, for 1 hour to 48 hours. After cooling down, water was added and compound **20E** could be collected by filtration.

In step 5, Compound **20E** was treated with Pd/C in a suitable solvent such as methanol, in a par shaker under H₂ atmosphere. After filtering off the catalyst and concentration of solvent, the product was used without further purification.

In step 6, the benzyl bromide (compound **20M**) was mixed with hydantoin methyl amine **20F**, DIPEA, and DMF. The solution was stirred at at room temperature to 80 °C for 12 to 24 hours. The product was either removed by filtration or purified by silica gel chromatography.

In step 7, compound **20G** was stirred with 2 to 20 equivalents of hydrogen chloride in dioxane for 1 to 12 hours. The solvent was removed and the compound **20H** was used without further purification.

In step 8, Compound **20H** was coupling with carboxylic acid to give compound **20J** which was purified by silica gel chromatography.

In step 9, Compound **20H** was coupling with sulphonyl chloride compound to give compound **20L** which was purified by silica gel chromatography.

In step 10, Compound **20H** was reacted with carbonyl compound under reductive amination condition to give compound **20I.** Alternatively, compound **20H** was treated with a suitable electrophile and a base to give the product **20I**, which was purified by silica gel chromatography.

In step 11, compound **20I** was reacted with carbonyl compound under reductive amination condition to give product **20K**. Alternatively, compound **20I** was treated with a suitable electrophile and a base to give the product **20K**, which was purified by silica gel chromatography.

### Example 21:

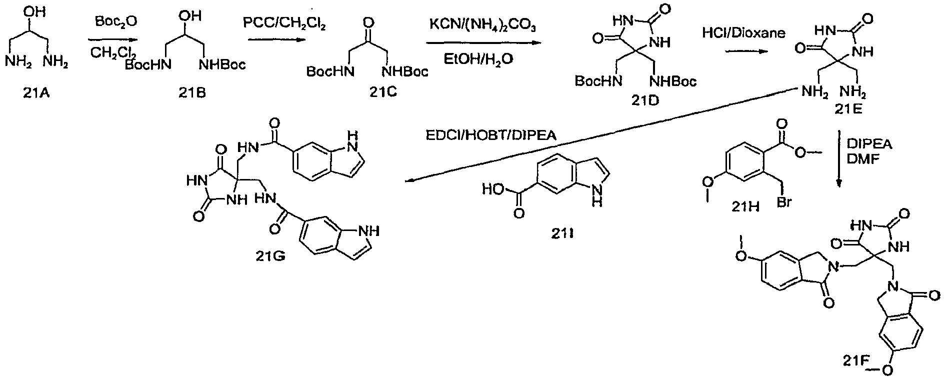

Compound **21B**: Compound **21A** (7 g, 77.7 mmol), and di-tert-butyl dicarbonate (35.6 g, 163 mmol) were stirred in methylene chloride (100 mL) at 25 °C for 2 hr. Saturated aqueous NaCl (150 mL) was added. The aqueous layer was extracted with CH₂Cl₂ (100 mL) twice. The organic phase was washed with brine (100 mL), dried over Na₂SO₄. The solvent was removed by rotary evaporator to give compound **21B** (17g, 76%) which was used without further purification.

Compound **21C**: compound **21B** (17 g, 58.6 mmol) was dissolved in methylene chloride (100 mL). PCC (25.2 g, 117 mmol) and celite (15 g) were added and the reaction mixture was stirred at 25 °C overnight. The solid was filtered off and the resulting solution was concentrated and purified via sgc (40% EtOAc/Hexanes) to give 3.62 g (22%) of compound **21C.**

Compound **21D:** Compound **21C** (3.62, 12.6 mmol), KCN (1.23g, 18.9 mmol), and (NH₄)₂CO₃ (3.62 g, 37.7 mmol) were suspended in a mixture of EtOH (30 mL) and water (30 mL). The solution was stirred at 80 °C overnight. After cooling down, water (35 mL) was added. The solid was filtered and washed with water three times. The solid was dried under vacuum to give compound **21D** (3 g, 67%).

Compound **21E**: Compound **21D** (3.0 g) was suspended in methanol (50 mL) and HCl (4M in dioxane, 20 mL) was added. The solution was stirred at 25 °C for 3 hours. Ethyl ether (50 ml) was added. The solid was filtered, washed by ethyl ether twice, and dried under vacuum compound **21E** (1.34 g, 70%).

Compound **21F**: Compound **21E** (130 mg, 0.82 mmol), compound **21H** (0.27 g, 1 mmol) and DIPEA (0.55 mL, 2 mmol) were mixed in DMF (5 mL). The solution was stirred at room temperature overnight. Solvent was removed and the crude material was and purified via sgc (5% NH₃•MeOH/CH₂Cl₂) to give 129 mg (35%) of compound **21E**.

### Example 22:

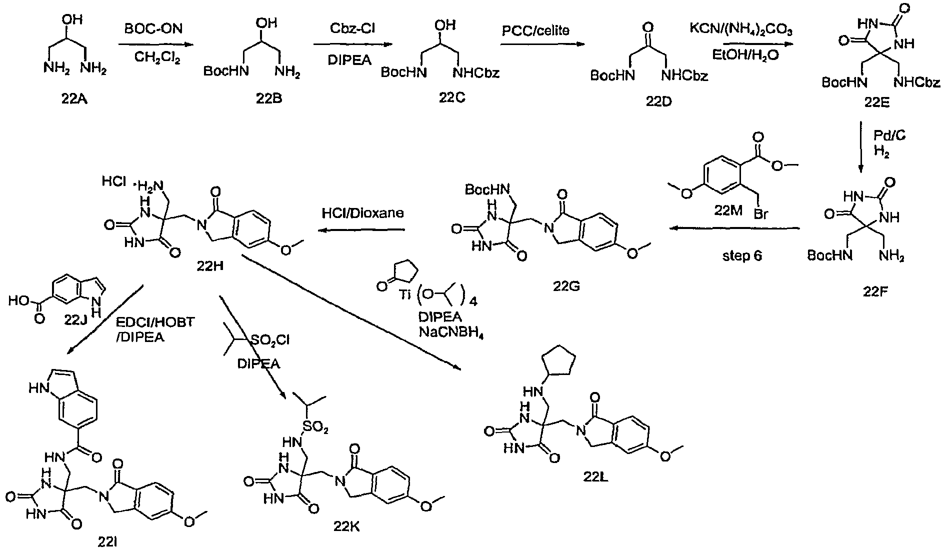

Compound **22B**: Compound **22A** (7.3 g, 81 mmol) was treated with BOC-ON (21.9 g, 89 mmol) in dichloromethane for 3 hr. Solvent was removed and the crude material was purified via sgc (10% NH₃•MeOH/CH₂Cl₂) to give 6.5 (42%) of compound **22B**.

Compound **22C**: Compound **22B** (1.5g, 7.9 mmol) was dissolved in dichloromethane (50 mL) at 0 °C. CbzCl (1.24 ml, 8.7 mmol) and DIPEA (1.52 ml, 8.7 mmol) were added and the reaction was stirred at 0 °C for 30 min. The reaction mixture was washed by HCl (1N, 50 mL) and brine (50 mL). The organic layer was dried and concentrated to give crude compound **22C** (2.6 g, 99%) which was used without further purification.

Compound **22D**: Compound **22C** (2.78g, 8.57 mmol) was dissolved in methylene chloride (100 mL). PCC (4.62 g, 21.4 mmol) and celite (4.6 g) were added and the reaction mixture was stirred at 25 °C overnight. Another 0.5 eq. of PCC (923 mg, 4.3 mmol) was added and it was stirred for 3 hr at room temperature. The solid was filtered off and the resulting solution was concentrated and purified via sgc (50% EtOAc/Hexanes) to give 1.86 g (73%) of compound **22D.**

Compound **22E:** Compound **22D** (1.86, 5.8 mmol), KCN (0.56 g, 8.65 mmol), and (NH₄)₂CO₃ (1.66 g, 17.3 mmol) were suspended in a mixture of EtOH (20 mL) and water (20 mL). The solution was stirred at 80 °C overnight. After cooling down, EtOH was removed. The solid was filtered and washed with water three times. The solid was dried under vacuum to give compound **22E** (1.45 g, 64%).

Compound **22F:** Compound **22E** (1.45 g, 3.68 mmol) was treated with Pd/C in methanol in a par shaker under H₂ atmosphere of 50 psi for 60 hr. After filtering off the catalyst and concentration of solvent, Compound **22E** (0.95 g, 99%) was used without further purification.

Compound **22G:** Compound **22F** (150 mg, 0.58 mmol), compound **22M** (170 mg, 0.64 mmol) and DIPEA (0.22 mL, 1.28 mmol) were mixed in DMF (5 mL). The solution was stirred at 70 °C overnight. Solvent was removed and the crude material was and purified via sgc (5% NH₃•MeOH/CH₂Cl₂) to give 166 mg (71%) of compound **22G**.

Compound **22H:** Compound **22G** (166 mg) was suspended in methanol (10 mL) and HCl (4M in dioxane, 4 mL) was added. The solution was stirred at 25 °C for 2 hours. Ethyl ether (50 ml) was added. Solvent was removed and give compound **22H** (0.14 g, 99%).

Compound **22I:** Compound **22H** (42 mg, 0.12 mmol) and compound **22J** (26 mg, 0.16 mmol) were dissolved in DMF (20 mL). EDCI (30 mg, 0.16 mmol), HOBT (21 mg, 0.16 mmol) and DIPEA (0.05 mL, 0.28 mmol) were added and the reaction mixture was stirred at room temperature for 2 hr. Solvent was removed and the crude material was and purified via sgc (10% NH₃•MeOH/CH₂Cl₂) to give 7 mg (13%) of compound **22I.**

Compound **22L:** Compound **22H** (25 mg, 0.073 mmol) and cyclopentanone (7.5 mg, 0.088 mmol) were stirred in methylene chloride (5 mL). Titanium tetraisopropoxide (0.043 mL, 0.15 mmol) was added followed by addition of DIPEA (0.015 mL, 0.088 mmol). The reaction mixture was stirred at room temperature for 2h. Then, Na(OAc)₃BH (31 mg, 0.15 mmol) was added and the mixture was stirred at rt overnight. Saturated K₂CO₃ aq. (20 mL) was added, and the mixture was stirred at rt briefly. The solid was filtered off through a celite pad. The filtrate was diluted with methylene chloride (30 mL), and it was extracted with brine. The organic layer was dried and concentrated to dryness. The crude material was purified via PTLC (10% NH₃•MeOH/CH₂Cl₂) to give 7 mg (26%) of compound **22L.**

Compound **22K:** Compound **22H** (20 mg, 0.06 mmol) and isopropyl sulphonyl (27 mg, 0.18 mmol) were dissolved in methylene chloride (10 mL). DIPEA (0.04 mL, 0.26 mmol) were added and the reaction mixture was stirred at room temperature for 48 hr. Solvent was removed and the crude material was and purified via sgc (10% NH₃•MeOH/CH₂Cl₂) to give 2 mg (8%) of compound **22K**.

The following compounds were prepared as described in **Examples 19-22.**

**Table 9**

| Compound # | Structure | Exact Mass | Mass Obsvd | Ki (nM) |
|---|---|---|---|---|
| **33** | 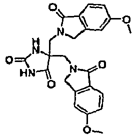 | 450.15 | 451.1 [M+H]⁺ | B |
| **34** | 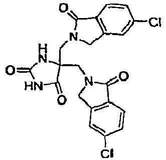 | 458.05 | 459.3 [M+H]⁺ | B |
| **35** | 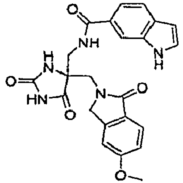 | 447.15 | 448.2 [M+H]⁺ | A |
| **36** | 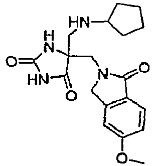 | 372.18 | 373.2 [M+H]⁺ | B |
| **37** | 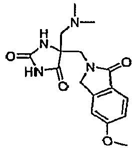 | 332.15 | 333.1 [M+H]⁺ | C |
| **38** | 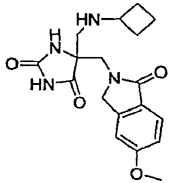 | 358.16 | 359.2 [M+H]⁺ | B |
| **39** | 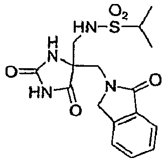 | 380.12 | 381.2 [M+H]⁺ | C |
| **40** | 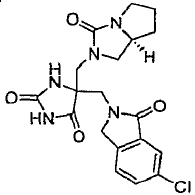 | 417.12 | 418.1 [M+H]⁺ | D |
| **41** | 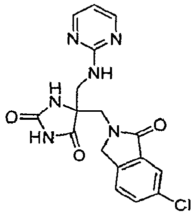 | 386.09 | 387.2 [M+H]⁺ | C |

### Example 1001:

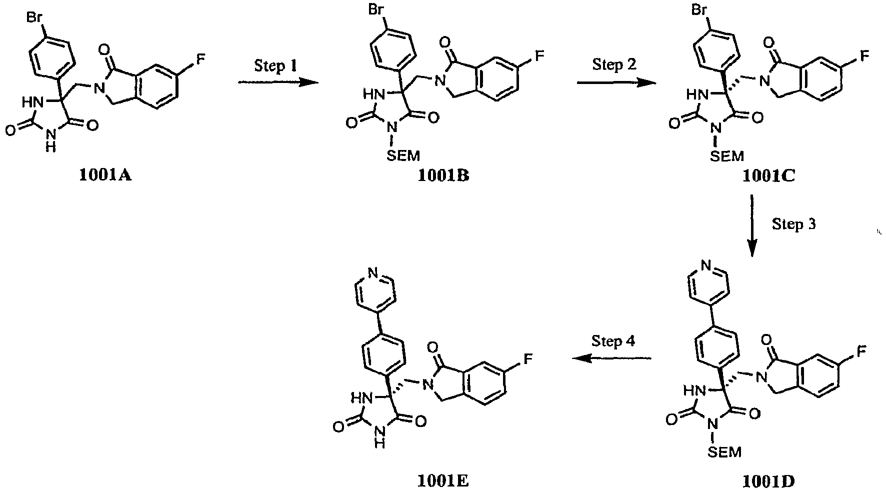

### Step 1

To a solution of compound **1001A** (1.65 g, 3.95 mmol) in anhydrous DMF (35 mL) was added 2-(trimethylsilyl)ethoxymethyl chloride (SEMCI, 0.93 mL, 4.73 mmol) and DIPEA (0.9 mL, 5.14 mmol). The solution was stirred at 25 °C for overnight. DMF was removed under vacuum. The product **1001B** was purified by SGC (Hexane/EtOAc, 2:1. yield: 1.6 g, 74%).

### Step 2

Compound **1001B** was resolved by Chiralcel OD column (Mobile phase: Hexane/2-propanol 3:1). The first peak was collected and concentrated to give compound **1001C.**

### Step 3

To a dry flask was added compound **1001C** (1.5 g, 2.73 mmol) and 4-pyridyl boronic acid (670 mg, 5.50 mmol). The flask was vacuumed and refilled with nitrogen three times. Pd(dppf)Cl₂ (220 mg, 0.30 mmol) was added and followed by addition of CH₃CN (20 mL) and aq. K₂CO₃ (1 M, 15 mL). The solution was stirred at 80 °C (oil bath) for 16 hours. After cooling down, CH₃CN (100 mL) was added and the solid was removed by filtration. The aqueous layer was separated and extracted with EtOAc (20 mL) once. The organic solution was combined and concentrated. The product was purified by SGC (CH₂Cl₂/MeOH/NH₄OH: 20:1:0.1) to give compound **1001D**.

### Step 4

Compound **1001D** was dissolved in a mixture of methanol and HCl (4M in dioxane) (2:1, 30 mL) and was stirred overnight in a sealed pressure flask at 90 °C (oil bath). After the solution was cooled, the solution was transferred into a 250 mL round bottom flask. It was concentrated and dried under vacuum. The crude mixture was dissolved in methanol (50 mL) and Et₃N (0.5 mL) was added and stirred overnight at 25 °C. The solvent was then removed and the product was purified by C18 reverse phase chromatography (CH₃CN/water 5% to 90%, with addition of 0.1% HCO₂H) to give compound **1001E** (815g, 71% from compound **1001C**).

### Example 1002

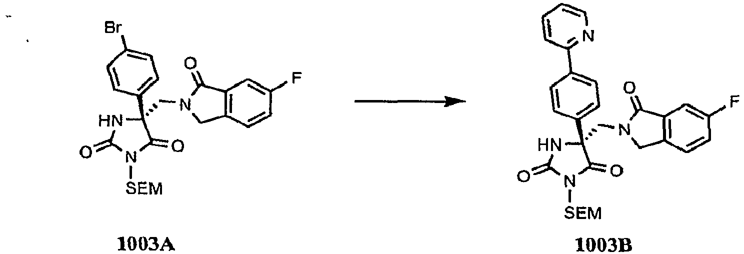

To a flamed dried flask was added compound **1003A** (100 mg, 0.182 mmol), [1,4-bis(diphenylphosphino)butane] palladium(II) dichloride [Pd(dppb)Cl₂, 12 mg, 0.02 mmol], and copper (II) oxide (15 mg, 0.18 mmol). The flask was vacuumed and refilled with nitrogen. 2-Tri-n-butylstannylpyridine (0.076 mL, 0.237 mmol) and DMF (1 mL) were added. The solution was stirred at 100 °C oil bath for 5 hours. After cooling, the DMF was removed by rotary evaporator. The product was purified by SGC (Hexane/EtOAc 2:1) to give **1003B** (84 mg, 84%).

### Example 1003

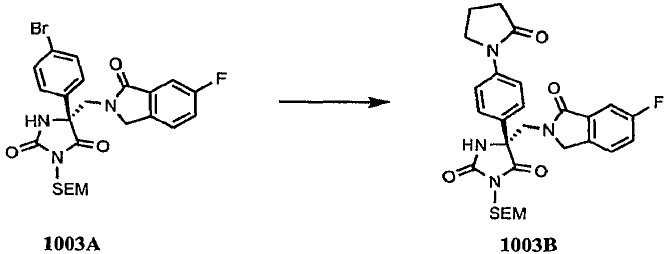

To a dry pressure tube was added compound **1003A** (50 mg, 0.091 mmol), bis(dibenzylideneacetone) palladium [Pd(dba)₂, 1.6 mg, 0.0018 mmol], 9,9-dimethyl-4,5-bis(diphenylphosphino)xanthene (Xantphos, 3.0 mg, 0.0055 mmol), and Cs₂CO₃ (60 mg, 0.182 mmol). The pressure tube was vacuumed and refilled with nitrogen. Pyrrolidinone (14 mg, 0.16 mmol) and dioxane(0.5 mL) were added. The tube was sealed and stirred overnight at 100 °C (oil bath). After cooling, dioxane (2 mL) was added and the solid was removed by filtration. The solution was concentrated and purified by SGC (CH₂Cl₂/MeOH: 40:1) to give compound **1003B** (27 mg).

### Example 1004:

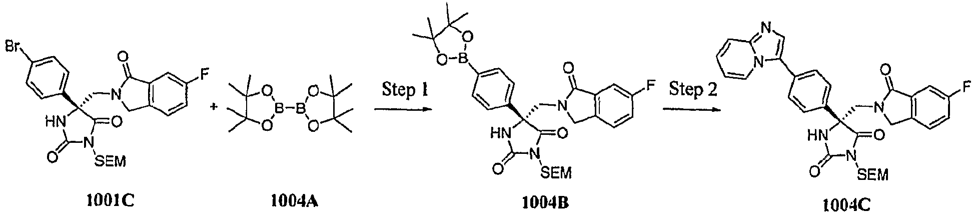

### Step 1

Compound **1001C** was prepared as described in Example 1001.

A mixture of compound **1001C** (0.3 g, 0.55 mmol), bis(pinacolato)diboron (**1004A**; 170 mg, 0.65 mmol), potassium acetate (170 mg, 1.70 mmol), and [PdCl₂(dppf)]CH₂Cl₂ (50 mg, 0.05 mmol) in 1,4-dioxane (10 mL) was vacuumed and refilled with argon three times. The reaction mixture was stirred at 100 °C (oil bath) for 1.5 hours. After cooling down, the mixture was diluted in EtOAc (50 mL) and filtered through a Celite pad. The filtrate was concentrated in vacuo and the residual material was purified by silica gel column chromatography (2% MeOH in CH₂Cl₂) to afford compound **1004B** (300 mg, 91% yield).

### Step 2

A solution of compound **1004B** (60 mg, 0.10 mmol), 3-bromoimidazo[1,2-a]pyridine (30 mg, 0.15 mmol), and [PdCl₂(dppf)]CH₂Cl₂ (8.2 mg, 0.01 mmol) in CH₃CN (3 mL) was treated with potassium carbonate (0.6 mL, 0.6 mmol, 1M in H₂O). The mixture was vacuumed and refilled with argon three times. The reaction mixture was stirred at 90 °C (oil bath) for 17 hours. After cooling, the mixture was diluted in EtOAc (20 mL) and filtered through a Celite pad. The filtrate was concentrated in vacuo and the residual material was purified by preparative TLC (10% MeOH in CH₂Cl₂) to afford compound **1004C** (42 mg, 71% yield).

The following compounds were prepared as described in **Examples 1001, 1002, 1003, or 1004.**

**Table 1000**

| **Compound #** | **Structure** | **Exact Mass** | **Mass Obsvd** | **Ki (nM)** |
|---|---|---|---|---|
| **110** | 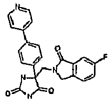 | **416.13** | **417.1 [M+H]⁺** | **A** |
| **111** | 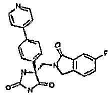 | **416.13** | **417.1 [M+H]⁺** | **A** |
| **112** | 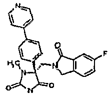 | **430.14** | **431.1 [M+H]⁺** | **B** |
| **113** | 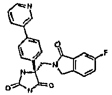 | **416.13** | **417.1 [M+H]⁺** | **A** |
| **114** | 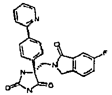 | **416.13** | **417.1 [M+H]⁺** | **A** |
| **115** | 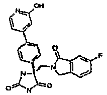 | **432.12** | **433.2 [M+H]⁺** | **A** |
| **116** | 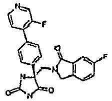 | **434.12** | **435.2 [M+H]⁺** | **A** |
| **117** | 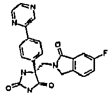 | **417.12** | **418.1 [M+H]⁺** | **A** |
| **118** | 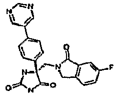 | **417.12** | **418.1 [M+H]⁺** | **A** |
| **119** | 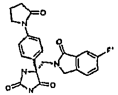 | **422.14** | **423.2 [M+H]⁺** | **A** |
| **120** | 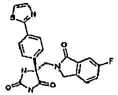 | **422.08** | **423.1 [M+H]⁺** | **A** |
| **121** | 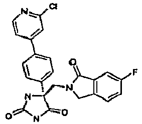 | **450.09** | **451.1 [M+H]⁺** | **D** |
| **122** | 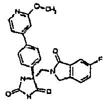 | **446.14** | **447.2 [M+H]⁺** | **A** |
| **123** | 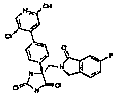 | **466.08** | **467.3 [M+H]⁺** | **A** |
| **124** | 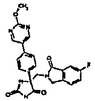 | **447.13** | **448.2 [M+H]⁺** | **A** |
| **125** | 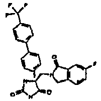 | **483.12** | **484.3 [M+H]⁺** | **D** |
| **126** | 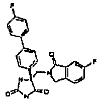 | **433.12** | **434.2 [M+H]⁺** | **A** |
| **127** | 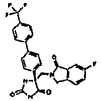 | **483.12** | **484.3 [M+H]⁺** | **B** |
| **128** | 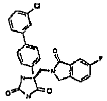 | **449.09** | **450.2 [M+H]⁺** | **A** |
| **129** | 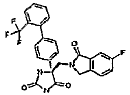 | **483.12** | **484.3 [M+H]⁺** | **C** |
| **130** | 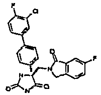 | **467.08** | **468.3 [M+H]⁺** | **C** |
| **131** | 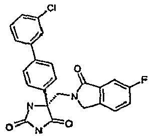 | **449.09** | **450.2 [M+H]⁺** | **A** |
| **132** | 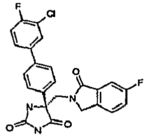 | **467.08** | **468.3 [M+H]⁺** | **A** |
| **133** | 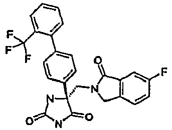 | **483.12** | **484.3 [M+H]⁺** | **A** |
| **134** | 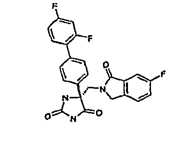 | **451.11** | **452.2 [M+H]⁺** | **A** |
| **135** | 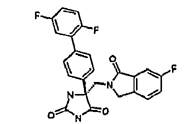 | **451.11** | **452.2 [M+H]⁺** | **A** |
| **136** | 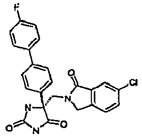 | **449.09** | **450.1 [M+H]⁺** | **n/a** |
| **137** | 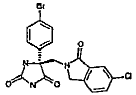 | **432.98** | **434.1 [M+H]⁺** | **C** |
| **138** | 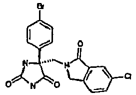 | **432.98** | **434.1 [M+H]⁺** | **A** |
| **139** | 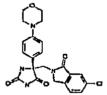 | **440.13** | **441.1 [M+H]⁺** | **A** |
| **140** | 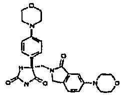 | **491.2** | **492.3 [M+H]⁺** | **C** |
| **141** | 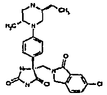 | **481.2** | **482.1 [M+H]⁺** | **A** |
| **141** | 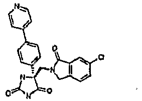 | **432.1** | **433.2 [M+H]⁺** | **D** |
| **143** | 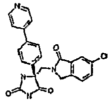 | **432.1** | **433.2 [M+H]⁺** | **A** |
| **144** | 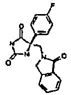 | **339.1** | **340 [M+H]⁺** | **C** |
| **145** | 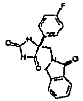 | **339.1** | **340 [M+H]⁺** | **A** |
| **146** | 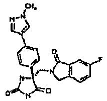 | **419.4** | **420.2 [M+H]⁺** | **A** |
| **147** | 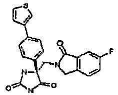 | **421.44** | **422.2 [M+H]⁺** | **A** |
| **148** | 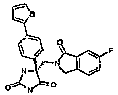 | **421.44** | **422.2 [M+H]⁺** | **A** |
| **149** | 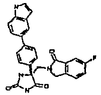 | **454.45** | **455.3 [M+H]⁺** | **A** |
| **150** | 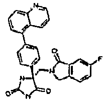 | **466.46** | **467.3 [M+H]⁺** | **A** |
| **151** | 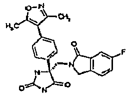 | **434.14** | **435.2[M+H]⁺** | **A** |
| **152** | 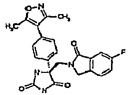 | **434.14** | **435.1[M+H]⁺** | **C** |
| **153** | 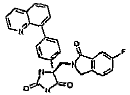 | **466.14** | **467.3[M+H]⁺** | **C** |
| **154** | 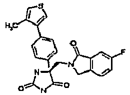 | **435.11** | **436.2[M+H]⁺** | **C** |
| **155** | 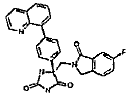 | **466.14** | **467.3[M+H]⁺** | **A** |
| **156** | 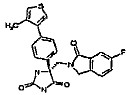 | **435.11** | **436.1 [M+H]⁺** | **A** |
| **157** | 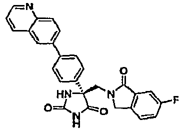 | **466.14** | **467.3[M+H]⁺** | **C** |
| **158** | 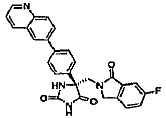 | **466.14** | **467.3[M+H]⁺** | **A** |
| **159** | 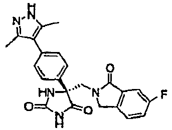 | **433.16** | **434.2[M+H]⁺** | **A** |
| **160** | 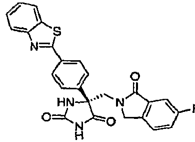 F | **472.10** | **473.3[M+H]⁺** | **A** |
| **161** | 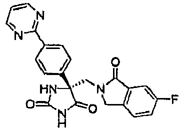 | **417.12** | **418.2[M+H]⁺** | **A** |
| **162** | 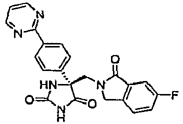 | **417.12** | **440.2[M+Na]⁺** | **C** |
| **163** | 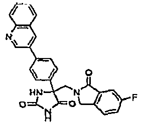 | **466.14** | **467.3[M+H]⁺** | **B** |
| **164** | 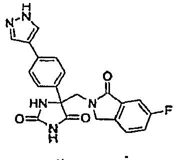 | **405.12** | **406.2[M+H]⁺** | **A** |
| **165** | 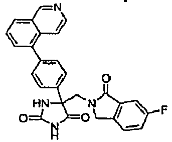 | **466.14** | **467.3[M+H]⁺** | **A** |
| **166** | 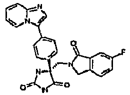 | **455.44** | **456.3 [M+H]⁺** | **n/a** |
| **167** | 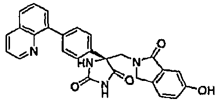 | **464.15** | **465.3 [M+H]⁺** | **A** |
| **168** | 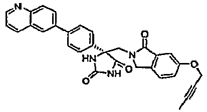 | **516.18** | **517.1 [M+H]⁺** | **C** |
| **169** | 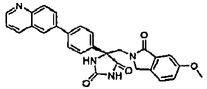 | **478.16** | **479.3 [M+H]⁺** | **A** |
| **170** | 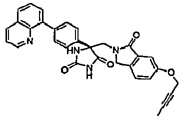 | **516.18** | **517.3 [M+H]⁺** | **n/a** |
| **171** | 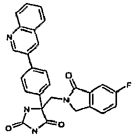 | **466.46** | **467.3 [M+H]⁺** | **B** |
| **172** | 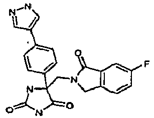 | **405.38** | **406.2 [M+H]⁺** | **A** |
| **173** | 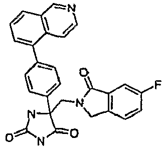 | **466.46** | **467.3 [M+H]⁺** | **A** |
| **174** | 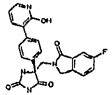 | **432.12** | **433.1 [M+H]⁺** | **A** |
| **175** | 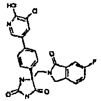 | **466.08** | **467.1 [M+H]⁺** | **A** |
| **176** | 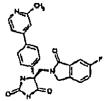 | **430.14** | **431.2 [M+H]⁺** | **D** |

**Proton NMR Spectral Data for Selected Compounds in Table 1000.**

**Compound 111.** ¹H-NMR (500 MHz, DMSO-d₆) δ 9.0 (s, 1H), 8.7 9(d, J = 6.0 Hz, 2H), 7.92 (d, J = 8.7 Hz, 2H), 7.79 (d, J = 8.7 Hz, 2H), 7.76 (d, J = 6.0 Hz, 2H), 7.65 (m, 1H), 7.48 (m, 2H), 4.40 (d, J = 17.3 H, 1H), 4.31 (d, J = 17.3 Hz, 1H), 4.27 (d, J = 14.2 Hz, 1H), 4.14 (d, J = 14.2 Hz, 1H).

**Compound 120.** ¹H-NMR (500 MHz, DMSO-d₆) 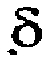 8.99 (s, 1H), 8.03 (d, J = 8.8 Hz, 2H), 7.96 (d, J = 3.3 Hz, 1H), 7.84 (d, J = 3.3 Hz, 1H), 7.77 (d, J = 8.8 Hz, 2H), 7.65 (s, 1H), 7.47 (m, 2H), 4.38 (d, J = 17.6 Hz, 1H), 4.28 (d, J = 17.6 Hz, 1H), 4.27 (d, J = 14.3 Hz, 1H), 4.13 (d, J = 14.3 Hz, 1H).

**Compound 123.** ¹H-NMR (500 MHz, DMSO-d₆) δ 8.99 (s, 1H), 7.84 (s, 1H), 7.74 (d, J = 8.4 Hz, 2H), 7.66 (dd, J = 8.5, 4.6 Hz, 1H), 7.54 (d, J = 8.4 Hz, 2H), 7.49 (m, 2H), 6.65 (s, 1H), 4.40 (d, J = 17.5 Hz, 1H), 4.31 (d, J = 17.5 Hz, 1H), 4.29 (d, J = 14.2 Hz, 1H), 4.10 9d, J = 14.2 Hz, 1H).

**Compound 139.** ¹H NMR (500 MHz, CD₃OD) 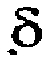 3.17-3.21 (m, 4H), 3.83-3.88 (m, 4H), 4.14-4.52 (m, 4H), 7.01 (d, J = 8.8 Hz, 2H), 7.47 (d, J = 8 Hz, 1H), 7.46-7.48 (m, 3H), 7.75 (s, 1H).

**Compound 143.** ¹H NMR (400 MHz, CDCl₃) δ 4.21-4.50 (m, 4H), 7.498 (d, J = 0.8 Hz, 1H), 7.52 (d, J = 0.4 Hz, 1H), 7.73-7.76 (m, 3H), 7.76-7.87 (m, 4H), 8.60 (d, J = 6 Hz, 2H).

**Compound 155.** ¹H NMR (500 MHz, CD₃OD) 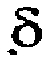 8.84 (dd, J = 1.89, 4.1 Hz, 1H); 8.43 (dd, J = 1.58, 8.2 Hz, 1H); 7.99 (dd, J = 1.58, 8.2 Hz; 1H); 7.85 (m, 3H); 7.8 (dd, J = 1.26 Hz, 6.94 Hz, 1H); 7.75 (m, 3H), 7.70 (dd, J = 7.25 Hz, 0.95 Hz, 1H); 7.59 (dd, J = 4.73 Hz, 7.57 Hz, 1H); 7.58 (dd, J = 4.4Hz, 8.2Hz, 1H); 7.51 (dd, J = 2.5 Hz, 7.8 Hz, 1H); 7.40 (m, 1H); 4.54 (d, J=17.0 Hz, 1H); 4.48 (d, J = 17.0 Hz, 1H); 4.48 (d, J = 14.5 Hz, 1H); 4.32 (1H, d, J = 14.5 Hz, 1H).

### Example 1005:

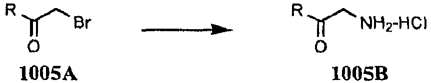

### General procedure for example 1005

Compound **1005A** was treated with one equivalent of hexamethylene tetraamine in chloroform or other suitable solvent for about 5 hours. The product was collected by filtration and then treated with HCl in ethanol for one day to three days. The solid was then collected by filtration to give compound **1005B.**

### Example 1006

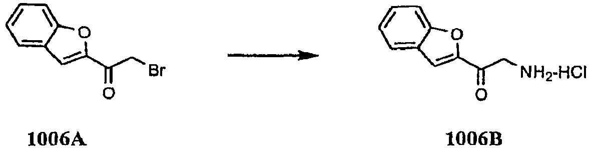

1-Benzofuran-2-yl-2-bromo-ethanone (**1006A,** 3.0g, 12.55 mmol), hexamethylene tetraamine (1.94 g, 13.80 mmol), and Nal (350 mg) were stirred in CHCl₃ (40 mL) for five hours. The solid was collected by filtration and dried under vacuum. The solid was then suspended in ethanol (30 mL) and HCl (conc, 36% in water, 10 mL) was added. The solution was stirred at 25 °C for 4 d. The solid was collected by filtration and washed by ethanol, dried under vacuum to give compound **1006B** (3.05 g, contained NH₄Cl).

### Example 1007

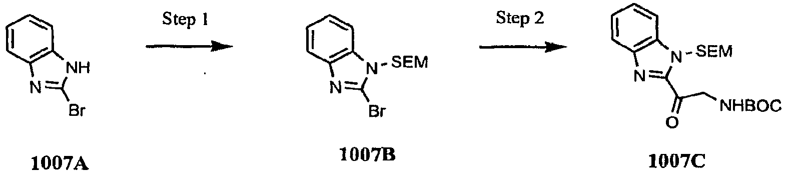

### Step 1

To a flame dried flask was added 2-bromo-1H-benzimidazole (**1007A**, 2.94 g, 14.92 mmol), anhydrous THF (75 mL), and NaH (95%, 490 mg, 19.4 mmol). The solution was stirred at 25 °C for 45 minutes; SEMCI (3.17 mL, 17.9 mmol) was added. The solution was stirred at 25 °C for 2.5 hours. Water (50 mL) and EtOAc (100 mL) were added. The aqueous layer was separated and extracted with EtOAc (100 mL) once. The organic layers were combined and concentrated under vacuum. The product was purified by SGC (Hexane/EtOAc: 3:1) to give compound **1007B** (3.6 g, 74%).

### Step 2

To a flame dried flask was added compound **1007B** (1.427 g, 4.35 mmol) and anhydrous ethyl ether/THF (2:1, 15 mL). The solution was cooled to -78°C. n-Butyllithium (1.6 M, 0.46 mL, 0.73 mmol) was added and stirred at -78 °C for 30 minutes. In another flamed dried pear shaped flask was added N-(tert-butoxycarbonyl)glycine-N'-methoxy-N'-methylamide (949 mg, 4.35 mmol) and anhydrous THF (2 mL). Isopropyl magnesium chloride (2 M, 2.5 mL, 5.0 mmol) was added at 0 °C. The solution was stirred at 0 °C for 5 minutes and was added into the compound **1003C** solution via cannula at -78 °C. The solution was then gradually warmed up to -20 °C and stirred between -20 °C and 10 °C for 4 hours. Saturated NH₄Cl solution was added and the aqueous solution was extracted with EtOAc (50 mL) three times. The organic phases were combined and concentrated. The product was purified by SGC (Hexane/EtOAc: 3:1) to give compound **1007C** (1.0 g, 57%).

The following compounds were prepared as described in **Example 1, 14. 1005, 1006, and/or 1007.**

**Table 1001**

| **Compound #** | **Structure** | **Exact Mass** | **Mass Obsvd** | **Ki (nM)** |
|---|---|---|---|---|
| **177** | 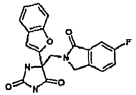 | **379.10** | **380.1 [M+H]⁺** | **A** |
| **178** | 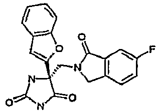 | **379.10** | **380.1 [M+H]⁺** | **A** |
| **179** | 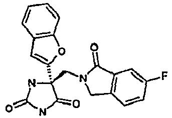 | **379.10** | **380.1 [M+H]⁺** | **D** |
| **180** | 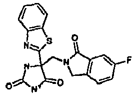 | **396.07** | **397.1 [M+H]⁺** | **B** |
| **181** | 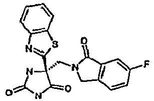 | **396.07** | **397.1 [M+H]⁺** | **A** |
| **182** | 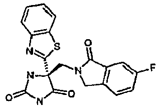 | **396.07** | **397.1 [M+H]⁺** | **B** |
| **183** | 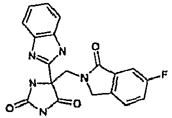 | **379.11** | **380.1 [M+H]⁺** | **A** |

**Proton NMR Spectral Data for Selected Compounds in Table 1003.**

**Compound 181.** ¹H-NMR (500 MHz, DMSO-d₆) δ 11.3 (s, 1H), 9.34 (s, 1H), 8.18 (d, J = 8.5 Hz, 1H), 8.12 (d, J = 7.6 Hz, 1H), 7.67 (m, 1H), 7.61 (m, 1H), 7.50 (m, 3H), 4.65 (d, J = 14.3 Hz, 1H), 4.44 (d, J = 17.3 Hz, 1H), 4.38 (d, J = 17.3 Hz, 1H), 4.34 (d, J = 14.3 Hz, 1H).

### Example 1008

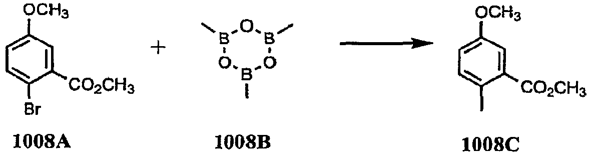

Compound **1008A** (20g, 81.61 mmol), **1008B** (13.36 mL, 97.93 mmol), Pd(dppf)Cl₂ (1.0g, 1.36 mmol), dioxane (350 mL), water (50 mL), and Cs₂CO₃ (22.5g, 163 mmol) were stirred at 110 °C (oil bath) under nitrogen for 16 hours. After cooling, the solid was removed by filtration. The solution was concentrated and purified by SGC (Hexane/EtOAc, 10:1) to give **1008C** (12.1 g, 80%).

The following compounds were prepared as described in **Examples 14 and 1008 and 1009.**

**Table 1002**

| **Compound** # | **Structure** | **Exact Mass** | **Mass Obsvd** | **Ki (nM)** |
|---|---|---|---|---|
| **184** | 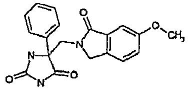 | **351.12** | **352.1 [M+H]⁺** | **A** |
| **185** | 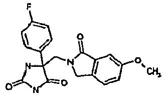 | **369.11** | **370.1 [M+H]⁺** | **A** |
| **186** | 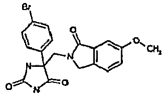 | **429.03** | **430.2 [M+H]⁺ 432.2 [M+Na]⁺** | **A** |

### Example 1009

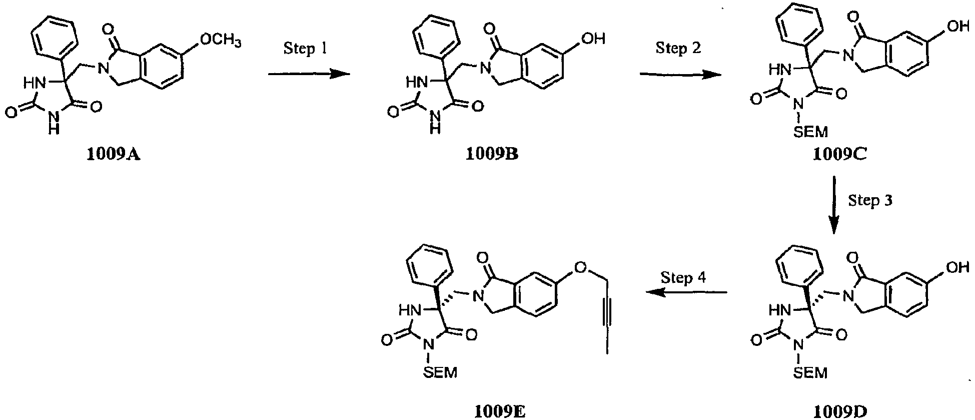

### Step1

Compound **1009A** (1.18g, 3.36mmol) and pyridine hydrochloride (2.33g, 20.17 mmol) were added into a 20 mL microwave reactor tube and reacted at 200 °C for 1 hour. After cooling down, the solid was dissolved in DMF and purified by C18 chromatography (CH₃CN/water 5% to 90%, with 0.1% HCO₂H) to give compound **1009B** (0.87 g, 77%).

### Step 2

Compound **1009B** (0.75 g, 2.22 mmol) was dissolved in DMF (12 mL). SEMCI (0.48 mL, 2.44 mmol) and DIPEA (0.775 mL, 4.44 mmol) were added and the solution was stirred at 25 °C for 4 hours. DMF was removed under vacuum and the product was purified by SGC (Hexane/EtOAc: 3:1 to 1:1) to give compound **1009C** (0.81 g, 78%).

### Step 3

Compound **1009C** was resolved on Chiralcel OD column by using Hexane and 2-propanol as mobile phase. The first peak was collected and concentrated to give compound **1009D**.

### Step 4

Compound **1009D** (100mg, 0.214 mmol), 1-bromo-2-butyne (34 mg, 0.257 mmol), and Cs₂CO₃ (140 mg, 0.428 mmol) were stirred in DMF (2 mL) at 0 °C for 2 hours, then at 25 °C for overnight. Water (5 mL) was added and the aqueous solution was extracted with EtOAc (10 mL) three times. The organic phases were combined and concentrated. The product was purified by SGC (Hexane/EtOAc: 3:1) to give compound **1009E** (81 mg).

### Example 1010

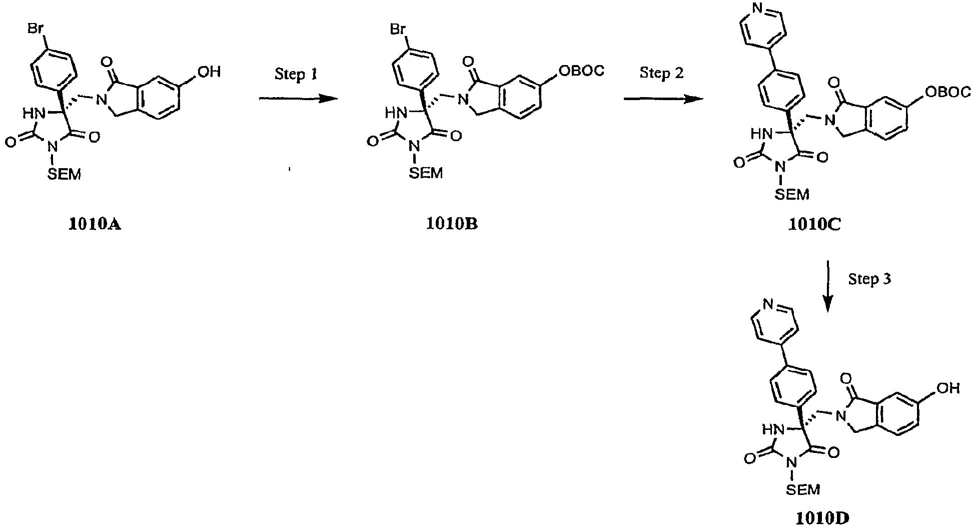

### Step 1

Compound **1010A** (1.03g, 1.88 mmol), (BOC)₂O (493 mg, 2.26 mmol), and Cs₂CO₃ (741 mg, 2.26 mmol) were stirred overnight in CHCl₃ (20 mL). Water was added. The aqueous layer was extracted with EtOAc (3×50 mL). The combined organic layers were concentrated and purified by SGC (Hexane/EtOAc 5% to 90%) to give compound **1010B** (1.01 g, 83%).

### Step 2

To a dry flask was added compound **1010B** (500 mg, 0.77 mmol) and 4-pyridyl boronic acid (190 mg, 1.55 mmol). The flask was vacuumed and refilled with nitrogen three times. Pd(dppf)Cl₂ (28 mg, 0.04 mmol) was added and followed by addition of CH₃CN (5 mL) and K₂CO₃ (1 M, 4 mL). The solution was stirred at 80 °C (oil bath) for 16 hours. After cooling down, CH₃CN (100 mL) was added and the solid was removed by filtration. The aqueous layer was separated and extracted once with EtOAc (20 mL). The organic solution was combined and concentrated. The product was purified by SGC (CH₂Cl₂/MeOH/NH₄OH: 20:1:0.1) to give compound **1010C.**

### Step 3

Compound **1010C** obtained in step 2 was dissolved in MeOH (10 mL) and HCl (4M in dioxane, 3 mL) was added and stirred overnight at 25 °C. MeOH was then removed and the product was dried under vacuum to give compound **1010D** (315 mg, 75% from compound **1010B**).

The following compounds were prepared as described in **Examples 14 and 1009 or 1010.**

**Table 1003**

| **Compound #** | **Structure** | **Exact Mass** | **Mass Obsvd** | **Ki (nM)** |
|---|---|---|---|---|
| **187** | 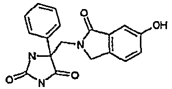 | **337.11** | **338.1 [M+H]⁺** | **B** |
| **188** | 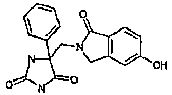 | **337.11** | **338.1 [M+H]⁺ 360.1 [M+Na]⁺** | **A** |
| **189** | 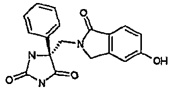 | **337.11** | **338.2 [M+H]⁺ 360.2 [M+Na]⁺** | **A** |
| **190** | 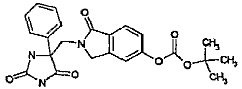 | **437.16** | **438.2 [M+H]⁺** | **A** |
| **191** | 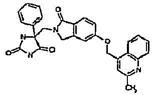 | **492.18** | **493.3 [M+H]⁺** | **A** |
| **192** | 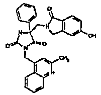 | **492.18** | **493.3 [M+H]⁺** | **C** |
| **193** | 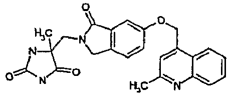 | **430.16** | **431.1 [M+H]⁺** | **A** |
| **194** | 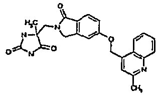 | **430.16** | **431.1 [M+H]⁺** | **A** |
| **195** | 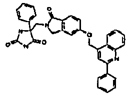 | **554.20** | **555.1 [M+H]⁺** | **B** |
| **196** | 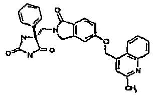 | **492.16** | **493.1 [M+H]⁺** | **A** |
| **197** | 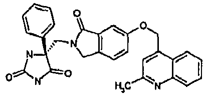 | **492.16** | **493.1 [M+H]⁺** | **A** |
| **198** | 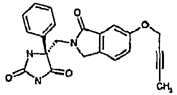 | **389.14** | **390.1 [M+H]⁺** | **A** |
| **199** | 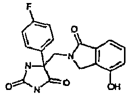 | **355.10** | **356.1 [M+H]⁺** | **C** |
| **200** | 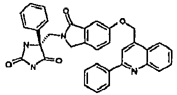 | **554.20** | **555.1 [M+H]⁺** | **B** |
| **201** | 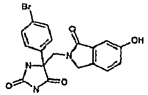 | **415.02** | **416.2 [M+H]⁺** | **B** |
| **202** | 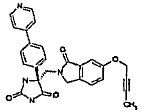 | **466.16** | **467.1 [M+H]⁺** | **A** |
| **203** | 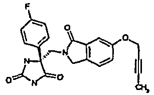 | **407.13** | **408.2 [M+H]⁺** | **A** |
| **204** | 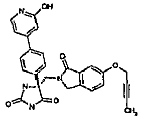 | **482.16** | **483.3 [M+H]⁺** | **A** |
| **205** | 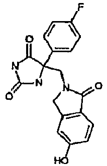 | **355.1** | **356 [M+H]⁺** | **A** |
| **206** | 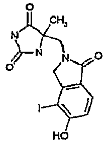 | **400.99** | **402 [M+H]⁺** | **C** |
| **207** | 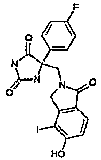 | **480.99** | **482 [M+H]⁺** | **D** |
| **208** | 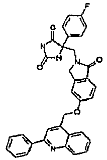 | **572.19** | **573 [M+H]⁺** | **B** |
| **209** | 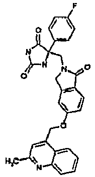 | **510.17** | **511 [M+H]⁺** | **A** |
| **210** | 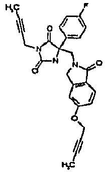 | **459.16** | **460 [M+H]⁺** | **D** |
| **211** | 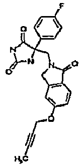 | **407.13** | **408 [M+H]⁺** | **A** |
| **212** | 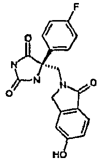 | **355.1** | **356 [M+H]⁺** | **C** |
| **213** | 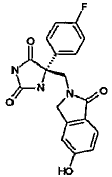 | **355.1** | **356 [M+H]⁺** | **A** |
| **214** | 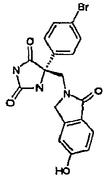 | **415.02** | **416.418 [M+H]⁺** | **A** |
| **215** | 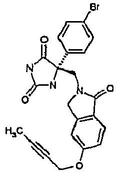 | **467.05** | **468.470 [M+H]⁺** | **A** |
| **216** | 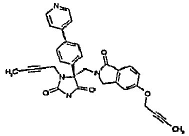 | **518.2** | **519 [M⁺H]⁺** | **C** |
| **217** | 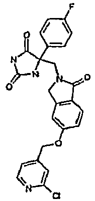 | **480.1** | **481 [M+H]⁺** | **A** |
| **218** | 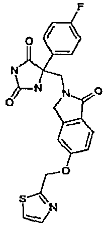 | **452.1** | **453 [M+H]⁺** | **B** |
| **219** | 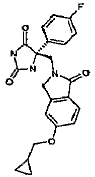 | **409.14** | **410 [M+H]⁺** | **A** |
| **220** | 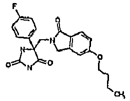 | **411.16** | **412 [M+H]⁺** | **A** |
| **221** | 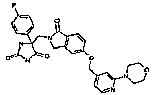 | **531.19** | **532 [M+H]⁺** | **B** |
| **222** | 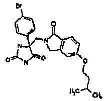 | **485.1** | **486.488 [M+H]⁺** | **A** |
| **223** | 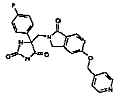 | **446.14** | **447 [M+H]⁺** | **A** |
| **224** | 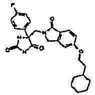 | **465.21** | **466 [M+H]⁺** | **C** |
| **225** | 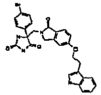 | **558.09** | **559.561 [M+H]⁺** | **A** |
| **226** | 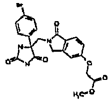 | **487.04** | **488.490 [M+H]⁺** | **A** |
| **227** | 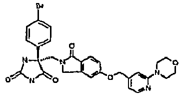 | **591.11** | **592.1 [M+H]⁺** | **B** |
| **228** | 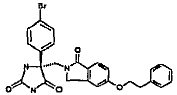 | **519.1** | **520.1 [M+H]⁺** | **C** |
| **229** | 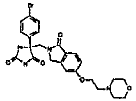 | **528.1** | **529.4 [M+H]⁺** | **B** |
| **230** | 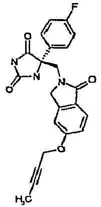 | **407.13** | **408 [M+H]⁺** | **A** |
| **231** | 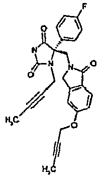 | **459.16** | **460 [M+H]⁺** | **C** |
| **232** | 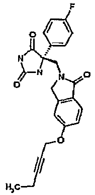 | **421.14** | **422 [M+H]⁺** | **A** |
| **233** | 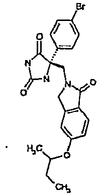 | **471.08** | **472.474 [M+H]⁺** | **C** |
| **234** | 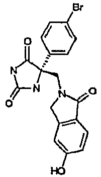 | **415.02** | **416.418 [M+H]⁺** | **A** |
| **235** | 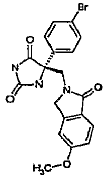 | **429.03** | **430.432 [M+H]⁺** | **A** |
| **236** | 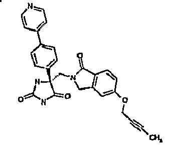 | **466.16** | **467 [M+H]⁺** | **A** |
| **237** | 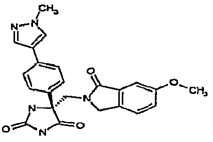 | **431.44** | **432.2 [M+H]⁺** | **A** |
| **238** | 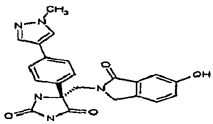 | **417.41** | **418.2 [M+H]⁺** | **n/a** |
| **239** | 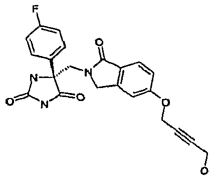 | **423.12** | **424.2 [M+H]⁺** | **A** |
| **240** | 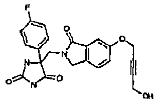 | **423.12** | **424.2 [M+H]⁺** | **A** |
| **241** | 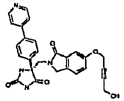 | **482.16** | **483.3 [M+H]⁺** | **A** |
| **242** | 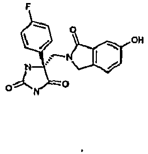 | **355.10** | **356.2 [M+H]⁺** | **A** |
| **243** | 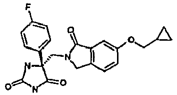 | **409.14** | **410.2 [M+H]⁺** | **A** |
| **244** | 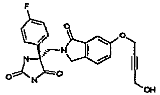 | **423.12** | **424.1 [M+H]⁺** | **n/a** |
| **245** | 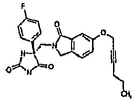 | **435.16** | **436.2 [M+H]⁺** | **n/a** |
| **246** | 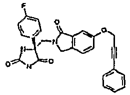 | **469.14** | **470.3 [M+H]⁺** | **n/a** |

**Proton NMR Spectral Data for Selected Compounds in Table 1003.**

**Compound 198.** ¹H-NMR (400 MHz, DMSO-d₆) δ 9.22 (s, 1H), 7.64 (m,2H). 7.43 (m, 4H), 7.22 (t, J = 2.2 Hz, 1H), 7.16 (dd, J = 9.6, 1.2 Hz, 1H). 4.82 (d, J = 2.0 Hz, 2H), 4.16 (m, 4H), 3.33 (s, 3H).

**Compound 203.** ¹H-NMR (400 MHz, DMSO-d₆) δ 7.63 (dd, J = 8.8, 5.6 Hz, 2H), 7.43 (d, J = 8.4 Hz, 1H), 7.13 (m, 4H), 4.80 (d, J = 0.8 Hz, 1H), 4.39 (d, J = 17.6 Hz, 1H), 4.17 (d, J = 17.6 Hz, 1H), 4.13 (d, J = 13.6 Hz, 1H), 3.71 (d, J = 13.6 Hz, 1H) 3.34 (s, 3H).

**Compound 213.** ¹H NMR (500 Hz, CD₃OD) δ 4.11 (d, J = 15Hz, 1H), 4.27 (d, J = 15Hz, 1H), 4.29 (d, J = 17Hz, 1H), 4.38 (d, J = 17Hz, 1H), 6.84-6.89 (m,2H), 7.16-7.21 (m,2H), 7.56-7.60 (m, 1H), 7.71-7.76 (m,2H)

**Compound 219.** ¹H NMR (500 Hz, CD₃OD) δ 0.36-0.40 (m,2H), 0.61-0.68 (m, 2H), 1.25-1.35 (m,1H), 3.91 (d, J = 7Hz, 2H), 4.14 (d, J = 15Hz,1H), 4.30 (d, J = 15Hz,1H), 4.34 (d, J = 17Hz,1H), 4.43 (d, J = 17Hz,1H), 7.01-7.05 (m,2H), 7.17-7.23 (m,2H), 7.65-7.69 (m,1H), 7.72-7.77 (m,2H)

**Compound 232.** ¹H NMR (500 Hz, CD₃OD) δ 1.13 (t, J = 8Hz, 3H), 2.21-2.27 (m, 2H), 4.15 (d, J = 14Hz, 1H), 4.31 (d, J = 14Hz, 1H), 4.36 (d, J = 17Hz, 1H), 4.45 (d, J = 17Hz,1H), 4.79 (t, J = 2Hz, 2H), 7.04-7.14 (m, 2H), 7.16-7.25 (m,2H), 7.64-7.79 (m, 3H).

**Compound 233.** ¹H NMR (500 Hz, CD₃OD) 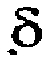 7.678 (d, J = 8.5Hz, 1H); 7.455 (d, J = 4.1Hz, 1H), 7.817 (d, J = 4.1Hz, 1H); 7.099 (s, 1H); 7.052 (dd, J = 2.207, 6.305Hz, 1H); 4.515 (d, J = 17.3 Hz, 1H), 4.450 (d, J = 17.3 Hz, 1H); 4.065 (d, J = 14.5 Hz, 1H); 3.89 (s, 3H); 3.87(d, J = 14.5 Hz, 1H); 3.85 (m, 1H); 2.46 (m. 2H); 2.09 (m, 1H)1.89 (m, 1H); 1.76 (m, 1H); 1.67(m, 1H); 1.54 (m, 1H); 1.32 (m, 1H).

**Compound 239.** ¹H NMR (500 Hz, DMSO-d₆) 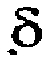 4.11 (d, J = 15 Hz, 1H), 4.27 (d, J = 15 Hz, 1H), 4.29 (d, J = 17 Hz, 1H), 4.38 (d, J = 17 Hz, 1H), 6.84-6.89 (m,2H), 7.16-7.21 (m,2H), 7.56-7.60 (m, 1H), 7.71-7.76 (m,2H)

**Compound 243.** ¹H-NMR (500 MHz, CD₃OD) δ 8.53 (s, 1H), 7.67 (dd, J = 8.5, 5 Hz, 2H), 7.46 (d, J = 8 Hz, 1H), 7.27 (t, J = 8.5 Hz, 2H), 7.15 (m, 2H), 4.31 9d, J = 17.0 Hz, 1H), 4.22 (d, J = 17 Hz, 1H), 4.13 (d, J = 14.2 Hz, 1H), 4.06 (d, J = 14.2 Hz, 1H), 3.88 9d, J = 6.5 Hz, 2H), 3.35 9m, 2H), 1.22 (m, 1H), 0.57 (d, J = 8 Hz, 1H), 0.33 (d, J = 5 Hz, 1H).

### Example 1011

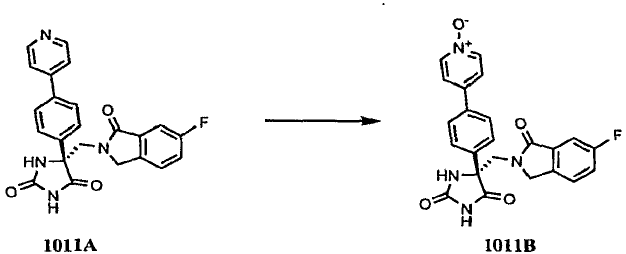

To a solution of compound **1011A** (100mg) in DMF (5 mL) was added m-chlorobenzoyl peroxide (MCPBA, 100mg). The solution was stirred overnight at 25 °C. The product was purified by C18 reverse phase chromatography (CH₃CN/water 5% to 90%, with 0.1% HCO₂H) to give compound **1011B** (73 mg).

The following compounds were prepared as described in **Examples 1010 and 1011.**

**Table 1004**

| **Compound #** | **Structure** | **Exact Mass** | **Mass Obsvd** | **Ki (nM)** |
|---|---|---|---|---|
| **247** | 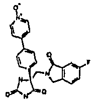 | **432.12** | **433.1 [M+H]⁺** | **A** |

### Example 1012.

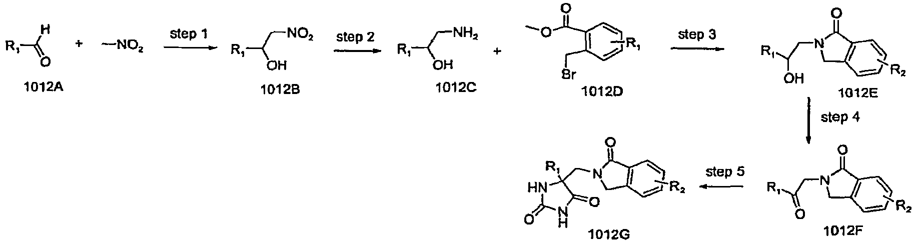

In step 1, Compound **1012A** was treated with nitromethane and KO*^{t}*Bu in a mixture of THF and *t*-BuOH for 2 to 12 h. Alternatively, compound **1012A** was treated with nitromethane and TBAF in a suitable solvent such as THF for 2 to 12 h. Compound **1012B** was purified by silica gel chromatography.

In step 2, Compound **1012B** was treated with Pd/C in a suitable solvent such as methanol, in a Parr shaker under H₂ atmosphere. After filtering off the catalyst and concentration of solvent, the product was used without further purification.

In step 3, the benzyl bromide (compound **1012D**) was mixed with compound **1012C,** DIPEA, and DMF. The solution was stirred at 0 °C to room temperature for 12 to 24 hours. The product was either removed by filtration or purified by silica gel chromatography.

In step 4, compound **1012E** was treated with PCC and Celite in a suitable solvent such as dichloromethane for 2 to 12 h. Compound **1012F** was purified by silica gel chromatography.

In step 5, compound **1012F** was reacted with potassium cyanide and ammonium carbonate in an appropriate alcohol and water solution, at 50 °C to 90 °C, for 5 to 48 hours. After cooling, water was added and compound **1012G** was collected by filtration.

### Example 1013.

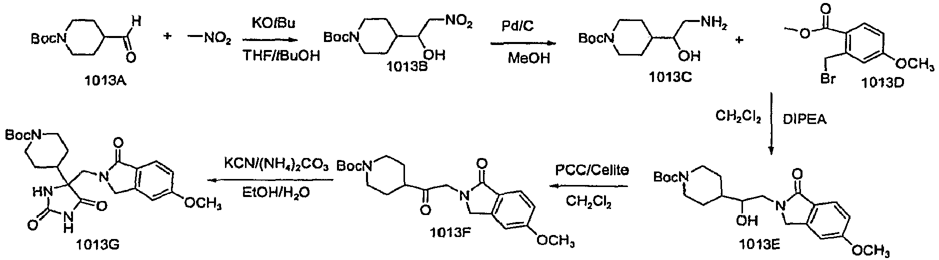

Compound **1013B**: To a solution of THF (15 mL) and t-BuOH (15 mL) was added compound **1013A** (1.2 g, 5.6 mmol) and nitromethane (0.61 mL, 11.2 mmol) followed by addition of KO*^{t}*Bu (0.63 g, 5.6 mmol). The reaction mixture was stirred at room temperature for 2 h. The reaction mixture was adjusted to pH 6 using HOAc. The reaction mixture was diluted with EtOAc (30 mL), and was extracted with brine. The aqueous layer was extracted with EtOAc (30 mL × 2) The combined organic layers were washed with brine, dried, and concentrated to dryness. The crude material was purified via PTLC (25% EtOAc/Hexanes) to give 1.24 g (81%) of compound **1013B**.

Compound **1013C**: Compound **1013B** (1.24 g, 4.5 mmol) was treated with Pd/C in methanol in a Parr shaker under H₂ atmosphere (50 psi) overnight. After filtering off the catalyst and concentration of solvent, compound **1013C** (1.1 g, 99%) was used without further purification.

Compound **1013E**: Compound **1013C** (1.02 g, 4.2 mmol) was dissolved in dichloromethane (30 mL) at 0 °C. Compound **1013D** (1.13 g, 4.2 mmol) and DIPEA (0.73 mL, 4.2 mmol) were added and the reaction was stirred at 0 °C and slowly warmed up to rt overnight. The reaction mixture was washed with HCl (1N, 50 mL) and brine (50 mL). The organic layer was dried and concentrated to dryness. The crude material was purified via PTLC (50% EtOAc/Hexanes) to give 0.88 g (54%) of compound **1013E.**

Compound **1013F**: Compound **1013E** (0.88 g, 2.25 mmol) was dissolved in methylene chloride (30 mL). PCC (1.22 g, 5.63 mmol) and Celite (1.22 g) were added and the reaction mixture was stirred at 25 °C overnight. The solid was filtered off and the resulting solution was concentrated and purified via sgc (90% EtOAc/Hexanes) to give 0.62 g (71%) of compound **1013F.**

Compound **1013G**: Compound **1013F** (1.01 g, 2.6 mmol), KCN (0.25 g, 3.9 mmol), and (NH₄)₂CO₃ (0.75 g, 7.8 mmol) were suspended in a mixture of NH₃ in Methanol (7 N, 10 mL) and water (10 mL). The solution was stirred at 90 °C overnight. After cooling, water (20 mL) was added. The solid was filtered and washed with water three times. The solid was dried under vacuum to give compound **1013G** (0.86 g, 72%).

### Example 1014.

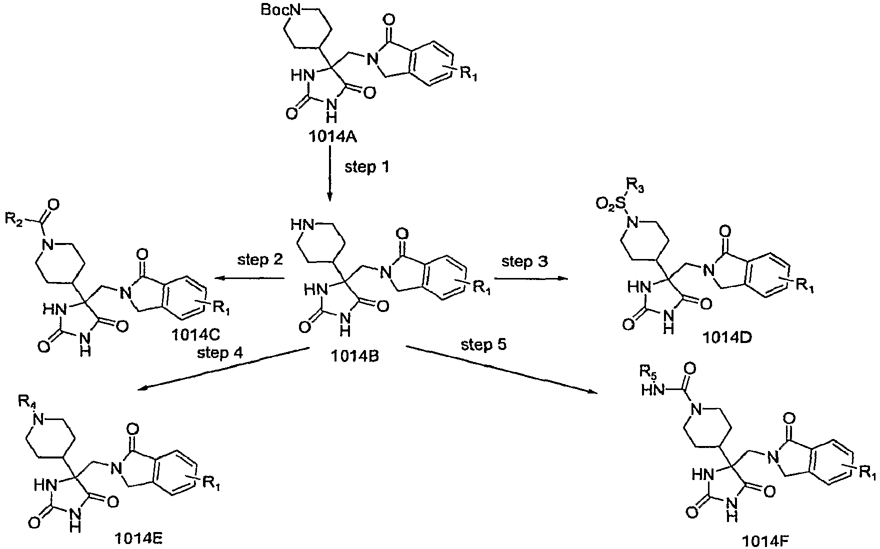

### Step 1.

Compound **1014A** was stirred with 2 to 20 equivalents of hydrogen chloride in methanol for 5 to 48 hours. The solvent was removed and the compound **1014B** could be used without further purification.

### Step 2.

Compound **1014B** was treated with carboxylic anhydride and DIPEA to give compound **1014C** which was purified by silica gel chromatography.

### Step 3.

Compound **1014B** was coupled with sulphonyl chloride compound to give compound **1014D**, which was purified by silica gel chromatography.

### Step 4.

Compound **1014B** was reacted with carbonyl compound under reductive amination conditions to give compound **1014E.** Alternatively, compound **1014B** was treated with a suitable electrophile and a base to give compound **1014E**, which was purified by silica gel chromatography.

### Step 5.

Compound **1014B** was reacted with isocyanate compound and DIPEA to give compound **1014F**, which was purified by silica gel chromatography.

### Example 1015.

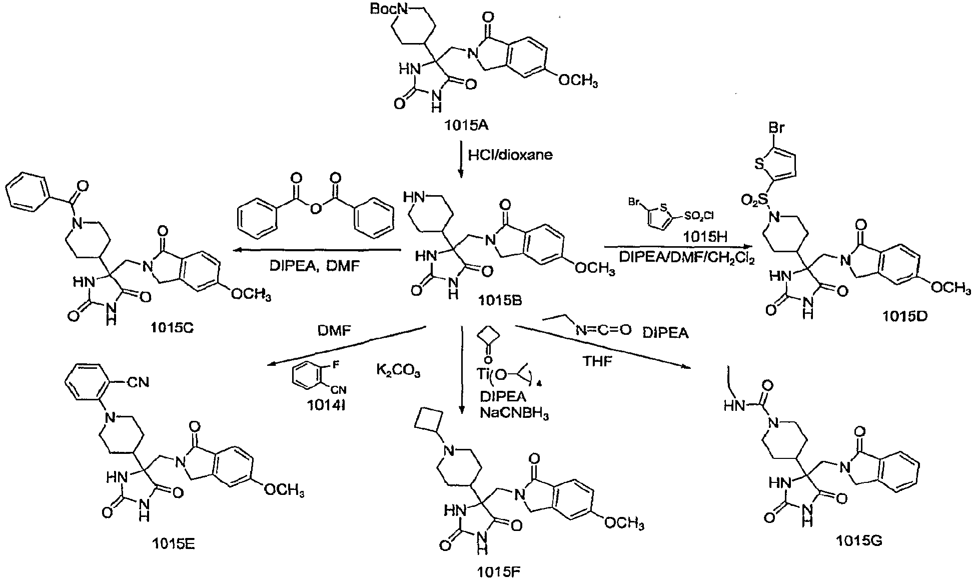

Compound **1015B**: Compound **1015A**(0.86 g) was suspended in methanol (10 mL) and HCl (4M in dioxane, 10 mL) was added. The solution was stirred at 25 °C for 3 hours. Solvent was removed and the material was dried under vacuum to give compound **1015B** (0.74 g, 99%).

Compound **1015C**: Compound **1015B** (40 mg, 0.11 mmol) and benzoic acid anhydride (25 mg, 0.11 mmol) were dissolved in DMF (1 mL). DIPEA (0.06 mL, 0.33 mmol) was added and the reaction mixture was stirred at room temperature overnight. Solvent was removed and the crude material was purified via sgc (5% NH₃•MeOH/CH₂Cl₂) to give 3.7 mg (7%) of compound **1015C.**

Compound **1015D:** Compound **1015B** (40 mg, 0.11 mmol) and compound **1015H** (30 mg, 0.11 mmol) were dissolved in DMF (1 mL). DIPEA (0.25 mL, 1.4 mmol) was added and the reaction mixture was stirred at room temperature overnight. Solvent was removed and the crude material was purified via sgc (5% NH₃•MeOH/CH₂Cl₂) to give 2.2 mg (3%) of compound **1015D.**

Compound **1015E**: Compound **1015B** (40 mg, 0.11 mmol) and compound **1015I** (0.024 mL, 0.22 mmol) were dissolved in DMF (1 mL). K₂CO₃ (46 mg, 0.33 mmol) was added and the reaction mixture was stirred at 90 °C overnight. Solvent was removed and the crude material was purified via sgc (5% NH₃•MeOH/CH₂Cl₂) to give 2.6 mg (5%) of compound **1015E**.

Compound **1015F**: Compound **1015B** (46 mg, 0.13 mmol) and cyclobutanone (0.2 mL) were stirred in methylene chloride (1 mL). Titanium tetraisopropoxide (0.045 mL, 0.15 mmol) was added followed by addition of DIPEA (0.027 mL, 0.16 mmol). The reaction mixture was stirred at room temperature for 2 h. Then, NaCNBH₃ (41 mg, 0.65 mmol) was added and the mixture was stirred at rt overnight. The solvent was removed. The crude material was purified via PTLC (10% NH₃•MeOH/CH₂Cl₂) to give 3.1 mg (6%) of compound **1015F.**

Compound **1015G**: Compound **1015B** (80 mg, 0.24 mmol) and ethyl isocyanate (0.018 mL, 0.24 mmol) were dissolved in DMF (1 mL). DIPEA (0.17 mL, 0.97 mmol) was added and the reaction mixture was stirred at room temperature overnight. Solvent was removed and the crude material was purified via sgc (9% NH₃•MeOH/CH₂Cl₂) to give 11 mg (11%) of compound **1015G.**

The following compounds were prepared as described in **Examples 1012 to 1015.**

**Table 1005**

| **Compound #** | **Structure** | **Exact Mass** | **Mass Obsvd** | **Ki (nM)** |
|---|---|---|---|---|
| **248** | 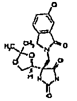 | **379.09** | **380.1 [M+H]⁺** | **C** |
| **249** | 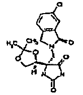 | **379.09** | **380.1 [M+H]⁺** | **C** |
| **250** | 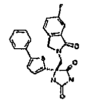 | **421.09** | **422.1 [M+H]⁺** | **B** |
| **251** | 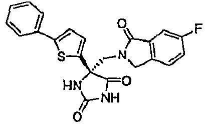 | **421.09** | **422.1 [M+H]⁺** | **A** |
| **252** | 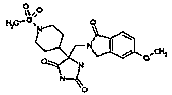 | **436.14** | **437.1 [M+H]⁺** | **B** |
| **253** | 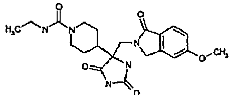 | **429.2** | **430.1 [M+H]⁺** | **B** |
| **254** | 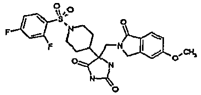 | **534.14** | **535.1 [M+H]⁺** | **B** |
| **255** | 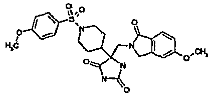 | **528.17** | **529.3[M+H]⁺** | **B** |
| **256** | 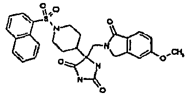 | **548.17** | **549.3[M+H]⁺** | **A** |
| **257** | 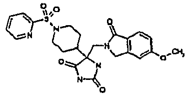 | **499.15** | **500.3[M+H]⁺** | **B** |
| **258** | 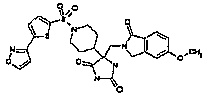 | **571.12** | **572.1[M+H]⁺** | **A** |
| **259** | 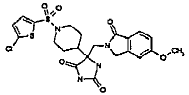 | **538.07** | **539.1[M+H]⁺** | **A** |
| **260** | 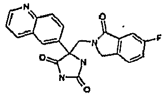 | **390.11** | **391.1[M+H]⁺** | **A** |
| **261** | 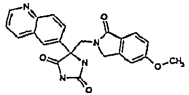 | **402.13** | **403.2[M+H]⁺** | **A** |
| **262** | 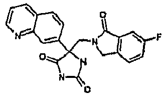 | **390.11** | **391.1[M+H]⁺** | **A** |
| **263** | 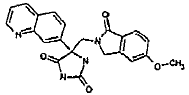 | **402.13** | **403.1 [M+H]⁺** | **A** |
| **264** | 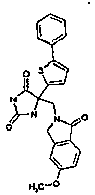 | **433.11** | **434.1 [M+H]⁺** | **A** |
| **265** | 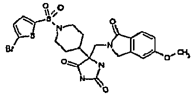 | **582.02** | **585.1 [M+H]⁺** | **A** |
| **266** | 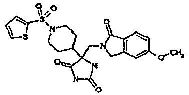 | **504.11** | **505.1 [M+H]⁺** | **A** |
| **267** | 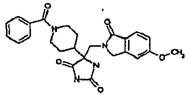 | **462.19** | **463.1 [M+H]⁺** | **B** |
| **268** | 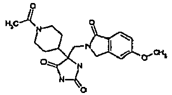 | **400.17** | **401.1[M+H]⁺** | **A** |
| **269** | 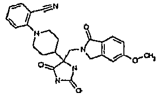 | **459.19** | **460.1 [M+H]⁺** | **B** |
| **270** | 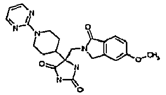 | **436.19** | **437.2[M+H]⁺** | **B** |
| **271** | 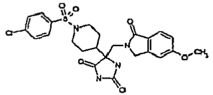 | **532.12** | **533.3[M+H]⁺** | **B** |
| **272** | 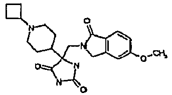 | **412.21** | **413.2[M+H]⁺** | **C** |
| **273** | 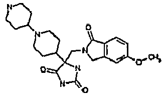 | **441.24** | **442.1 [M+H]⁺** | **C** |
| **274** | 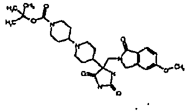 | **541.29** | **542.3[M+H]⁺** | **C** |
| **275** | 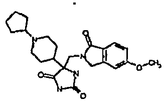 | **426.23** | **427.2[M+H]⁺** | **C** |
| **276** | 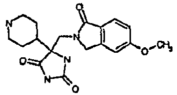 | **358.16** | **359.1 [M+H]⁺** | **C** |
| **277** | 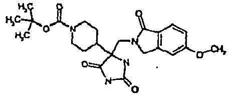 | **458.22** | **459.1 [M+H]⁺** | **B** |

**Proton NMR Spectral Data for Selected Compounds in Table 1005.**

**Compound 262.** ¹H NMR (500 Hz, CD₃OD) 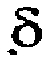 8.921 (m, 1H); 8.433 (d, J = 8.6 Hz, 1H); 8.357 (s, 1H); 8.072 (m, 4H); 7.622 (m, 1H); 7.545 (m, 1H); 7.476 (m, 1H); 7.369 (m, 1H); 4.522 (d, J = 17 Hz, 1H); 4.510 (d, J = 14.5 Hz, 1H); 4.425 (d, J = 17 Hz, 1H), 4.350 (d, J = 14.5 Hz, 1H).

### Example 1016.

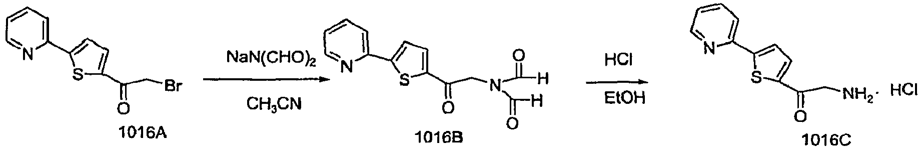

Compound **1016B**: Compound **1016A** (500 mg, 1.77 mmol) was suspended in CH₃CN (5 mL) followed by addition of NaN(CHO)₂ (202 mg, 2.12 mmol). The reaction mixture was stirred at rt for 30 min before warmed up to 70 °C and stirred for 2 h. Solid was collected by suction filtration and washed with acetonitrile to give **1016B** (380 mg, 78%) as brown solid.

Compound **1016C**: Compound **1016B** (380 mg, 1.38 mmol) was stirred with HCl (36% aq., 1 mL) and EtOH (10 mL) at rt for 2 days. It was then heated to 60 °C for 2 hr. Solvent was removed and it was dried under vacuum to give **1016C** (345 mg, 98%). The material was used without further purification.

The following compounds were prepared as described in **Example 1016**, **Example 2** and **Example 8.**

**Table 1006**

| **Compound #** | **Structure** | **Exact Mass** | **Mass Obsvd** | **Ki (nM)** |
|---|---|---|---|---|
| **278** | 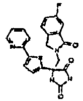 | **422.08** | **423.1 [M+H]⁺** | **A** |
| **279** | 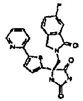 | **422.08** | **423.1 [M+H]⁺** | **C** |
| **280** | 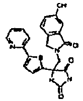 | **420.9** | **421.1[M+H]⁺** | **A** |
| **281** | 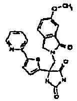 | **434.1** | **435.2[M+H]⁺** | **A** |

**Proton NMR Spectral Data for Selected Compounds in Table 1006.**

**Compound 278.** ¹H NMR (500 Hz, CD₃OD) 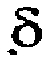 8.503 (d, J = 4.73 Hz, 1H); 7.84 (m, 2H); 7.67 (d, J = 3.8 Hz, 1H); 7.56 (dd, J = 4.4 Hz, 8.5 Hz, 1H); 7.50 (dd, J = 2.5 Hz,7.8 Hz, 1H); 7.38 (m, 1H); 7.33 (d, J = 4.1 Hz, 2H); 7.3 (m, 1H); 4.52 (d, J = 17 Hz, 1H); 4.45 (d, J = 17 Hz, 1H); 4.43 (d, J = 14.2 Hz, 1H); 4.28 (d, J = 14.2 Hz, 1H).

### Example 1017.

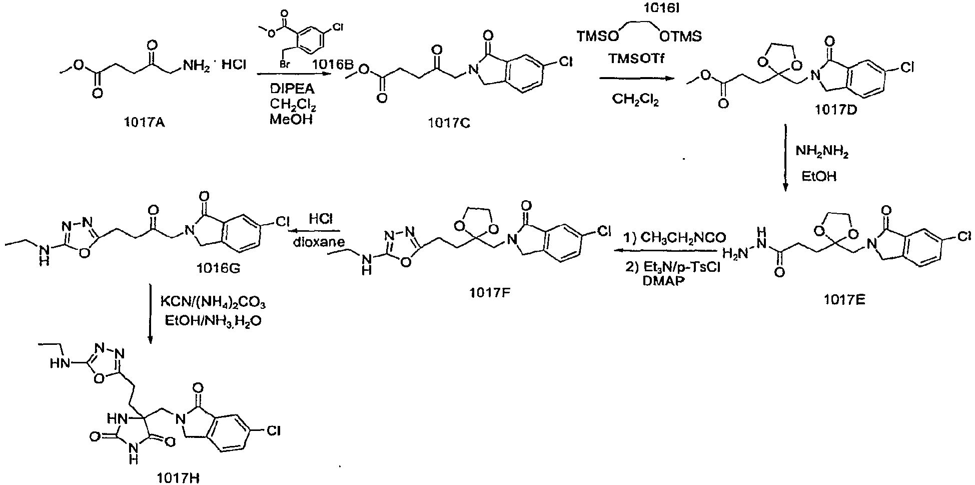

Compound **1017C**: Compound **1017A** (1.5 g, 8.26 mmol) was dissolved in dichloromethane (20 mL) and methanol (10 mL) at 0 °C. Compound **1017B** (2.64 g, 10 mmol) and DIPEA (2.9 mL, 16.5 mmol) were added and the reaction was stirred at 0 °C and slowly warmed up to rt overnight. The reaction mixture was then heated to 50 °C and stirred for 2 h. The reaction mixture was washed with brine (50 mL). The organic layer was dried and concentrated to dryness. The crude material was purified via PTLC (50% EtOAc/hexanes) to give 0.7g (29%) of compound **1017C.**

Compound **1017D:** Compound **1017C** (200 mg, 0.68 mmol) was stirred in CH₂Cl₂ (15 mL) at 0 °C followed by addition of compound **1017I** (0.5 mL, 2.04 mmol) and TMS-OTf (13 □L, 0.07 mmol). The reaction mixture was stirred at 0 °C to 5 °C for 6 hr before warmed up to rt and stirred overnight. The solvent was removed and the crude material was purified via PTLC (EtOAc) to give 0.21g (91%) of compound **1017D.**

Compound **1017E:** Compound **1017D** (210 mg, 0.62 mmol) was heated in a sealed tube with NH₂NH₂ (0.2 mL, 6.2 mmol) and EtOH (2 mL) at 60 °C overnight. Solvent was removed and gave crude material **1017E** (210 mg, 99%) which was used without further purification.

Compound **1017F**: Compound **1017E** (210 mg, 0.62 mmol) and ethyl isocyanate (59 µL, 0.74 mmol) were dissolved in CH₂Cl₂ (10 mL). The reaction mixture was stirred at room temperature overnight. To this mixture was added Et₃N (0.43 mL, 3.1 mmol), DMAP (15 mg, cat.) and *p*-TsCl (141 mg, 0.74 mmol). The reaction was stirred at rt overnight. Solvent was removed and the crude material was purified via sgc (10% NH₃•MeOH/CH₂Cl₂) to give 60 mg (25%) of compound **1017F.**

Compound **1017G**: Compound **1017F** (60 mg, 0.15 mmol) was heated in a sealed tube with HCl (3 mL, 4N in dioxane) at 65 °C for 48 hr. Solvent was removed and the crude material was purified via sgc (5% NH₃•MeOH/CH₂Cl₂) to give 35 mg (66%) of compound **1017G.**

Compound **1017H**: Compound **1017G** (34 mg, 0.1 mmol), KCN (10 mg, 0.15 mmol), and (NH₄)₂CO₃ (30 mg, 0.3 mmol) were suspended in a mixture of NH₃•H₂O (1 mL) and ethanol (1mL). The solution was stirred at 90 °C overnight. Solvent was removed and the crude material was purified via sgc (10% NH₃•MeOH/CH₂Cl₂) to give 6 mg (15%) of compound **1017H**.

The following compounds were prepared as described in **Example 1017**

**Table 1007**

| **Compound #** | **Structure** | **Exact Mass** | **Mass Obsvd** | **Ki (nM)** |
|---|---|---|---|---|
| **282** | 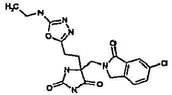 | **418.12** | **419.1[M+H]⁺** | **B** |

### Example 1018.

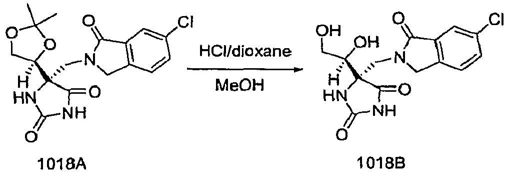

Compound **1018A:** Compound **1018A** was synthesized following procedures in **Example 1012.**

Compound **1018B:** Compound **1018A** (180 mg, 047 mmol) was stirred in MeOH (1 mL) at rt. HCl (3 mL, 4N in dioxane) was added and the reaction mixture was heated to 70 °C overnight. Solvent was evaporated. The crude material was taken up in water and the solid was collected by suction filtration to give **1018B** (115 mg, 71%).

### Example 1019.

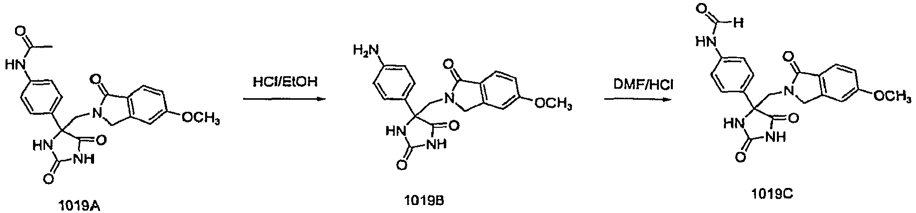

Compound **1019A**: Compound **1019A** was synthesized following procedures described in Example **1012.**

Compound **1019B**: Compound **1019A** (74 mg, 0.18 mmol) was dissolved in EtOH (2 mL) and HCl (0.4 mL, aq. 36%) was added and the reaction mixture was heated to 70 °C overnight. Solvent was removed and gave **1019B** as a light yellow solid (74 mg, 99%).
Compound **1019C**: Compound **1019B** (20 mg, 0.05 mmol) was stirred in DMF (1 mL) and HCl (cat., 4 N in dioxane) at 120 °C overnight. Solvent was removed and the crude material was purified via PTLC (9% NH₃•MeOH/CH₂Cl₂) to give 8 mg (37%) of Compound **1019C.**

The following compounds were prepared as described in **Example 1012, 1018 and 1019.**

**Table 1008**

| **Compound #** | **Structure** | **Exact Mass** | **Mass Obsvd** | **Ki (nM)** |
|---|---|---|---|---|
| **283** | 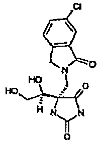 | **339.06** | **340.2[M+H]⁺** | **B** |
| **284** | 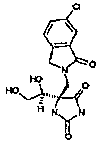 | **339.06** | **340.2[M+H]⁺** | **D** |
| **285** | 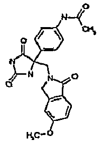 | **408.14** | **409.2[M+H]⁺** | **A** |
| **286** | 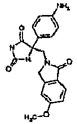 | **366.13** | **367.1 [M+H]⁺** | **A** |
| **287** | 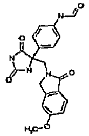 | **394.13** | **395.2[M+H]⁺** | **B** |

### Example 1020.

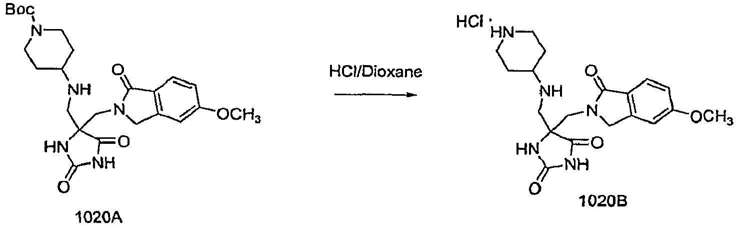

Compound **1020A**: Compound **1020A** was synthesized following the procedures described in Example **22.**

Compound **1020B:** Compound **1020A** (855 mg, 1.86 mmol) was stirred in MeOH (10 mL) and HCl (10 mL, 4N in dioxane) at rt for 2 hr. Solvent was removed and the material was dried to give **1020B** (735 mg, 99%).

The following compounds were prepared as described in **Example 22** and **Example 1020.**

**Table 1009**

| **Compound #** | **Structure** | **Exact Mass** | **Mass Obsvd** | **Ki (nM)** |
|---|---|---|---|---|
| **288** | 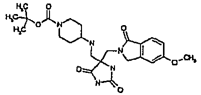 | **487.24** | **488.3[M+H]⁺** | **B** |
| **289** | 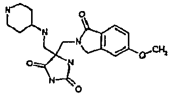 | **387.19** | **388.1 [M+H]⁺** | **C** |
| **290** | 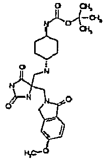 | **501.26** | **502.3[M+H]⁺** | **B** |
| **291** | 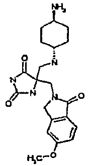 | **401.21** | **402.2[M+H]⁺** | **C** |
| **292** | 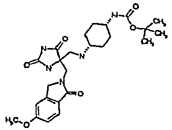 | **501.26** | **502.3[M+H]⁺** | **B** |
| **293** | 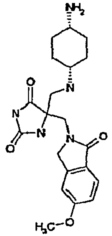 | **401.21** | **402.1 [M+H]⁺** | **B** |

### Example 1021

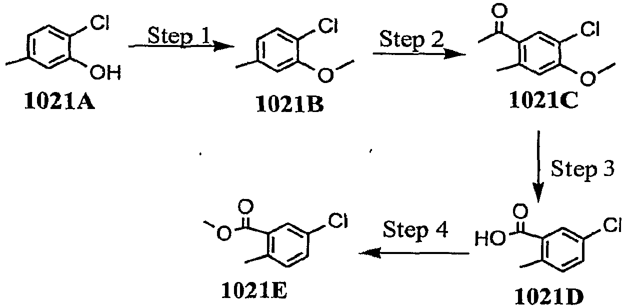

### Step 1

DMF ( 100 mL), cesium carbonate (41.13 g, 126 mmol), and 2-chloro-5-methylphenol (**1021A**) (15.0 g, 105 mmol) were added to a flask. Methyl iodide (17.92 g, 126 mmol) was added dropwise via addition funnel. The reaction mixture was stirred overnight at room temperature. The reaction mixture was diluted with EtOAc, washed with water and brine, and dried with Na₂SO₄. The resulting material was filtered, and concentrated to dryness. The crude product was purified via flash sgc using 1:4 EtOAc:hexanes as the mobile phase to give 15.93 g of **1021B.**

### Step 2

A flask containing AlCl₃ (2.55 g, 19.1 mmol), and LiCl (0.41 g, 9.6 mmol) was placed in a -30 °C cold bath. A solution of **1021B** (1.0 g, 6.38 mmol) and acetyl chloride (0.75 g, 9.5 mmol) in 20 mL of CH₂Cl₂ was added dropwise. The reaction mixture was stirred for 1 h at -30 °C, then allowed to warm to rt and stirred overnight at rt. The reaction mixture was poured into a mixture of ice and EtOAc. The organic layer was washed with water, saturated aq NaHCO₃, and water, then dried with Na₂SO₄, and concentrated to dryness to give 1.18 g of Compound **1021C**.

### Step 3

Sodium hydroxide (58 g, 1.45 mol) was dissolved in water (260 mL) and the flask was cooled in an ice-water bath. Bromine (19 mL) was added dropwise to the flask with stirring. The reaction mixture was stirred for 0.5 h after the addition was complete. The resulting solution was added dropwise to an ice-water cooled flask containing Compound **1021C** (18.5 g, 93.1 mmol). After the addition was complete, the reaction mixture was allowed to warm to rt and left stirring overnight. The reaction mixture was heated at 40 °C for 2 h. NaHSO₃ (55 g) was added. The reaction mixture was stirred for 1 h. The resulting material was diluted with 10% aq NaOH and extracted with EtOAc to remove starting material. The aqueous layer was adjusted to pH 1 and extracted with additional EtOAc. The organic layer was dried with Na₂SO₄, filtered, and concentrated to dryness to give 12.31 g of **1021D.**

### Step 4

DMF (10 mL), Compound **1021D** (0.50 g, 2.49 mmol), and K₂CO₃ (0.41 g, 2.96 mmol) were added to a flask. Methyl iodide (0.42 g, 2.96 mmol) was added dropwise. The reaction mixture was stirred overnight at room temperature. The reaction mixture was concentrated to dryness to give 0.52 g of **1021E**.

The following compounds were prepared as described in step 1 in **Example 14** and **Example 1021.**

**Table 1010**

| **Compound #** | **Structure** | **Exact Mass** | **Mass Obsvd** | **Ki (nM)** |
|---|---|---|---|---|
| **296** | 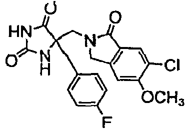 | **403.07** | **404.2 [M+H]⁺** | **A** |
| **297** | 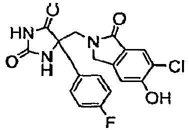 | **389.06** | **390.2 [M+H]⁺** | **A** |
| **298** | 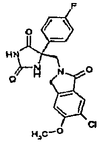 | **403.07** | **404.1 [M+H]⁺** | **n/a** |

**Proton NMR Spectral Data for Selected Compounds in Table 1010.**

**Compound 296.** ¹H NMR (500 Hz, DMSO-d₆) 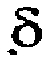 3.93 (s, 3H), 4.00 (d, J = 14 Hz, 1H), 4.19 (d, J = 14 Hz, 1H), 4.23 (d, J = 18 Hz,1H), 4.34 (d, J = 18 Hz, 1H), 7.24-7.34 (m, 2H), 7.42 (s, 1H), 7.62-7.73 (m, 3H), 8.92 (s, 1H), 10.95 (s, 1H).

Specific TACE inhibitory activity (Ki values) of some representative compounds of the present invention are set forth below.

**Table 1011**

| **Compound #** | **Structure** | **Ki (nM)** |
|---|---|---|
| **111** | 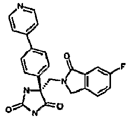 | **0.48** |
| **120** | 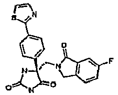 | **4** |
| **213** | 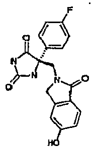 | **0.8** |
| **181** | 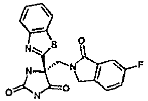 | **3.17** |
| **262** | 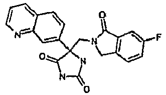 | **4.91** |
| **198** | 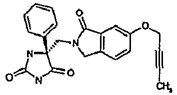 | **1.29** |
| **143** | 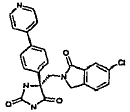 | **1.87** |
| **219** | 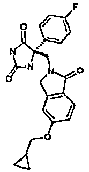 | **2.4** |
| **155** | 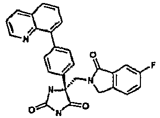 | **1.05** |
| **296** | 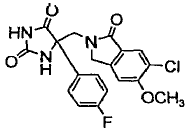 | **1.01** |
| **123** | 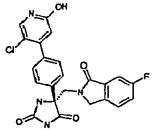 | **1.2** |
| **232** | 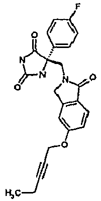 | **1.0** |
| **233** | 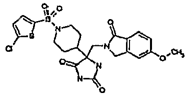 | **2.35** |
| **278** | 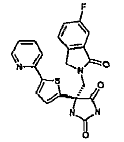 | **2.2** |
| **139** | 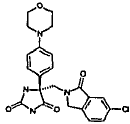 | **2.8** |
| **25** | 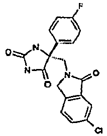 | **0.43** |
| **203** | 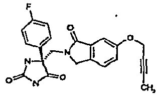 | **0.23** |
| **239** | 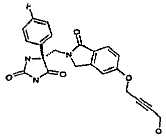 | **0.11** |
| **243** | 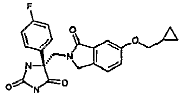 | **3.00** |

The following additional compounds were also prepared by procedures described above as well as in the description discussed later.

**Table 3000**

| **Compound ID** | **Structures** | **Exact Mass** | **Mass Obsvd** | **Ki Rating** |
|---|---|---|---|---|
| **3000** | 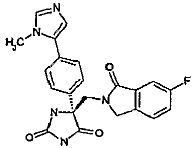 | **419.14** | **420.1 [M+H]⁺** | **C** |
| **3001** | 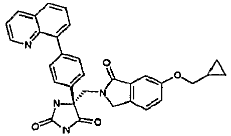 | **518.20** | **519.1[M+H]⁺** | **B** |
| **3002** | 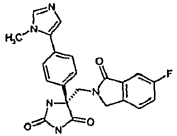 | **419.14** | **420.1[M+H]⁺** | **A** |
| **3003** | 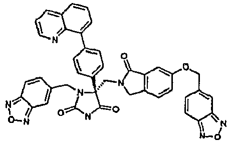 | **728.71** | **729.2[M+H]⁺** | **D** |
| **3004** | 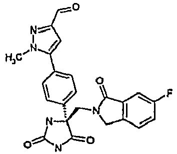 | **447.13** | **448.2[M+H]⁺** | **C** |
| **3005** | 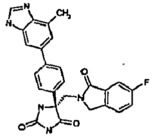 | **469.16** | **470.3[M+H]⁺** | **A** |
| **3006** | 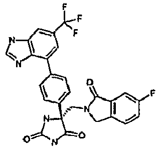 | **523.13** | **524.3[M+H]⁺** | **A** |
| **3007** | 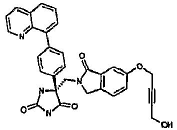 | **532.20** | **533.3[M+H]^{*}** | **A** |
| **3008** | 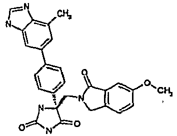 | **481.20** | **482.3[M+H]⁺** | **B** |
| **3009** | 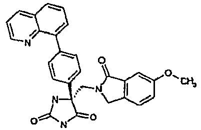 | **478.16** | **479.3[M+H]⁺** | **A** |
| **3010** | 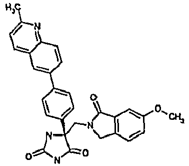 | **492.20** | **493.3[M+H]⁺** | **A** |
| **3011** | 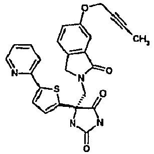 | **472.10** | **473.3[M+H]⁺** | **A** |
| **3012** | 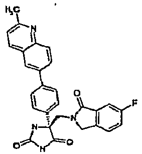 | **480.20** | **481.3[M+H]⁺** | **D** |
| **3013** | 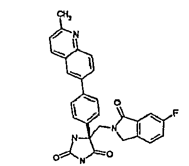 | **480.20** | **481.3[M+H]⁺** | **A** |
| **3014** | 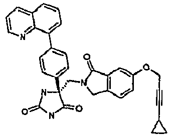 | **542.20** | **543.3[M+H]⁺** | **B** |
| **3015** | 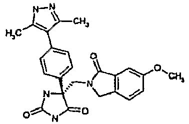 | **445.20** | **446.2[M+H]⁺** | **A** |
| **3016** | 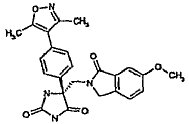 | **446.20** | **447.2[M+H]⁺** | **A** |
| **3017** | 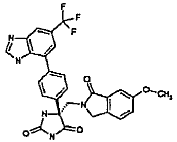 | **535.15** | **536.3[M+H]⁺** | **B** |
| **3018** | 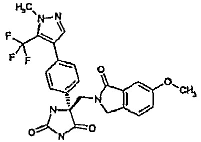 | **499.10** | **500.3[M+H]⁺** | **B** |
| **3019** | 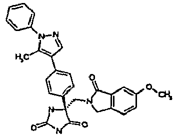 | **507.20** | **508.3[M+H]⁺** | **A** |
| **3020** | 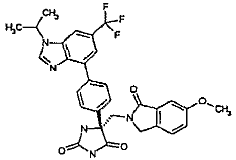 | **577.19** | **578.3[M+H]⁺** | **B** |
| **3021** | 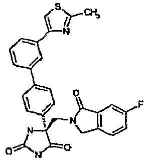 | **512.13** | **513.3[M+H]⁺** | **D** |
| **3022** | 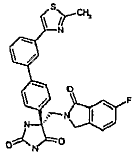 | **512.13** | **513.3[M+H]⁺** | **B** |
| **3023** | 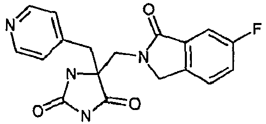 | **354.34** | **355.2[M+H]⁺** | **D** |
| **3024** | 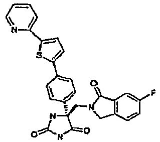 | **498.12** | **499.3[M+H]⁺** | **C** |
| **3025** | 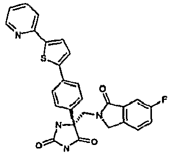 | **498.12** | **499.3[M+H]⁺** | **B** |
| **3026** | 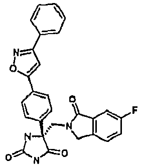 | **482.14** | **483.3[M+H]⁺** | **B** |
| **3027** | 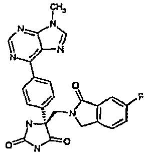 | **471.15** | **472.3[M+H]⁺** | **D** |
| **3028** | 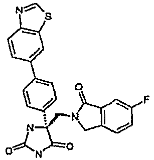 | **472.10** | **473.3[M+H]⁺** | **C** |
| **3029** | 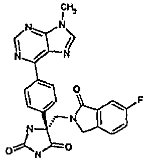 | **471.15** | **472.3[M+H]⁺** | **A** |
| **3030** | 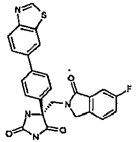 | **472.10** | **473.3[M+H]⁺** | **A** |
| **3031** | 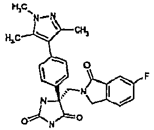 | **447.17** | **448.2[M+H]⁺** | **A** |
| **3032** | 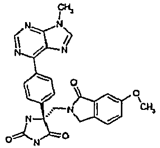 | **483.17** | **484.3[M+H]⁺** | **A** |
| **3033** | 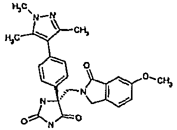 | **459.19** | **460.3[M+H]⁺** | **A** |
| **3034** | 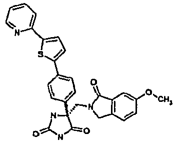 | **510.14** | **511.3[M+H]⁺** | **A** |
| **3035** | 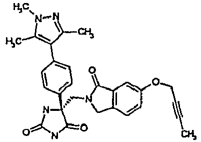 | **497.21** | **498.3[M+H]⁺** | **A** |
| **3003** | 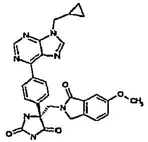 | **523.20** | **524.3[M+H]⁺** | **A** |
| **3037** | 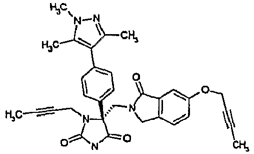 | **549.24** | **550.3[M+H]⁺** | **A** |
| **3038** | 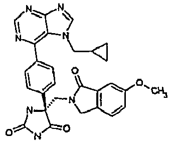 | **523.20** | **524.3[M+H]⁺** | **A** |
| **3039** | 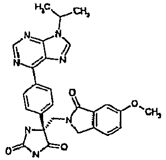 | **511.20** | **512.3[M+H]⁺** | **A** |
| **3040** | 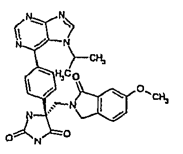 | **511.20** | **512.3[M+H]⁺** | **A** |
| **3041** | 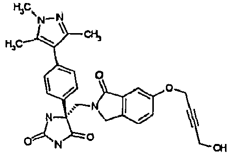 | **513.20** | **514.3[M+H]⁺** | **A** |
| **3042** | 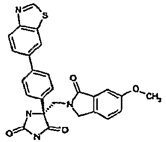 | **584.12** | **585.3[M+H]⁺** | **A** |
| **3043** | 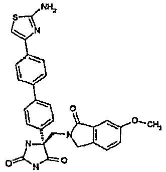 | **525.14** | **526.3[M+H]⁺** | **C** |
| **3044** | 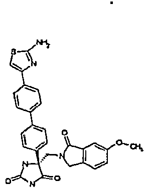 | **525.14** | **526.3[M+H]⁺** | **A** |
| **3045** | 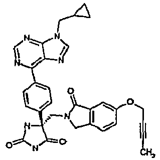 | **561.21** | **562.3[M+H]⁺** | **A** |
| **3046** | 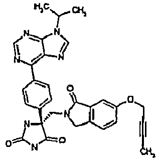 | **549.21** | **550.3[M+H]⁺** | **A** |
| **3047** | 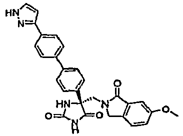 | **493.18** | **494.3[M+H]⁺** | **A** |
| **3048** | 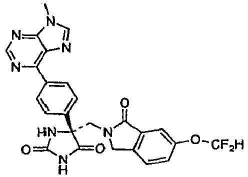 | **519.15** | **520.3[M+H]⁺** | **A** |
| **4001** | 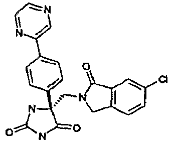 | **433.1** | **434.2 [M+H]⁺** | **A** |
| **4002** | 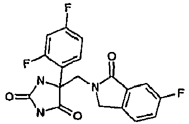 | **375.1** | **376.1 [M+H]⁺** | **A** |
| **4003** | 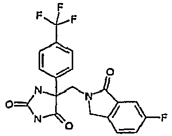 | **407.1** | **408.2 [M+H]⁺** | **A** |
| **4004** | 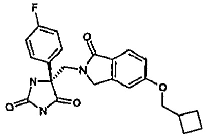 | **423.2** | **424.2 [M+H]⁺** | **A** |
| **4005** | 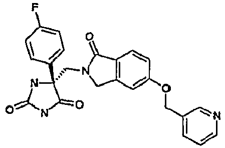 | **446.1** | **447.2 [M+H]⁺** | **A** |
| **4006** | 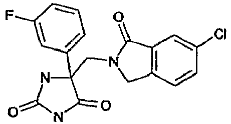 | **373.1** | **374.2 [M+H]⁺** | **C** |
| **4007** | 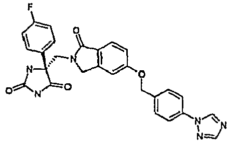 | **512.2** | **513.3 [M+H]⁺** | **A** |
| **4008** | 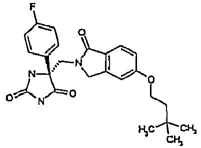 | **439.2** | **440.2 [M+H]⁺** | **B** |
| **4009** | 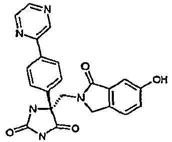 | **415.1** | **416.2 [M+H]⁺** | **B** |
| **4010** | 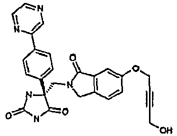 | **483.2** | **484.3 [M+H]⁺** | **A** |
| **4011** | 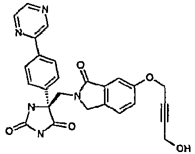 | **483.2** | **484.3 [M+H]⁺** | **C** |
| **4012** | 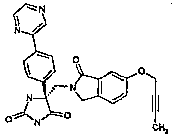 | **467.2** | **468.3 [M+H]⁺** | **A** |
| **4013** | 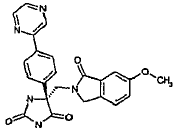 | **429.2** | **430.2 [M+H]⁺** | **A** |
| **4014** | 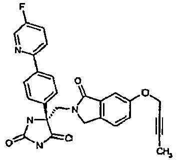 | **484.2** | **485.3 [M+H]⁺** | **A** |
| **4015** | 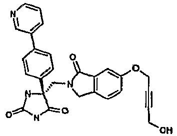 | **482.2** | **483.3 [M+H]⁺** | **A** |
| **4016** | 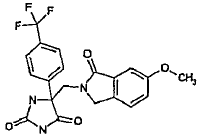 | **419.1** | **420.0 [M+H]⁺** | **A** |
| **4017** | 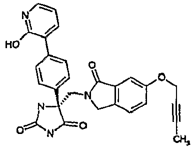 | **482.2** | **483.3 [M+H]⁺** | **A** |
| **4018** | 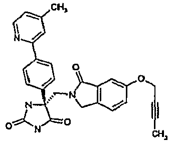 | **480.2** | **481.3 [M+H]⁺** | **A** |
| **4019** | 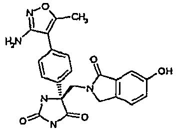 | **433.1** | **434.2 [M+H]⁺** | **C** |
| **4020** | 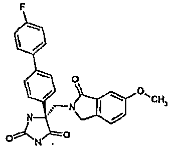 | **445.1** | **446.2 [M+H]⁺** | **A** |
| **4021** | 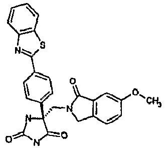 | **484.1** | **485.3 [M+H]⁺** | **A** |
| **4022** | 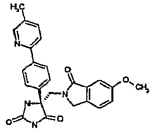 | **442.2** | **443.2 [M+H]⁺** | **A** |
| **4023** | 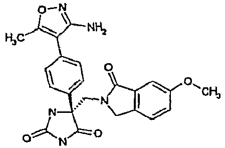 | **447.2** | **448.2 [M+H]⁺** | **A** |
| **4024** | 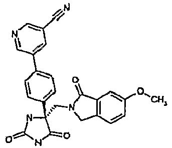 | **453.1** | **454.2 [M+H]⁺** | **A** |
| **4025** | 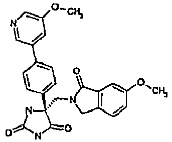 | **458.2** | **459.3 [M+H]⁺** | **A** |
| **4026** | 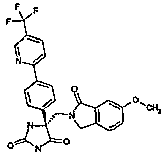 | **496.2** | **497.3 [M+H]⁺** | **A** |
| **4027** | 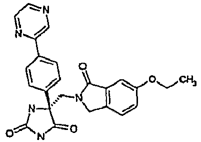 | **443.2** | **444.2 [M+H]⁺** | **A** |
| **4028** | 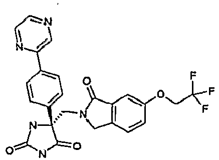 | **497.1** | **498.3 [M+H]⁺** | **B** |
| **4029** | 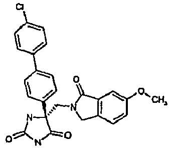 | **461.1** | **462.3 [M+H]⁺** | **A** |
| **4030** | 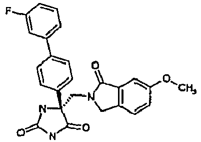 | **445.1** | **446.2 [M+H]⁺** | **A** |
| **4031** | 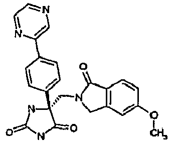 | **429.1** | **430.2 [M+H]⁺** | **A** |
| **4032** | 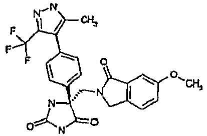 | **499.1** | **500.3 [M+H]⁺** | **A** |
| **4033** | 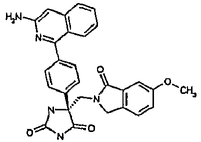 | **493.2** | **494.3 [M+H]⁺** | **A** |
| **4034** | 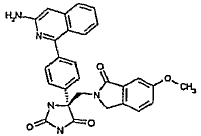 | **493.2** | **494.3 [M+H]⁺** | **B** |
| **4035** | 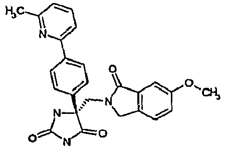 | **442.2** | **443.2 [M+H]⁺** | **A** |
| **4036** | 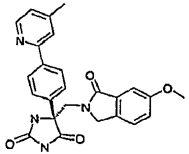 | **442.2** | **443.2 [M+H]⁺** | **n/a** |
| **4037** | 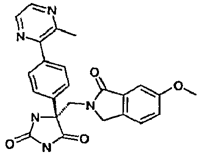 | **443.2** | **444.2 [M+H]⁺** | **n/a** |
| **4038** | 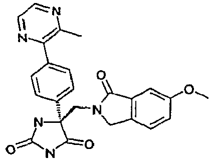 | **443.2** | **444.2 [M+H]⁺** | **n/a** |
| **4039** | 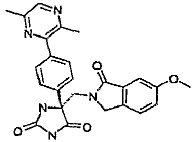 | **457.2** | **458.3 [M+H]⁺** | **n/a** |
| **4040** | 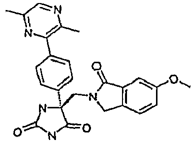 | **457.2** | **458.3 [M+H]⁺** | **n/a** |
| **4041** | 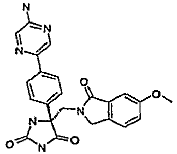 | **444.1** | **445.2 [M+H]⁺** | **n/a** |
| **4042** | 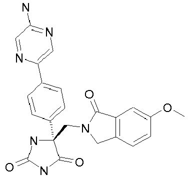 | **444.1** | **445.2 [M+H]⁺** | **n/a** |
| | | | | |
| **5000** | 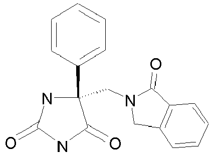 | **321.1** | **322.2[M+H]⁺** | |
| **5001** | 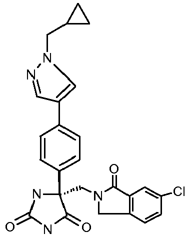 | **475.1** | **476.3[M+H]⁺** | |
| **5003** | 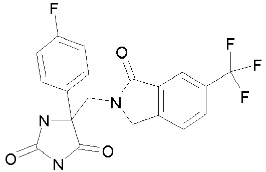 | **407.1** | **408.2[M+H]⁺** | **B** |
| **5005** | 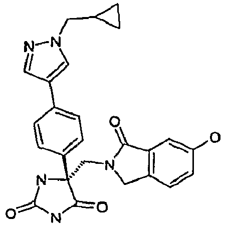 | **457.2** | **458.3[M+H]⁺** | **A** |
| **5006** | 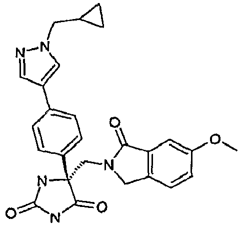 | **471.2** | **472.1 [M+H]⁺** | **A** |
| **5007** **1338095** | 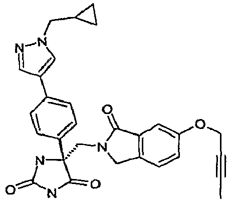 | **509.2** | **510.3[M+H]⁺** | **A** |
| **5008** | 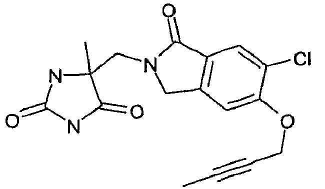 | **361.08** | **362.2[M+H]⁺** | **B** |
| **5009** | 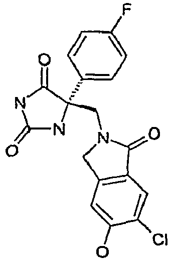 | **389.1** | **390.2[M+H]⁺** | **A** |
| **5010** | 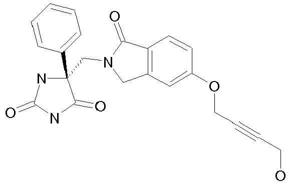 | **405.1** | **406.2[M+H]⁺** | **A** |
| **5016** | 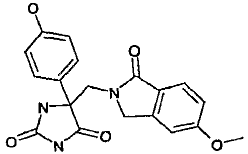 | **367.1** | **368.2[M+H]⁺** | **B** |
| **5017** | 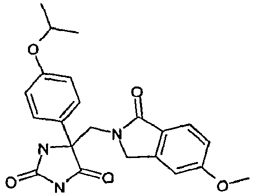 | **409.2** | **410.2[M+H]⁺** | **A** |
| **5018** | 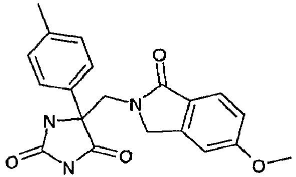 | **365.14** | **366.2[M+H]⁺** | **A** |
| **5019** | 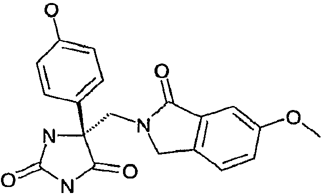 | **367.12** | **368.2[M+H]⁺** | **A** |
| **5020** | 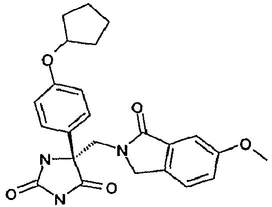 | **435.2** | **436.2[M+H]⁺** | **A** |
| **5021** | 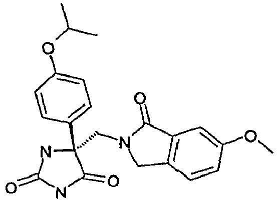 | **409.2** | **410.2[M+H]⁺** | **A** |
| **5022** | 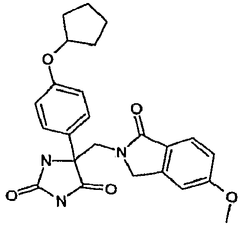 | **435.2** | **436.2[M+H]⁺** | **A** |
| **5023** | 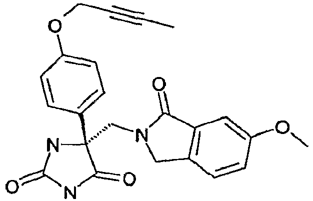 | **419.2** | **420.2[M+H]⁺** | **A** |
| **5024** | 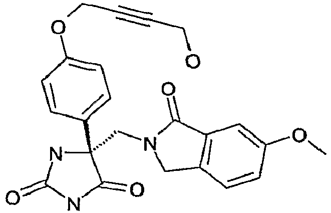 | **435.1** | **436.2[M+H]** | **A** |
| **5025** | 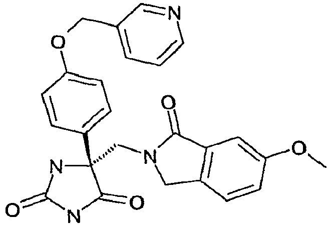 | **458.2** | **459.3[M+H]⁺** | **A** |
| | | | | |
| **6000** | 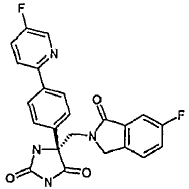 | **434.39** | **435.4 [M+H]⁺** | **A** |
| **6001** | 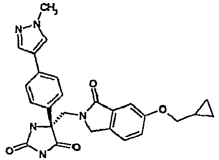 | **471.50** | **472.5 [M+H]⁺** | **A** |
| **6002** | 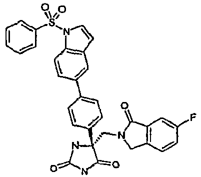 | **594.61** | **595.7 [M+H]⁺** | **B** |
| **6003** | 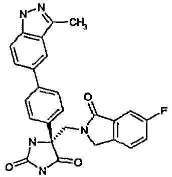 | **469.46** | **470.5 [M+H]⁺** | **A** |
| **6004** | 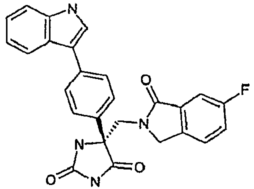 | **454.45** | **455.5 [M+H]⁺** | **A** |
| **6005** | 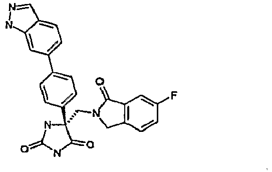 | **455.44** | **456.3 [M+H]⁺** | **A** |
| **6006** | 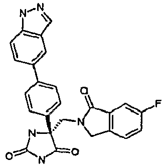 | **455.44** | **456.3 [M+H]⁺** | **A** |
| **6007** | 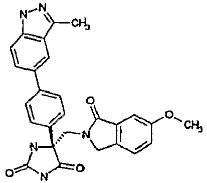 | **481.50** | **482.6 [M+H]⁺** | **A** |
| **6008** | 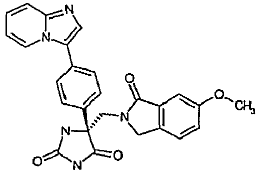 | **467.47** | **468.5 [M+H]⁺** | **A** |
| **6009** | 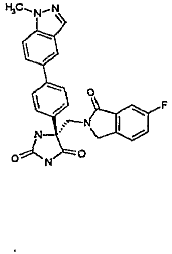 | **469.46** | **470.5 [M+H]⁺** | **A** |
| **6010** | 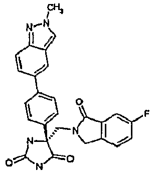 | **469.46** | **470.5 [M+H]⁺** | **A** |
| **6011** | 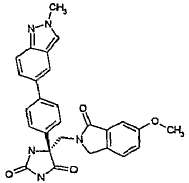 | **481.50** | **482.4 [M+H]⁺** | **A** |
| **6012** | 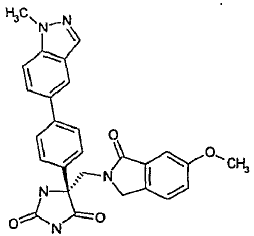 | **481.50** | **482.4 [M+H]⁺** | **A** |
| **6013** | 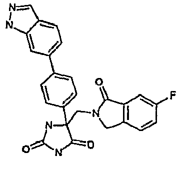 | **455.44** | **456.3 [M+H]⁺** | **A** |
| **6014** | 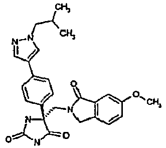 | **473.52** | **474.5 [M+H]⁺** | **A** |
| **6015** | 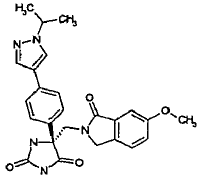 | **459.49** | **460.5 [M+H]⁺** | **A** |
| **6016** | 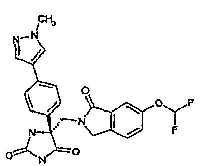 | **503.89** | **504.9 [M+H]⁺** | **A** |
| **6017** | 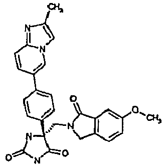 | **481.50** | **482.5 [M+H]⁺** | **A** |
| **6018** | 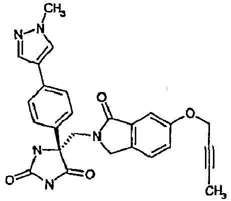 | **469.49** | **470.5 [M+H]⁺** | **A** |
| **6019** | 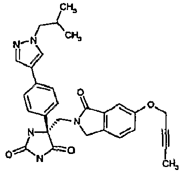 | **511.57** | **512.6 [M+H]⁺** | **A** |
| **6020** | 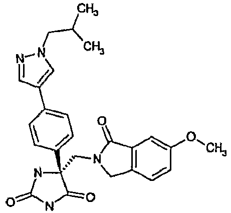 | **473.52** | **474.5 [M+H]⁺** | **A** |
| **6021** | 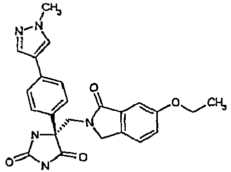 | **445.47** | **446.5 [M+H]⁺** | **A** |
| **6022** | 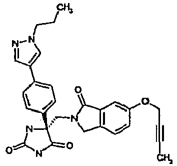 | **497.54** | **498.6 [M+H]⁺** | **A** |
| **6023** | 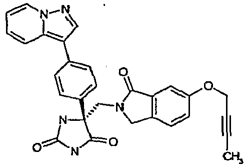 | **505.5** | **506.5 [M+H]⁺** | **A** |
| **6024** | 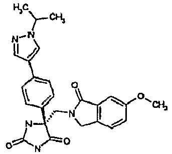 | **459.49** | **460.6 [M+H]⁺** | **A** |
| **6025** | 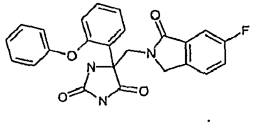 | **431.41** | **432.5 [M+H]⁺** | **B** |
| **6026** | 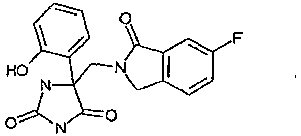 | **355.31** | **356.4 [M+H]⁺** | **A** |
| **6027** | 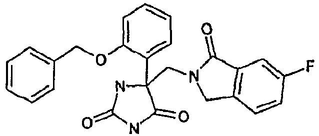 | **445.44** | **446.5 [M+H]⁺** | **B** |
| **6028** | 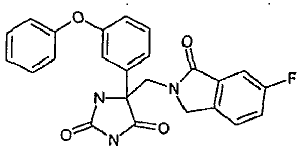 | **431.41** | **432.4 [M+H]⁺** | **B** |
| **6029** | 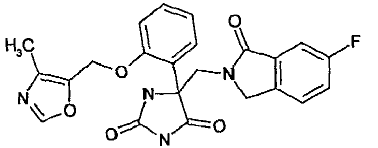 | **450.42** | **451.4 [M+H]⁺** | **B** |
| **7000** | 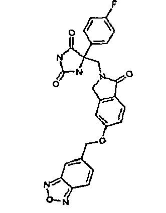 | **487.43 93** | **488.3[M+H]⁺** | **C** |
| **7001** | 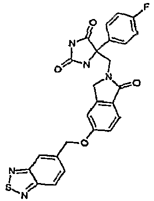 | **503.50 49** | **504.3[M+H]⁺** | **C** |
| **7002** | 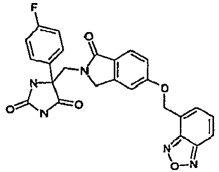 | **487.43 93** | **488.3[M+H]⁺** | **B** |
| **7003** | 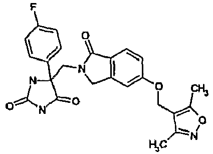 | **464.44 57** | **465.3[M+H]⁺** | **C** |
| **7004** | 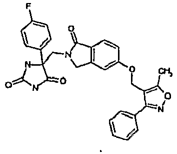 | **526.51 51** | **527.3[M+H]⁺** | **D** |
| **7005** | 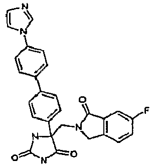 | **481.47 78** | **482.3[M+H]⁺** | **B** |
| **7006** | 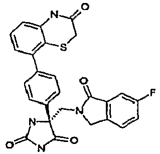 | **502.51 69** | **503.3[M+H]⁺** | **A** |
| **7007** | 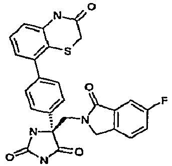 | **502.51 69** | **503.3[M+H]⁺** | **C** |
| **7008** | 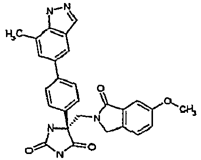 | **481.50 26** | **482.3[M+H]⁺** | **A** |
| **7009** | 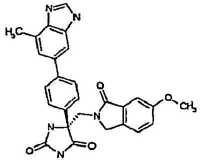 | **481.50 26** | **482.3[M+H]⁺** | **A** |
| **7010** | 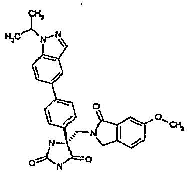 | **509.55 58** | **510.3[M+H]⁺** | **A** |
| **7011** | 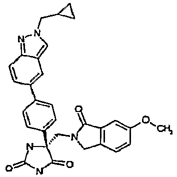 | **521.56 65** | **522.3[M+H]⁺** | **A** |
| **7012** | 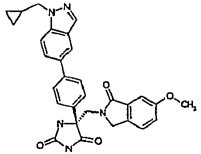 | **521.56 65** | **522.3[M+H]⁺** | **A** |
| **7013** | 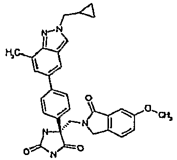 | **535.59 31** | **536.3[M+H]⁺** | **A** |
| **7014** | 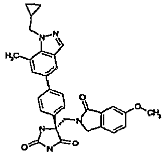 | **535.59 31** | **536.3[M+H]⁺** | **B** |
| **7015** | 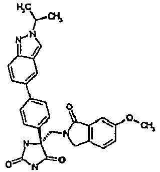 | **509.55 58** | **510.3[M+H]⁺** | **A** |
| **7016** | 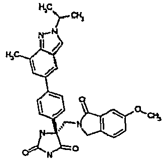 | **523.58 24** | **524.3[M+H]⁺** | **A** |
| **7017** | 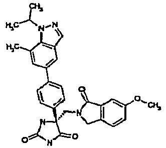 | **523.58 24** | **524.3[M+H]⁺** | **A** |
| **7018** | 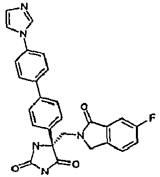 | **481.47 78** | **482.3[M+H]⁺** | **A** |
| **7019** | 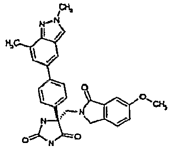 | **495.52 92** | **496.3[M+H]⁺** | **A** |
| **7020** | 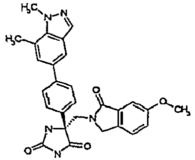 | **495.52 92** | **496.3[M+H]⁺** | **A** |
| **7021** | 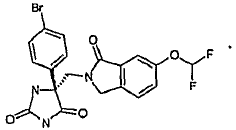 | **466.23 3** | **468.3[M+H]⁺** | **A** |
| **7022** | 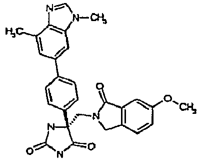 | **495.52 92** | **496.3[M+H]⁺** | **A** |
| **7023** | 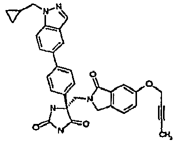 | **559.61 45** | **560.3[M+H]⁺** | **B** |
| **7024** | 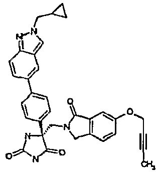 | **559.61 45** | **560.3[M+H]⁺** | **A** |
| **7025** | 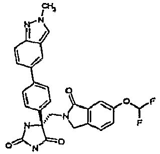 | **517.48 35** | **518.3[M+H]⁺** | **A** |
| **7026** | 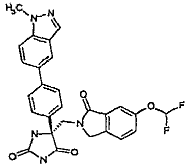 | **517.48 35** | **518.3[M+H]⁺** | **A** |
| **8001** | 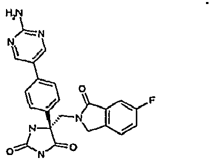 | **432.13** | **433.2[M+H]⁺** | **A** |
| **8002** | 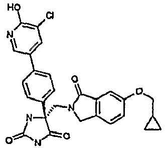 | **518.14** | **519.3[M+H]⁺** | **A** |
| **8003** | 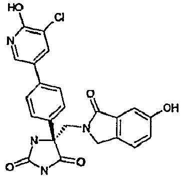 | **464.09** | **465.3[M+H]⁺** | **A** |
| **8004** | 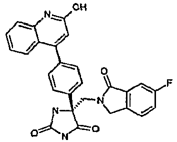 | **482.14** | **483.3[M+H]⁺** | **A** |
| **8005** | 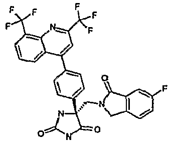 | **602.12** | **603.3[M+H]⁺** | **C** |
| **8006** | 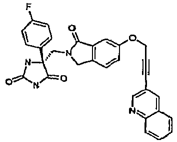 | **520.15** | **521.3[M+H]⁺** | **B** |
| **8007** | 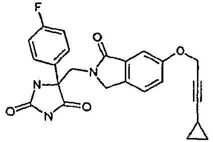 | **433.14** | **434.2[M+H]⁺** | **A** |
| **8008** | 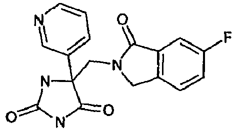 | **340.1** | **341.2[M+H]⁺** | **B** |
| **8009** | 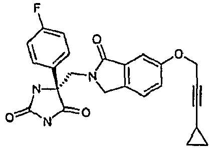 | **433.14** | **434.2[M+H]⁺** | **A** |
| **8010** | 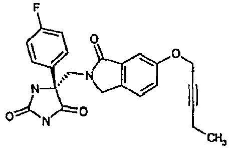 | **421.14** | **422.2[M+H]⁺** | **A** |
| **8011** | 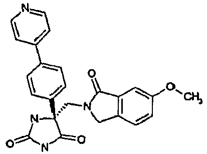 | **428.15** | **429.2[M+H]⁺** | **A** |
| **8012** | 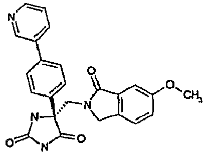 | **428.15** | **429.2[M+H]⁺** | **A** |
| **8013** | 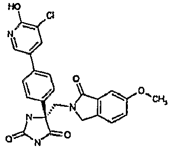 | **478.1** | **479.3[M+H]⁺** | **A** |
| **8014** | 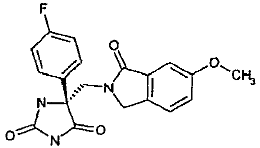 | **369.11** | **370.2[M+H]⁺** | **A** |
| **8015** | 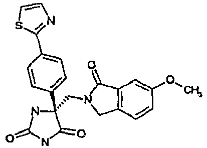 | **434.1** | **435.2[M+H]⁺** | **A** |
| **8016** | 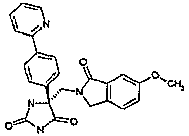 | **428.15** | **429.2[M+H]⁺** | **A** |
| **8017** | 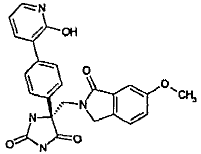 | **444.14** | **445.2 [M+H]⁺** | **A** |
| **8018** | 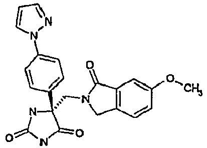 | **417.14** | **418.2[M+H]⁺** | **A** |
| **8019** | 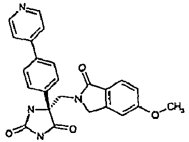 | **428.15** | **429.2[M+H]⁺** | **A** |
| **8020** | 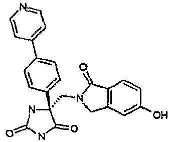 | **414.13** | **415.2[M+H]⁺** | **A** |
| **8021** | 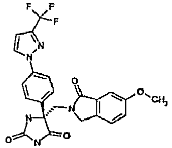 | **485.13** | **486.3[M+H]⁺** | **A** |
| **8022** | 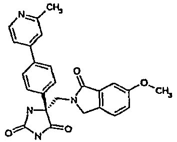 | **442.16** | **443.2[M+H]⁺** | A |
| **8023** | 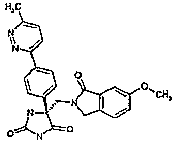 | **443.16** | **444.2[M+H]⁺** | |
| **8024** | 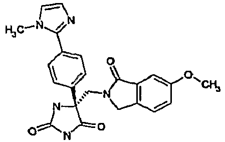 | **431.16** | **432.2[M+H]⁺** | |
| **8025** | 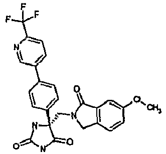 | **496.14** | **497.3[M+H]⁺** | |
| **8027** | 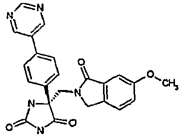 | **429.14** | **430.2[M+H]⁺** | |
| **2021F** | 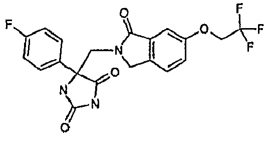 | **437.1** | **438.1 M+H]⁺** | **C** |
| **2023C** | 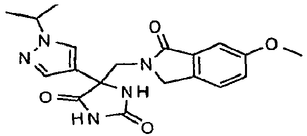 | **383.1 6** | **384.1 [M+H] ⁺** | **A** |
| **2024D** | 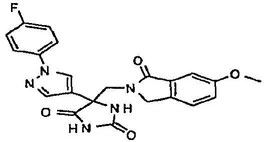 | **435.1 3** | **436.1 [M+H]⁺** | **A** |
| **2022G** | 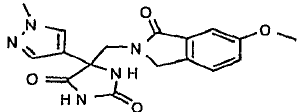 | **355.1 3** | **356.1 [M+H]⁺** | **A** |
| **2025C** | 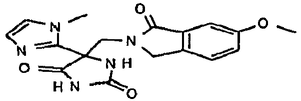 | **355.1 3** | **356.1 [M+H]⁺** | **A** |
| **2028** | 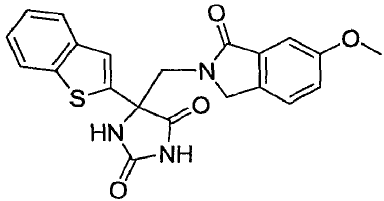 | **407.1** | **408.1 [M+H]⁺** | A |
| 2026H | 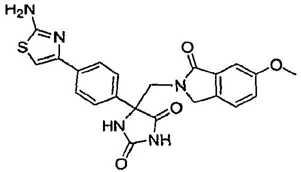 | **449.1** | **450.1 [M+H]⁺** | A |
| **2030A** | 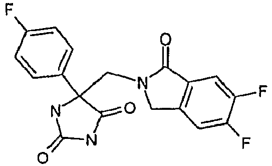 | **375.0 8** | **376.1 [M+H ]⁺** | C |
| **2030C** | 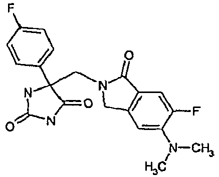 | **400.1 3** | **401.2[M+H ]⁺** | C |
| 2030B | 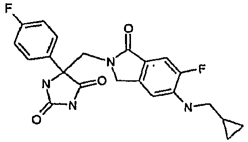 | **426.1 5** | **427.1 [M+H ]⁺** | B |
| 2031C | 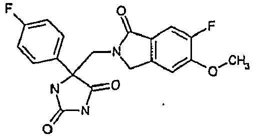 | **387.1 0** | **388.1 [M+H ]⁺** | A |
| 2031D | 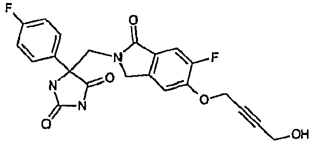 | **441.1 1** | **442.0[M+H ]⁺** | A |
| 2030D | 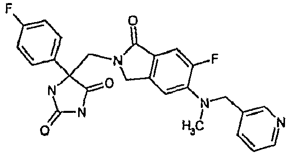 | **477.1 6** | **478.1 [M+H ]⁺** | B |
| 2031A | 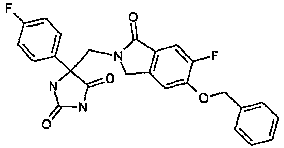 | **463.1 3** | **464.0[M+H ]⁺** | B |
| 2031B | 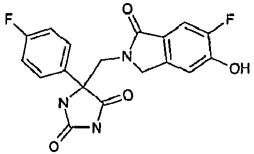 | **373.0 9** | **374.0[M+H ]⁺** | B |
| 2032B | 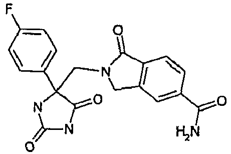 | **382.1 1** | **383.1 [M+H ]⁺** | B |
| 2031E | 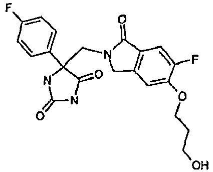 | **431.1 3** | **432.1 [M+H ]⁺** | B |
| 2031F | 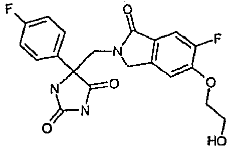 | **417.1 1** | **418.1 [M+H ]⁺** | B |

### Procedures

### Example 4000:

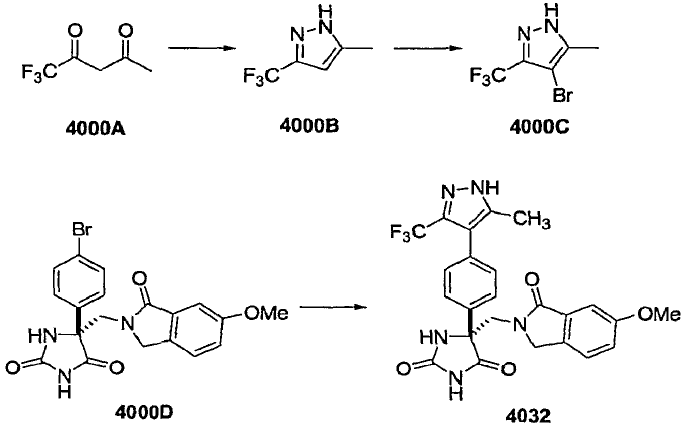

Compound **4000C** was prepared from commercially available **4000A** according to a published two-step procedure: Ebenbeck, W.; Rampf, F.; Marhold, A. PCT Intl. Appl. US 2004/0142820 A1 (July 22, 2004). Compound 4000D was prepared by procedures given in Examples 14,1008, 9 and 1001. Compound **4032** was prepared from Compound **4000D** as described in Example 1004, but substituting Compound **4000C** for 3-bromoimidazo[1,2-a]pyridine in Step 2.

### Example 4100:

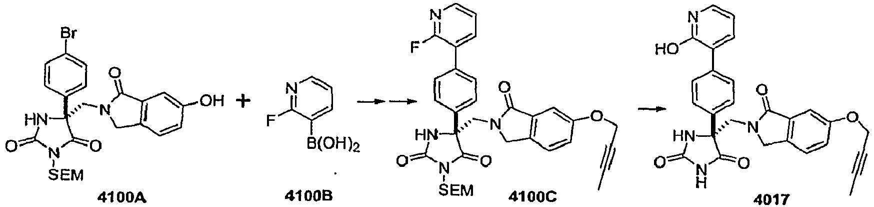

Compound **4100C** was prepared from compound **4100A** and commercially available Compound **4100B** by procedures given in Example 14 and 1009. Subsequently, compound **4100C** (123 mg, 0.2 mmol) was dissolved in methanol (1 mL) in a pressure tube and treated with HCl (0.4 mL, 4 M in dioxane). The tube was sealed and heated with stirring at 90 °C for 18 h. The reaction mixture was allowed to cool to rt and the solvent was then removed under reduced pressure. The residue was re-dissolved in methanol (1 mL) and DIPEA (0.27 mL, 0.20 mg, 1.6 mmol) was added. The reaction mixture was stirred overnight at rt. The volatile components were removed by evaporation and the residue was purified by PTLC (8% MeOH-CH₂Cl₂) to give Compound **4017** (59 mg, 61% yield) as a beige solid.

### Example 1022

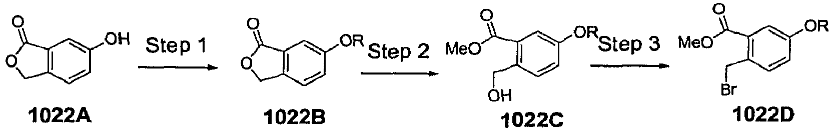

### Step 1

To a solution of **1022A** (500 mg, 3.33 mmol) in acetone (40 mL) were added potassium carbonate (920 mg, 6.7 mmol) and 1-bromo-2-butyne (0.32 mL, 3.7 mmol, in case of R = CH₂CCCH₃). The reaction mixture was heated to reflux for 2 h. After cooling to RT, the mixture was added to ice water/CH₂Cl₂. The organic layers were extracted with CH₂Cl₂ and the combined organic solution was washed with saturated aqueous NaCl solution, dried (Na₂SO₄), and concentrated *in vacuo* to afford **1022B** (674 mg, quantitative yield).

### Steps2 and 3

A suspension of **1022B** (100 mg, 0.5 mmol) in 1N NaOH solution (0.5 mL) was heated to 100 °C. The reaction mixture was stirred for 1 h at the temperature and concentrated *in vacuo.* The residue was dried by azeotropic distillation with toluene and the resulting solid was dissolved in DMF (0.6 mL) followed by addition of xs. Mel (0.1 mL, 1.5 mmol). The mixture was stirred at RT for 2 h and diluted in EtOAc. The organic solution was washed with water, saturated aqueous NaCl solution, dried (Na₂SO₄), and concentrated *in vacuo* to give crude **1022C** (117 mg). The crude **1022C** (67 mg, 0.28 mmol) was dissolved in CH₂Cl₂ (2 mL) and the solution was treated with PPh₃ (150 mg, 0.57 mmol) and CBr₄ (189 mg, 0.57 mmol) at RT. The mixture was stirred at RT for 1 h and concentrated *in vacuo.* The residue was purified by preparative TLC (CH₂Cl₂) to afford **1022D** (50 mg, 60% yield).

### Example 1023

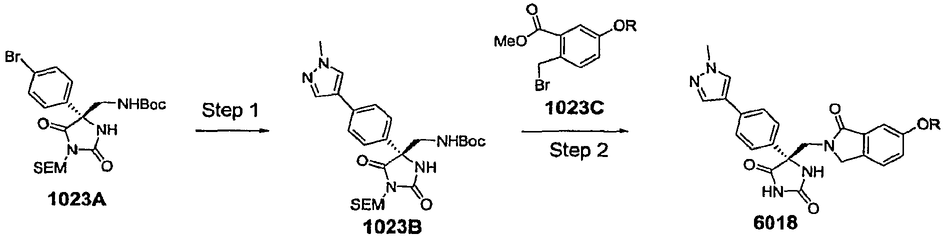

### Step 1

A mixture of **1023A** (370 mg, 0.72 mmol), 1-methyl-4-(4,4,5,5-tetramethyl-1,3,2-dioxaborolan-2-yl)-1H-pyrazole (225 mg, 1.08 mmol), potassium carbonate (1 M aqueous solution, 2.9 mL, 2.9 mmol), and [PdCl₂(dppf)]CH₂Cl₂ (59 mg, 0.072 mmol) in acetonitrile (12 mL) was vacuumed and refilled with argon three times. The reaction mixture was stirred at 80 °C oil bath for 17 hours. After cooling down, the mixture was diluted in EtOAc (50 mL) and filtered through a celite pad. The filtrate was concentrated *in vacuo* and the residual material was purified by silica gel column chromatography (1% to 1.5% MeOH in CH₂Cl₂) to afford compound **1023B** (369 mg, 99% yield).

### Step 2

A solution of **1023B** (109 mg, 0.21 mmol) in EtOAc/MeOH (4/1, 5 mL) was treated with 4N HCl in dioxane (2 mL). The reaction mixture was stirred at RT for 15 h and concentrated *in vacuo.* The residue was dissolved in DMF (1 mL) and treated with **1022D** (75 mg, 0.25 mmol, R = CH₂CCCH₃) and diisopropylethyl amine (0.22 mL, 1.26 mmol). The mixture was stirred at 60 °C for 9.5 h and concentrated *in vacuo.* The residue was dissolved in MeOH (5 mL) and treated with 4N HCl in dioxane (1 mL) at 70 °C for 17 h in a pressure vessel. After cooling to RT, the mixture was concentrated and the residue was treated with ammonia in MeOH for 0.5 h. The precipitate was filtered off and the filtrate was concentrated. The residue was dissolved in DMF (3-4 mL) and purified by reverse phase column chromatography (0.01% HCO₂H in water-0.01% HCO₂H in acetonitrile) to afford **6018** (48 mg, 49% yield).

### Example 1024

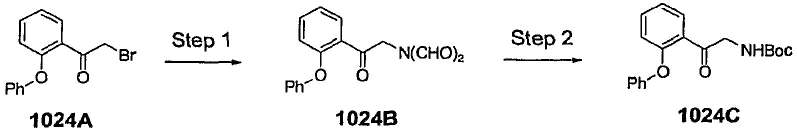

### Step 1

A mixture of **1024A** (811 mg, 2.8 mmol) and sodium diformyl amide (291 mg, 3.0 mmol) in acetonitrile (15 mL) was stirred at RT for 19 h. The resulting suspension was filtered through celite and the filtrate was concentrated *in vacuo.* The residue was purified by SiO₂ column chromatography (CH₂Cl₂) to give **1024B** (611 mg, 77% yield).

### Step 2

A solution of **1024B** (611 mg, 2.16 mmol) in EtOH (40 mL) was treated with 4N HCl in dioxane (8 mL) at RT. The resulting solution was stirred at RT for 16 h and concentrated *in vacuo.* The residue was dissolved in dioxane/water (5/1, 24 mL) and the solution was stirred at RT for 2 h. The mixture was added to water and the organic layers were extracted with CH₂Cl₂ and the combined organic solution was washed with saturated aqueous NaCl solution, dried (Na₂SO₄), and concentrated *in vacuo.* The residue was purified by SiO₂ column chromatography (CH₂Cl₂/hexane=1/1 to CH₂Cl₂ only) to give **1024C** (679 mg, 96% yield).

### Example 1025

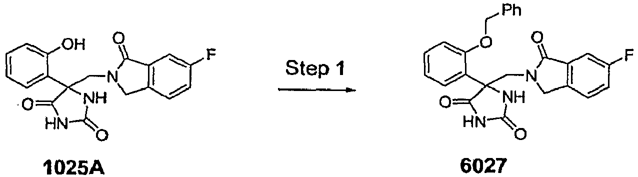

### Step 1

A mixture of **1025A** (52 mg, 0.11 mmol), benzyl bromide (13 □L, 0.11 mmol), and cesium carbonate (108 mg, 0.33 mmol) in DMF (0.5 mL) was stirred at RT for 2 h. The mixture was diluted in EtOAc and the organic solution was washed with water, saturated aqueous NaCl solution, dried (Na₂SO₄), and concentrated *in vacuo.* The residue was purified by preparative TLC (5% MeOH in CH₂Cl₂ ) to give **6027** (34 mg, 54% yield).

### Example 1026 -

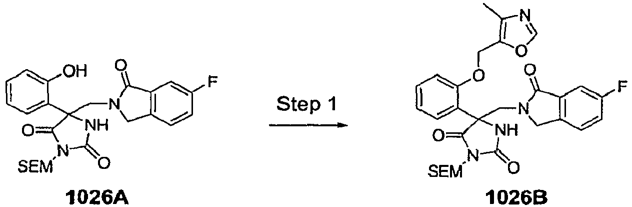

### Step1

To a solution of **1026A** (41 mg, 0.084 mmol), 5-hydroxymethyl-4-methyl-1,3-oxazole (19 mg, 0.17 mmol), and PPh₃ (66 mg, 0.25 mmol) in THF (1 mL) was added diisopropy azodicarboxylate (50 □L, 0.25 mmol) dropwisely at 0 °C. The mixture was stirred at 0 °C for 10 min and was allowed to warm to RT. After stirring for 2 h at RT, the mixture was concentrated *in vacuo* and the residue was purified by preparative TLC (CH₂Cl₂) to afford **1026B** (38 mg, 77% yield).

### Example 5021

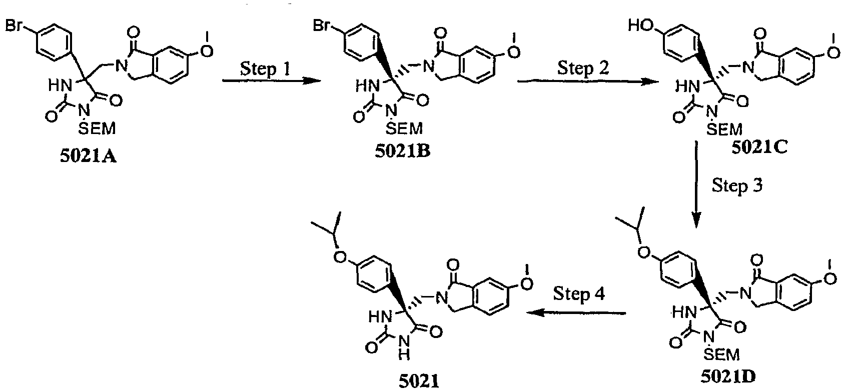

Compound **5021 A** was prepared using chemistry described in Examples 14, 1001, and 1008.

### Step 1

Compound **5021A** was resolved by Chiralcel OD column (Mobile phase: Hexane:2-propanol 4:1). The first peak was collected and concentrated to give compound **5021B.**

### Step 2

Compound **5021B** (1.82 g, 3.25 mmol), bis(pinacolato)diboron (2.89 g, 11.4 mmol), potassium acetate (1.5 g, 15 mmol), and [PdCl₂(dppf)]CH₂Cl₂ (0.27 g, 0.3 mmol) were added to a roundbottomed flask and placed under N₂. The flask was cycled three times between vacuum and nitrogen. Dioxane (30 mL, Aldrich anhydrous) was added via syringe. The reaction mixture was stirred at 80 °C (oil bath) for 4 hours. The reaction mixture was allowed to cool to rt. Water (30 mL) was added, followed by sodium perborate-(5.0g, 32 mmol). The reaction mixture was left stirring overnight at room temperature. The resulting mixture was diluted with EtOAc, washed with water and brine, dried with MgSO₄, and concentrated to dryness. The crude product was purified via flash silica gel chromatography using a 30% to 100% EtOAc-Hexanes gradient as the mobile phase. A white solid (1.28 g )was collected as product **5021C.**

### Step 3

Compound **5021C** (40 mg, 0.12 mmol), cesium carbonate ( 59 mg, 1.5 eq), and DMF (1 mL) were added to a round bottomed flask. The flask was sonicated for 30 min. 2-bromopropane (18 mg, 1.2 eq) was added and the reaction mixture was stirred at rt overnight. The resulting mixture was diluted with EtOAc, washed with water, dried with MgSO₄, and concentrated to dryness. The crude product was purified via flash chromatography using a 10% to 100% EtOAc-Hexanes gradient as the mobile phase. Compound **5021 D** (28mg) was obtained as product.

### Step 4

Compound **5021 D** was converted to compound 5021 using procedures similar to those described in Example 1001.

### Example 7000

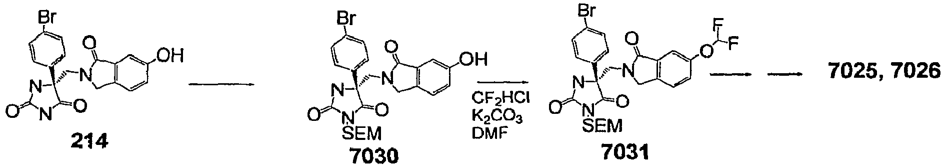

**7025** was synthesized from **7031** using appropriate heterocyclic bromide using the procedure similar to synthesis of **169.**

**7031** was prepared from **7030** using the procedure described below:

**7030** (0.273 g, 0.5 mmol) in 5 mL anhydrous DMF was treated with potassium carbonate (0.2 g, 0.15 mmol). The flask was equipped with a dry ice acetone trap and difluoro choro methane gas was bubbled for 2 h. The bubbling was stopped and excess reagent was removed by bubbling nitrogen. The reaction was diluted with 50 mL ethyl acetate and washed with water (2 X 50 mL) and brine (1x25 mL). The organics were dried and concentrated to yield a crude which was purified by silica-gel prep plate chromatography using 1:1 ethyl acetate: hexane to yield 0.0 37 g of pure product.

**7030** itself was prepared from **214,** using standard procedure reported previously in this case.

### Example 8001:

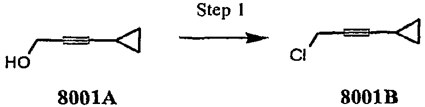

Compound **8001B** was prepared according to a literature procedure (Munyemana, F.; Frisque-Hesbain, A.; Devos, A.; and Ghosez, L. Tetrahedron Letters 30(23), 3077 - 3080, 1989).

### Example 8002:

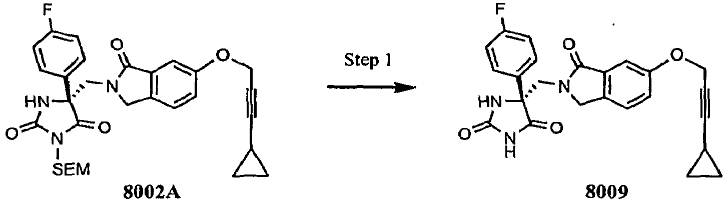

Compound **8002A** (746 mg, 1.32 mmol) was dissolved in anhydrous acetonitrile (10 mL) and the solution was colled to 0 °C by ice water bath. BF₃-Et₂O (0.84 mL, 6.62 mmol) was added dropwise via syringe. The solution was stirred at 0 °C for two hours. DIPEA (1 mL) was added followed by NaOH (1N, 1 mL). The solution was stirred at 25 °C for two hours. The solvent was removed and the product was purified by C18 reverse phase chromatography (CH₃CN/water, 5% to 90%, with 0.5% HCO₂H) to give **8009. 8009** was dissolved in methanol and NaOH (1N, 1.0 mL, 1.0 mmol, 0.95 equivalent) was added. The solution was stirred at 25 °C for 30 minutes. The solvent was removed to give the sodium salt form of **8009** (495 mg).

### Example 2021:

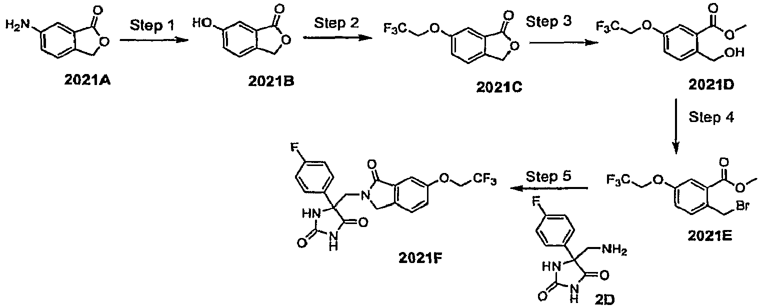

### Step 1:

To a solution of compound **2021A** (4 g, 26.8 mmol) in water (25 mL) and concentrated sulfuric acid (1 mL) was added sodium nitrite (2.2 g, 31.8 mmol) in water (10 mL) with ice bath cooling. The reaction mixture was diluted with concentrated sulfuric acid (20 mL). The reaction mixture was added to 50% sulfuric acid (50 mL) at reflux and boiled for 2 minutes. The reaction mixture was cooled to room temperature and diluted with water (250 mL). The mixture was extracted with diethyl ether (5 × 100 mL). The combined organic layers were washed with sodium bicarbonate solution and brine, dried over sodium sulfate and concentrated to afford **2021B** (1.6 g) as a yellow solid.

### Step 2:

A mixture of compound **2021B** (790 mg, 5.3 mmol), cesium carbonate (1.90 g, 5.8 mmol) and 2,2,2-trifluoroethyl trifluoromethanesulfonate (1.47 g, 6.3 mmol) in NMP (15 mL) was stirred overnight at room temperature. The reaction mixture was filtered and the solids were washed with ethyl acetate. The filtrate was poured into water and extracted with ethyl acetate. The combined organic layers were dried over sodium sulfate and concentrated. Recrystallization from 30% ethyl acetate/hexane afford **2021C** (728 mg) as a yellow solid. HPLC-MS t_{R} = 1.54 min (UV_{254 nm}); mass calculated for formula C10H7F3O3 232.03, observed LCMS m/z 233.1 (M+H).

### Step 3:

A suspension of **2021C** (168 mg, 0.72 mmol) and 1.0 N sodium hydroxide (0.72 mL, 0.72 mmol) in water (0.8 mL) was heated at 100 °C for 1 hour. The reaction mixture was concentrated and the residue was azeotroped with toluene. The sodium salt was dissolved in DMF (1 mL) and methyl iodide (0.135 mL, 2.16 mmol) was added. The reaction mixture was stirred for 2 hours at room temperature. The reaction mixture was diluted with ethyl acetate and water. The layers were separated and the aqueous layer was extracted with ethyl acetate. The combined organic layers were washed with brine, dried over sodium sulfate and concentrated. Purification by chromatography (SiO₂, 30% ethyl acetate/hexane) afforded **2021D** (149 mg) as a solid. HPLC-MS t_{R} = 1.56 min (UV_{254 nm}); mass calculated for formula C11H11F3O4 264.06, observed LCMS m/z 287.1 (M+Na).

### Step 4:

A mixture of **2021D** (149 mg, 0.56 mmol), carbon tetrabromide (371 mg, 1.12 mg), and triphenylphosphine (294 mg, 1.12 mmol) in dichloromethane (5 mL) was stirred for 40 minutes at room temperature. The mixture was concentrated and purified by chromatography (SiO₂, 5% to 10% ethyl acetate/hexane) to afford **2021E** (153) mg as an oil. HPLC-MS t_{R} = 2.04 min (UV_{254 nm}); mass calculated for formula C11H10BrF303 325.98, observed LCMS m/z 349 (M+Na).

### Step 5:

Compound **2021F** (136 mg) was prepared from **2021E** (151 mg, 0.46 mmol) and **2D** (118 mg, 0.45 mmol) using previously described procedures. HPLC-MS t_{R} = 1.60 min (UV_{254 nm}); mass calculated for formula C20H15F4N3O4 437.1, observed LCMS m/z 438.1 (M+Na).

### Example 2022:

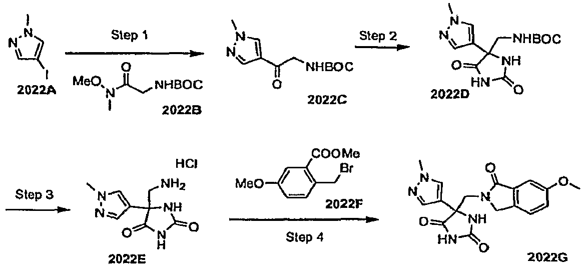

### Step 1:

Compound **2022C** was prepared according to a modification of a procedure by Felding, J. et al. (*J*. *Org. Chem.* **1999,** *64*, 4196-4198) using 4-Iodo-1-methyl-1H-pyrazole **2022A** and Weinreb amide **2022B** as starting materials. The crude reaction mixture was chromatographed (SiO₂, 60% - 80% ethyl acetate/hexane) to give compound **2022C** (62%). HPLC-MS t_{R} = 1.18 min (UV_{254 nm}); mass calculated for formula C11H17N3O3 239.1, observed LCMS m/z 184.1 (M-tBu+H).

### Step 2:

BOCamino hydantoin **2022D** was prepared using procedures described in Example 1, Step 2. (81%) HPLC-MS t_{R} = 0.94 min (UV_{254 nm}): mass calculated for formula C13H19N5O4 309.1, observed LCMS m/z 310.1 (M+H).

### Step 3:

Amino hydantoin **2022E** was prepared using procedures described in Example 1, Step 3. HPLC-MS t_{R} = 0.18 min (UV_{254 nm}); mass calculated for formula C8H11N5O2 209.1, observed LCMS m/z 210.1 (M+H).

### Step 4:

Hydantoin **2022G** was prepared using procedures described in Example 8. HPLC-MS t_{R} = 2.23 min (UV_{254 nm}; 10 min); mass calculated for formula C17H17N5O4 355.1, observed LCMS m/z 356.1 (M+H).

### Example 2023:

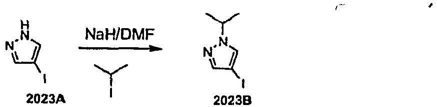

### Step 1:

To a slurry of sodium hydride (95%, 0.58 g, 23 mmol) in DMF (20 mL) was added a solution of 4-Iodo-1H-pyrazole (2023A) (4.07 g, 21 mmol) in DMF (20 mL) and the resulting mixture was stirred at rt for 10 min. Then 2-iodopropane (2.52 mL, 25.2 mmol) was added dropwise and the resulting mixture was stirred at rt overnight. The reaction mixture was concentrated, diluted with ethyl acetate, washed with water (4 times), brine, dried and concentrated to give an oily residue which was chromatographed (SiO₂, 10% - 20% ethyl acetate/hexane) to give 4-Iodo-1-isopropyl-1H-pyrazole **2023B** (3.27 g, 66%). HPLC-MS t_{R} = 1.66 min (UV_{254 nm}); mass calculated for formula C6H9lN2 235.98, observed LCMS m/z 237.0 (M+H).

### Example 2024:

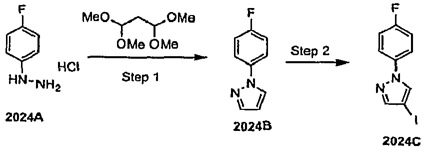

### Step 1:

Compound **2024B** was prepared according to a modification of a procedure by Roppe, J. et al. (*J. Med. Chem.* **2004,** *47*, 4645-4648) using 4-fluorophenylhydrazine hydrochloride **2024A** and malondialdehyde-bis-(dimethylacetal) as starting materials (95% yield). HPLC-MS t_{R} = 1.62 min (UV_{254 nm}); mass calculated for formula C9H7FN2 162.1, observed LCMS m/z 163.1 (M+H).

### Step 2:

Compound **2024C** was prepared according to a modification of a procedure by Rodriguez-Franco, M. I. et al. (Tetrahedron. Lett. 2001, 42, 863-865) (85% yield). HPLC-MS t_{R} = 1.98 min (UV_{254 nm}); mass calculated for formula C9H6FIN2 287.96, observed LCMS m/z 288.9 (M+H).

### Example 2025:

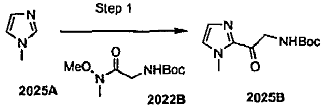

### Step 1:

Compound **2025B** was prepared according to a modification of a procedure by Evans, D.A. et al. (J. Am. Chem. Soc. 2005, 127, 8942-8943) using 1-Methyl-1H-imidazole **2025A** and Weinreb amide **2022B** as starting materials (42%). HPLC-MS t_{R} = 1.24 min (UV_{254 nm}); mass calculated for formula C11H17N3O3 239.1, observed LCMS m/z 240.1 (M+H).

The following compounds were prepared using procedures described in Examples 2021 to 2025.

| Compound # | Structure | Exact mass | MS m/e (M+H) |
|---|---|---|---|
| **2021F** | 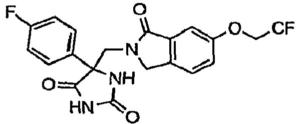 | 437.1 | 438.1 |
| **2023C** | 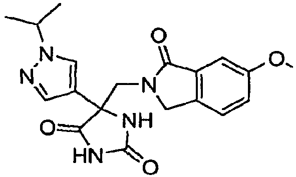 | 383.16 | 384.1 |
| **2024D** | 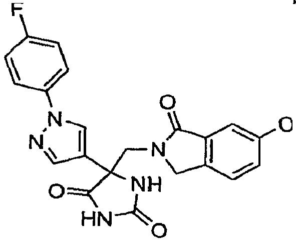 | 435.13 | 436.1 |
| **2022G** | 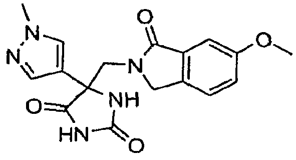 | 355.13 | 356.1 |
| **2025C** | 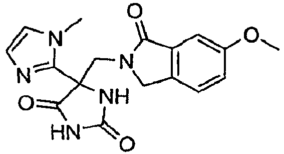 | 355.13 | 356.1 |

### Example 2026:

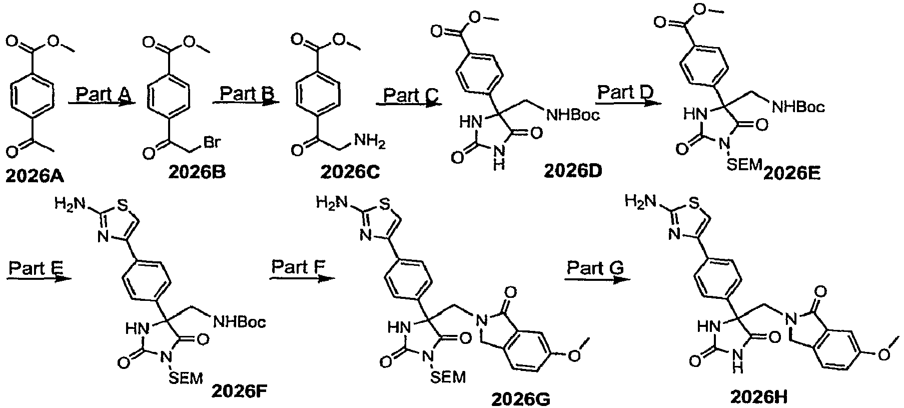

### Part A

To a solution of methyl 4-acetylbenzoate (2026A) (1.9 g, 10.6 mmol) in acetic acid (10 mL) was added dropwise bromine (1.7 g, 21.3 mmol). The mixture was heated at 60 °C for 30 min, then stirred at room temperature for 1 hour, and poured into cold water (30 mL). The light yellow precipitate was collected, washed with water and dried (2.6 g, 96%).

### Part B

Compound **2026C** was prepared from compound **2026B** following the procedure described in Example 1005.

### Part C

Compound **2026D** was prepared following the procedures described in Example 1: HPLC-MS t_{R} = 1.36 min (UV_{254 nm}); mass calculated for formula C17H21N306 363.1, observed LCMS m/z 386.0 (M+Na).

### Part D

To a mixture of **2026D** (7.87 g, 21.7 mmol) and diisopropylethylamine (7.5 mL, 43.4 mmol) in DMF (80 mL) was added 2-trimethylsilylethoxy methyl chloride (4.7 mL, 23.8 mmol). The mixture was stirred at room temperature overnight, diluted with water and extracted with ethyl acetate. The combined organic layers were washed with water, brine, dried over sodium sulfate and concentrated. The residue was purified by column chromatography (SiO₂, 15% EtOAc/hexane to 30 % EtOAc/hexane) to afford **2026E** as a white solid (10.2 g, 95%): HPLC-MS t_{R} = 2.17 min (UV_{254 nm}); mass calculated for formula C23H35N3O7Si 493.2, observed LCMS m/z 516.1 (M+Na).

### Part E

Compound **2026F** was prepared from ester **2026E** following a procedure as described in Guo, Z. et. al (WO 2005/121130A2).

### Part F

Compound **2026G** was prepared following a previously described procedure. HPLC-MS t_{R} = 1.67 min (UV_{254 nm}); mass calculated for formula C28H33N5O5SSi 579.2, observed LCMS m/z 580.3 (M+H).

### Part G

Compound **2026G** (65 mg, 0.11 mmol) was heated in a sealed tube in MeOH (2 mL) and 4 N HCl in 1,4-dioxane (2 mL) overnight at 90 °C. Solvent was evaporated and the residue was stirred in MeOH (2 mL) and triethylamine (2 mL) at room temperature for 4 hours. The solvent was removed and the residue was purified by reverse phase chromatography to give **2026H** (11 mg, 20%): HPLC-MS t_{R} = 1.00 min (UV_{254 nm}); mass calculated for formula C22H19N5O4S 449.1, observed LCMS m/z 450.1 (M+H).

| **Compound #** | Structure | Exact mass | MS m/e (M+H) |
|---|---|---|---|
| **2026H** | 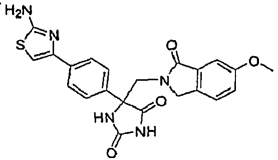 | 449.1 | 450.1 |

### Example 2030:

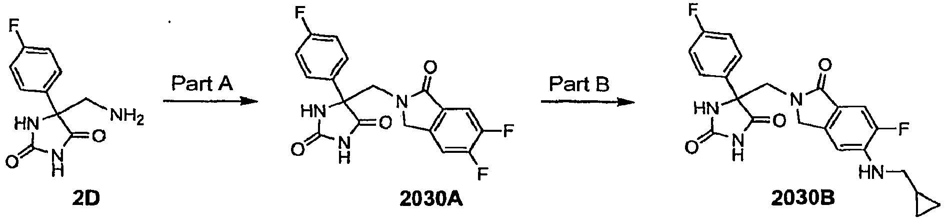

### Part A:

Compound **2030A** was prepared using previously described methods from **2D**. HPLC-MS t_{R} = 3.80 min (UV_{254 nm}), Mass calculated for formula C₁₈H₁₂F₃N₃O₃ 375.1, observed LCMS m/z 376.1 (M+H).

### Part B:

Compound **2030A** (100 mg, 0.27 mmol) and cyclopropylmethylamine (0.140 mL) in NMP (0.5 mL) was heated at 100 °C for 12 h. After cooling to room temperature, the reaction mixture was diluted with EtOAc, washed with water and brine, dried over Na₂SO₄, and concentrated. Recrystallization from EtOAc / hexane yielded **2030B** as a white solid (75 mg, 66%). HPLC-MS t_{R} = 4.06 min (UV_{254 nm}), Mass calculated for formula C₂₂H₂₀F₂N₄O₃ 426.2, observed LCMS m/z 427.1 (M+H).

### Example 2031:

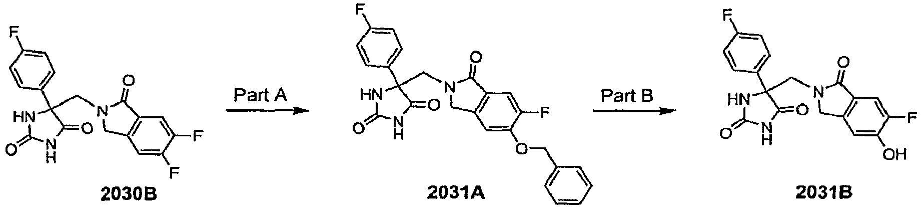

### Part A:

Compound **2030B** (250 mg, 0.67 mmol) in 1 mL of benzyl alcohol was added with powdered KOH (75 mg, 1.33 mmol). The reaction mixture was stirred at 100 °C for 2h and cooled to room temperature. It was diluted with EtOAc, washed with 1 N HCl, H₂O, and brine, dried over Na₂SO₄, and concentrated. Flash column chromatography with EtOAc afforded **2031A** as a white solid (280 mg, 91%). HPLC-MS t_{R} = 4.29 min (UV_{254 nm}), Mass calculated for formula C₂₅H₁₉F₂N₃O₄ 463.1, observed LCMS m/z 464.0 (M+H).

### Part B:

Compound **2031A** (250 mg, 0.54 mmol) in EtOH (30 mL) was added with 10% palladium on carbon (100 mg). The reaction mixture was stirred at room temperature for 2 h under 1 atmosphere of hydrogen. It was then filtered through celite, washed with EtOH, and concentrated, affording **2031B** as a white solid (120 mg, 60%). HPLC-MS t_{R} = 2.66 min (UV_{254 nm}), Mass calculated for formula C₁₈H₁₃F₂N₃O₄ 373.1, observed LCMS m/z 374.0 (M+H).

### Example 2032:

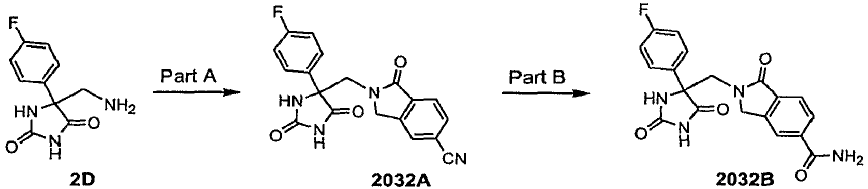

### Part A:

Compound **2032A** was prepared using previously described method from **2D.** HPLC-MS t_{R} = 2.94 min (UV_{254 nm}), Mass calculated for formula C₁₉H₁₃FN₄O₃ 364.1, observed LCMS m/z 365.0 (M+H).

### Part B:

Compound **2032A** (100 mg, 0.27 mmol) was dissolved in 2 mL of 90% H₂SO₄. After stirring at 60 °C for 10 h, the reaction mixture was poured into 50 g of ice, and white solid was precipitated. Filtration and drying under vacuum gave **2032B** as a white solid (80 mg, 78%). HPLC-MS t_{R} = 2.01 min (UV_{254 nm}), Mass calculated for formula C₁₉H₁₅FN₄O₆ 382.1, observed LCMS m/z 383.1 (M+H).

The following compounds were prepared as described in Examples 2030 to 2032.

| **Compound #** | Structure | Exact mass | MS m/e (M+H) |
|---|---|---|---|
| **2030A** | 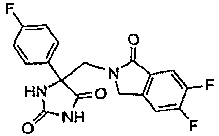 | 375.08 | 376.1 |
| **2030C** | 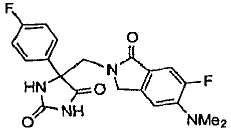 | 400.13 | 401.2 |
| **2030B** | 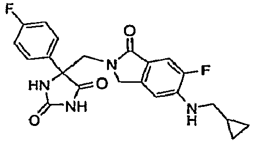 | 426.15 | 427.1 |
| **2031C** | 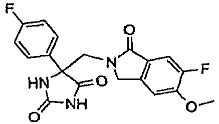 | 387.10 | 388.1 |
| **2031D** | 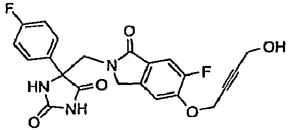 | 441.11 | 442.0 |
| **2030D** | 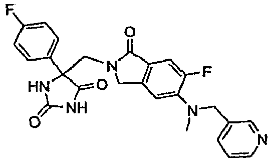 | 477.16 | 478.1 |
| **2031A** | 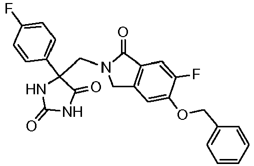 | 463.13 | 464.0 |
| **2031B** | 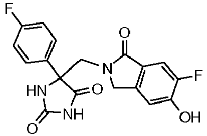 | 373.09 | 374.0 |
| **2032B** | 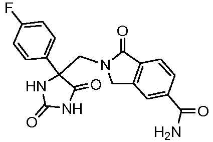 | 382.11 | 383.1 |
| **2031E** | 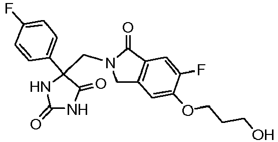 | 431.13 | 432.1 |
| **2031F** | 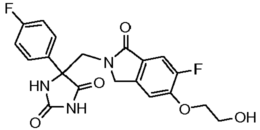 | 417.11 | 418.1 |

The Table below lists compounds prepared by the procedures set forth below: **Table 6000**

| **Compound ID** | **Structures** | **Exact Mass** | **Mass Obsvd** | **Ki (nM)** |
|---|---|---|---|---|
| **9200** | 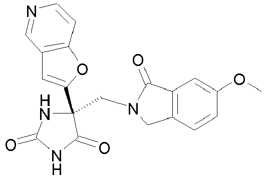 | **392.11** | **392.11** | **A** |
| **9205** | 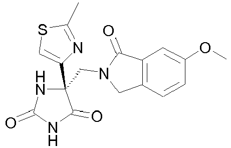 | **372.09** | **373.0[M+H]⁺** | **A** |
| **9206** | 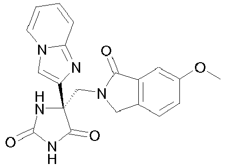 | **391.13** | **392.0[M+H]⁺** | **A** |
| **9207** | 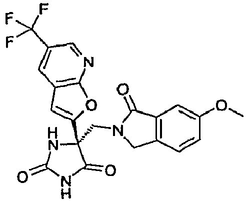 | **460.10** | **461.0[M+H]⁺** | **A** |
| **9208** | 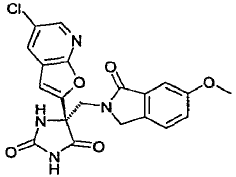 | **426.07** | **427.1 [M+H]⁺** | **A** |
| **9209** | 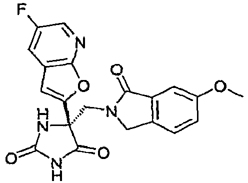 | **410.10** | **411.1[M+H]⁺** | **A** |
| **9210** | 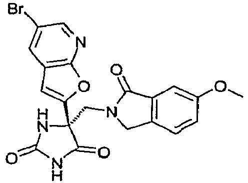 | **470.02** | **471.1[M+H]⁺** | **A** |
| **9211** | 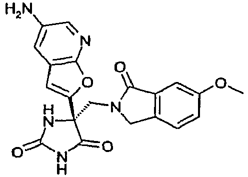 | **407.12** | **408.1[M+H]**⁺ | **A** |
| **9212** | 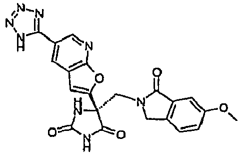 | **460.10** | **461.2[M+H]⁺** | **A** |
| **9213** | 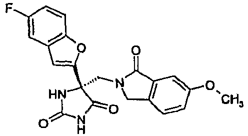 | **409.37** | **410.1 [M+H]⁺** | **A** |
| **9214** | 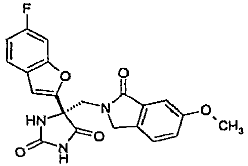 | **409.37** | **410.1[M+H]**⁺ | **A** |
| **9215** | 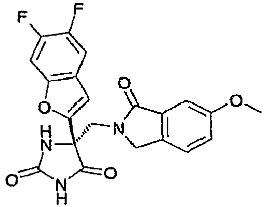 | **427.10** | **428.0[M+H]⁺** | **A** |
| **9216** | 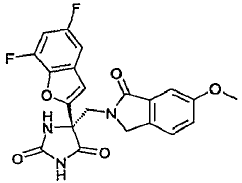 | **427.10** | **428.0[M+H]⁺** | **A** |
| **9217** | 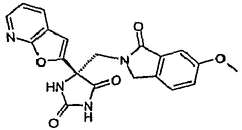 | **392.11** | **393.1[M+H]⁺** | **A** |
| **9218** | 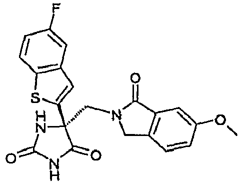 | **425.08** | **426.1[M+H]⁺** | **A** |
| **9219** | 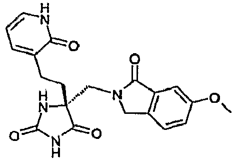 | **396.14** | **397.1[M+H]⁺** | **A** |
| **9220** | 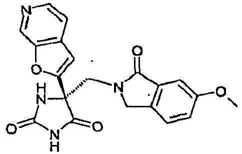 | **392.11** | **393.1[M+H]**⁺ | **A** |
| **9221** | 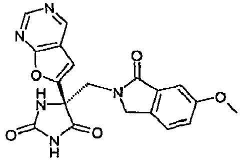 | **393.11** | **394.1[M+H]⁺** | **A** |
| **9222** | 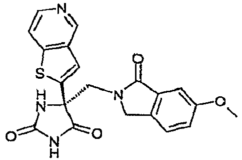 | **408.09** | **409.1[M+H]⁺** | **A** |
| **9223** | 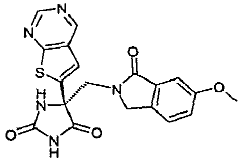 | **409.08** | **410.2[M+H]⁺** | **A** |
| **9224** | 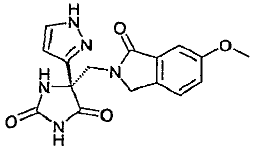 | **341.11** | **342.1[M+H]⁺** | **A** |
| **9225** | 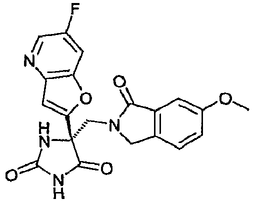 | **410.10** | **411.1[M+H]⁺** | **A** |
| **9226** | 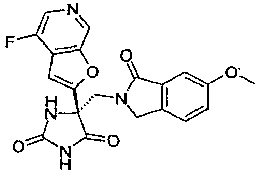 | **410.10** | **411.1[M+H]⁺** | **A** |
| **9227** | 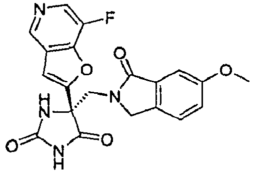 | **410.10** | **411.1[M+H]⁺** | **A** |
| **9228** | 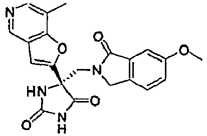 | **406.13** | **407.1[M+H]⁺** | **A** |
| **9229** | 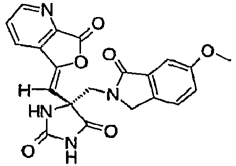 | **420.11** | **421.1[M+H]**⁺ | **B** |
| **9230** | 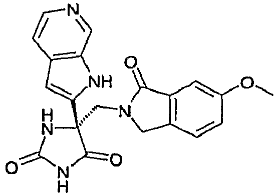 | **391.13** | **392.2[M+H]⁺** | **A** |
| **9231** | 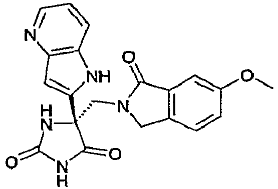 | **391.13** | **392.2[M+H]⁺** | **A** |
| **9232** | 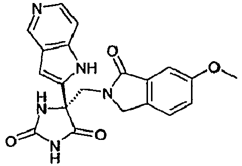 | **391.13** | **392.2[M+H]⁺** | **A** |
| **9233** | 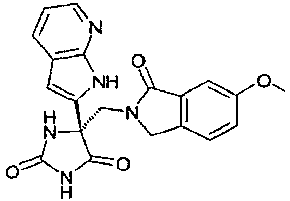 | **391.13** | **392.2[M+H]⁺** | **A** |
| **9234** | 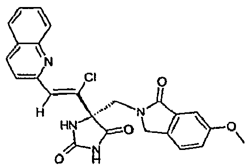 | **462.11** | **463.1[M+H]⁺** | **A** |
| **9235** | 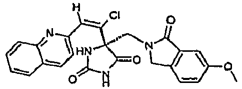 | **462.11** | **463.1[M+H]⁺** | **A** |
| **9236** | 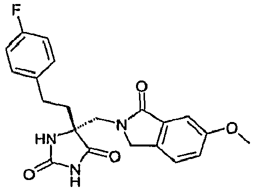 | **397.40** | **398.1[M+H]⁺** | **A** |
| **9237** | 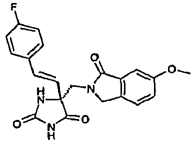 | **395.13** | **396.2 [M+H]⁺** | **A** |
| **9238** | 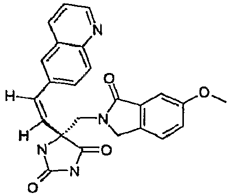 | **419.19** | **420.2[M+H]⁺** | **A** |
| **9239** | 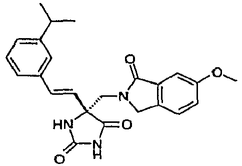 | **419.18** | **420.2[M+H]⁺** | **A** |
| **9240** | 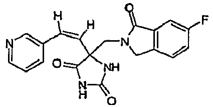 | **366.3** | **367.2[M+H]⁺** | **B** |
| **9241** | 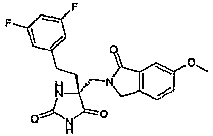 | **415.13** | **416.2 [M+H]⁺** | **A** |
| **9242** | 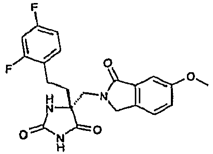 | **415.13** | **416.2 [M+H]⁺** | **A** |
| **9243** | 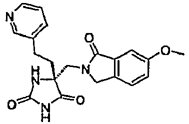 | **380.15** | **381.2 [M+H]⁺** | **A** |
| **9244** | 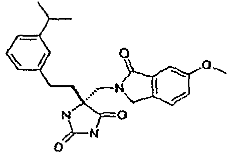 | **421.20** | **422.2[M+H]⁺** | **A** |
| **9245** | 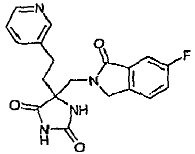 | **368.4** | **389.99[M+H]⁺** | **B** |
| **9246** | 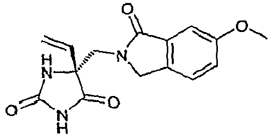 | **301.1** | 302.2 | **B** |
| **9247** | 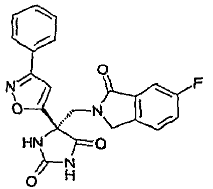 | **406.4** | **407.1[M+H]⁺** | **A** |
| **9248** | 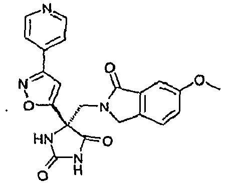 | **419.4** | **420.1[M+H]⁺** | **A** |
| **9249** | 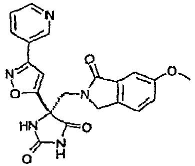 | **419.4** | **420.2[M+H]⁺** | **A** |
| **9250** | 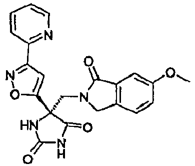 | **419.4** | **420.2[M+H]⁺** | **A** |
| **9251** | 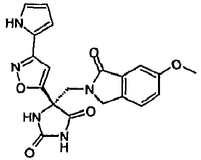 | **407.4** | **408.2[M+H]⁺** | **A** |
| **9252** | 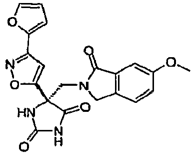 | **408.4** | **409.2[M+H]⁺** | **A** |
| **9253** | 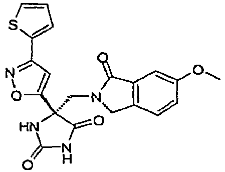 | **424.4** | **425.2[M+H]⁺** | **A** |
| **9254** | 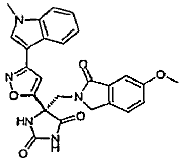 | **471.5** | **472.3[M⁺H]⁺** | **A** |
| **9255** | 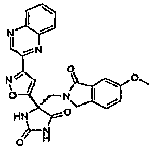 | **470.4** | **471.3[M+H]⁺** | **A** |
| **9256** | 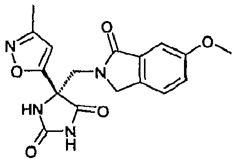 | **356.3** | **357.2[M+H]⁺** | **A** |
| **9257** | 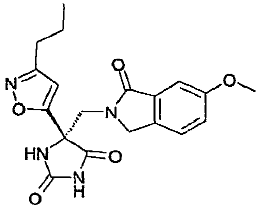 | **384.9** | **385.2[M+H]⁺** | **A** |
| **9258** | 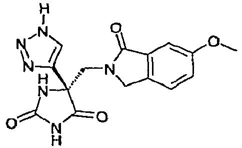 | **342.3** | **343.2[M+H]⁺** | **B** |
| **9259** | 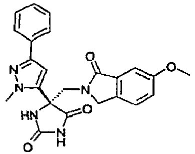 | **431.4** | **432.2[M+H]⁺** | **A** |
| **9260** | 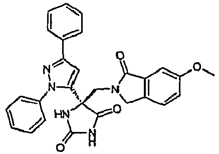 | **493.5** | **494.3[M+H]⁺** | **B** |
| **9261** | 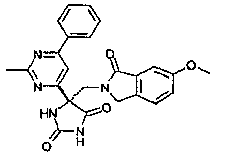 | **443.5** | **444.2[M+H]⁺** | **A** |
| **9262** | 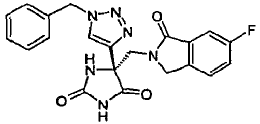 | **420.4** | **421.2[M+H]⁺** | **B** |
| **9263** | 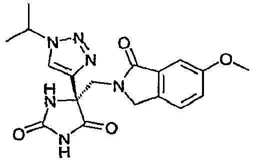 | **384.4** | **385.2[M+H]⁺** | **A** |
| **9264** | 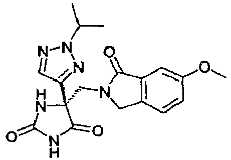 | **384.4** | **385.2[M+H]⁺** | **A** |
| **9265** | 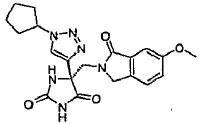 | **410.4** | **411.2[M+H]⁺** | **A** |
| **9266** | 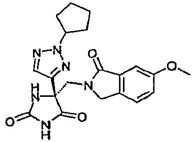 | **410.4** | **411.2[M+H]⁺** | **A** |
| **9267** | 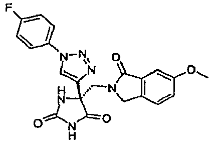 | **436.4** | **437.2[M+H]⁺** | **A** |
| **9268** | 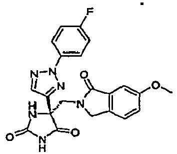 | **436.4** | **437.2[M+H]⁺** | **A** |
| **9269** | 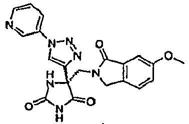 | **419.4** | **420.2[M+H]⁺** | **A** |
| **9270** | 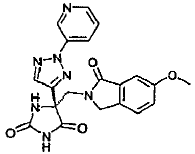 | **419.4** | **420.2[M+H]^{*}** | **A** |
| **9271** | 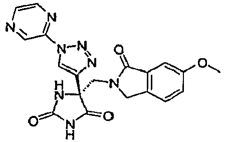 | **420.4** | **421.2[M+H]⁺** | **A** |
| **9272** | 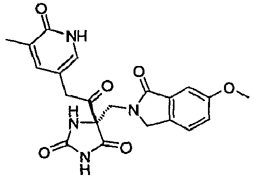 | **424.40** | **425.5[M+H]⁺** | **A** |
| **9273** | 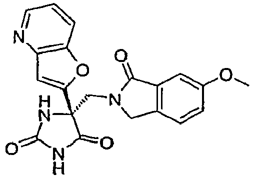 | **392.36** | **393.4[M+H]⁺** | **A** |
| **9274** | 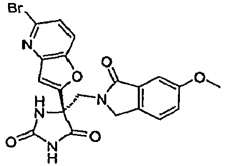 | **471.26** | **472.3[M+H]⁺** | **A** |
| **9275** | 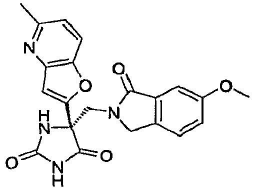 | **406.39** | **407.4[M+H]⁺** | **A** |
| **9276** | 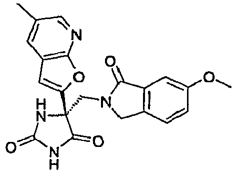 | **406.39** | **407.5[M+H]⁺** | **A** |
| **9277** | 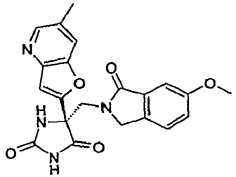 | **406.39** | **407.5[M+H]⁺** | **A** |
| **9278** | 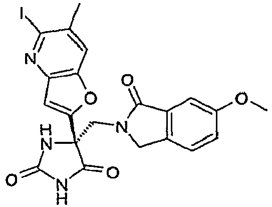 | **532.29** | **533.4[M+H]⁺** | **A** |
| **9500** | 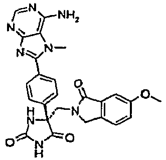 | **498.18** | **499.3[M+H]⁺** | **A** |
| **9501** | 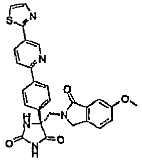 | **511.13** | **512**.**3[M**+**H**]⁺ | **A** |
| **9502** | 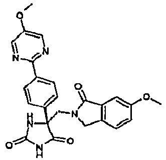 | **459.15** | **460.3[M+H]⁺** | **A** |
| **9504** | 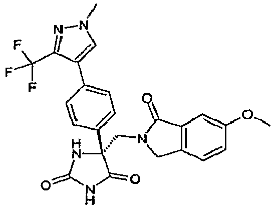 | **499.15** | **500.3[M+H]⁺** | **A** |
| **9505** | 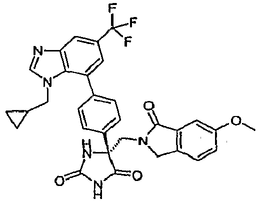 | **589.19** | **590.3[M+H]⁺** | **A** |
| **9506** | 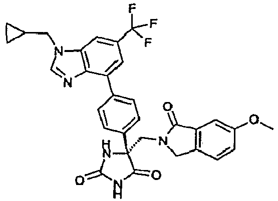 | **589.19** | **590.3[M+H]⁺** | **A** |
| **9507** | 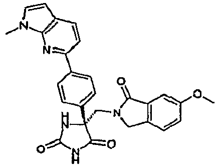 | **481.18** | **482**.**3[M**+**H]⁺** | **A** |
| **9508** | 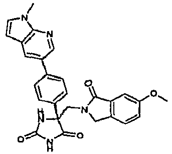 | **481.18** | **482**.**3**[**M**+**H]⁺** | **A** |
| **9509** | 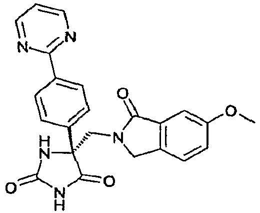 | **429.14** | **430.2[M+H]⁺** | **A** |
| **9510** | 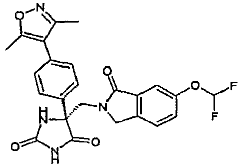 | **482.14** | **483.3[M+H]⁺** | **A** |
| **9511** | 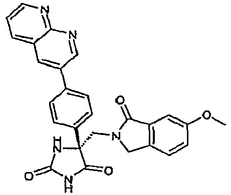 | **479.16** | **480.3** | **A** |
| **9512** | 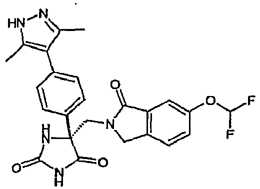 | **481.16** | **482.3[M+H]⁺** | **A** |
| **9513** | 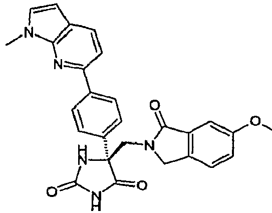 | **481.18** | **482.3[M+H]⁺** | **B** |
| **9514** | 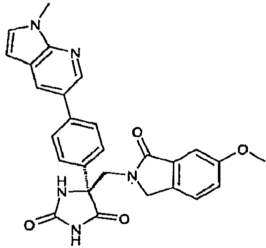 | **481.18** | **482.3[M+H]⁺** | **B** |
| **9515** | 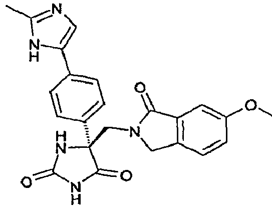 | **431.16** | **432.2[M+H]⁺** | **C** |
| **9516** | 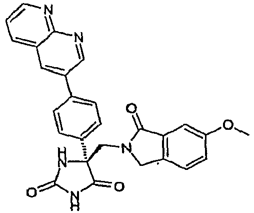 | **479.16** | **480.3[M+H]⁺** | **N/A** |
| **9517** | 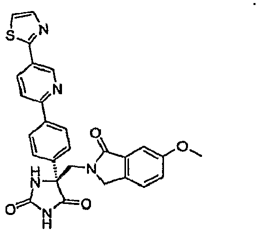 | **511.13** | **512.3[M+H]⁺** | C |
| | | | | |
| **9901** | 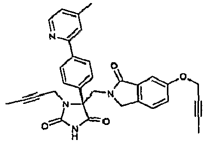 | 532.59 | 533.3 [M+H]⁺ | C |
| **9902** | 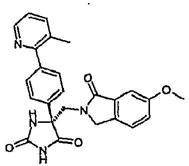 | 442.47 | 443.2 [M+H]⁺ | A |
| **9906** | 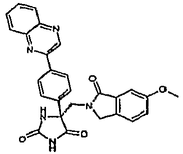 | 479.49 | 480.3 [M+H]⁺ | A |
| **9908** | 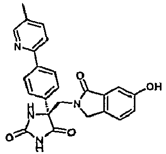 | 428.44 | 429.2 [M+H]⁺ | C |
| **9909** | 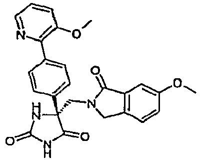 | 458.47 | 459.3 [M+H]⁺ | A |
| **9913** | 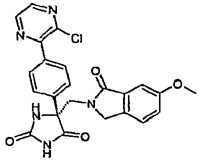 | 463.87 | 464.3 [M+H]⁺ | A |
| **9914** | 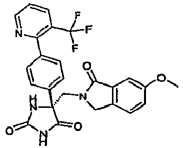 | 496.44 | 497.3 [M+H]⁺ | A |
| **9915** | 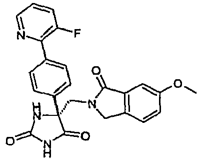 | 446.43 | 447.2 [M+H]⁺ | A |
| **9916** | 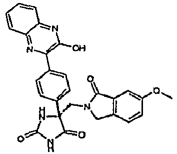 | 495.49 | 479.3 [(M-OH)+H]⁺ | A |
| **9923** | 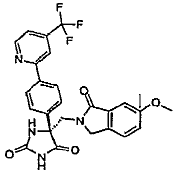 | 496.44 | 497.3 [M+H]⁺ | A |
| **9928** | 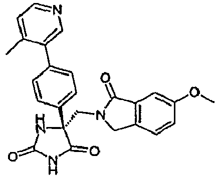 | 442.47 | 443.2 [M+H]⁺ | A |
| **9520** | 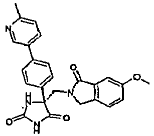 | 442.16 | 443.2 [M+H]⁺ | A |
| **9521** | 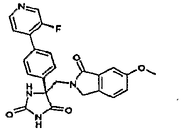 | 446.14 | 447.2 [M+H]⁺ | A |
| **9522** | 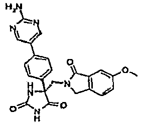 | 444.15 | 445.2 [M+H]⁺ | A |
| **9523** | 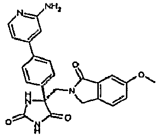 | 443.16 | 444.2 [M+H]⁺ | A |
| **9636** | 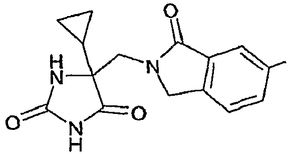 | **315.1** | **316.2**[M+H]⁺ | **B** |
| **9637** | 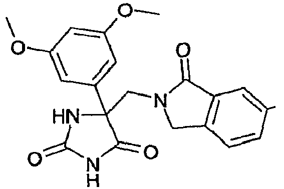 | **411.1** | **412.2**[M+H]⁺ | **A** |
| **9700** | 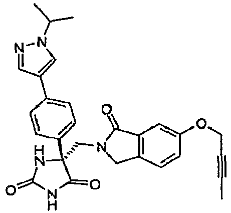 | 497.54 | **498.5[M+H]⁺** | A |
| **9701** | 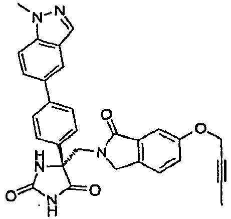 | 519.55 | **520.5[M+H]⁺** | A |
| **9702** | 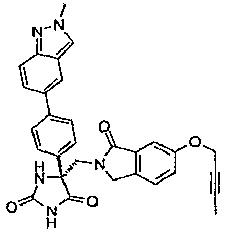 | 519.55 | **520.5[M+H]⁺** | A |
| 9703 | 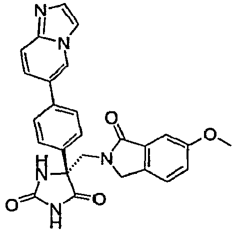 | 467.47 | **468.5[M+H]⁺** | A |
| 9704 | 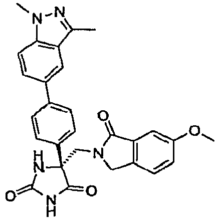 | 495.52 | **496.5[M+H]⁺** | A |
| 9705 | 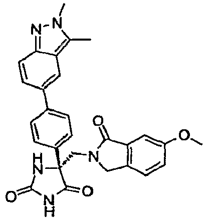 | 495.52 | **496.5[M+H]⁺** | A |
| 9706 | 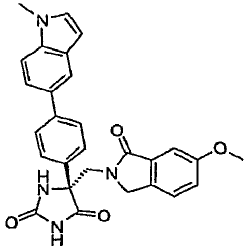 | 480.51 | **481.5[M+H]⁺** | A |
| **9707** | 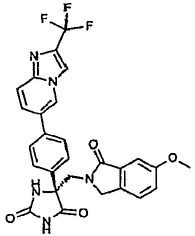 | 535.47 | **536.4[M+H]⁺** | A |
| **9708** | 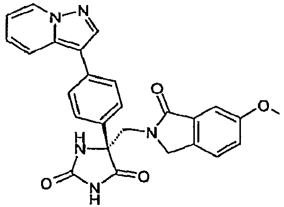 | 467.47 | **468.5[M+H]⁺** | A |
| **9709** | 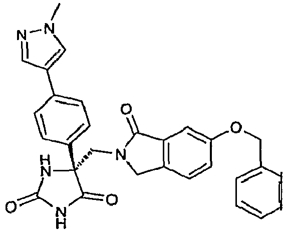 | 507.53 | **508.5[M+H]⁺** | A |
| **9710** | 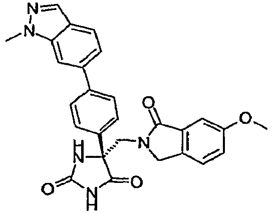 | 481.50 | **482.5[M+H]⁺** | A |
| 9711 | 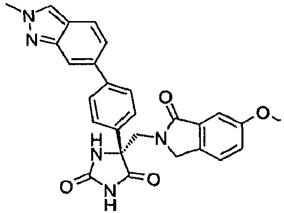 | 481.50 | **482.5[M+H]⁺** | A |
| 9712 | 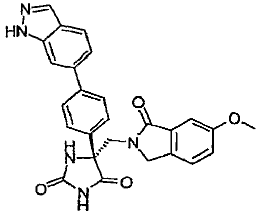 | 467.47 | **468.4[M+H]⁺** | A |
| 9714 | 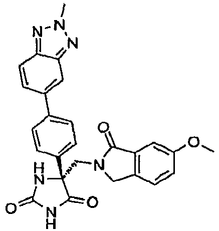 | 482.49 | **483.5[M+H]⁺** | A |
| 9715 | 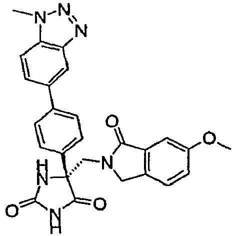 | 482.49 | **483.5[M+H]⁺** | A |
| 9716 | 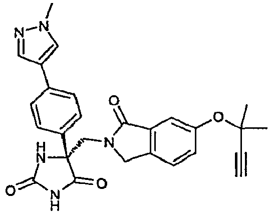 | 483.51 | **484.5[M+H]⁺** | C |
| 9717 | 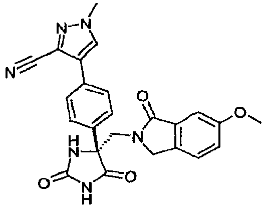 | 456.45 | **457.5[M+H]⁺** | A |
| **9730** | 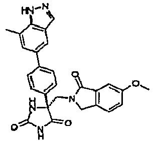 | **481.5** | **482.3[M+H]⁺** | A |
| **9731** | 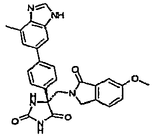 | **481.5** | **482**.**3**[**M**+**H]⁺** | A |
| **9732** | 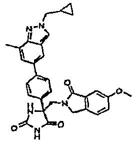 | **535.6** | **536.3[M+H]⁺** | **A** |
| **9733** | 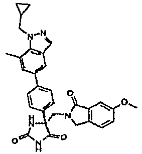 | **535.6** | **536.3[M+H]⁺** | **B** |
| **9734** | 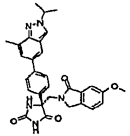 | **523.6** | **524.3[M+H]⁺** | **A** |
| **9735** | 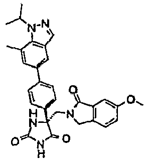 | **523.6** | **524.3[M+H]⁺** | **A** |
| **9736** | 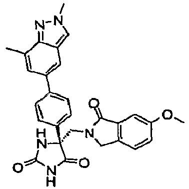 | **495.5** | **496.3[M+H]⁺** | **A** |
| **9737** | 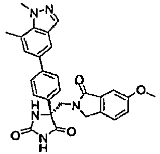 | **495.5** | **496.3[M+H]⁺** | **A** |
| **9738** | 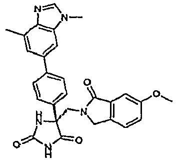 | **495.5** | **496.3[M+H]⁺** | **A** |
| **9739** | 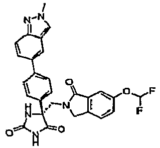 | **517.5** | **518.3[M+H]⁺** | **A** |
| **9740** | 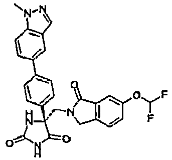 | **517.5** | **518.3[M+H]⁺** | **A** |
| **9741** | 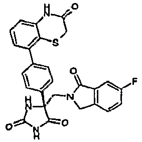 | **502.5** | **380.2[M+H]⁺** | **A** |
| **9742** | 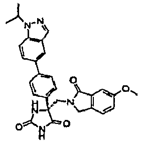 | **509.6** | **380.2[M+H]⁺** | **A** |
| **9743** | 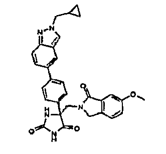 | **521.6** | **380.2[M+H]⁺** | **A** |
| **9744** | 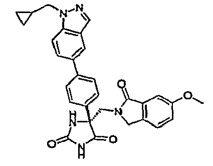 | **521.6** | **380.2[M+H]⁺** | **A** |
| **9745** | 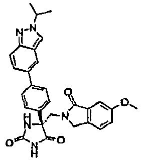 | **509.6** | **380.2[M+H]⁺** | **A** |
| **9746** | 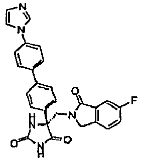 | **481.5** | **380.2[M+H]⁺** | **A** |
| **9748** | 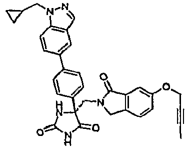 | **559.6** | **380.2[M+H]⁺** | **B** |
| **9749** | 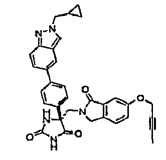 | **559.6** | **380.2[M+H]⁺** | **A** |
| **9750** | 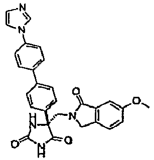 | **493.5** | **494.3[M+H]⁺** | **A** |
| **9751** | 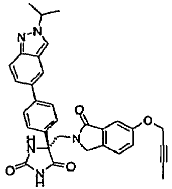 | **547.6** | **548.3[M+H]⁺** | **A** |
| **9752** | 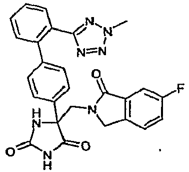 | **497.5** | **498.3[M+H]⁺** | **A** |
| **9753** | 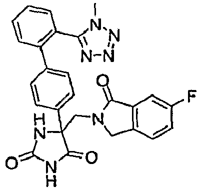 | **497.5** | **498.3[M+H]⁺** | **B** |
| **9754** | 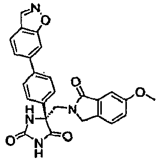 | **468.5** | **469.3[M+H]⁺** | **A** |
| **9755** | 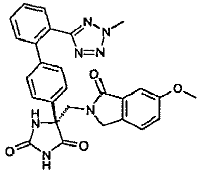 | **509.5** | **510.3[M+H]⁺** | **A** |
| **9756** | 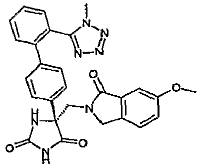 | **509.5** | **510.3[M+H]⁺** | **A** |
| **9757** | 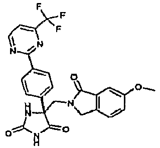 | **497.4** | **498.3[M+H]⁺** | **A** |
| **9758** | 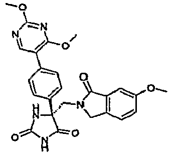 | **489.5** | **490.3[M+H]⁺** | **A** |
| **9759** | 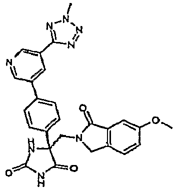 | **510.5** | **511.3[M+H]⁺** | **A** |
| **9760** | 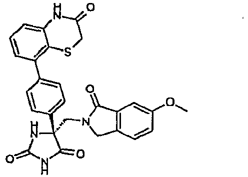 | **514.6** | **515.3[M+H]⁺** | **A** |
| **9600** | 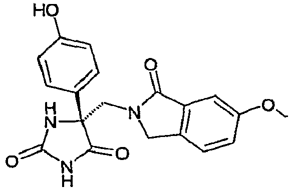 | **367.1** | **368.2[M+H]⁺** | **A** |
| **9601** | 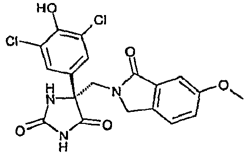 | **435.0** | **436.2[M+H]⁺** | **A** |
| **9602** | 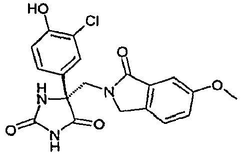 | **401.1** | **402.2[M+H]⁺** | **A** |
| **9603** | 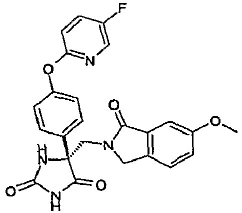 | **462.1** | **463.2[M+H]⁺** | **A** |
| **9607** | 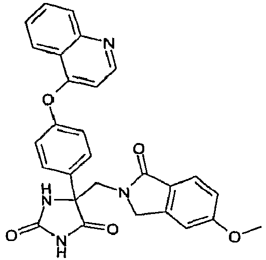 | **494.2** | **495.3[M+H]⁺** | **A** |
| **9608** | 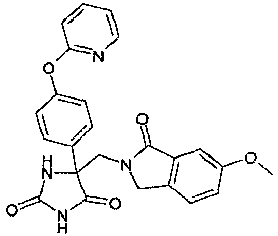 | **444.1** | **445.2[M+H]⁺** | **B** |
| **9609** | 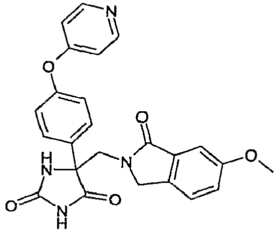 | **444.1** | **445.2[M+H]⁺** | **C** |
| **9610** | 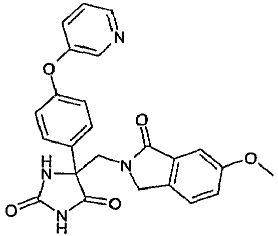 | **444.1** | **445.2[M+H]⁺** | **A** |
| **9611** | 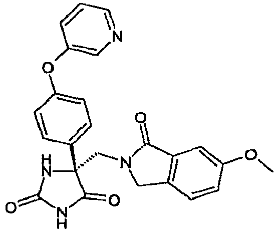 | **444.1** | **445.2[M+H]⁺** | **A** |
| **9612** | 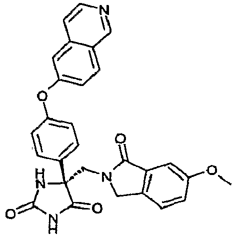 | **494.1** | **495.3[M+H]⁺** | **A** |
| **9613** | 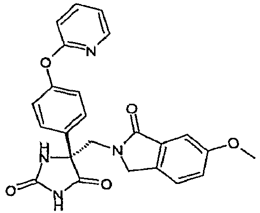 | **444.1** | **445.2[M+H]**⁺ | **A** |
| **9614** | 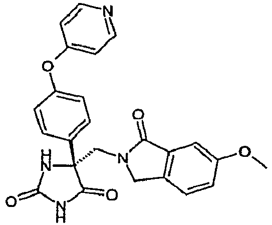 | **444.1** | **445.2[M+H]⁺** | **A** |
| **9615** | 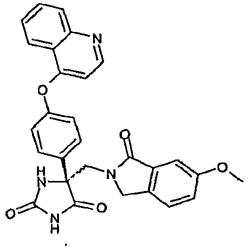 | **494.2** | **495.3[M+H]⁺** | **A** |
| **9618** | 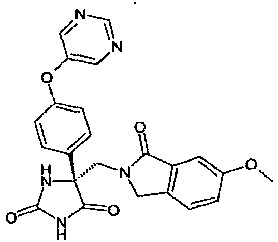 | **445.1** | **446.2[M+H]⁺** | **A** |
| **9619** | 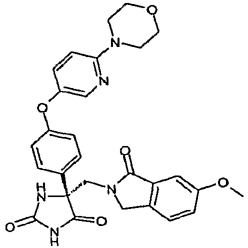 | **529.2** | **530.3[M+H]⁺** | **A** |
| **9624** | 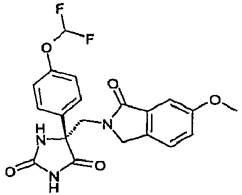 | **417.1** | **418.2[M+H]⁺** | **A** |
| **9625** | 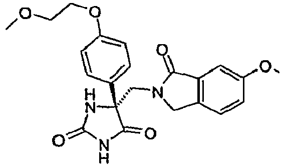 | **425.2** | **426.2[M+H]⁺** | **A** |
| **9626** | 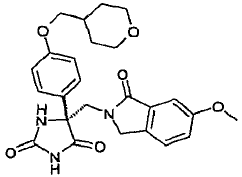 | **465.2** | **466.3[M+H]⁺** | **A** |
| **9627** | 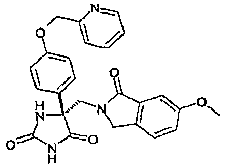 | **458.2** | **459.3[M+H]⁺** | **A** |
| **9628** | 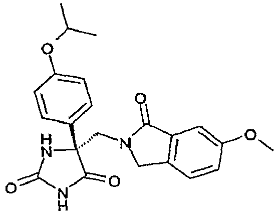 | **409.1** | **410.2[M+H]⁺** | **A** |
| **9629** | 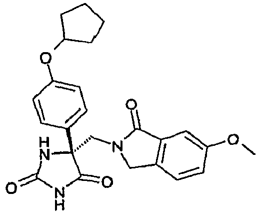 | **435.2** | **436.2[M+H]⁺** | **A** |
| **9632** | 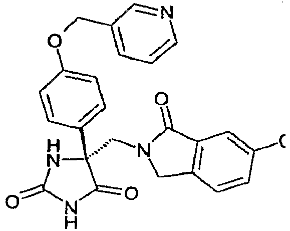 | **458.2** | **459.3[M+H]⁺** | **A** |
| **9633** | 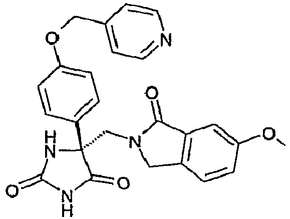 | **458.2** | **459.3[M+H]⁺** | **A** |
| **9634** | 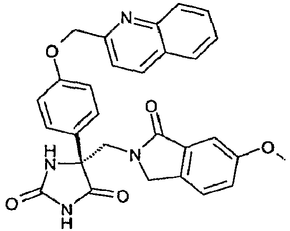 | **508.2** | **509.3[M+H]⁺** | **A** |
| **9638** | 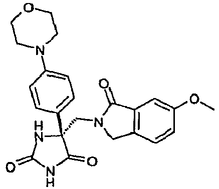 | **436.2** | **437.2[M+H]⁺** | **A** |
| **9650** | 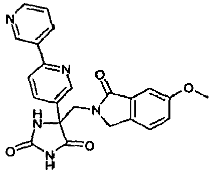 | **429.14** | **430.2 [M+H]⁺** | **A** |
| **9651** | 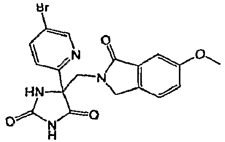 | **430.03** | **431.2 [M+H]**⁺ | **A** |
| **9652** | 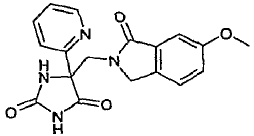 | **352.12** | **353.2 [M+H]⁺** | **A** |
| **9653** | 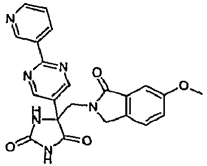 | **430.14** | **431.2 [M+H]⁺** | **A** |
| **9002C** | 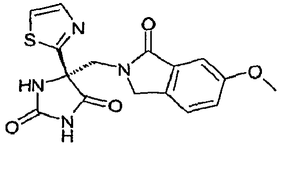 | **358.07** | **359.0[M+H]⁺** | **A** |
| **9002D** | 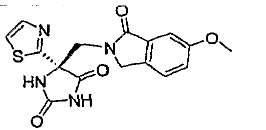 | **358.07** | **359.0[M+H]⁺** | **B** |
| **9003D** | 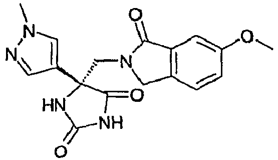 | **355.13** | **356.1[M+H]⁺** | **A** |
| **9003E** | 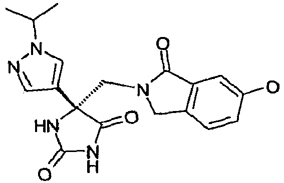 | **383.16** | **384.2[M+H]⁺** | **A** |
| **9003F** | 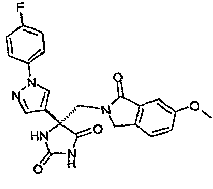 | **435.13** | **436.1[M+H]⁺** | **A** |
| **9003G** | 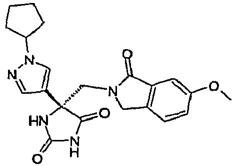 | **409.17** | **410.2[M+H]⁺** | **A** |
| **9003H** | 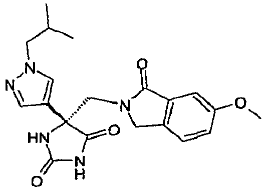 | **397.17** | **398.2[M+H]⁺** | **A** |
| **90031** | 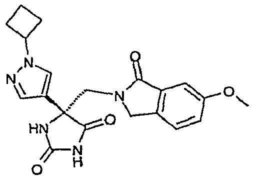 | **395.16** | **396.1[M+H]⁺** | **A** |
| **9003J** | 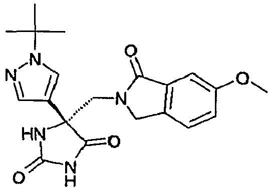 | **397.17** | **398.2[M+H]⁺** | **A** |
| **9003K** | 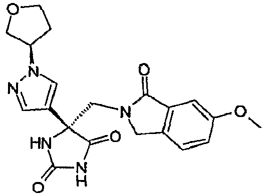 | **411.15** | **412.1[M+H]⁺** | **A** |
| **9003L** | 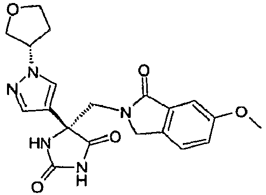 | **411.15** | **412.1[M+H]**⁺ | **A** |
| **9003M** | 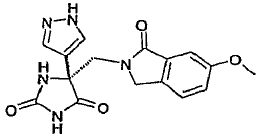 | **341.11** | **342.2[M+H]⁺** | **A** |
| **9004B** | 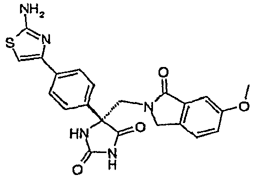 | **449.12** | **450.1[M+H]⁺** | **A** |
| **9004C** | 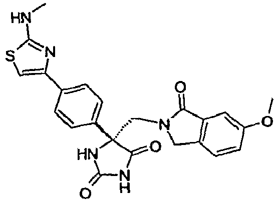 | **463.12** | **464.1 [M+H]⁺** | **A** |
| **9004D** | 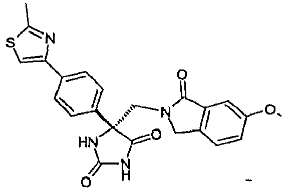 | **448.12** | **449.1 [M+H]⁺** | **A** |
| **9004E** | 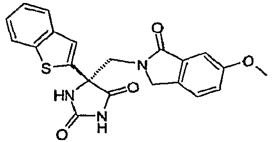 | **407.20** | **408.1 [M+H]⁺** | **A** |
| **9005** | 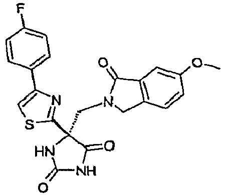 | **452.10** | **453.1 [M+H]⁺** | **A** |
| **9930** | 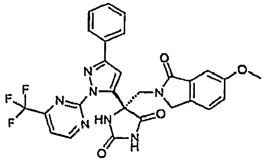 | **563.15** | **564.3[M+H]⁺** | **A** |
| **9931** | 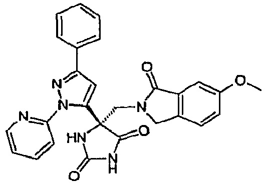 | **494.17** | **495.3[M+H]⁺** | **A** |
| **9932** | 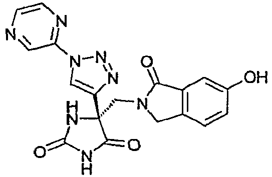 | **406.35** | **407.2[M+H]⁺** | **A** |
| **9933** | 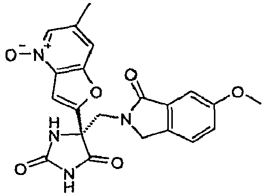 | **422.12** | **423.2[M+H]⁺** | **A** |
| **9934** | 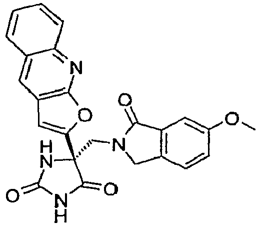 | **442.13** | **443.2[M+H]⁺** | **A** |
| **9935** | 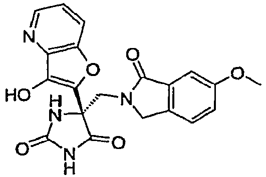 | **408.11** | **409.1 [M+H]⁺** | **A** |
| **9937** | 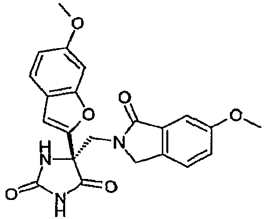 | **421.13** | **422.2[M+H]⁺** | **A** |
| **9938** | 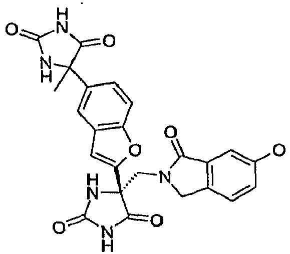 | **503.14** | **504.3[M+H]⁺** | **A** |
| **9939** | 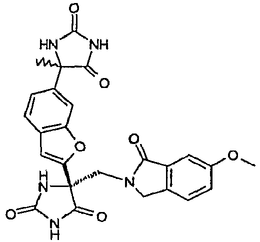 | **503.14** | **504.3[M+H]⁺** | **A** |
| **9940** | 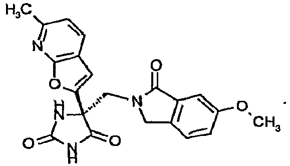 | **406.13** | **407.2[M+H]⁺** | **A** |
| **9941** | 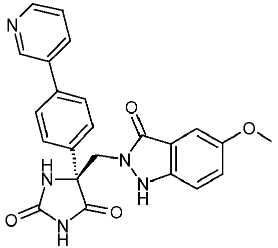 | **429.14** | **430.12[M+H]⁺** | >C |

### Example 9000:

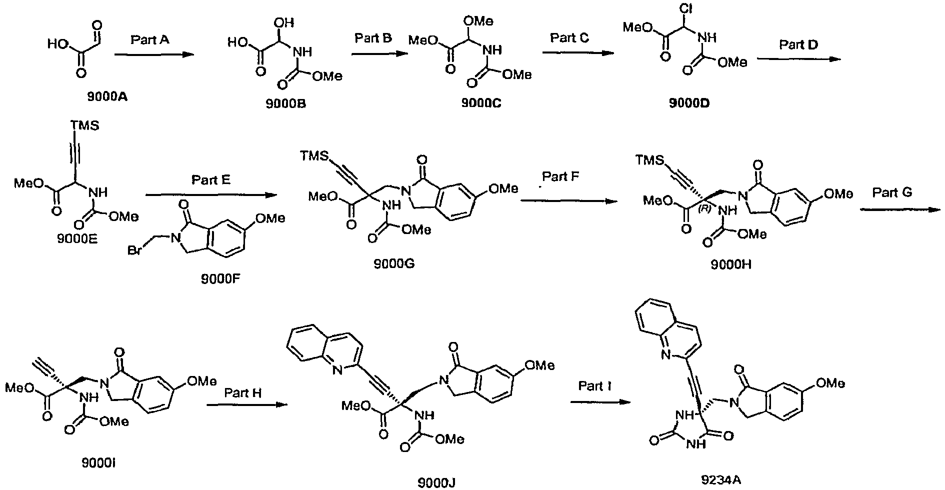

### Part A:

Glyoxylic acid monohydrate (20.0 g, 218 mmol) and methyl carbamate (16.3 g, 218 mmol) were dissolved in diethyl ether (200 mL) and stirred overnight. The solids were filtered to provide the desired product **9000B** (32.0 g, 98%).

### Part B:

Compound **9000B** (32.0 g, 214 mmol) was dissolved in MeOH (200 mL) and cooled in an ice bath. Concentrated sulfuric acid (8 mL) was added dropwise and the reaction was stirred overnight. The reaction mixture was diluted with ethyl acetate and water. The organic layer was washed with brine, dried over sodium sulfate, and concentrated to provide compound **9000C** that was used without purification (27.0 g, 71%).

### Part C:

Compound **9000C** (27.0 g, 152 mmol) was dissolved in carbon tetrachloride (700 mL). Phosphorus pentachloride (50 g, 240 mmol) was added and the suspension was stirred for 18 hours (solution became clear over time). The solvent was removed under reduced pressure and the residue was stirred in petroleum ether (500 mL) overnight. The solids were filtered to provide compound **9000D** with no need for purification (26.5 g, 96%). Trituration step was repeated if mass yield was too high.

### Part D:

Compound **9000D**(15.0 g, 82.7 mmol) was dissolved in methylene chloride (140 mL) and cooled in an ice bath. Bis(trimethylsilyl)acetylene (15.0 g, 88.2 mmol) was added in methylene chloride (20 mL). Freshly crushed aluminum chloride (11.0 g, 82.7 mmol) was added in portions over 20 minutes. The reaction mixture was allowed to slowly warm to room temperature and stirred overnight. The reaction was cooled in an ice bath and slowly quenched with water. The organic layer was washed several times with water, dried over sodium sulfate, and concentrated. The residue was triturated/recrystallized from hexanes to provide the desired product **9000E** (14.8 g, 69%). HPLC-MS t_{R} = 1.84 min (ELSD); mass calculated for formula C₁₀H₁₇NO₄Si 243.09, observed LCMS m/z 244.1 (M+H).

### Part E:

Compound **9000E** (24.0 g, 98.7 mmol) and compound **9000F** (25.1 g, 99.0 mmol) were dissolved in THF (300 mL) and cooled to -78°C. A 1 M solution of LiHMDS (198 mL, 198 mmol) was added dropwise over 30 minutes and the reaction mixture was stirred for 2 hours. Saturated ammonium chloride solution was added slowly and the reaction was allowed to warm to room temperature. The aqueous layer was extracted with ethyl acetate. The combined organic layers were washed with water, brine, dried over sodium sulfate, and concentrated. Purification by column chromatography (SiO₂, 33% ethyl acetate/hexanes to 50% ethyl acetate/hexanes) afforded the desired product **9000G** (26.0 g, 63%). HPLC-MS t_{R} = 1.90 min (UV_{254 nm}); mass calculated for formula C₂₀H₂₆N₂O₆Si 418.15, observed LCMS m/z 419.2 (M+H).

### Part F:

The two isomers were separated using a chiral OD column. One gram of material was injected into the column and the two peaks were separated by using a solvent mixture of 85% hexanes/ethanol. The second isomer was the desired compound **9000H** (400 mg, 80%).

### Part G:

Compound **9000H** (8.0 g, 19.1 mmol) was dissolved in THF (250 mL) and cooled to 0°C. Tetrabutylammonium fluoride (1 M in THF, 22.9 mL, 22.9 mmol) was added dropwise and the reaction was stirred for 1 hour at room temperature. The reaction mixture was diluted with ethyl acetate and water. The organic layer was washed with water, saturated sodium bicarbonate, brine, dried over sodium sulfate and concentrated to provide compound **9000I** (5.8 g, 88%). The product was used without purification.

### Part H:

Compound **9000I** (75 mg, 0.22 mmol) was combined with 3-bromoquinoline (0.032 mL, 0.24 mmol), Pd(PPh₃)₂Cl₂ (3 mg, 0.0044 mmol), Cul (2 mg, 0.009 mmol), diisopropylamine (0.062 mL, 0.44 mmol) in DMF (1 mL) and stirred overnight at 80 °C. The reaction mixture was diluted with ethyl acetate and water. The organic layer was washed with water, brine, dried over sodium sulfate and concentrated. Purification by column chromatography (SiO₂, 50% ethyl acetate/hexane to 80% ethyl acetate/hexane) afforded the desired product **9000J** (93 mg, 89%). HPLC-MS t_{R} = 1.66 min (UV_{254 nm}); mass calculated for formula C₂₆H₂₃N₃O₆ 473.16, observed LCMS m/z 474.1 (M+H).

### Part I:

Compound **9000J** (77 mg, 0.16 mmol) was dissolved in 7 M ammonia solution (3 mL) and stirred in a sealed pressure tube at 90 °C overnight. The reaction mixture was cooled to room temperature and concentrated to afford compound **9234A.** HPLC-MS t_{R} = 1.41 min (UV_{254 nm}); mass calculated for formula C₂₄H₁₈N₄O₄ 426.13, observed LCMS m/z 427.0 (M+H).

### Example 9001:

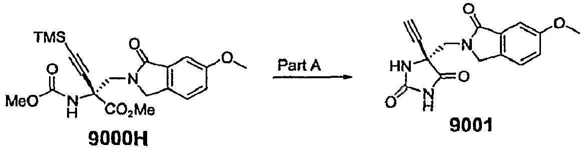

### Part A:

Compound **9000H** (1.26 g, 3.0 mmol) in 7 M ammonia in methanol (20 mL) was heated to 85 °C in a pressure bottle overnight. The reaction mixture was concentrated to afford **9001** (900 mg, 100%) which was used without further purification. HPLC-MS t_{R} = 1.00 min (UV_{254 nm}); mass calculated for formula C₁₅H₁₃N₃O₄ 299.09, observed LCMS m/z 300.1 (M+H).

### Example 9243

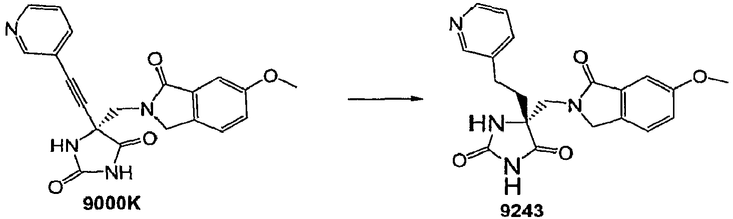

Compound **9000K** (33.5 mg, 0.089 mmol) was dissolved in 5 mL MeOH. Lindlar's catalyst (25 mg) was added. A hydrogen balloon was attached and the solution was stirred under H₂ for 3 days. The catalyst was removed by filtration. The solvent was removed by rotary evaporator. The product was purified by sgc (CH₂Cl₂/MeOH/NH₃-H₂O: 15:1:0.1) to give compound **9243** (12.5 mg, 36.9%).

### Example 9234:

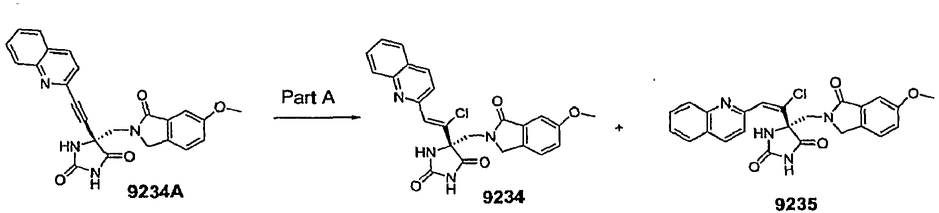

### Part A:

Compound **9234A** (prepared using previously described procedures) (68 mg, 0.16 mmol) was dissolved in methanol (2 mL) and treated with 4 M HCl in dioxane (1 mL). The reaction mixture was stirred a few minutes and concentrated. Purification by reverse phase prep HPLC afforded the **9234** (24 mg) and **9235** (7 mg). **9234:** HPLC-MS t_{R} = 1.43 min (UV_{254 nm}); mass calculated for formula C₂₄H₁₉ClN₄O₄ 462.11, observed LCMS m/z 463.1 (M+H). **9235:** HPLC-MS t_{R} = 1.51 min (UV_{254 nm}); mass calculated for formula C₂₄H₁₉ClN₄O₄ 462.11, observed LCMS m/z 463.1 (M+H).

**Compounds 9234 and 9235 was synthesized using example 9234 and procedures described previously**

### Example 9237

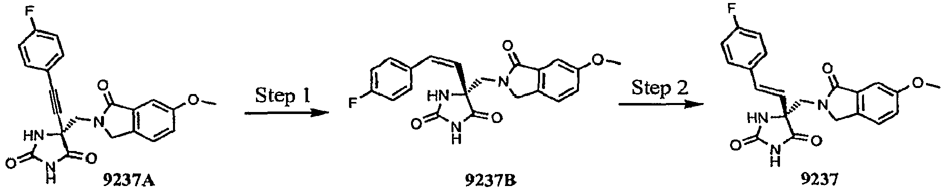

### Step 1

Compound **9237A** was dissolved in 2 mL MeOH. Lindar's catalyst (10%) (5 mg) was added. The solution was stirred under H₂ balloon for 1.5 h. It was diluted with MeOH (3 mL) and the catalyst was removed by filtration. The solvent was removed by rotary evaporator to give compound **9237B,** which was used without further purification.

### Step 2

Compound **9237B** was dissolved in CH₂Cl₂ (0.5 mL) and Pd(CH₃CN)₂Cl₂ was added. The solution was stirred overnight and the product was purified by silica gel prep TLC (CH₂Cl₂/MeOH/NH₃.H₂O: 20:1:0.1) to give compound **9237** (1.3 mg).

### Example 9246

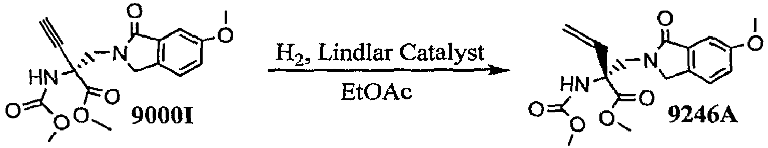

Compound **9000I** (34 mg) was added to a 25 mL Schlenck tube equipped with a stir bar, and dissolved in EtOAc. Lindlar catalyst was added. The flask was placed under balloon pressure of H₂. The reaction mixture was stirred at rt overnight. The reaction mixture was filtered and concentrated to dryness. The crude product was purified via sgc using a 5% to 50% (5%MeOH in EtOAc)/Hexanes gradient as the mobile phase. Compound **9246A** was collected as a clear oil (20 mg).

Compound **9246A** was dissolved in 8 mL of methanolic ammonia (7N). The solution was added to a pressure tube equipped with a stir bar and capped tightly. The tube was placed in an oil bath, heated to 75 C, and stirred ON at that temperature. After 15h, the reaction mixture was allowed to cool to rt. The reaction mixture was concentrated to dryness. The crude product was purified via sgc using a 5% to 60% (5%MeOH in EtOAc)/Hexanes gradient as the mobile phase. Compound **9246** was obtained as a clear oil.

**Compound 9246 was synthesized using example 9246 and procedures described previously**

### Example 9202:

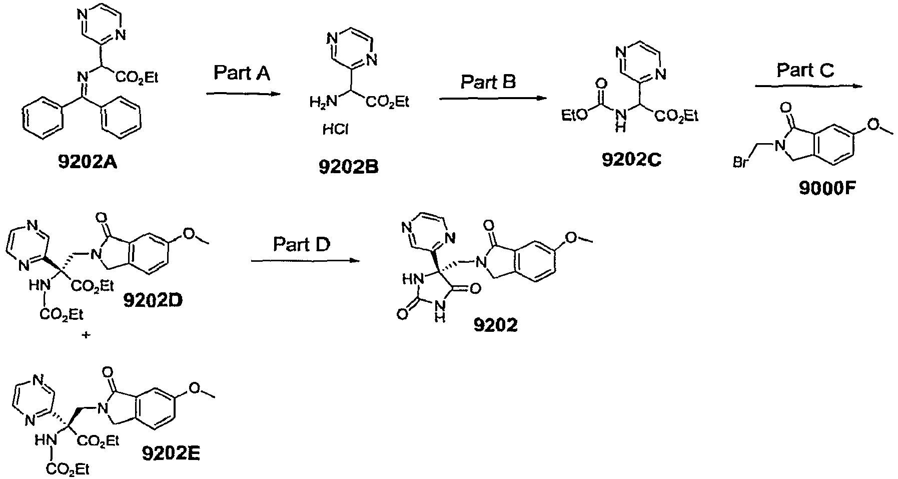

### Part A:

Compound **9202A** (prepared according to the procedures described in J. Med Chem. 1994, 37, 4567) (6.6 g, 19.1 mmol) was dissolved in diethyl ether (100 mL) and treated with 1 M HCl (aq. 23 mmol). The reaction mixture was stirred overnight at room temperature. The layers were separated and the aqueous layer was washed with diethyl ether (3 x 30 mL). The aqueous layer was concentrated and the residue was triturated with ethanol and acetone to afford **9202B** (3.14 g, 75%) as an off white powder. HPLC-MS t_{R} = 0.21 min (UV_{254 nm}); mass calculated for formula C₈H₁₁N₃O₂ 181.09, observed LCMS m/z 182.1 (M+H).

### Part B:

A mixture of **9202B** (2.17 g, 10 mmol), ethyl chloroformate (1.05 mL, 11 mmol) and DIEA (2.14 mL, 12 mmol) in THF (20 mL) was stirred at room temperature overnight. The reaction mixture was diluted with ethyl acetate and water. The layers were separated and the aqueous layer was extracted with ethyl acetate. The combined organic layers were washed with 1.0 M HCl (aq.) and brine, dried over sodium sulfate and concentrated. Purification by silica gel chromatography (50% ethyl acetate/hexane) afforded **9202C** (1.47 g, 58%) as an orange oil. HPLC-MS t_{R} = 1.19 min (UV_{254 nm}); mass calculated for formula C₁₁H₁₅N₃O₄ 253.11, observed LCMS m/z 254.2 (M+H).

### Part C:

Compound **9202C** (1.00 g, 3.95 mmol) in THF (10 mL) and HMPA (5 ml) was deprotonated with freshly prepared LDA solution (11.85 mmol) at -78 °C. The anion solution was stirred for 1 hr before **9000F** (1.13 g, 4.41 mmol) in THF (20 mL) was added at -78 °C. The reaction mixture was stirred 2.5 hrs and quenched at -78 °C with HOAc (1 mL). The reaction mixture was purified silica gel chromatography (ethyl acetate/hexane to methanol/ethyl acetate) to afford the c-alkylated product (103 mg, 6%). The isomers were separated by chiral chromatography with an AD column to afford **9202E** (36 mg) and **9202D** (35 mg). HPLC-MS t_{R} = 1.60 min (UV_{254 nm}); mass calculated for formula C₂₁H₂₄N₄O₆ 428.17, observed LCMS m/z 429.1 (M+H).

### Part D:

Compound **9202D** (35 mg, 0.08 mmol) was heated in 7 M ammonia in methanol (5 mL) at 90 °C for 3 days. The reaction mixture was concentrated and lypholized to afford **9202** (29 mg, 97%). HPLC-MS t_{R} = 0.95 min (UV_{254 nm}); mass calculated for formula C₁₇H₁₅N₅O₄ 353.11, observed LCMS m/z 354.1 (M+H).

**Compound 9202 was synthesized using example 9202 and procedures described previously**

### Example 9203:

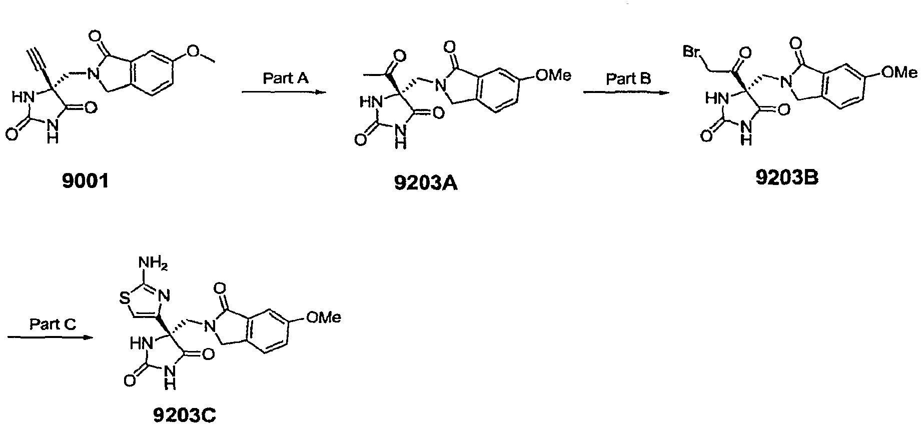

### Part A:

Compound **9001** (600 mg, 2.01 mmol) in MeOH (5 mL) was added with 2N H₂SO₄ (5 mL) and HgO (100 mg) and stirred at 80 °C for 1 h. The reaction mixture was diluted with EtOAc. The solid was removed by filtration; and the filtrate was washed with H₂O, brine, dried over Na₂SO₄, and concentrated to dryness, affording crude compound **9203A** as a white solid which was utilized for subsequent reaction (480 mg, 76%). HPLC-MS t_{R} = 1.39 min (UV_{254 nm}); mass calculated for formula C₁₅H₁₅N₃O₅ 317.10, observed LCMS m/z 318.1 (M+H).

### Part B:

Crude compound **9203A** (480 mg) in acetic acid (8 mL) was added dropwise with bromine (194 mg, 1.21 mmol) at room temperature. After stirring for 3 h at 50°C, the reaction mixture was concentrated to dryness, resulting in the crude product of compound **9203B** (500 mg). HPLC-MS t_{R} = 1.136 min (UV_{254 nm}); mass calculated for formula C₁₅H₁₄BrN₃O₅ 395.01, observed LCMS m/z 396.0 (M+H).

### Part C:

Crude compound **9203B** (100 mg) and thiourea (100 mg) in DMF (1 mL) was stirred at room temperature for 12 h. After concentrating to dryness, the residue was dissolved in DMSO/ACN (3:1) and purified by reverse phase HPLC, resulting in compound **9203** as a white solid (25 mg). HPLC-MS (10 min) t_{R} = 2.238 min (UV_{254 nm}); mass calculated for formula C₁₆H₁₅N₅O₄S 373.08, observed LCMS m/z 374.0 (M+H).

**Compound 9203C was synthesized using example 9203 and procedures described previously**

### Example 9200:

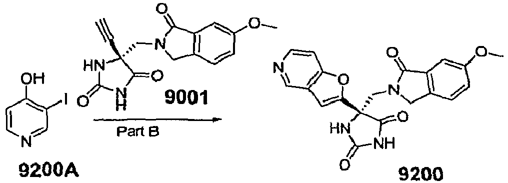

### Part B:

A mixture of **9200A** (prepared according to the procedures in WO 9846609) (412 mg, 1.38 mmol), **9001** (305 mg, 1.38 mmol), copper iodide (16 mg, 0.083 mmol), Pd(PPh₃)₂Cl₂ (48 mg, 0.069 mmol) and triethylamine (0.232 mL, 1.66 mmol) in DMF (8 mL) was heated at 80 °C overnight. The reaction mixture was concentrated, purified by reverse-phase chromatography converted to an HCl salt and lypholized to afford **9200** (252 mg, 42%) as a pale yellow powder.

HPLC-MS t_{R} = 0.75 min (UV_{254 nm}); mass calculated for formula C₂₀H₁₆N₂O₅ 392.11, observed LCMS m/z 393.1 (M+H).

**Compound 9200 was synthesized using example 9200 and procedures described previously**

### Example 9207:

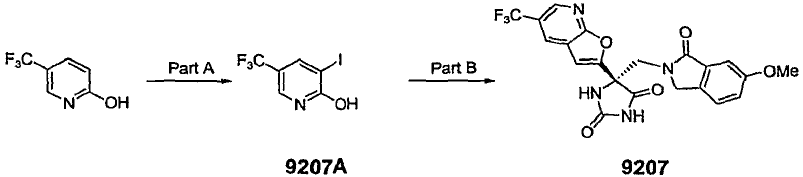

### Part A:

Compound **9207A** was prepared from 5-trifluoromethyl-2-hydroxypyridine and NIS according to a procedure in the literature (A. Meana, J.F.Rodriguez, M.A. Sanz-Tejedor, J. L. Garcia-Ruano, Synlett, 2003, 1678-1682).

### Part B:

Under argon, compound **9207A** (87 mg, 0.3 mmol) and compound **9001** (60 mg, 0.2 mmol) in DMF (1mL) were added with Pd₂(dba)₃ (4.6 mg, 0.005 mmol), dppf (5.5 mg, 0.01 mg), CuI (1.9 mg, 0.01 mmol) and DIEA (105 µL). After stirring at 100°C for 12 h, the reaction mixture was diluted with acetonitrile (1 mL). Reverse phase HPLC purification afforded compound **9207** (38 mg, 41%) as a white solid. HPLC-MS (10 min) t_{R} = 3.870 min (UV_{254 nm}); mass calculated for formula C₂₂H₁₆F₃N₃O₅ 460.10, observed LCMS m/z 461.0 (M+H).

**Compound 9207 was synthesized using example 9207 and procedures described previously**

### Example 9216:

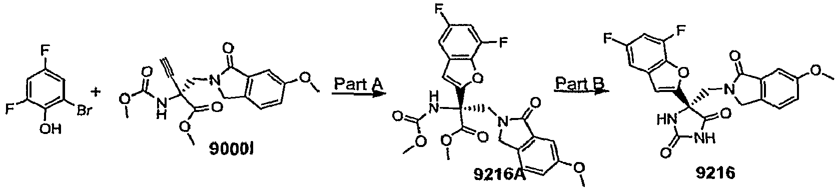

### Part A:

Compound **9216A** was prepared from compound **9000I** and 2-bromo-4, 6-difluorophenol following the procedure described in Example 400 part H.

### Part B:

Compound **9216A** (80 mg, 0.19 mmol) was dissolved in 7 M ammonia solution (5 mL) in MeOH and stirred in a sealed pressure tube at 90 °C for 2 days. The reaction mixture was cooled to room temperature and concentrated. The residue was purified by RP-HPLC to provide compound **9216** (42 mg, 52%). HPLC-MS t_{R} = 1.54 min (UV_{254 nm}); mass calculated for formula C₂₁H₁₅F₂N₃O₅ 427.10, observed LCMS m/z 428.0 (M+H).

**Compound 9216 was synthesized using example 9216 and procedures described previously**

### Example 9217:

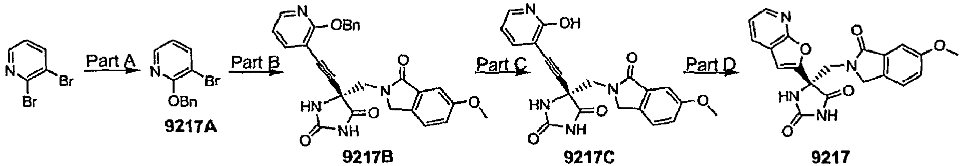

### Part A:

To a solution of benzyl alcohol (270 mg, 2.53 mmol) in THF (10 mL) at 0 °C was added sodium hydride (101 mg, 2.53 mmol). After 15 min, 2, 3-dibromopyridine (500 mg, 2.11 mmol) was added and the resulting mixture was warmed to 50 °C overnight. The reaction mixture was diluted with EtOAc, washed with water and brine, dried over sodium sulfate and concentrated. The residue was purified by column chromatography (SiO₂, 2% EtOAc/hexane to 5 % EtOAc/hexane) to afford **9217A** as a colorless oil (235 mg, 42%). HPLC-MS t_{R} = 2.21 min (UV_{254 nm}); mass calculated for formula C₁₂H₁₀BrNO 262.99, observed LCMS m/z 264.0 (M+H).

### Part B:

Compound **9217B** was prepared following the procedures described in Example **9000** part H and I.

### Part C:

Compound **9217B** (66 mg, 0.14 mmol) was treated with trifluoroacetic acid (3 mL) and CH₂Cl₂ (3 mL) overnight and the reaction mixture was concentrated. The residue was dissolved in acetonitrile (3 mL) and water (3 mL). The solvent was removed by lyophilization to provide compound **9217C** (53 mg, 97%). HPLC-MS t_{R} = 0.99 min (UV_{254 nm}); mass calculated for formula C₂₀H₁₆N₄O₅ 392.11, observed LCMS m/z 393.1 (M+H).

### Part D

To compound **9217C** (25 mg, 0.064 mmol) in DMF (2 mL) was added CuI (26 mg, 0.14 mmol) and the resulting mixture was heated in a microwave at 100 °C for 30 min. The reaction mixture was concentrated and the residue was purified by RP-HPLC to provide compound **9217** (10 mg, 40%). HPLC-MS t_{R} = 1.17 min (UV_{254 nm}); mass calculated for formula C₂₀H₁₆N₄O₅ 392.11, observed LCMS m/z 393.1 (M+H).

**Compounds 9217 was synthesized using example 9217 and procedures described previously**

### Example 9220

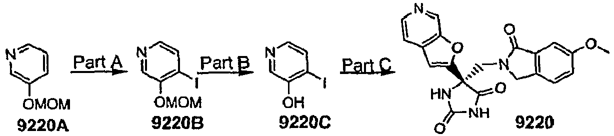

### Part A:

To **9220A** (prepared from 3-hydoxypyridine following a procedure described in Shimano, M. et al. (Tetrahedron, 1998, 54, 12745-12774)) (300 mg, 2.16 mmol) in Et₂O (20 mL) at -78 °C was added ^{t}BuLi (1.7 M in hexane, 2.5 mL, 4.32 mmol). After 30 min, a solution of 1-chloro-2-iodoethane (492 mg, 2.59 mmol) in THF (5 mL) was added dropwise. The resulting mixture was stirred for 30 min and treated with water and EtOAc. The organic layer was washed with water, brine, 9220(478 mg), which was used without purification in the next step.

### Part B:

Compound **9220B** (87 mg, 0.33 mmol) was treated with trifluoroacetic acid (2 mL) and CH₂Cl₂ (2 mL) for 3 hours and the reaction mixture was concentrated to give **9220C** (67 mg, 100%). HPLC-MS t_{R} = 0.52 min (UV_{254 nm}); mass calculated for formula C₅H₄INO 220.93, observed LCMS m/z 222.1 (M+H).

### Part C:

Compound 9001 (60 mg, 0.20 mmol) was combined with **9220C** (57 mg, 0.26 mmol), Pd(PPh₃)₂Cl₂ (7.0 mg, 0.01 mmol), CuI (4.0 mg, 0.02 mmol), triethylamine (0.055 mL, 0.40 mmol) in THF (2 mL) and stirred overnight at 60 °C. The reaction mixture was concentrated and the residue was purified by RP-HPLC to provide compound **9220** (43 mg, 54%). HPLC-MS t_{R} = 0.75 min (UV_{254 nm}); mass calculated for formula C₂₀H₁₆N₄O₅ 392.11, observed LCMS m/z 393.1 (M+H).

**Compound 9220 was synthesized using example 9220 and procedures described previously**

### Example 9225:

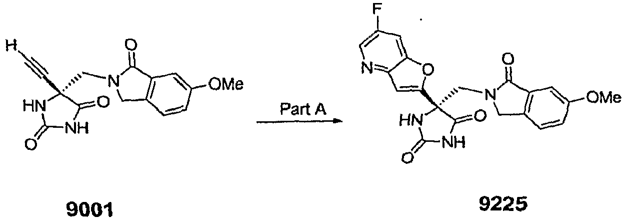

### Part A:

Under argon, compound **9001** (90 mg, 0.9 mmol) and 2-chloro-5-fluoro-3-hydroxypyridine (89 mg, 0.6 mmol), in DMF (1 mL) were added with Cs₂CO₃ (390 mg, 1.2 mmol) and bis(tri-*tert*-butylphosphine)palladium (7.7 mg, 0.015 mmol). After stirring at 100 °C for 12 h, the reaction mixture was diluted with acetonitrile (1 mL). Reverse phase HPLC purification afforded compound **9225** (41 mg, 34%) as a white solid. HPLC-MS (10 min) t_{R} = 3.037 min (UV_{254 nm}); mass calculated for formula C₂₀H₁₅FN₄O₅ 410.10, observed LCMS m/z 411.1 (M+H).

**Compound 9225 was synthesized using example 9225 and procedures described previously**

### Example 9228:

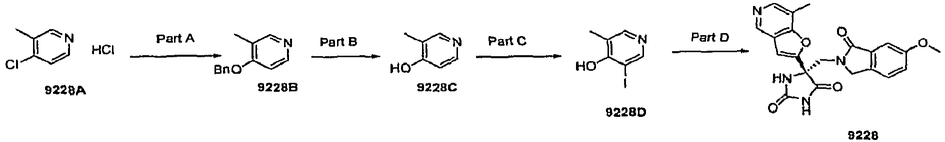

### Part A:

Benzyl alcohol (3.3 g, 30 mmol) was dissolved in THF (20 mL) and NaH (1.34 g, 33 mmol) was added slowly. After the bubbling ceased compound **9228A** (2.0 g, 12.1 mmol) was added and the mixture was refluxed for 48 hours. The reaction mixture was quenched with water and extracted with ethyl acetate. The organic layer was dried over sodium sulfate and concentrated. Column chromatography (1:1 hexanes/ethyl acetate) provided compound **9228B** (1.6 g, 67%). ¹H NMR (400 MHz, CDCl₃) δ 8.35 (m, 1H), 8.3 (s, 1H), 7.4-7.3 (m, 5 H), 6.85 (d, 1H), 5.15 (s, 2H).

### Part B:

Compound **9228B** (120 mg, 0.61 mmol) was dissolved in methanol (10 mL) and Pd-C (20 mg) was added. The reaction mixture was stirred overnight under a hydrogen atmosphere at room temperature. The reaction mixture was filtered and the solvent was removed to provide the desired product (60 mg, 92%). ¹H NMR (400 MHz, CDCl₃) δ 7.5 (m, 2H), 6.0 (s, 1H), 3.1 (s, 1H).

### Part C:

Compound **9228C** (60 mg, 0.56 mmol) was suspended in acetonitrile (5 mL) and N-iodosuccinimide (182 mg, 0.67 mmol) was added. The reaction mixture was refluxed for 2 hours. The solvent was evaporated and the remaining solid residue was triturated with methanol to provide the desired product (50 mg, 39%). ¹H NMR (400 MHz, CDCl₃) δ 8.2 (d, 1H), 7.6 (s, 1H), 1.75 (s, 3H).

### Part D:

Compound **9001** (410 mg, 1.37 mmol), compound **9228D** (334 mg, 1.43 mmol), Pd(PPh₃)₂Cl₂ (30 mg), CuI (15 mg), and triethylamine (0.5 mL) were dissolved in DMF and stirred under an inert atmosphere at 80 °C overnight. The solvent was removed and the material was purified by reverse phase chromatography to provide the desired product (168.3 mg, 30%). HPLC-MS t_{R} = 0.75 min (UV_{254 nm}); mass calculated for formula C₂₁H₁₈N₄O₅ 406.39, observed LCMS m/z 407.1 (M+H). ¹H NMR (400 MHz, DMSO-d6) δ 11.4 (s, 1H), 9.3 (s, 1H), 9.15 (s, 1H), 8.75 (s, 1H), 7.5 (m, 2H), 7.15 (m, 2H), 4.55 (m, 2H), 4.35 (m, 2H), 3.8 (s, 3H), 2.55 (s, 3H).

**Compound 9228 was synthesized using example 9228 and procedures described previously**

### Example 9600

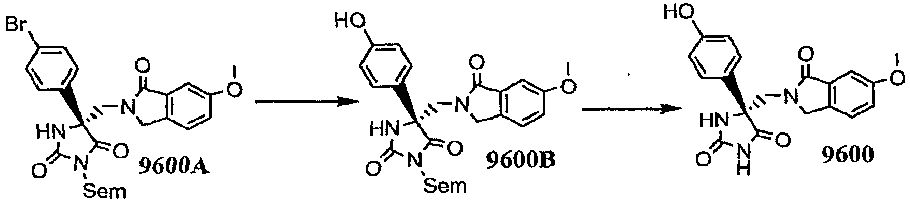

Compound **9600A** (1.50 g/ 2.68 mmol), pinocolatodiboron (816 mg, 3.21 mmol), potassium acetate (785 mg, 8.0 mmol), and palladium (II) dichloride(dppf) CH₂Cl₂ complex (250 mg, 0.306 mmol) were added to a 100 mL Schlenck flask equipped with a stir bar. The flask was caped with a septum, then cycled between vacuum and nitrogen four times. Dioxane (20 mL, Aldrich anhydrous) was added via syringe. The flask was cycled between vacuum and nitrogen three times, then placed in an 85°C oil bath. The bath was heated to 100°C, then stirred for 1.5 h. the reaction mixture was allowed to cool to RT and diluted with EtOAc (80 mL). The resulting mixture was filtered through Celite. The Celite was rinsed with additional EtOAc. The combined filtrate was concentrated to near dryness then redissolved in EtOAc. The organic solution was washed with 1.0 M aq pH 7 sodium phosphate buffer, water, and brine. After drying with MgSO₄, the organic layer was concentrated to dryness. The crude product was purified via sgc using a 2%-4% MeOH/ CH₂Cl₂ gradient as the mobile phase. A brown solid was obtained (1.9 g). The solid was dissolved in dioxane (16 mL) and water (11 mL) was added. Sodium perborate (3.0 g, 19.5 mmol) was added and the reaction mixture was stirred at rt overnight. The reaction mixture was diluted with EtOAc and aq 1 M NH₄Cl. The layers were separated. The organic layer was washed with water and brine, dried with MgSO₄, filtered and concentrated to dryness, giving an off white solid (1.37 g). SGC using a gradient of 25% to 100% (5%Methanol in EtOAc)/Hexanes as the mobile phase gave 0.25 g of pure **9600B** and 0.62 g of impure **9600B.**

Compound **9600B** (0.70 g, 1.40 mmol) was dissolved in 50 mL of Aldrich 4N HCl in dioxane and 50 mL of methanol. The solution was added to a pressure tube equipped with a stir bar. The tube was capped, placed in an oil bath, and heated to 95 C. The reaction was stirred at 95°C for 4 h, then allowed to cool to rt. The reaction mixture was concentrated to dryness. Methanol was added and the reaction mixture was reconcentrated. Methanol (50 mL) was added, followed by triethylamine (5 mL). The reaction mixture was stirred at rt for 1 h, then concentrated to dryness. EtOAc and 1.0 M aq pH 5.5 sodium phosphate buffer were added. The layers were separated. The organic layer was washed with water and brine, dried with MgSO₄, filtered, and concentrated to dryness. The crude product was purified via SiO₂ chromatography. The mobile phase was a gradient of 10% to 100% of (100:10:1-CH₂Cl₂:MeOH:concentrated NH₄OH) in CH₂Cl₂.. The main UV active peak was isolated as product giving 0.42 g of compound 9600 as white solid.

**Compound 9600 was synthesized using example 9600 and procedures described previously**

### Example 9601

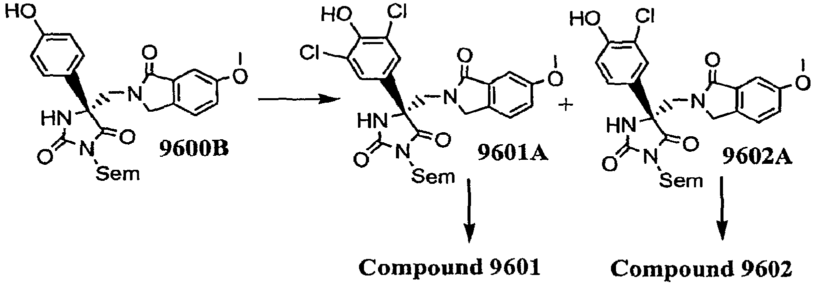

Compound **9600B** (218 mg, 0.44 mmol) was partially dissolved in THF (4 mL) and pyridine (3 mL). N-Chlorosuccinimide (62 mg) was added and the reaction mixture was left stirring ON at rt. After approximately 24 h, the reaction mixture was heated to 40°C and stirred for 2 h. The reaction mixture was allowed to cool to rt and quenched with 1 M aq sodium bisulfite. The resulting mixture was diluted with EtOAc. The layers were separated. The aq layer was extracted with additional EtOAc. The combined organic layer was washed with water and brine, dried with MgSO₄, filtered, and concentrated to an off white solid (0.22 g). The crude product was purified via prep TLC, giving starting material, **Compound 9601A** and **Compound 9602A**. The two products were converted to compounds **9601** and **9602** using SEM deprotection procedures similar to those described previously.

**Compound 9600 to compound 9602 were synthesis using example 9600 and example 9601 and procedures described previously.**

### Example 9603

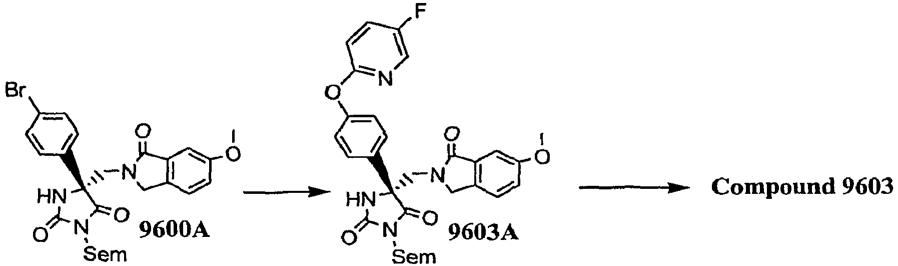

The aryl ether compounds **9603** to **9621** were prepared from compound **9600A** using a procedure based on that described by E. Buck and Z. J. Song in Organic Synthesis Vol 82, p. 69, followed by a standard SEM deprotection sequence. An example is provided below.

Compound **9600A** (0.248 g, 0.442 mmol), 5-Fluoro-2-hydroxypyridine (128 mg, 1.13 mmol), cesium carbonate (374 mg, 1.14 mmol), and copper (I) chloride (48 mg, 0.48 mmol) were added to a 10 mL Schlenck tube equipped with a stir bar. The tube was capped with a septum and cycled between vacuum and N₂ three times. N-methyl-2-pyrrolidinone (2mL) was added via syringe and the Schlenck tube was cycled between vacuum and N₂ three times. 2,2,6,6,6-Tetramethyl heptane-3,5-dione (33 µL) was added via syringe. The Schlenck tube was placed in a 100°C oil bath and heated to 150°C. The reaction mixture was stirred for 23 h at 150°C. The reaction mixture was allowed to cool to rt, then diluted with EtOAc and water. Aqueous 1% EDTA was added and the layers were separated. The organic layer was washed with 1% aq EDTA, water, and brine. The resulting organic solution was dried with MgSO₄, filtered, and concentrated to dryness. A brown solid was obtained. The crude product was purified via sgc using a Biotage SiO₂ cartridge and a 1%-2.5% MeOH/CH₂Cl₂ gradient as the mobile phase. The major spot was collected as product, giving 0.04 g of compound **9603A**.

Compound **9603A** (0.04g) was dissolved in (10 mL) anhydrous acetonitrile and concentrated to dryness on the rotovap. This step was repeated. The compound was redissolved in anhydrous acetonitrile (3 mL) and placed under N₂. The flask was cooled in an ice water bath. BF₃ etherate (90µL) was added, the ice bath was removed, and the reaction mixture was stirred at rt for 7 h. The reaction mixture was capped and stored in a 4°C freezer overnight. The reaction mixture was cooled in an ice-water bath. Diisopropylethylamine (1.5 mL) was added, followed by aq 3.0 M sodium hydroxide. The reaction was stirred for 15 min. The ice bath was removed, and the reaction mixture was stirred for 3 h at rt. Acetic acid was added until the reaction mixture was weakly acidic. The reaction mixture was partially concentrated on the rotovap. EtOAc and water were added. The layers were separated. The organic layer was washed with water and brine, dried with MgSO₄, filtered, and concentrated to dryness. The crude product was purified via reverse phase chromatography using an Isco C-18 cartridge (43g). The mobile phase was a 15% to 80% CH₃CN/H₂O gradient with 0.1% (volume) formic acid added to both components of the mobile phase. The main peak was isolated as product giving compound **9603**.

**Compound 9603 to compound 9619 were synthesis using example 9603 and procedures described previously.**

### Example 9624

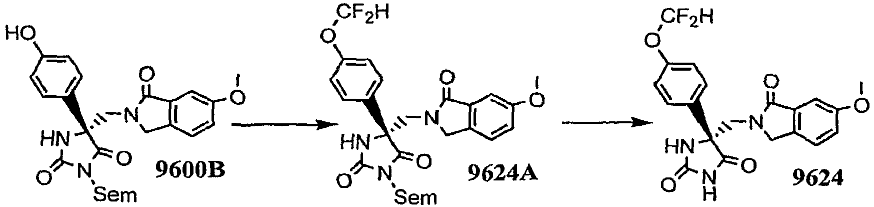

Compound **9600B** (168 mg, 0.337) and cesium carbonate (342 mg, 1.0 mmol) were added to a 15 mL 2 necked flask. DMF (3.5 ml) was added. The flask was equipped with a septum in one outlet and cold finger condenser in the other. The condenser was filled with dry ice and 2-propanol and the flask was placed in a room temperature water bath. Chlorodifluoromethane gas was bubbled into the reaction mixture through a needle in the septum for ca 15 minutes. The reaction mixture was allowed to stir at rt. When the rate of condensation of the chlorodifluromethane gas on the cold finger condenser decreased, additional chlorodifluromethane was added. After approx 5 h stirring at rt, the gas was stopped and the reaction mixture was diluted with EtOAc. Aqueous pH 5.5 sodium phosphate buffer was added and the layers were separated. The organic layer was washed with water and brine, dried with MgSO₄, filtered, and concentrated to a yellow oil (0.28 g). The crude product was partially purified ve sgc using a 0% to 6% MeOH/CH₂Cl₂ gradient as the mobile phase. The resulting impure product was columned on SiO₂ using a gradient of 20% to 30% (5% MeOH in EtOAc)/Hexanes as the mobile phase to give 0.06 g of **9624A**.

Compound **9624A** was converted to compound **9624** via a procedure that is similar to the SEM deprotection procedure using the HCl in dioxane and methanol, followed by triethylamine in methanol described previously.

### Example 9625

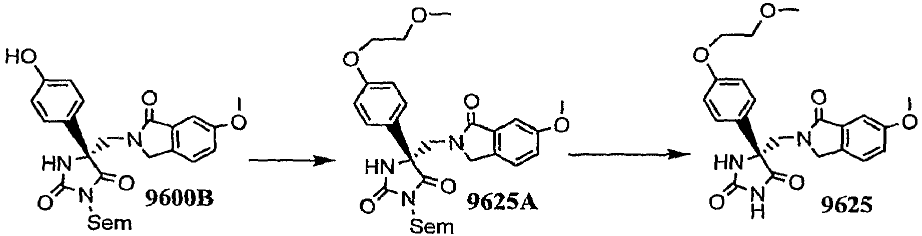

Compound **9600B** (0.255 mg, 0.51 mmol) was dissolved in DMF (2.6 mL). Cesium carbonate was added (366 mg, 1.1 mmol). the reaction mixture was stirred at rt under N₂ for 20 min. 2-Bromoethyl methyl ether (112 mg, 0.81 mmol) was added and the reaction mixture was stirred at rt under N₂ over the weekend. The reaction mixture was diluted with EtOAc and 1.0 M aq pH 5.5 sodium phosphate buffer, the layers were separated. The organic layer was washed with water and brine, dried with MgSO₄, filtered, and concentrated to dryness. The crude product was purified via sgc using a 1 % to 5% MeOH/CH₂Cl₂ gradient as the mobile phase to give compound **9625A.**

Compound **9625A** (0.1 g) was dissolved in 6 mL of Aldrich 4N HCl in dioxane and 3 mL of methanol. The solution was added to a pressure tube equipped with a stir bar. The tube was capped and placed in an 80°C oil bath. The reaction was stirred at 80°C for 21 h, then allowed to cool to rt. The reaction mixture was concentrated to dryness. Methanol was added and the reaction mixture was reconcentrated. Methanol (8 mL) was added, followed by triethylamine (3 mL).The reaction mixture was stirred at rt for 3 h, then concentrated to dryness. EtOAc and 1.0 M aq pH 5.5 sodium phosphate buffer were added. The layers were separated. The organic layer was washed with water and brine, dried with MgSO₄, filtered, and concentrated to dryness. The crude product was purified via reverse phase chromatography using an Isco C-18 cartridge (43g). The mobile phase was a 15% to 80% CH₃CN/H₂O gradient with 0.1% (volume) formic acid added to both components of the mobile phase. The main peak was isolated as product giving 43 mg of **9625** as white solid.

**Compound 9624 to compound 9634 were synthesis using example 9624 and example 9625 and procedures described previously.**

### Example 9638

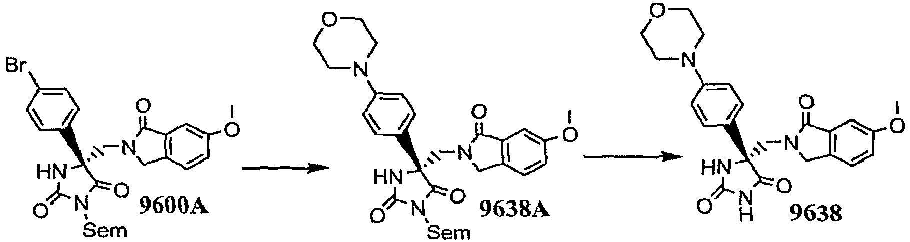

Compound **9638A** (233 mg, 0.416 mmol), and palladium (II) acetate (15 mg, 0.067 mmol) were added to a Schlenck tube equipped with a stir bar. The flask was placed under N₂ flow and sodium tert butoxide was added (96 mg, 0.99 mmol), followed by ortho biphenyl-di-tert-butylphosphine (38 mg, 0.13 mmol). The flask was capped with a septum, then cycled between vacuum and N₂ four times. Toluene was added via syringe and the flask was cycled between vacuum and N₂ two times. The reaction mixture was stirred at rt for 20 min. Morpholine (45 mg, 0.52 mmol) was added via syringe. The tube was cycled between vacuum and N₂ once, then placed in a 90 C oil bath. The reaction mixture was stirred for 2.5 h, then allowed to cool to rt. The reaction mixture was stirred at rt ON. The reaction mixture was diluted with EtOAc and 1.0 M aqueous pH 5.5 sodium phosphate buffer. The layers were separated. The organic layer was washed with aq NH₄Cl, water and brine. The resulting organic solution was filtered, dried with MgSO₄, filtered, and concentrated to dryness. The crude product was purified via flash sgc using a 90g SiO₂ cartridge. The mobile phase used was 30% (5% MeOH in EtOAc)/Hexanes, followed by 50% (5% MeOH in EtOAc)/Hexanes. The major UV active material was isolated as a white solid (0.16 g). This material was repurified via prep TLC using 95:5 CH₂Cl₂:MeOH as the mobile phase. Compound **9638A** was isolated as clear oil that crystallized on standing.

Compound **9638A** was converted to compound **9638** using SEM deprotection procedures similar to those described previously.

**Compound 9638 was synthesis using example 9638 and procedures described previously.**

### Example 9650

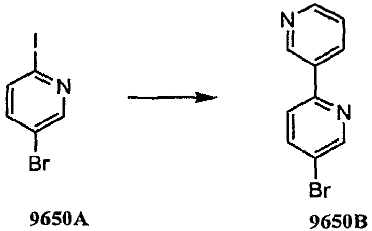

Compound **9650A** (5.0 g, 17.61 mmol), 4-pyridine boronic acid (2.06 g, 16.73 mmol), and Pd(dppf)Cl₂-CH₂Cl₂ (644 mg, 0.88 mmol) were placed in a 500 mL flask. The flask was vacuumed for 1 minutes and then it was filled with N₂. This process was repeated for twice. CH₃CN (200 mL) and K₂CO₃ (1M, 100 mL) were added. The solution was stirred in at 35 °C for two days. Additional Pd(dppf)Cl₂-CH₂Cl₂ (400mg) was added in the second day. The aqueous layer was separated and it was extracted with EtOAc (50 mL) once. The organic layers were combined, washed with brine (100 mL) and dried over Na₂SO₄. The solution was concentrated and purified by sgc (Hexane/EtOAc 3:1 to 2:1) to give compound **9650B** (3.1g, 74.9%).

**Compound 9650 to compound 9653 were synthesis using example 9650 and procedures described previously.**

### Example 9002:

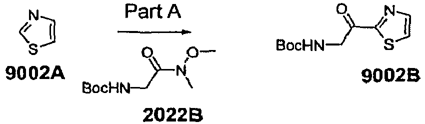

### Part A:

To thiazole (9002A) (1.50 g, 17.6 mmol) in THF (20 mL) at -78 °C was added n-BuLi (2.5 M, 7.2 mL, 18 mmol). The reaction mixture was stirred for 1 hour then Weinreb amide **2022B** (1.92 g, 8.8 mmol) in THF (10 ml) was added. The reaction mixture was warmed to room temperature and stirred overnight. The mixture was quenched with ammonium chloride solution and extracted with ethyl acetate. The combined organic layers were washed with brine, dried over sodium sulfate and concentrated. Purification by silica gel chromatography (30% ethyl acetate/hexane) afforded **9002B** (980 mg, 46%) as an orange oil. HPLC-MS t_{R} = 1.45 min (UV_{254 nm}); mass calculated for formula C₁₀H₁₄N2O₃S 242.07, observed LCMS m/z 265.1 (M+Na).

**Compound 9002C and compound 9002D were synthesis using example 9002 and procedures described previously.**

### Example 9003:

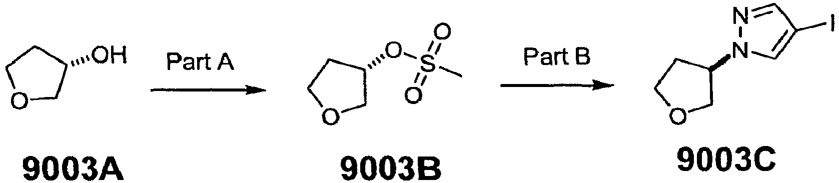

### Part A:

According to a modification of a procedure by Ghosh, A. K. et. al. (J. Med. Chem. 1993, 36, 2300-2310) to an ice cold solution of (S)-(+)-3-hydroxytetrahydrofuran (9003A) (4.5 mL, 66.3 mmol) in DCM (50 mL) was added triethylamine (13.8 mL, 99.4 mmol) followed by methanesulfonyl chloride (5.64 mL, 73 mmol), and the resulting mixture was stirred at 0°C for 1.5 h. The mixture was then quenched with water (15 mL), stirred at 0°C for 10 min, and diluted with DCM (100 mL). After phase separation the organics were washed with 12% HCl solution, saturated sodium bicarbonate solution, brine, and then concentrated to give desired product **9003B** as a dark yellow oil (5.3 g, 48%), which was used in the next step without further purification.

### Part B:

To an ice cold mixture of sodium hydride (770 mg, 30.5 mmol) in DMF (20 mL) was added a solution of 4-iodopyrazole (5.38 g, 27.8 mmol) in DMF (10 mL) and the resulting slurry was stirred at 0°C for 20 min. Then a solution of mesylate **9003B** (5.3 g, 31.9 mmol) in DMF (10 mL) was added and the resulting mixture was allowed to warm up to rt, stirred at rt for 30 min, and then heated at 80°C for 1.5 h. The reaction mixture was then concentrated and partitioned between ethyl acetate and water. The organics were washed with water, brine, dried and concentrated to give an oil, which was chromatographed (SiO₂, 20% to 30% ethyl acetate/hexanes) to afford a white solid. This solid was triturated with ether, and the resulting solid was filtered off to give desired **9003C.** HPLC-MS t_{R} = 1.32 min (UV); mass calculated for formula C₇H₉IN₂O 263.98, observed LCMS m/z 265.0 (M+H).

**Compound 9003D and compound 9003M were synthesis using example 9003 and procedures described previously.**

### Example 9004:

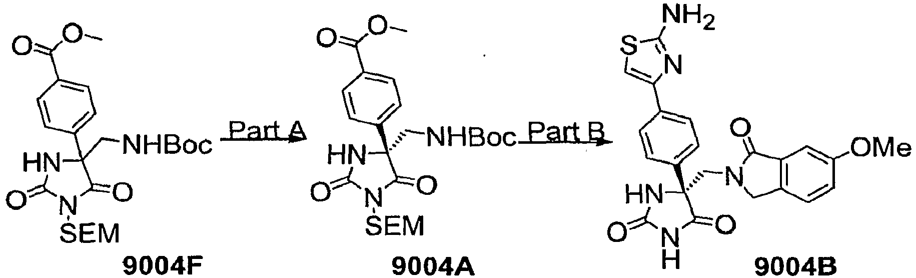

### Pa rt A:

The two isomers of **29004F** were separated using a chiral AD column. One gram of material was injected into the column and the two peaks were separated by using a solvent mixture of 80% hexanes/2-propanol. The second isomer was the desired compound **9004A** (400 mg, 80%).

### Part B:

Compound **9004B** was prepared from compound **9004A** according to the procedures described in Example 2026 part E, F and G: HPLC-MS t_{R} = 0.98min (UV_{254 nm}): mass calculated for formula C₂₂H₁₉N₅O₄S 449.12, observed LCMS m/z 450.1 (M+H).

**Compound 9004B and compound 9004E were synthesis using example 9004 and procedures described previously.**

### Example 9005

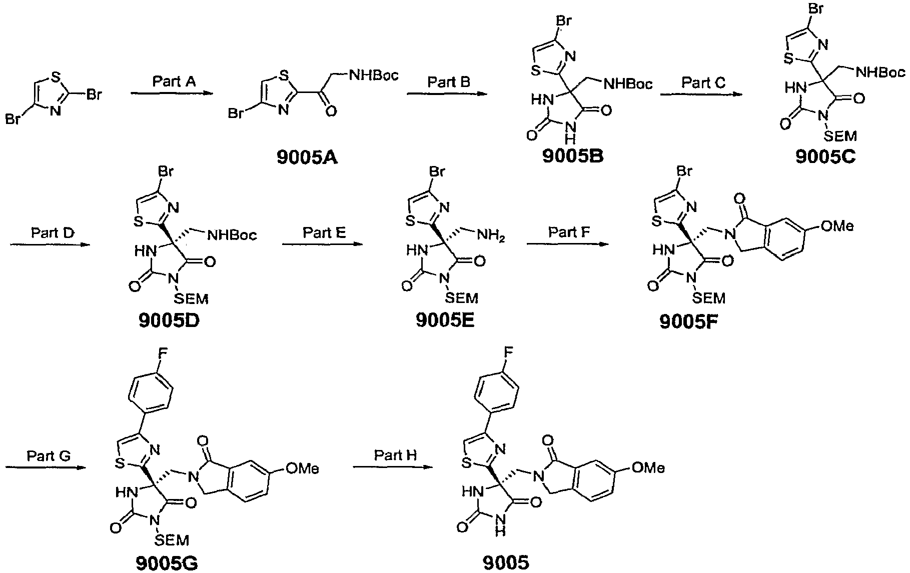

### Part A:

*i*-PrMgCl (2M in Et₂O, 10.4 mL, 20.8 mmol) was added dropwise to a solution of 2,4-dibromothiazole (5.0 g, 20.75 mmol) in Et₂O (40 mL) at 0 °C. After stirring for 15 min at this temperature, a solution of the Weinreb amide of N-Boc glycine (2.26 g, 10.4 mmol) in Et₂O (40 mL) and THF (20 mL) was added dropwise. The reaction mixture was then allowed to warm to room temperature and stirred for 1.5 h, then quenched with 1 N NH₄Cl aqueous solution. It was diluted with EtOAc; the organic layer was washed with H₂O, NaHCO₃, brine, dried over Na₂SO₄, and concentrated. Flash column chromatography afforded compound **9005A** as a white solid (3.04 g, 89%) HPLC-MS t_{R} = 1.75 min (UV_{254 nm}); mass calculated for formula C₁₀H₁₃BrN₂O₃S 319.98, observed LCMS m/z 321.0 (M+H).

### Part B:

Compound **9005A** (3.20 g, 10 mmol) in 20 mL of 7 N NH₃ in MeOH was added into a mixture of KCN (0.98 g, 15 mmol) and (NHa)₂CO₃ (3.36 g, 35 mmol) in H₂O (20 mL). The reaction mixture was stirred at 75 °C for 3 hours in a sealed pressure flask. After cooling to room temperature, the solution was transferred to a round bottom flask and concentrated to dryness. The residue was dissolved with EtOAc, washed with H₂O, brine, dried over Na₂SO₄, and concentrated to afford compound **9005B** as a yellow solid (1.85 g, 47%). HPLC-MS t_{R} = 1.32 min (UV_{254 nm}); mass calculated for formula C₁₂H₁₅BrN₄O₄S 390.0, observed LCMS m/z 413.0 (M+Na).

### Part C:

SEM-Cl (1 mL) was added dropwise to a solution of compound **9005B** (1.85 g, 4.73 mmol) and DIEA (1.65 mL) in DMF (30 mL). After stirring for 2h at room temperature, the reaction mixture was concentrated to dryness. It was then dissolved with EtOAc and H₂O. The organic layer was washed with H₂O, saturated Na₂HCO₃, brine, dried over Na₂SO₄, and concentrated. Flash column chromatography over silica gel (EtOAc/hexane 35:65) afforded compound **9005C** (2.22g, 90%) as a white solid. HPLC-MS t_{R} = 2.18 min (UV_{254 nm}): mass calculated for formula C₁₈H₂₉BrN₄O₅SSi 520.1, observed LCMS m/z 543.1 (M+Na).

### Part D:

The two isomers were separated using a chiral OD column. The racemate (400 mg) in 5 mL of IPA/hexane (1:1) was injected into the column and the two peaks were separated using a solvent mixture of 85% hexanes/isopropanol. The second isomer was the desired compound **9005D** (112 mg, 56%).

### Part E:

Compound **9005D** (460 mg, 0.88 mmol) in dioxane (6 mL) was treated with 4 mL of 25% HCl in H₂O. After stirring at room temperature for 6h, the reaction mixture was concentrated to dryness, giving rise to compound **9005E** as a white solid (345 mg). HPLC-MS t_{R} = 1.267 min (UV_{254 nm}); mass calculated for formula C₁₃H₂₁BrN4O₃SSi 420.0, observed LCMS m/z 421.0 (M+H).

### Part F:

A mixture of compound **9005E** (445 mg), compound **305** (342 mg, 1.32 mmol) and DIEA (384 µL, 2.2 mmol) in DMF (5 mL) was heated at 90°C overnight. The solution was concentrated to dryness; the residue was then dissolved with EtOAc and H₂O. The organic layer was washed with 1 N HCl, saturated NaHCO₃, brine, dried over Na₂SO₄, and concentrated. Flash column chromatography over SiO₂ (EtOAc / hexane 50:50) afforded compound **9005F** (170 mg. 34%). HPLC-MS t_{R} = 2.181 min (UV_{254 nm}); mass calculated for formula C₂₂H₂₇BrN₄O₅SSi 566.1, observed LCMS m/z 567.1 (M+H).

### Part G:

Under argon, a mixture of compound **9005F** (70 mg, 0.123 mmol), 4-fluorophenylboronic acid (26 mg, 0.185 mmol), K₃PO₄.H₂O (85 mg, 0.37 mmol), and PdCl₂(dppf) (5 mg, 0.0062 mmol) in dioxane (5 mL) was stirred at 60°C for 12 h. The reaction mixture was filtered through a plug of celite with the aid of EtOAc, and the solution was then concentrated to dryness. The residue was purified by flash column chromatography over SiO₂ (EtOAc/hexane 55:45), resulting in compound **9005G** (50 mg, 70%). HPLC-MS t_{R} = 2.15 min (UV_{254 nm}); mass calculated for formula C₂₈H₃₁FN₄O₅SSi 582.2, observed LCMS m/z 583.3 (M+H).

### Part H:

Compound **9005G** (50 mg, 0.086 mmol) in CH₃CN (5 mL) was added dropwise with BF₃.OEt₂ (55 µL, 0.43 mmol) at 0°C. After stirring for 2h at this temperature, DIEA (1 mL) and 1 N NaOH (2 mL) were subsequently added. The reaction mixture was allowed to stir at room temperature for 12 h, then concentrated to dryness. The resulting solid was treated with H₂O (10 mL), 1 N HCl (6 mL). The precipitate was collected by filtration. HPLC purification afforded pure compound **9005** (27 mg) as a white solid. HPLC-MS (10 min) t_{R} = 3.85 min (UV_{254 nm}); mass calculated for formula C₂₂H₁₇FN4O₄S 452.10, observed LCMS m/z 453.1 (M+H).

**Compound 9005 was synthesized using example 9005 and procedures described previously.**

### Example 9901

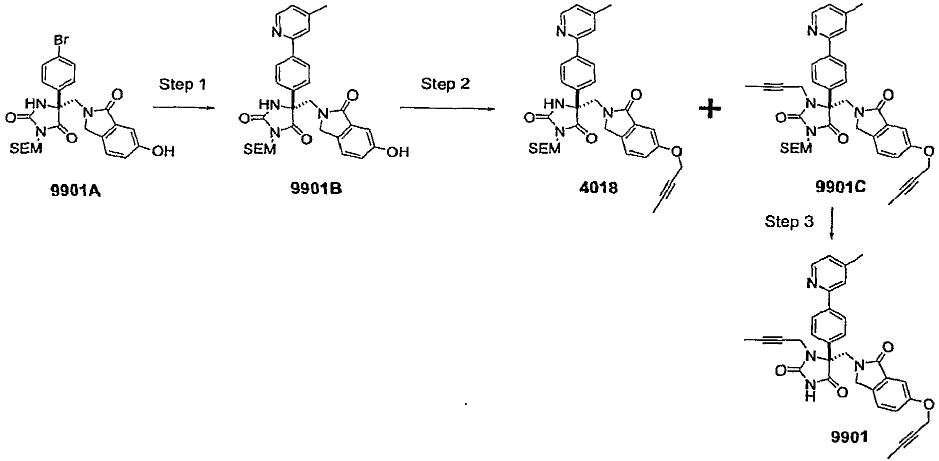

### Step 1

Compound **9901A** was converted to Compound **9901B** as described in Example 1004.

### Step 2

A mixture of Compound **9901B** (200 mg, 0.358 mmol) and cesium carbonate (175 mg, 0.537 mmol) in DMF (3.0 mL) was stirred for 30 min at 0 °C. A solution of 1-bromo-2-butyne (57 mg, 0.430 mmol) in DMF (0.5 mL) was added at 0 °C and the reaction mixture was stirred for 1 h at 0 °C, then overnight at rt. The reaction mixture was diluted with EtOAc and washed sequentially with saturated aq ammonium chloride, water, and brine. The organic phase was dried over MgSO₄, filtered, and concentrated. The residue was purified by sgc (0-100% EtOAc/hexanes gradient) to yield, in order of elution, Compound **9801** (110 mg, 51%) and then Compound **9901C** (67 mg, 28%).

### Step 3

Compound **9901C** was converted to Compound **9901** as described in Example 1001, Step 4.

**Compound 9901 was synthesized using example 9901 and procedures described previously.**

### Example 9916

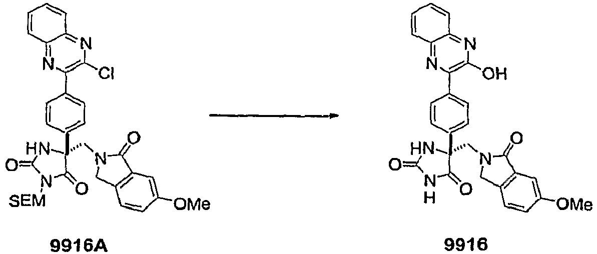

Compound **9916A** was prepared according to procedures given in Examples 14, 1001, 1004 and 1008. Compound **9916A** was converted to Compound **9916** using the procedure given in Example 1001, Step 4.

**Compound 9916 was synthesized using example 9916 and procedures described previously.**

### Example 9229

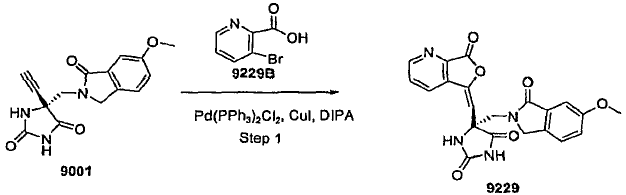

Step1. Compound **9001** (100 mg, 0.33 mmol) was combined with compound **9229B** (80 mg, 0.4 mmol), Pd(PPh₃)₂Cl₂ (8 mg, 0.012 mmol), CuI (17 mg, 0.1 mmol), diisopropylamine (0.08 mL, 0.58 mmol) in DMF (1 mL) and stirred at 85°C for 2 h. The reaction mixture was purified on a Gilson reverse phase HPLC (0-40% acetonitrile in H₂O with formic acid 0.1%) afforded the desired product **9229**18 mg, 13%).

**Compound 9229 was synthesized using example 9229 and procedures described previously**

### Example 9272

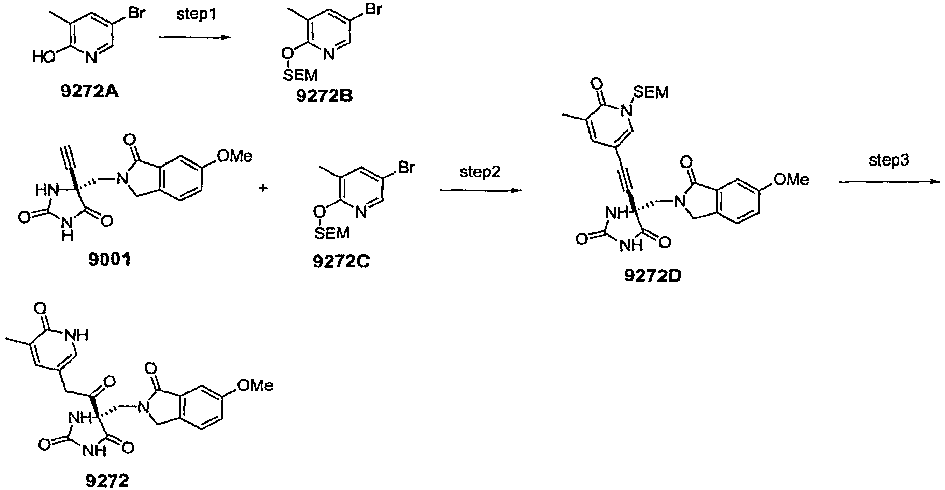

### Step 1

A mixture of **9272A** (161 mg, 0.86 mmol), SEMCI (0.17 mL, 0.94 mmol), and diisopropylethylamine (0.22 mL, 1.28 mmol) in CH₂Cl₂ (3 mL) was stirred at 25 °C for 2 h. The mixture was added to an aqueous NaHCO₃ solution and the organic layers were extracted with CH₂Cl₂. The combined organic solution was washed with brine solution, dried (Na₂SO₄), and concentrated in vacuo. The residue was purified by SiO₂ column chromatography (CH₂Cl₂/hexane = 2:1). to afford **9272B** (200 mg, 74% yield).

### Step 2

A mixture of **9001** (100 mg, 0.33 mmol), **9272C** (165 mg, 0.52 mmol), Pd(PPh₃)₂Cl₂ (4.9 mg, 7 µmol), CuI (1.9 mg, 10 µmol), and diisopropylethylamine (0.17 mL, 0.99 mmol) in DMF (1.5 ml) was purged with N₂ and heated to 70 °C. After heating for 17 h, the mixture was cooled to 25 °C and purified by column chromatography on a reverse phase C-18 column (0.01%HCO₂H in water/0.01% HCO₂H in CH₃CN) to afford **9272D** (78 mg, 44% yield).

### Step 3

**9272D** (78 mg, 0.14 mmol) was dissolved in MeOH (15 mL) and treated with 4 N HCl in dioxane (3 mL). The mixture was heated to 60 °C in a pressure vessel for 16 h and cooled to 25 °C. The mixture was neutralized with NH₃-MeOH (7 N solution) and the resulting precipitate was filtered off. The filtrate was concentrated in vacuo and the residue was purified by preparative TLC (10% MeOH in CH₂Cl₂) to afford 9272 (25 mg, 40% yield).

**Compound 9272 was synthesized using example 9272and procedures described previously.**

### Example 9218:

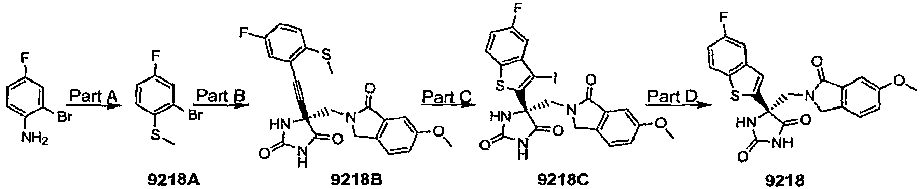

### Part A:

Compound **9218A** was synthesized from 2-bromo-4-fluoroaniline according to a procedure described in Allaire, F.S. et al. (Syn. Commun., 2001, 31, 1857-1861.).

### Part B:

Compound **9218B** was prepared from **9218A** following procedures described in Example **9000** part H and I. HPLC-MS t_{R} = 1.60 min (UV_{254 nm}); mass calculated for formula C₂₂H₁₈FN₃O₄S 439.10, observed LCMS m/z 440.0 (M+H).

### Part C:

To compound **9218B** (78 mg, 0.18 mmol) in CH₂Cl₂ (5 mL) was added iodine (90 mg, 0.36 mmol) in CH₂Cl₂ (5 mL) and the resulting mixture was stirred for 3 h. The mixture was diluted with CH₂Cl₂ and washed with 5% NaHSO₃, water and brine, dried over sodium sulfate and concentrated to give a white solid (92 mg) which was used directly in the next step without purification. HPLC-MS t_{R} = 1.71 min (UV_{254 nm}); mass calculated for formula C₂₁H₁₆FIN₃O₄S 550.98, observed LCMS m/z 552.0 (M+H).

### Part D:

Compound **9218C** (92 mg, 0.17 mmol) and palladium on carbon (10%, 10 mg) in MeOH (3 mL) were stirred under H₂ for 4 days. The mixture was filtered through Celite and concentrated. The residue was purified through RP-HPLC to provide recovered starting **9218C** (33 mg) and compound **9218** (7.4 mg, 16% based on conversion). HPLC-MS t_{R} = 1.55 min (UV_{254 nm}); mass calculated for formula C₂₁H₁₆FN₃O₄S 425.08, observed LCMS m/z 426.1 (M+H).

**Compound 92186 was synthesized using example 99218 and procedures described previously**

### Example 9223:

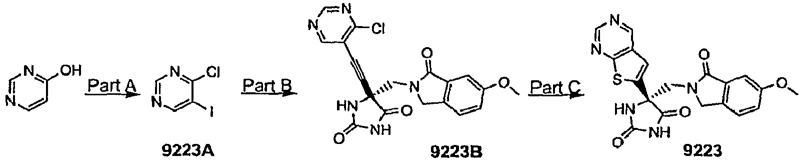

### Part A:

Compound **9223A** was synthesized from 4(3H)-pyrimidone according to a procedure described in Sakamoto, T. et al. (Chem. Pharm. Bull., 1986, 34, 2719-2724.).

### Part B:

Compound **9223B** was prepared from compound **9001** and compound **9223A** according to the procedure described in Example **9000** part H: HPLC-MS t_{R} = 1.21 min (UV_{254 nm}); mass calculated for formula C₁₉H₁₄CIN₅O₄S 411.07, observed LCMS m/z 412.1 (M+H).

### Part C:

Compound **9223B** (82 mg, 0.20 mmol) and NaSH (56 mg, 1.0 mmol) were heated in EtOH (3 mL) at 80 °C for 2h. After the mixture was concentrated and ice water was added, the aqueous mixture was extracted with chloroform and the combined organic layers were washed with brine, dried over sodium sulfate and concentrated. The resulting residue was purified by RP-HPLC to provide **9223** (44 mg, 54%). HPLC-MS t_{R} = 1.09 min (UV_{254 nm}); mass calculated for formula C₁₉H₁₅N₅O₄S 409.08, observed LCMS m/z 410.2 (M+H).

**Compound 9223 was synthesized using example 9223 and procedures described previously**

### Example 9231

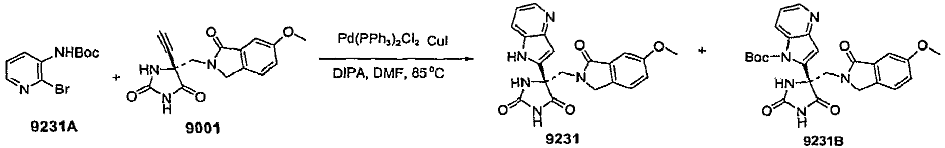

### Step 1

Compound **9231A** (80 mg, 0.29 mmol) was combined with compound **9001** (100 mg, 0.33 mmol), Pd(PPh₃)₂Cl₂ (5 mg, 0.007 mmol), CuI (12 mg, 0.06 mmol), diisopropylamine (0.16 mL, 1.13 mmol) in DMF (1 mL) and stirred at 85 °C. The reaction mixture was neutralized with acetic acid and purified with Gilson reverse phase (0-40% acetonitrile in H₂O with 0.1% formic acid) afforded the desired product **9231** (3 mg, 3%) mass calculated for formula C₂₀H₁₇N₅O₄ 391.13, observed LCMS m/z 392.2. (M+H) and compound **9231B** (22 mg, 15%), mass calculated for formula C₂₅H₂₅N₅O₆ 491.18, observed LCMS m/z 492.2. (M+H)

**Compound 9231 was synthesized using example 9231 and procedures described previously.**

### Example 9273

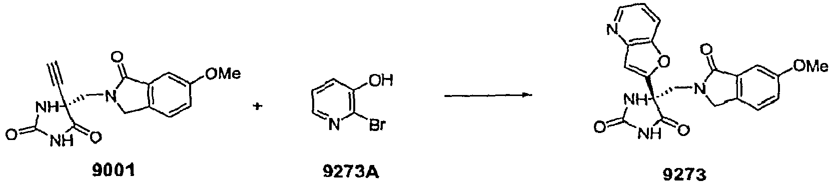

A mixture of **9001** (1.17 g, 3.91 mmol), **9273A** (2.04g, 11.7 mmol), Pd(PPh₃)₂Cl₂ (55 mg, 78 µmol), CuI (15 mg, 78 µmol), and diisopropylethylamine (3.0 mL, 17.6 mmol) in DMF (10 ml) was purged with N₂ and heated to 60 °C. After heating for 17 h, the mixture was cooled to 25 °C and a half of the solvent was removed by evaporation. The reaction mixture in DMF was purified by column chromatography on a reverse phase C-18 column (0.01%HCO₂H in water/0.01% HCO₂H in CH₃CN) to afford 9273 (1.27 g, 84% yield).

**Compound 9273 was synthesized using example 9273 and procedures described previously**

### Example 9277

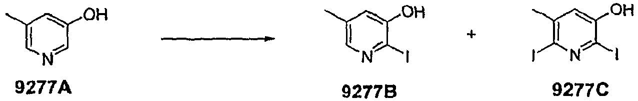

A suspension of **9277A** (202 mg, 1.85 mmol) in water (5 mL) was treated with Na₂CO₃ (411 mg, 3.89 mmol) and iodine (470 mg, 1.85 mmol) at 25 °C. The mixture was stirred for 3 h at the temperature and acidified to pH ∼ 4 by 1 N HCl solution. The resulting precipitate was filtered, washed with water, and purified by SiO₂ column chromatography (0 to 5% MeOH in CH₂Cl₂) to afford **9277B** (204 mg, 47% yield) and **9277C** (58 mg, 9% yield).

**Compound 9277was synthesized using example 9277 and procedures described previously**

### Example 9276

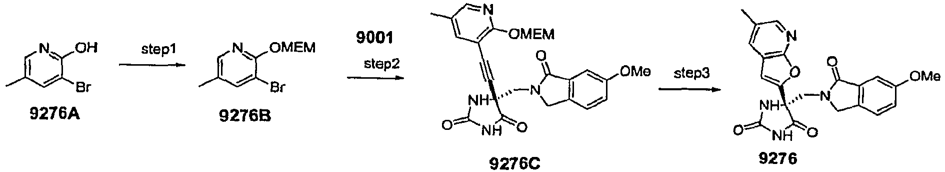

### Step1

A mixture of **9276A** (200 mg, 2.12 mmol), MEMCI (0.31 mL, 2.76 mmol), and diisopropylethylamine (1.1 mL, 6.38 mmol) in CH₂Cl₂ (10 mL) was stirred at 25 °C for 26 h. The mixture was added to aqueous NaHCO₃ solution and the organic layers were extracted with EtOAc. The combined organic solution was washed with brine solution, dried (Na₂SO₄), and concentrated in vacuo. The residue was purified by SiO₂ column chromatography to afford **9276B** (160 mg, 27% yield).

### Step2

A mixture of **9001** (80 mg, 0.26 mmol), **9276B** (81 mg, 0.29 mmol), Pd(PPh₃)₂Cl₂ (5.6 mg, 8 µmol), CuI (5 mg, 26 µmol), and diisopropylethylamine (0.09 mL, 0.53 mmol) in DMF (3 ml) was purged with N₂ and heated to 70 °C. After heating for 20 h, the mixture was cooled to 25 °C and the solvent was removed by evaporation. The residue was purified by preparative TLC (7% MeOH in CH₂Cl₂) to afford **9276C** (20 mg, 16% yield).

### Step3

A solution of **9276C** (20 mg, 0.04 mmol) in CH₂Cl₂ (2 mL) was treated with trifluoroacetic acid (0.5 mL) and the mixture was stirred at 25 °C for 16 h. The mixture was concentrated in vacuo and the residue was purified by preparative TLC to afford **9276** (3 mg, 18% yield).

**Compound 9276 was synthesized using Example 9276 and procedures described previously**

### Example 9224:

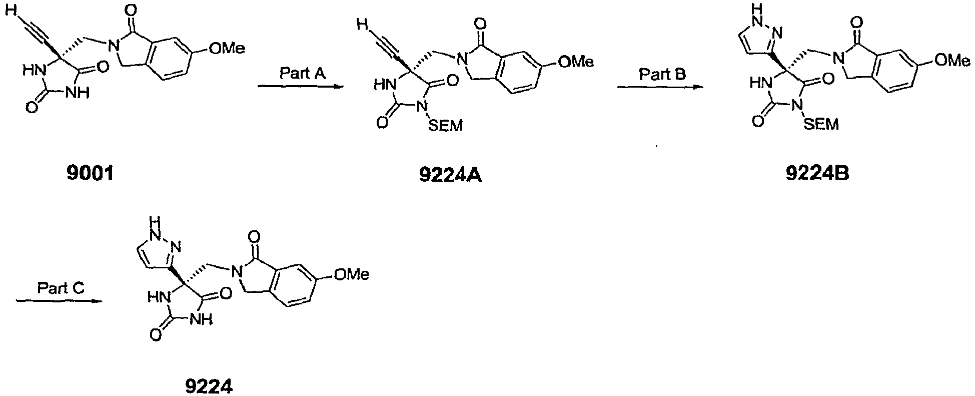

### Part A:

SEM-CI (627 mg, 3.76 mmol) was added dropwise to a solution of compound **9001** (940 mg, 3.14 mmol) and DIEA (487 mg, 3.77 mmol) in DMF (10 mL) at 0 °C. After stirring at room temperature for 12 h, the reaction mixture was concentrated; the residue was dissolved with EtOAc and H₂O. The aqueous phase was extracted with EtOAc (20 mL x 3); the organics were combined, washed with saturated NaHCO₃, brine, dried over Na₂SO₄, and concentrated. Flash column chromatography over SiO₂ (EtOAc/hexane: 60:40) afforded compound **9224A** as a white solid (1.22 g, 91%). HPLC-MS t_{R} = 1.894 min (UV_{254 nm}); mass calculated for formula C₂₁H₂₇N₃O₅Si 429.27, observed LCMS m/z 452.2 (M+Na).

### Part B:

A solution of trimethylsilyldiazomethane (1 mL, 2.0 M, 2.0 mmol) in Et₂O was added into compound **9224A** (430 mg, 1 mmol) in THF (5 mL). After stirring at 50°C for 12 h, the reaction mixture was concentrated to dryness. Flash column chromatography over SiO₂ (EtOAc) afforded compound **9224B** as a white solid (320 mg, 68%). HPLC-MS t_{R} = 1.722 min (UV_{254 nm}); mass calculated for formula C₂₂H₂₉N₅O₅Si 471.19, observed LCMS m/z 943.3 (2M+H).

### Part C:

Trifluoroboron etherate (0.1 mL) was added dropwise to a solution of compound **9224B** (85 mg, 0.18 mmol) in acetonitrile (5 mL) at 0°C. The reaction mixture was allowed to stir at room temperature for 30 min, then cooled to 0°C. DIEA (0.5 mL) and 2 mL of 1 N NaOH solution was subsequently added. After stirring at room temperature for 12 h, the solution was concentrated, and extracted with EtOAc (20 mL x 3). The organic phase was washed with brine, dried over Na₂SO₄, and concentrated. Reverse phase HPLC purification afforded compound **9224** (27 mg, 44%) as a white solid. HPLC-MS (10 min) t_{R} = 2.189 min (UV_{254 nm}); mass calculated for formula C₁₆H₁₅N₅O₄ 341.11, observed LCMS m/z 342.1 (M+H). ¹H NMR (400 MHz, DMSO-d⁶): δ 10.78 (s, 1H), 8.56 (d, *J* = 1.20 Hz, 1H), 7.73 (d, *J* = 2.43 Hz, 1H), 7.48 (d, *J* = 8.27 Hz, 1H), 7.18 - 7.14 (m, 2H), 6.21 (d, *J* = 2.20 Hz, 1H), 4.34 -4.25 (m, 2H), 4.33 - 4.02 (m, 2H), 3.81 (s, 3H).

**Compound 9224 was synthesized using example 9224 and procedures described previously**

### Example 9247

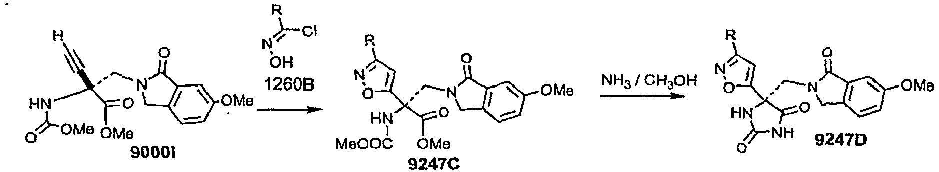

General Procedure for the Synthesis of Isoxazoles from Alkyne **9000I**

As exemplified for phenylisoxazole derivative **9247:** N-Hydroxybenzenecarboximidoyl chloride **9247B** (70 mg, 0.17 mmol) and acetylene **9000I** (30 mg, 0.2 mmol) were dissolved in 3 mL of a 1:1 tert-BuOH/H₂O mixture. While the mixture was being stirred, sodium ascorbate (1 M solution in water, 100 µ L, 10 mol %) was added, followed by copper(II) sulfate pentahydrate (3 mg in 100 µL of H₂O, 2 mol %). The reaction mixture was then treated with KHCO₃ (100 mg, 1 mmol) and left stirring for 1 h at ambient temperature, after which time it was diluted with water and the product was extracted with 2 X 50 mL ethyl acetate. The crude product was subjected to silical gel chromatography using 30% ethyl acetate / n-hexane to provide pure product **9247C** (62 mg).

Derivative **9247C** was converted to **9247D** using conditions described previously.

### Example 9267

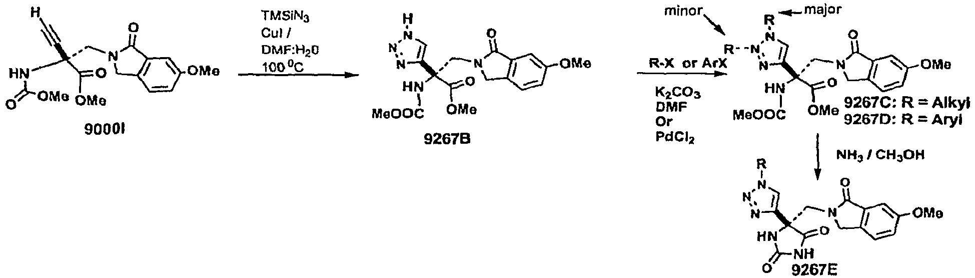

9267 **1280B** from **9000I:** (Scheme 2). Trimethylsilyl azide (0.1 mL, 0.75 mmol) was added to a DMF and MeOH solution (1 mL, 9:1) of CuI (4.8 mg, 0.025 mmol) and acetylene **9000I** (58 mg, 0.5 mmol) under N₂ in a pressure vial. The reaction mixture was stirred at 100 °C for 12 h. After consumption of **9000I**, the mixture was cooled to room temperature. The residue was purified with silica gel column chromatography (*n*-hexane/EtOAc, 10:1 to 1:1) to afford 1,2,3-triazole **9267B**.

Procedure for converting **9267B** to **9267C**: A mixture of Triazole (60 mg, 0.15mmole) and K₂CO₃ (42 mg, 0.30mmole) in DMF (3ml) was stirred at r.t. for 10 minutes, followed by addition of lodomethane (0.0115mL, 0.18mmole). Stirred at r.t. for 24 hours. By this time no more starting material left (checked by TLC). Reaction was diluted with water and extracted with EtOAc. Combined organic extract was washed with water (2 x 15mL) and saline (1 x 15mL), dried (Na₂SO₄), filtered and conc.. The crude was separated by preparative TLC (using 5%MeOH/CH₂Cl₂) to give 1-substituited triazole, 0.03g and 2-substituted triazole, 0.015g .

Procedure for converting **9267B** to **9267D:** Following the reference procedure
* , triazole (75mg, 0.19mmole) was coupled with 4-Flouro-1- iodo benzene( 51 mg, 0.23 mmole) using K₃PO₄(81 mg, 0.38mmole), CuI (1.8mg, 0.0094mmole) and Ligand (N,N-dimethyl cyclohexane diamine) (2.74mg, 0.0192mmole) in DMF ().5mL). Heated at 80°C for 18 hours. Preparative chromatography using 5% MeOH/CH₂Cl₂ gave 1-substituted triazole ,0.03g and 2-substituted substituted triazole (0.01g).
*Reference: Jon C. Antilla, Jeremy M. Baskin, Timothy E. Barder, and Stephen L. Buchwald, J. Org. Chem 2004, 69, 5578-5587

Derivatives **9267C and 9267B** were converted to **9267E** using conditions described above.

### Example 9259

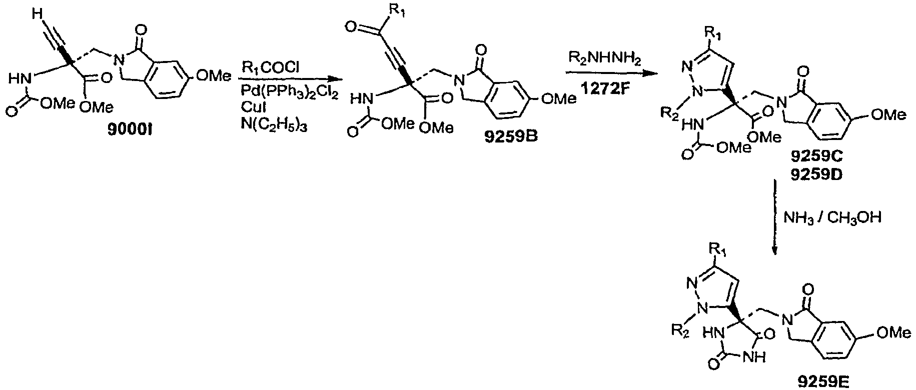

Procedure for synthesizing pyrazoles 10 (Scheme 3): A stirred mixture of Pd(PPh₃)₂Cl₂ (7 mg, 0.01 mmol) and CuI (4 mg, 0.025 mmol) in THF (2 mL) was degassed for 5 min. Then Et₃N (0.07 mL, 0.5 mmol), acid chloride (1 equiv) and acetylene **9000I** (1 equiv) were added. The reaction mixture was stirred for 1 h under N₂ at r.t. until the alkyne was completely consumed. The solvent was removed and the product was isolated using 1:1 hexanes: ethyl acetate as the eluting solvent to provide **9259B** (0.15g).

To a solution of **9259B** (75 mg, 0.16 mmol) in EtOH (3 mL) at r.t. was added methylhydrazine **9259F** (0.008 mL, 0.17 mmol). The reaction was left for 1 h after which time EtOH was removed in vacuo to afford an orange oil which was purified by column chromatography (5% EtOAc in hexane) to afford 1-methyl-3-phenyl pyrazole (**9259C**, 31 mg).

To a solution of **9259B** (75 mg, 0.16 mmol) in EtOH (3 mL) at r.t. under nitrogen was added phenylhydrazine (0.009 mL, 0.17 mmol). The solution was heated at reflux for 3-4 h and the EtOH was removed in vacuo. The residual crude was purified by column chromatography (40% EtOAc in hexane) to afford 1,5-diphenyl-pyrazole (**9259D**, 25 mg).

Derivatives **9259C** and **9259D** were converted to **9259E** using conditions described above.

**Compounds 9247-9271 were synthesized using examples 9247, 9267 and 9259 and procedures described previously.**

### Example 9933

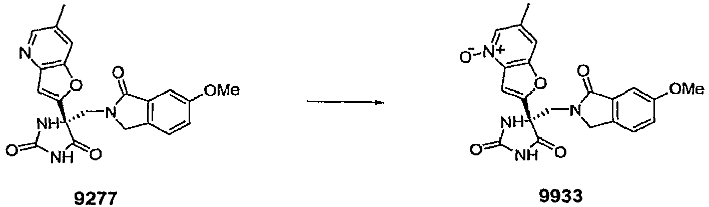

To a solution of compound **9277** (70 mg, 0.13 mmol)in CH₂Cl₂ (3 mL) was added *m*-Chloroperbenzoic acid (70 % purity, 45 mg, 0.16 mmol) at 25 °C. The mixture was stirred for 20 h at the temperature and diluted in EtOAc. The organic solution was washed with aqueous NaHCO₃ solution and brine solution, dried (Na₂SO₄), and concentrated in vacuo. The residue was purified by reverse phase C-18 column chromatography (0.1 % HCO₂H in H₂O - 0.1 % HCO₂H in CH₃CN) to afford compound **9933** (65 mg, 90 % yield).

### Example 9935

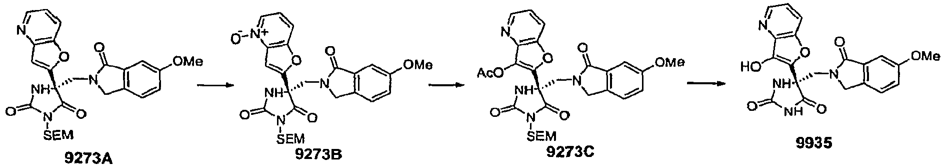

### Step1

To a solution of compound **9273A** (95 mg, 0.18 mmol)in CH₂Cl₂ (3 mL) was added *m*-Chloroperbenzoic acid (70 % purity, 54 mg, 0.22 mmol) at 25 °C. The mixture was stirred for 20 h at the temperature and diluted in EtOAc. The organic solution was washed with aqueous NaHCO₃ solution and brine solution, dried (Na₂SO₄), and concentrated in vacuo. The residue was purified by SiO₂ column chromatography (0 % to 5 % MeOH in CH₂Cl₂) to afford compound **9273B** (75 mg, 77 % yield).

### Step2

Compound **9273B** (37 mg, 0.07 mmol) was dissolved in Ac₂O (1 mL) and the solution was heated to 80 °C. After stirring for 24 h, the mixture was further stirred at 120 °C for 6 h. The mixture was cooled to 25 °C and concentrated in vacuo. The residue was purified by preparative TLC (5 % MeOH in CH₂Cl₂) to afford **9273C** (20.4 mg, 51 % yield).

### Step3

A solution of **9273C** (20 mg, 0.034 mmol) in MeOH (2.5 mL) was treated with 4N HCl in dioxane (0.6 mL). The mixture was stirred at 40 °C in a pressure vessel for 18 h and at 70 °C for 24 h. The mixture was cooled to 25 °C and concentrated in vacuo and the residue was purified by preparative TLC (5 % MeOH in CH₂Cl₂) to afford compound **9935** (9.3 mg, 67 % yield).

**Compounds 9933 and 9935 were synthesized using examples 9933, 9935 and procedures described previously.**

### Example 9651

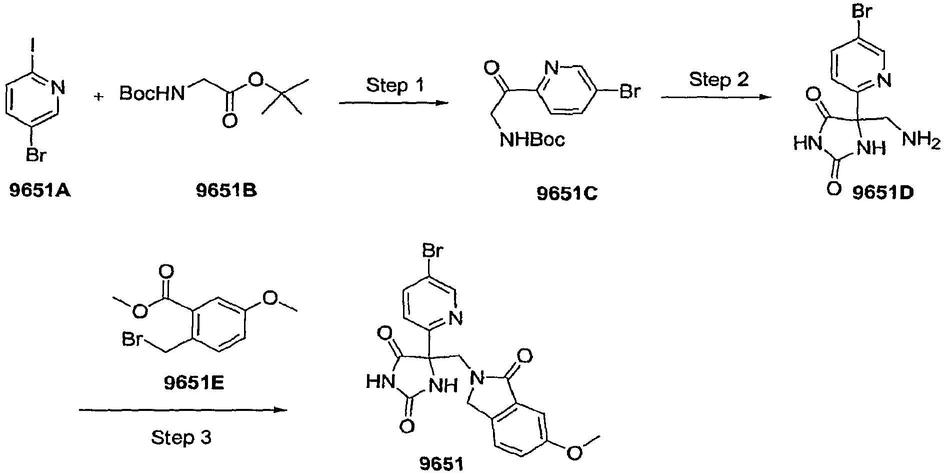

### Step 1

Compounds **9651A** (500 mg, 1.76 mmol) and **9651B** (385 mg, 1.76 mmol) were added to a flame-dried 50 mL flask and dissolved in anhydrous THF (15 mL) under a nitrogen atmosphere. The solution was cooled to 0°C and isopropyl magnesium chloride (2M in THF, 2.64 mL, 5.28 mmol) was added slowly, dropwise. The yellow solution was allowed to warm to 23°C and stir for 20 h. Saturated aqueous NH₄Cl (35 mL) was added slowly to the solution and a white precipitate appeared which was dispersed by the addition of excess water. The solution was partitioned against EtOAc (50 mL) and saturated NaCl (50 mL) twice. The organic layers were combined and dried over Na₂SO₄. The solvent was removed *in vacuum* to give a viscous orange oil which was purified by SiO₂ column chromatography (Gradient: 0-30% EtOAc/Hexanes) to afford **9651C** (2.16 g, 39%) as a white solid.

### Step 2

Compound **9651C** (2.21 g, 7.16 mmol), KCN (684 mg, 10.5 mmol), and (NH₄)₂CO₃ (2.75 g, 28.6 mmol) were added to a 150 mL pressure tube and dissolved in 7N NH₃/MeOH (16 mL) and water (16 mL). The flask was sealed and the solution was allowed to stir at 80°C for 18 h. The yellow solution was immediately purified by C18 chromatography (Gradient: 5-95% H₂O/MeCN). The solvent was removed *in vacuum* and the solid was dissolved in MeOH (10 mL). 4M HCl in dioxanes (12 mL) was added and the solution was allowed to stir for 1 h at 23°C. The solvent was removed *in vacuum* to give **9651D** (1.21 g, 60%) as a white solid.

### Step 3

Compounds **9651D** (797 mg, 2.02 mmol) and **9651E** (562 mg, 2.02 mmol) were dissolved in anhydrous DMF (10 mL) in a 25 mL flame-dried flask. DIPEA (1.76 mL, 10.1 mmol) was added and the solution was allowed to stir at 60°C for 18 h. The solvent was removed *in vacuo* and the crude product was applied to C18 chromatography (Gradient: 5-95% H₂O/MeCN). The solvent was removed *in vacuo* to afford compound **9651** (480 mg, 55%) as a white solid.

### Example 9652

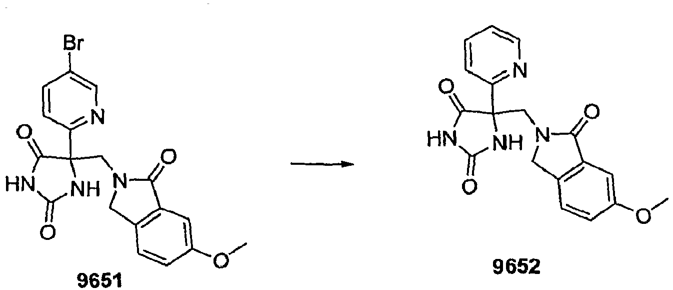

Compound **9651** was dissolved in MeOH (3 mL) and Pd/C (10%, 5 mg) was added. The suspension was allowed to stir for 5 h at 23°C. The solution was filtered and washed with DMF. C18 chromatography (Gradient: 5-90% MeCN/H₂O) afforded **9652** (7.9 mg, 33%) as a white solid.

### Example 9938

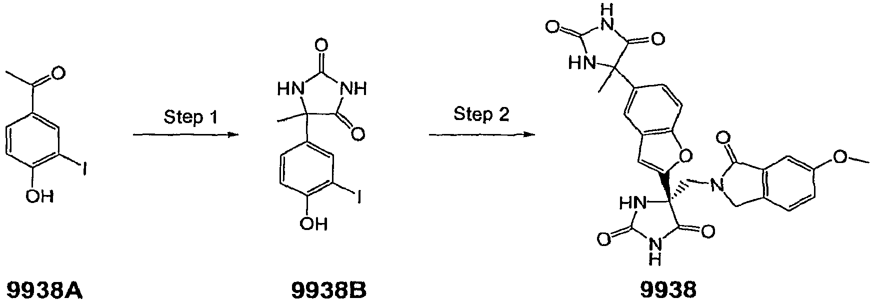

### Step 1

Compound 9938A was prepared as described in Journal of Chemical Society, Perkin Trans 1, 90, 1977**.** Compound **9938A** (4.06 g, 15.49 mmol), KCN (1.21g, 18.59 mmol), (NH₄)₂CO₃ (6.0g, 61.96 mmol), EtOH (20 mL), and water (20 mL) was suspended in a 125 mL flask and the solution was stirred at 80 °C for overnight. After cooling down, half the solution was applied to C18 reverse phase chromatography (130g, CH₃CN/water 5% to 90%) to give compound **9938B** (850mg, 33%).

### Step 2

Compound 9001 (100mg, 0.288 mmol), 9938B (96 mmol, 0.288 mmol), Pd(PPh₃)₂Cl₂ (5 mg), CuI (3 mg), and DIPA (0.2 mL) were dissolved in DMF (2 mL). The solution was degassed and filled with N₂. The solution was stirred at 60 °C for overnight. The solution was directed applied to C18 reverse phase chromatography (43g, CH₃CN/water 5% to 90%) to give crude compound **9938**, which was combined with the crude **9938** obtained from a separated reaction in the scale of 50 mg of compound **9001**, and further purified by sgc (CH₂Cl₂/MeOH/NH₃-H₂O: 15:1:0.1 to 10:1:0.1) to give compound **9938** (24.5 mg, 11.3%).

Compound **9938** and **9939** was prepared as described in example **9000**, **9001**, and **9938**.

The rest of compounds were synthesized using procedures described previously.

NMR spectral data of the some of the compounds above are given below:
**Compound 9200.** ¹H NMR (400 Hz, DMSO-d₆) δ 11.36 (s, 1H), 9.31 (s, 1H, NH), 9.10 (d, 1H, J = 1.4 Hz), 8.8 (bs, 1H), 8.23 (d, 1H, J = 6.2 Hz), 7.50 (m, 2H), 7.18 (m, 2H), 4.39 (m, 4H), 3.80 (s, 3H).
**Compound 9204**. ¹H NMR (400 Hz, DMSO-d₆) **Compound 9200**. ¹H NMR (400 Hz, DMSO-d₆) δ 11.36 (s, 1H), 9.31 (s, 1H, NH), 9.10 (d, 1H, J = 1.4 Hz), 8.8 (bs, 1H), 8.23 (d, 1H, J = 6.2 Hz), 7.50 (m, 2H), 7.18 (m, 2H), 4.39 (m, 4H), 3.80 (s, 3H).
**Compound 9217.** ¹H NMR (400 Hz, DMSO-d₆) (free forum) 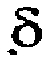 11.20 (s, 1H), 8.94 (d, J = 1.2 Hz, 1H), 8.32 (m, 1H), 8.15 (m, 1H), 7.48 (d, J = 8.0 Hz, 1H), 7.37 (m, 1H), 7.17 (m, 3H), 4.39 (dd, J = 32.8 Hz, 17.2 Hz, 2H), 4.29 (dd, J = 25.6 Hz, 14.8 Hz, 2H), 3.80 (s, 3H).
**Compound 9209**. ¹H NMR (400 Hz, DMSO-d₆) 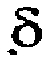 11.26 (s, 1H), 9.00 (s, 1H), 8.36 (s, 1H), 8.12 (dd, *J* = 8.34, 2.57 Hz, 1H), 7.50 (d, *J* = 8.38 Hz, 1H), 7.21-7.16 (m, 3H), 4.48 -4.25 (m, 4H), 3.81 (s, 3H).
**Compound 9221.** ¹H NMR (400 Hz, DMSO-d₆) 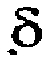 11.24 (s, 1H), 9.20 (s, 1H), 9.02 (d, J = 1.2 Hz, 1H), 8.99 (s, 1H), 7.49 (m, 1H), 7.30 (s, 1H), 7.16 (m, 3H), 4.42 (dd, J = 34 Hz, 18 Hz, 2H), 4.29 (dd, J = 30 Hz, 14 Hz, 2H), 3.80 (s, 3H).
**Compound 9222.** ¹H NMR (400 Hz, DMSO-d₆) 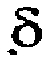 11.24 (s, 1H), 9.28 (m, 2H), 8.51 (d, J = 5.6 Hz, 1H), 8.25 (m, 1H), 7.85 (s, 1H), 7.48 (m, 1H), 7.16 (m, 2H), 4.37 (m, 1H), 4.16 (m, 1H), 3.78 (s, 3H).
**Compound 9224.** ¹H NMR (400 Hz, DMSO-d₆) 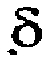 10.78 (s, 1H), 8.56 (d, *J* = 1.20 Hz, 1H), 7.73 (d, *J* = 2.43 Hz, 1H), 7.48 (d, *J* = 8.27 Hz, 1H), 7.18 - 7.14 (m, 2H), 6.21 (d, *J* = 2.20 Hz, 1H), 4.34 -4.25 (m, 2H), 4.33 - 4.02 (m, 2H), 3.81 (s, 3H).
**Compound 9225.** ¹H NMR (400 Hz, DMSO-d₆) 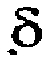 11.27 (s, 1H), 8.95 (d, *J* = 1.08 Hz, 1H), 8.62 (dd, *J* = 2.63, 1.58 Hz, 1H), 8.28-8.25 (m, 1H), 7.51 (d, *J* = 8.30 Hz, 1H), 7.42 (s, 1H), 7.19-7.16 (m, 2H), 4.50 - 4.27 (m, 4H), 3.82 (s, 3H).
**Compound 9226.** ¹H NMR (400 Hz, DMSO-d₆) 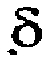 11.32 (s, 1H), 9.02 (s, 1H), 8.96 (s, 1H), 8.47 (s, 1H), 7.51 (d, *J* = 8.23 Hz, 1H), 7.46 (s, 1H), 7.21-7.16 (m, 2H), 4.49 - 4.30 (m, 4H), 3.81 (s, 3H).
**Compound 9227.** ¹H NMR (400 Hz, DMSO-d₆) 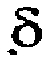 11.31 (s, 1H), 9.05 (d, *J* = 1.28 Hz, 1H), 8.88 (d, *J* = 1.18 Hz, 1H), 8.62 (d, *J* = 2.87 Hz, 1H), 7.52 - 7.50 (m, 1H), 7.42 (d, *J* = 2.82 Hz, 1H), 7.21-7.16 (m, 3H), 4.50 - 4.27 (m, 4H), 3.81 (s, 3H).
**Compound 9228.** ¹H NMR (400 Hz, DMSO-d₆) 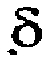 11.4 (s, 1H), 9.3 (s, 1H), 9.15 (s, 1H), 8.75 (s, 1H), 7.5 (m, 2H), 7.15 (m, 2H), 4.55 (m, 2H), 4.35 (m, 2H), 3.8 (s, 3H), 2.55 (s, 3H).
**Compound 9230**. ¹H NMR (500 Hz, MeOH-d₄) 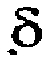 8.89 (bs, 1H,), 8.19 (bs, 1H), 7.89 (bs, 1H), 7.43 (d, 1H, J =8.5 Hz), 7.29 (d, 1H, J = 2.5 Hz), 7.195 (dd, 1H, J =8.5 Hz, 2 Hz), 6.93 (bs, 1 H), 4.51 (dd, 2H, J =16.5Hz, 9.5 Hz), 4.40 (dd, 2H, J =16 Hz, 28 Hz), 3.86 (s, 3H).
**Compound 9220.** ¹H NMR (400 MHz, DMSO-d₆) 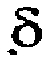 11.36 (s, 1H), 9.43 (bs, 1H), 9.13 (d, J = 1.2 Hz, 1H), 8.61 (m, 1H), 8.12 (m, 1H), 7.48 (m, 2H), 7.16 (m, 2H), 4.34 (m, 4H), 3.80 (s, 3H).
**Compound 9273.** ¹H NMR (500 MHz, CDCl₃) 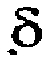 8.53 (brs, 1H), 8.25 (s, 1H), 7.81 (d, 1H, J = 8.4 Hz), 7.27 (s, 1H), 7.25 (brs, 1H), 7.15 (d, 1H, J = 8.2 Hz), 7.04 (d, 1H, J = 2.0 Hz), 6.98 (dd, 1H, J = 8.1 Hz, 2.0 Hz), 4.53 (d, 1H, J = 14.5 Hz), 4.43 (d, 1H, J = 14.5 Hz), 4.38 (d, 1H, J = 16.7 Hz), 4.15 (d, 1H, J = 16.7 Hz), 3.71 (s, 3H).
**Compound 9275.** ¹H NMR(500 MHz, DMSO-d₆) 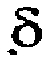 11.23 (s, 1H), 8.92 (s, 1H), 7.97 (dd, 1H, J = 8.1 Hz, 1.0 Hz), 7.51 (d, 1H, J = 8.1 Hz), 7.25 (d, 1H, J = 1.0 Hz), 7.24 (d, 1H, J = 8.6 Hz), 7.21 (d, 1H, J = 2.6 Hz), 7.18 (dd, 1H, J = 8.6 Hz, 2.6 Hz), 4.47 (d, 1H, J = 17.0 Hz), 4.38 (d, 1H, J = 17.0 Hz), 4.36 (d, 1H, J = 14.5 Hz), 4.29 (d, 1H, J = 14.5 Hz), 3.82 (s, 3H), 2.56 (s, 3H).
**Compound 9248.** ¹H NMR (500 Hz, dmso-d₆) 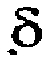 9.1 (bs, 1H,), 8.76 (s, 2H), 7.9 (d, 2H, J = 4Hz), 7.54 (d, 2H, J = 6 Hz), 7.48 (s, 1H), 7.19 (m, 3H), 4.2-4.5 (m, 4H), 3.8 (s, 3H).
**Compound 9256.** ¹H NMR (500 Hz, dmso-d₆) 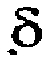 8.98 (s, 1H,), 7.52 (d, 1H, J = 6Hz), 7.16-7.2 (m, 2H), 6.58 (s, 1H), 4.35 (dd, 2H, J =16 Hz, 24 Hz), 4.21 (dd, 2H, J =16 Hz, 9 Hz), 3.82 (s, 3H), 2.22(s, 3H).
**Compound 9259.** ¹H NMR (500 Hz, dmso-d₆) 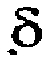 8.8 (bs, 1H,), 7.18-7.85 (m, 9H), 6.75 (s, 1H), 4.45 (dd, 2H, J = 4 Hz, 8 Hz), 4.35 (dd, 2H, J = 6 Hz, 8 Hz), 3.86 (s, 3H), 3.82 (s, 3H).
**Compound 9264.** ¹H NMR(500Hz, dmso-d₆), 10.93(s,1H), 8.66(s,1H), 8.34(s,1H), 7.52 (d,1H, J=8.197Hz), 7.18(dd, 2H, , J= 8.197Hz, J=2.52Hz), 4.88-4.81 (quintet, 1H) 4.39(s, 2H), 4.24(dd, 2H, J=14.187Hz, J= 45.38Hz), 3.8(s,3H), 1.50(dd, 6H, J=1.57Hz, J=6.62Hz)
**Compound 9271.** ¹H NMR(500Hz, dmso-d₆), 8.59(s,1H), 7.926(d, 1H, J=2.52Hz), 7.82-7.8(m, 1H), 7.48(s,1H), 6.654(d, 1H, J=8.512Hz), 6.487(d, 1H, J=2.20 Hz), 6.4(dd,1H, J= 2.52Hz, J=8.197Hz), 3.74(d, 2H, J=4.41 Hz), 3.664(s, 2H), 3.058(s, 3H)
**Compound 9003E.** ¹H NMR (400 Hz, DMSO-d₆) 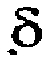 10.81 (d, 1H, J=1.2 Hz); 8.59 (d, 1H, J = 1.5 Hz); 7.82 (d, 1H, J=0.8 Hz); 7.49 (d, 1H, J=0.8 Hz); 7.51-7.49 (m, 1H); 7.18-7.15 (m, 2H); 4.48 (quintet, 1H, J=6.6 Hz); 4.39-4.30 (d of d, 2H, J=0.5, 17.4); 4.05-3.96 (d of d, 2H, J=6.9, 13.8); 3.81 (s, 3H); 1.39 (d of d, 6H, J=0.8 Hz, 6.6 Hz).
**Compound 9625.** ¹H NMR (500 MHz, MeOH-d₆) 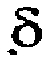 7.56-7.52 (m, 2H), 7.35-7.31(m, 1H), 7.23-20 (m, 1H), 7.12-7.10 (m, 1H), 6.97-6.94 (m, 2 H), 4.40-4.10 (m, 2H), 4.09-4.06 (m, 2H), 3.79 (s, 3H), 3.70-3.68 (m, 2H), 3.37 (s, 3H), 3.19-3.12 (m, 2H).
**Compound 9243.** ¹H NMR (500 Hz, DMSO-d₆) δ 10.89(s, 1H), 8.74 (br s, 1H), 8.29 (br. s, 1H), 8.15 (s, 1H), 7.88 (br. s, 1H), 7.53 (d, 1H, J = 9 Hz), 7.19 (m, 2H), 4.47 (s, 2H), 3.90 (d, 1H, J = 14.5 Hz), 3.82 (s, 3H), 3.73 (d, 1H, J = 14.5 Hz), 2.80 (m, 1H), 2.67 (m, 1H), 2.11 (m, 1H), 1.96 (m, 1H).
**Compound 9938**. : ¹H NMR (500 Hz, DMSO-d₆) δ 7.63 (s, 1H), 7.45 (d, 1H, J = 8.5 Hz), 7.44 (d, 1H, J = 8.5 Hz), 7.34 (d, 1H, J = 8.5Hz), 7.18 (d, 1H, J = 2.5 Hz), 7.12 (dd, 1H, J = 8.5, 2.5 Hz), 6.81 (s, 1H), 4.55 (d, 1H, J = 17.5 Hz), 4.37 (dd, 1H, J = 5, 14 Hz), 4.25 (d, 1H, J = 17.5 Hz), 3.81 (s, 3H), 3.75 (dd, 1H, J = 5.5, 14 Hz), 1.59 (s, 3H).
**Compound 9759** ¹H NMR (500 Hz, DMSO-d₆) δ 10.97(S,1H), 9.24(d, 1H, J=1.89Hz), 9.12(d, 1H, J=2.20Hz), 8.99(d, 1H, J=1.26Hz), 8.65(t, 1H, J=2.20Hz & J= 2.20 Hz), 7.96(d, 2H, J=8.82Hz), 7.82(d, 2H, J= 8.51 Hz), 7.50(d, 1H, J= 8.51 Hz), 7.2(d, 1H, J=2.20 Hz), 7.17(dd, 1H, J=2.52Hz, J=8.19 Hz), 3.82(s, 3H), 3.74-3.66(m, 2H), 3.52-3.44(m, 2H), 3.58(s, 3H)
**Compound 9711.** ¹H NMR (500 Hz, DMSO-d₆) 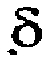 10.92 (s, 1H), 8.92 (s, 1H), 8.36 (s, 1H), 7.88 (2, 1H), 7.83-7.79 (m, 3H), 7.73 (d, 2H, J = 8.2 Hz), 7.50 (d, 1H, J = 8.6 Hz), 7.39 (d, 1H, J = 8.8 Hz), 7.20 (d, 1H, J = 2.4 Hz), 7.17 (dd, 1H, J = 8.6 Hz, 2.6 Hz), 4.36 (d, 1H, J = 17.0 Hz), 4.30 (d, 1H, J = 17.0 Hz), 4.27 (d, 1H, J = 14.2 Hz), 4.20 (s, 3H), 4.14 (d, 1H, J = 14.2 Hz), 3.82 (s, 3H).
   10.74 (s, 1H), 8.39 (d, *J* = 1.84 Hz, 1H), 7.02 (br s, 1H), 7.50 (d, *J* = 8.28 Hz, 1H), 7.19 - 7.15 (m, 2H), 6.71 (s, 1H), 4.37 (s, 2H), 4.17 (dd, *J* = 19.23, 13.97 Hz, 2H), 3.82 (s, 3H), 1.60 (d, *J* = 6.79 Hz, 6H).
**Compound 9217.** ¹H NMR (400 Hz, DMSO-d₆) (free form) 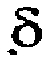 11.20 (s, 1H), 8.94 (d, J = 1.2 Hz, 1H), 8.32 (m, 1H), 8.15 (m, 1H), 7.48 (d, J = 8.0 Hz, 1H), 7.37 (m, 1H), 7.17 (m, 3H), 4.39 (dd, J = 32.8 Hz, 17.2 Hz, 2H), 4.29 (dd, J = 25.6 Hz, 14.8 Hz, 2H), 3.80 (s, 3H).
**Compound 9209.** ¹H NMR (400 Hz, DMSO-d₆) 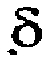 11.26 (s, 1H), 9.00 (s, 1H), 8.36 (s, 1H), 8.12 (dd, *J* = 8.34, 2.57 Hz, 1H), 7.50 (d, *J* = 8.38 Hz, 1H), 7.21-7.16 (m, 3H), 4.48 - 4.25 (m, 4H), 3.81 (s, 3H).
**Compound 9221.** ¹H NMR (400 Hz, DMSO-d₆) 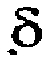 11.24 (s, 1H), 9.20 (s, 1H), 9.02 (d, J = 1.2 Hz, 1H), 8.99 (s, 1H), 7.49 (m, 1H), 7.30 (s, 1H), 7.16 (m, 3H), 4.42 (dd, J = 34 Hz, 18 Hz, 2H), 4.29 (dd, J = 30 Hz, 14 Hz, 2H), 3.80 (s, 3H).
**Compound 9222.** ¹H NMR (400 Hz, DMSO-d₆) 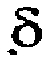 11.24 (s, 1H), 9.28 (m, 2H), 8.51 (d, J = 5.6 Hz, 1H), 8.25 (m, 1H), 7.85 (s, 1H), 7.48 (m, 1H), 7.16 (m, 2H), 4.37 (m, 1H), 4.16 (m, 1H), 3.78 (s, 3H).
**Compound 9224.** ¹H NMR (400 Hz, DMSO-d₆) 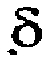 10.78 (s, 1H), 8.56 (d, *J* = 1.20 Hz, 1H), 7.73 (d, *J* = 2.43 Hz, 1H), 7.48 (d, *J* = 8.27 Hz, 1H), 7.18 - 7.14 (m, 2H), 6.21 (d, *J* = 2.20 Hz, 1H), 4.34 -4.25 (m, 2H), 4.33 - 4.02 (m, 2H), 3.81 (s, 3H).
**Compound 9225.** ¹H NMR (400 Hz, DMSO-d₆) δ 11.27 (s, 1H), 8.95 (d, *J* = 1.08 Hz, 1H), 8.62 (dd, *J* = 2.63, 1.58 Hz, 1H), 8.28-8.25 (m, 1H), 7.51 (d, *J* = 8.30 Hz, 1H), 7.42 (s, 1H), 7.19-7.16 (m, 2H), 4.50 - 4.27 (m, 4H), 3.82 (s, 3H).
**Compound 9226.** ¹H NMR (400 Hz, DMSO-d₆) 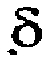 11.32 (s, 1H), 9.02 (s, 1H), 8.96 (s, 1H), 8.47 (s, 1H), 7.51 (d, *J* = 8.23 Hz, 1H), 7.46 (s, 1H), 7.21-7.16 (m, 2H), 4.49 - 4.30 (m, 4H), 3.81 (s, 3H).
**Compound 9227.** ¹H NMR (400 Hz, DMSO-d₆) δ 11.31 (s, 1H), 9.05 (d, *J* = 1.28 Hz, 1H), 8.88 (d, *J* = 1.18 Hz, 1H), 8.62 (d, *J* = 2.87 Hz, 1H), 7.52 - 7.50 (m, 1H), 7.42 (d, *J* = 2.82 Hz, 1H), 7.21-7.16 (m, 3H), 4.50 - 4.27 (m, 4H), 3.81 (s, 3H).
**Compound 9228.** ¹H NMR (400 Hz, DMSO-d₆) 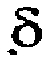 11.4 (s, 1H), 9.3 (s, 1H), 9.15 (s, 1H), 8.75 (s, 1H), 7.5 (m, 2H), 7.15 (m, 2H), 4.55 (m, 2H), 4.35 (m, 2H), 3.8 (s, 3H), 2.55 (s, 3H).
**Compound 9230**. ¹H NMR (500 Hz, MeOH-d₄) 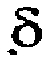 8.89 (bs, 1H,), 8.19 (bs, 1H), 7.89 (bs, 1H), 7.43 (d, 1H, J =8.5 Hz), 7.29 (d, 1H, J = 2.5 Hz), 7.195 (dd, 1H, J =8.5 Hz, 2 Hz), 6.93 (bs, 1 H), 4.51 (dd, 2H, J =16.5Hz, 9.5 Hz), 4.40 (dd, 2H, J =16 Hz, 28 Hz), 3.86 (s, 3H).
**Compound 9220**. ¹H NMR (400 MHz, DMSO-d₆) 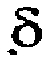 11.36 (s, 1H), 9.43 (bs, 1H), 9.13 (d, J = 1.2 Hz, 1H), 8.61 (m, 1H), 8.12 (m, 1H), 7.48 (m, 2H), 7.16 (m, 2H), 4.34 (m, 4H), 3.80 (s, 3H).
**Compound 9273**. ¹H NMR (500 MHz, CDCl₃) 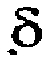 8.53 (brs, 1H), 8.25 (s, 1H), 7.81 (d, 1H, J = 8.4 Hz), 7.27 (s, 1H), 7.25 (brs, 1H), 7.15 (d, 1H, J = 8.2 Hz), 7.04 (d, 1H, J = 2.0 Hz), 6.98 (dd, 1H, J = 8.1 Hz, 2.0 Hz), 4.53 (d, 1H, J = 14.5 Hz), 4.43 (d, 1H, J = 14.5 Hz), 4.38 (d, 1H, J = 16.7 Hz), 4.15 (d, 1H, J = 16.7 Hz), 3.71 (s, 3H).
**Compound 9275.** ¹H NMR (500 MHz, DMSO-d₆) δ11.23 (s, 1H), 8.92 (s, 1H), 7.97 (dd, 1H, J = 8.1 Hz, 1.0 Hz), 7.51 (d, 1H, J = 8.1 Hz), 7.25 (d, 1H, J = 1.0 Hz), 7.24 (d, 1H, J = 8.6 Hz), 7.21 (d, 1H, J = 2.6 Hz), 7.18 (dd, 1H, J = 8.6 Hz, 2.6 Hz), 4.47 (d, 1H, J = 17.0 Hz), 4.38 (d, 1H, J = 17.0 Hz), 4.36 (d, 1H, J = 14.5 Hz), 4.29 (d, 1H, J = 14.5 Hz), 3.82 (s, 3H), 2.56 (s, 3H).
**Compound 9248.** ¹H NMR (500 Hz, dmso-d₆) 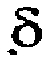 9.1 (bs, 1H,), 8.76 (s, 2H), 7.9 (d, 2H, J = 4Hz), 7.54 (d, 2H, J = 6 Hz), 7.48 (s, 1H), 7.19 (m, 3H), 4.2-4.5 (m, 4H), 3.8 (s, 3H).
**Compound 9256.** ¹H NMR (500 Hz, dmso-d₆) 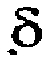 8.98 (s, 1H,), 7.52 (d, 1H, J = 6Hz), 7.16-7.2 (m, 2H), 6.58 (s, 1H), 4.35 (dd, 2H, J =16 Hz, 24 Hz), 4.21 (dd, 2H, J =16 Hz, 9 Hz), 3.82 (s, 3H), 2.22(s, 3H).
**Compound 9259.** ¹H NMR (500 Hz, dmso-d₆) 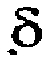 8.8 (bs, 1H,), 7.18-7.85 (m, 9H), 6.75 (s, 1H), 4.45 (dd, 2H, J = 4 Hz, 8 Hz), 4.35 (dd, 2H, J = 6 Hz, 8 Hz), 3.86 (s, 3H), 3.82 (s, 3H).
**Compound 9264.** ¹H NMR(500Hz, dmso-d₆), δ 10.93(s,1H), 8.66(s,1H), 8.34(s,1H), 7.52 (d,1H, J=8.197Hz), 7.18(dd, 2H,, J= 8.197Hz, J=2.52Hz), 4.88-4.81 (quintet, 1H) 4.39(s, 2H), 4.24(dd, 2H, J=14.187Hz, J= 45.38Hz), 3.8(s,3H), 1.50(dd, 6H, J=1.57Hz, J=6.62Hz)
**Compound 9271.** ¹H NMR(500Hz, dmso-d₆), δ 8.59(s,1H), 7.926(d, 1H, J=2.52Hz), 7.82-7.8(m,1H), 7.48(s,1H), 6.654(d, 1H, J=8.512Hz), 6.487(d, 1H, J=2.20 Hz), 6.4(dd,1H, J= 2.52Hz, J=8.197Hz), 3.74(d, 2H, J=4.41Hz), 3.664(s, 2H), 3.058(s, 3H)
**Compound 9003E**. ¹H NMR (400 Hz, DMSO-d₆) 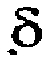 10.81 (d, 1H, J=1.2 Hz); 8.59 (d, 1H, J = 1.5 Hz); 7.82 (d, 1H, J=0.8 Hz); 7.49 (d, 1H, J=0.8 Hz); 7.51-7.49 (m, 1H); 7.18-7.15 (m, 2H); 4.48 (quintet, 1H, J=6.6 Hz); 4.39-4.30 (d of d, 2H, J=0.5, 17.4); 4.05-3.96 (d of d, 2H, J=6.9, 13.8); 3.81 (s, 3H); 1.39 (d of d, 6H, J=0.8 Hz, 6.6 Hz).
**Compound 9625.** ¹H NMR (500 MHz, MeOH-d₆) 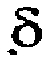 7.56-7.52 (m, 2H), 7.35-7.31 (m, 1H), 7.23-20 (m, 1H), 7.12-7.10 (m, 1H), 6.97-6.94 (m, 2 H), 4.40-4.10 (m, 2H), 4.09-4.06 (m, 2H), 3.79 (s, 3H), 3.70-3.68 (m, 2H), 3.37 (s, 3H), 3.19-3.12 (m, 2H).
**Compound 9243.** ¹H NMR (500 Hz, DMSO-d₆) 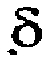 10.89(s, 1H), 8.74 (br s, 1H), 8.29 (br. s, 1H), 8.15 (s, 1H), 7.88 (br. s, 1H), 7.53 (d, 1H, J = 9 Hz), 7.19 (m, 2H), 4.47 (s, 2H), 3.90 (d, 1H, J = 14.5 Hz), 3.82 (s, 3H), 3.73 (d, 1H, J = 14.5 Hz), 2.80 (m, 1H), 2.67 (m, 1H), 2.11 (m, 1H), 1.96 (m, 1H).
**Compound 9938.** : ¹H NMR (500 Hz, DMSO-d₆) 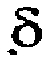 7.63 (s, 1H), 7.45 (d, 1H, J = 8.5 Hz), 7.44 (d, 1H, J = 8.5 Hz), 7.34 (d, 1H, J = 8.5Hz), 7.18 (d, 1H, J = 2.5 Hz), 7.12 (dd, 1H, J = 8.5, 2.5 Hz), 6.81 (s, 1H), 4.55 (d, 1H, J = 17.5 Hz), 4.37 (dd, 1H, J = 5, 14 Hz), 4.25 (d, 1H, J = 17.5 Hz), 3.81 (s, 3H), 3.75 (dd, 1H, J = 5.5, 14 Hz), 1.59 (s, 3H).
**Compound 9759** ¹H NMR (500 Hz, DMSO-d₆) 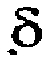 10.97(S,1H), 9.24(d, 1H, J=1.89Hz), 9.12(d, 1H, J=2.20Hz), 8.99(d, 1H, J=1.26Hz), 8.65(t, 1H, J=2.20Hz & J= 2.20 Hz), 7.96(d, 2H, J=8.82Hz), 7.82(d, 2H, J= 8.51Hz), 7.50(d, 1H, J= 8.51 Hz), 7.2(d, 1H, J=2.20 Hz), 7.17(dd, 1H, J=2.52Hz, J=8.19 Hz), 3.82(s,3H), 3.74-3.66(m,2H), 3.52-3.44(m,2H), 3.58(s, 3H)
**Compound 9711.** ¹H NMR (500 Hz, DMSO-d₆) 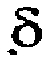 10.92 (s, 1H), 8.92 (s, 1H), 8.36 (s, 1H), 7.88 (2, 1H), 7.83-7.79 (m, 3H), 7.73 (d, 2H, J = 8.2 Hz), 7.50 (d, 1H, J = 8.6 Hz), 7.39 (d, 1H, J = 8.8 Hz), 7.20 (d, 1H, J = 2.4 Hz), 7.17 (dd, 1H, J = 8.6 Hz, 2.6 Hz), 4.36 (d, 1H, J = 17.0 Hz), 4.30 (d, 1H, J = 17.0 Hz), 4.27 (d, 1H, J = 14.2 Hz), 4.20 (s, 3H), 4.14 (d, 1H, J = 14.2 Hz), 3.82 (s, 3H).

Specific TACE inhibitory activity (Ki values) of some representative compounds of the present invention described above is set forth below:

| **Compounds** | **Structures** | **Exact Mass** | **Mass Obsvd** | **Ki (nM)** |
|---|---|---|---|---|
| **8009** | 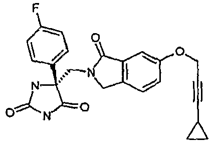 | **433.14** | **434.2[M+H]⁺** | **0.79** |
| **3009** | 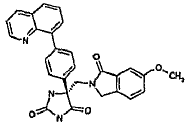 | **478.16** | **479.3[M+H]⁺** | **1.47** |
| **3010** | 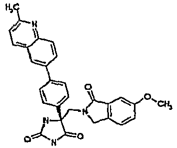 | **492.20** | **493.3[M+H]⁺** | **5** |
| **8011** | 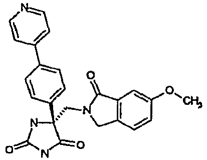 | **428.15** | **429.2[M+H]⁺** | **0.64** |
| **8012** | 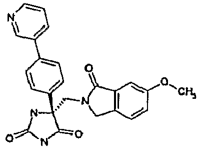 | **428.15** | **429.2[M+H]⁺** | **0.8** |
| **7011** | 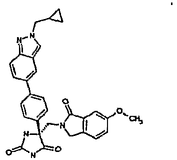 | **521.5665** | **522.3[M+H]⁺** | **2.24** |
| **7012** | 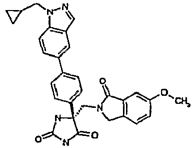 | **521.5665** | **522.3[M+H]⁺** | **2.75** |
| **8015** | 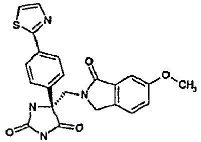 | **434.1** | **435.2[M+H]⁺** | **0.63** |
| **7016** | 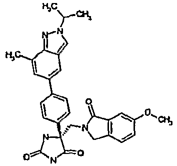 | **523.5824** | **524.3[M+H]⁺** | **3.08** |
| **7017** | 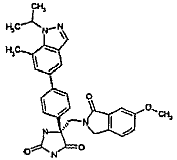 | **523.5824** | **524.3[M+H]⁺** | **3.79** |
| **4013** | 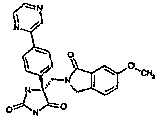 | **429.2** | **430.2 [M+H]⁺** | **1.2** |
| **7018** | 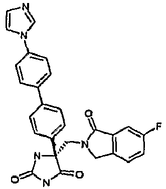 | **481.4778** | **482.3[M+H]⁺** | **3.25** |
| | | | | |
| **8016** | 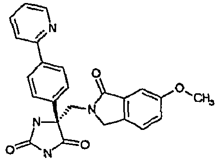 | **428.15** | **429.2[M+H]⁺** | **0.48** |
| **8019** | 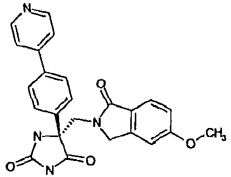 | **428.15** | **429.2[M+H]⁺** | **1** |
| **5006** | 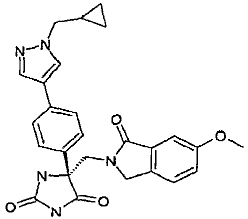 | **471.2** | **472.1 [M+H]⁺** | **0.5** |
| **7020** | 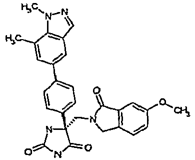 | **495.5292** | **496.3[M+H]⁺** | **2.85** |
| **4025** | 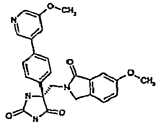 | **458.2** | **459.3 [M⁺H]⁺** | **0.84** |
| **5019** | 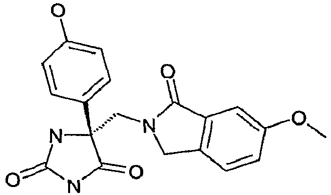 | **367.12** | **368.2[M+H]⁺** | **2** |
| **5010** | 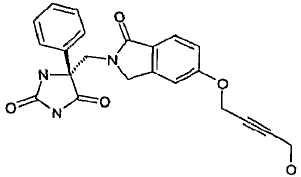 | **405.1** | **406.2[M+H]⁺** | **0.2** |
| **3041** | 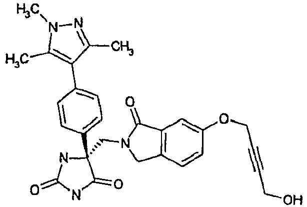 | **513.20** | **514.3[M+H]⁺** | **1.44** |
| **4012** | 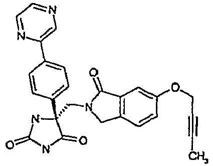 | **467.2** | **468.3 [M+H]⁺** | **0.5** |
| | | | | |
| **9003E** | 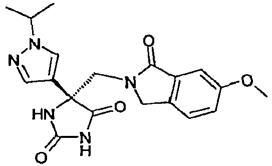 | **383.16** | **384.2[M+H]⁺** | **2.8** |
| **9938** | 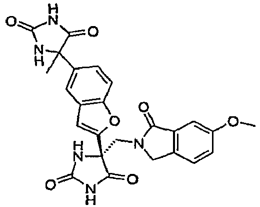 | **503.14** | **504.3[M+H]⁺** | **0.37** |
| **9217** | 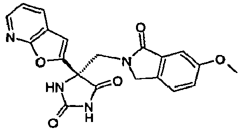 | **392.11** | **393.1 [M+H]⁺** | **0.70** |
| **9273** | 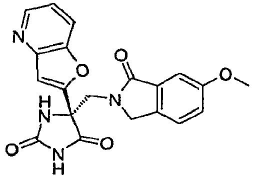 | **392.36** | **393.4[M+H]⁺** | **1.84** |
| **9220** | 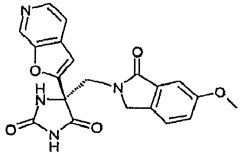 | **392.11** | **393.1 [M+H]⁺** | **0.75** |
| **9200** | 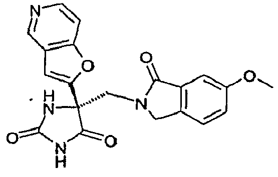 | **392.11** | **392.11** | **0.78** |
| **9225** | 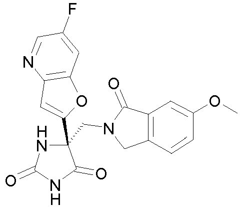 | **410.10** | **411.1[M+H]⁺** | **1.18** |
| **9227** | 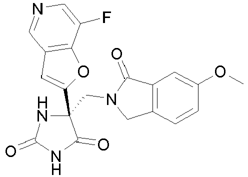 | **410.10** | **411.1[M+H]⁺** | **2.14** |
| **9275** | 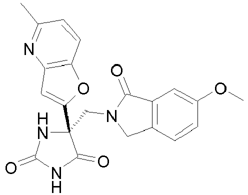 | **406.39** | **407.4[M+H]⁺** | **0.98** |
| **9256** | 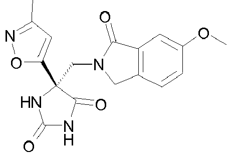 | **356.3** | **380.2[M+H]⁺** | **2.02** |

## Claims

1. A compound selected from the group consisting of:
| **Compound ID** | **Structure** |
|---|---|
| 3000 | 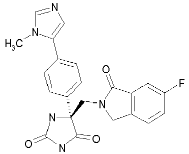 |
| 3001 | 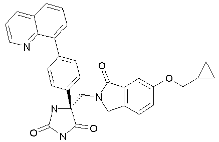 |
| 3002 | 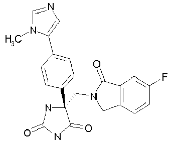 |
| 3003 | 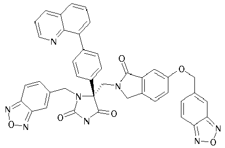 |
| 3004 | 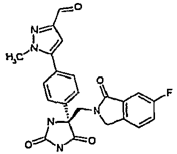 |
| 3005 | 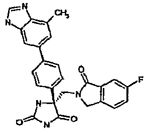 |
| 3006 | 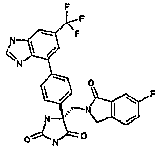 |
| 3007 | 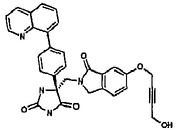 |
| 3008 | 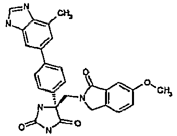 |
| 3009 | 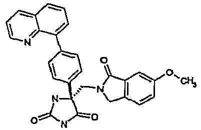 |
| 3010 | 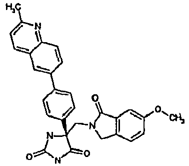 |
| 3011 | 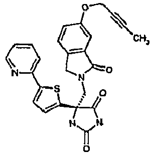 |
| 3012 | 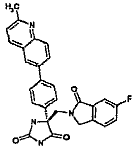 |
| 3013 | 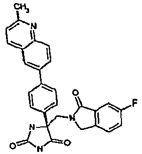 |
| 3014 | 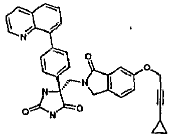 |
| 3015 | 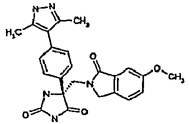 |
| 3016 | 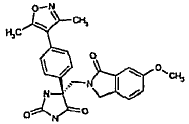 |
| 3017 | 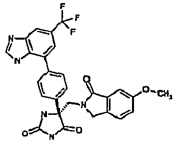 |
| 3018 | 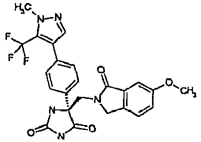 |
| 3019 | 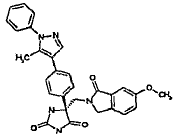 |
| 3020 | 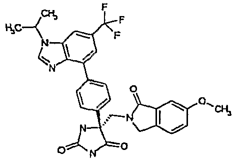 |
| 3021 | 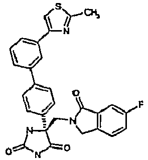 |
| 3022 | 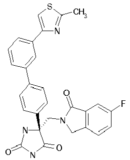 |
| 3024 | 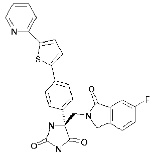 |
| 3025 | 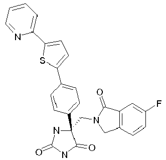 |
| 3026 | 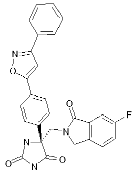 |
| 3027 | 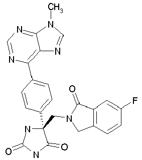 |
| 3028 | 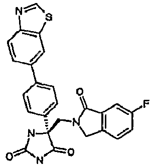 |
| 3029 | 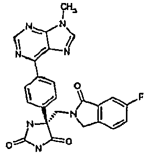 |
| 3030 | 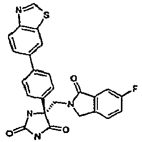 |
| 3031 | 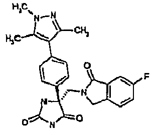 |
| 3032 | 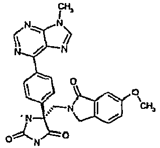 |
| 3033 | 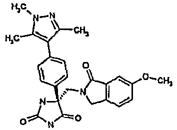 |
| 3034 | 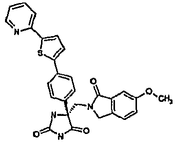 |
| 3035 | 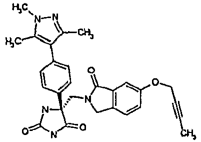 |
| 3003 | 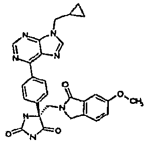 |
| 3037 | 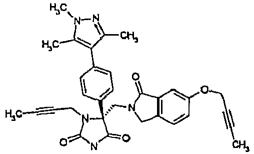 |
| 3038 | 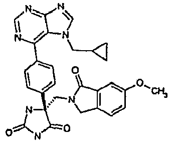 |
| 3039 | 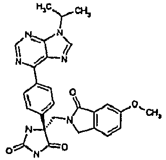 |
| 3040 | 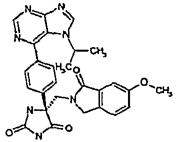 |
| 3041 | 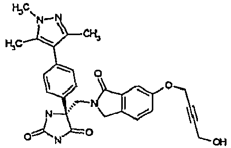 |
| 3042 | 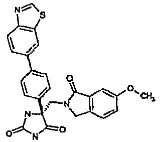 |
| 3043 | 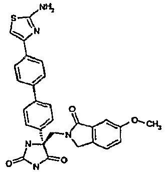 |
| 3044 | 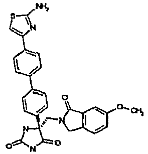 |
| 3045 | 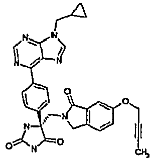 |
| 3046 | 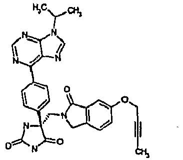 |
| 3047 | 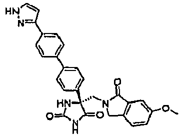 |
| 3048 | 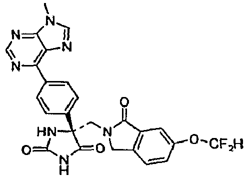 |
| 4001 | 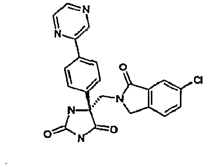 |
| 4002 | 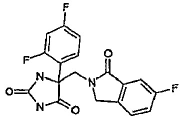 |
| 4003 | 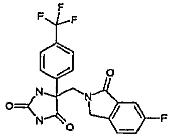 |
| 4004 | 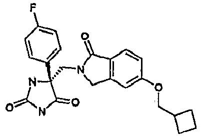 |
| 4005 | 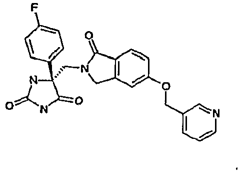 |
| 4006 | 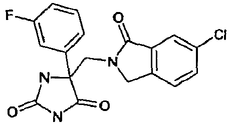 |
| 4007 | 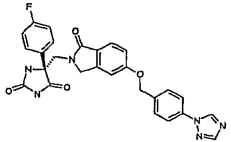 |
| 4008 | 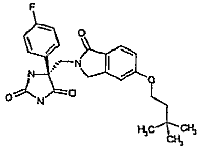 |
| 4009 | 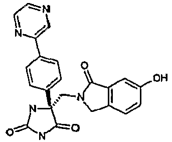 |
| 4010 | 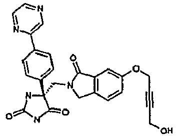 |
| 4011 | 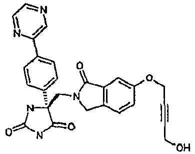 |
| 4012 | 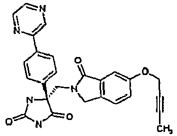 |
| 4013 | 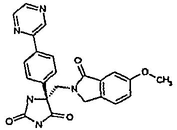 |
| 4014 | 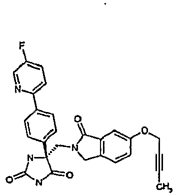 |
| | |
| 4015 | 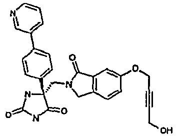 |
| 4016 | 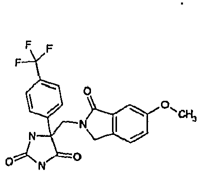 |
| 4017 | 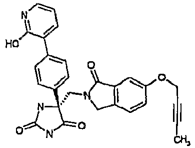 |
| 4018 | 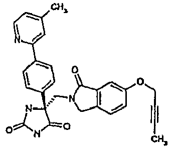 |
| 4019 | 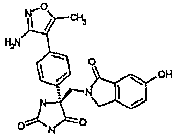 |
| 4020 | 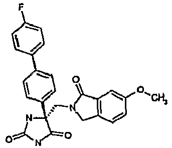 |
| 4021 | 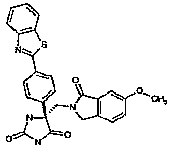 |
| 4022 | 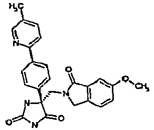 |
| 4023 | 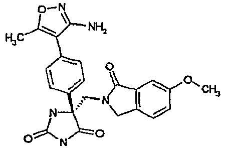 |
| 4024 | 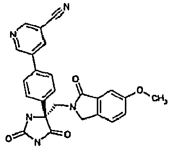 |
| 4025 | 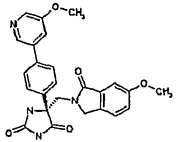 |
| | |
| 4026 | 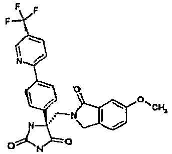 |
| 4027 | 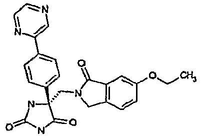 |
| 4028 | 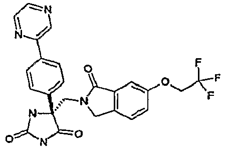 |
| 4029 | 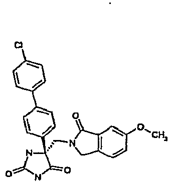 |
| 4030 | 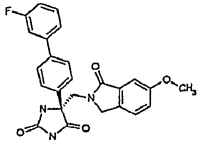 |
| | |
| 4031 | 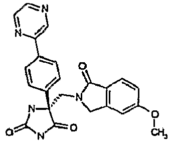 |
| 4032 | 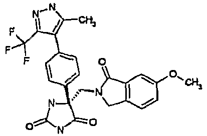 |
| 4033 | 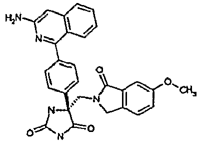 |
| 4034 | 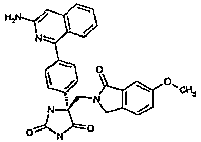 |
| 4035 | 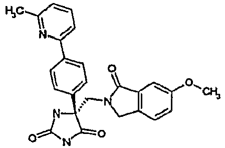 |
| 4036 | 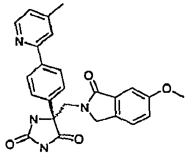 |
| 4037 | 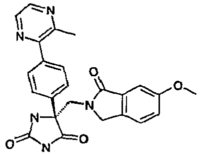 |
| 4038 | 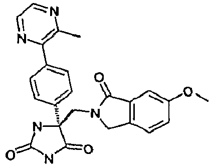 |
| 4039 | 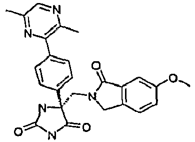 |
| 4040 | 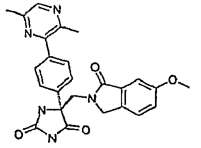 |
| 4041 | 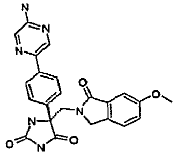 |
| 4042 | 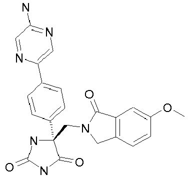 |
| 5000 | 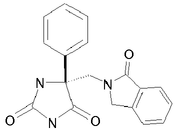 |
| 5001 | 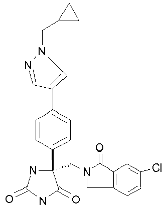 |
| 5003 | 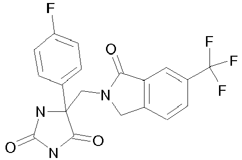 |
| 5005 | 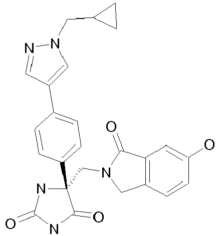 |
| 5006 | 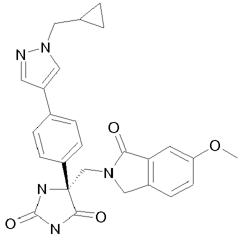 |
| 5007 | 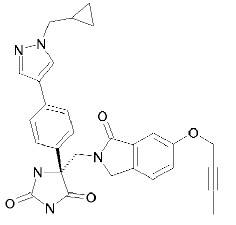 |
| 5009 | 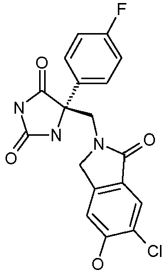 |
| 5010 | 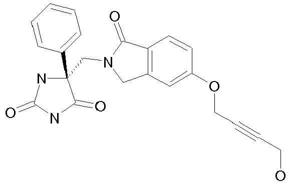 |
| 5011 | 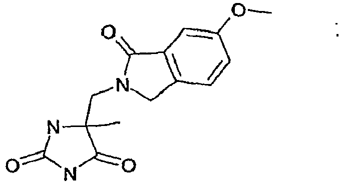 |
| 5012 | 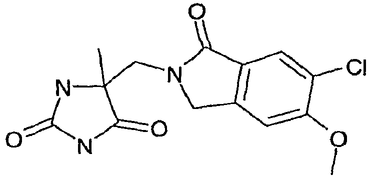 |
| 5013 | 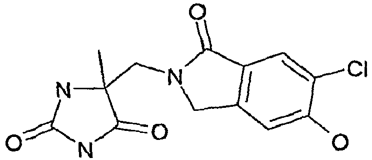 |
| 5014 | 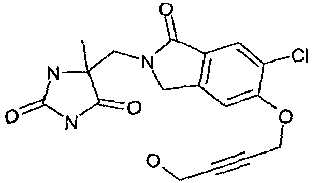 |
| 5015 | 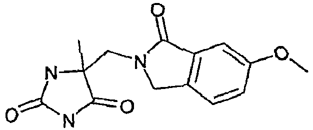 |
| 5016 | 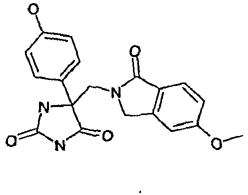 |
| 5017 | 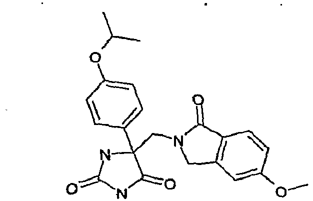 |
| 5018 | 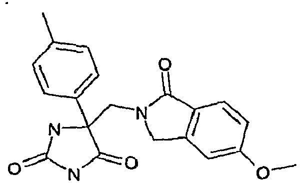 |
| 5019 | 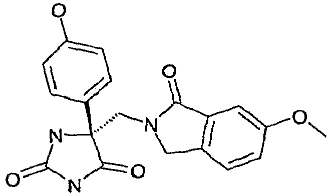 |
| 5020 | 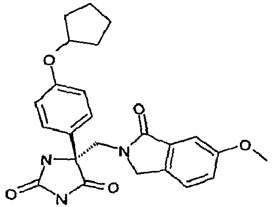 |
| 5021 | 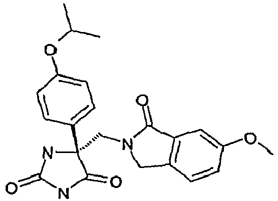 |
| 5022 | 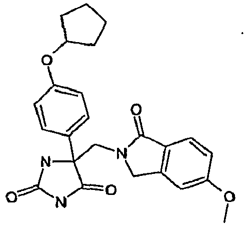 |
| 5023 | 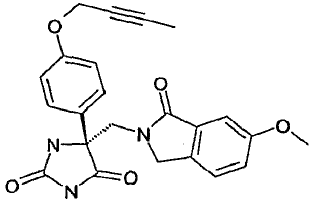 |
| 5024 | 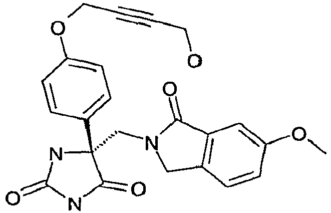 |
| 5025 | 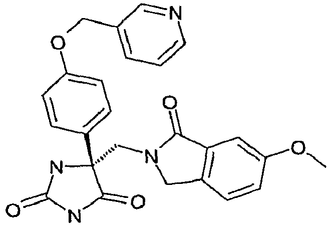 |
| 6000 | 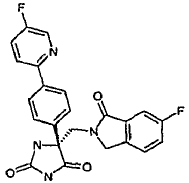 |
| 6001 | 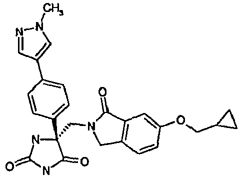 |
| 6002 | 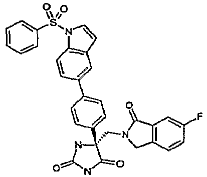 |
| 6003 | 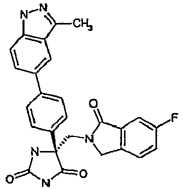 |
| 6004 | 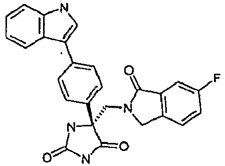 |
| 6005 | 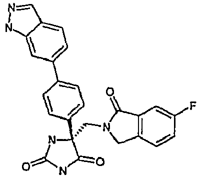 |
| 6006 | 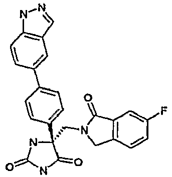 |
| 6007 | 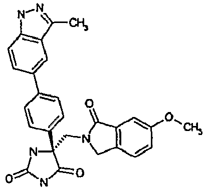 |
| 6008 | 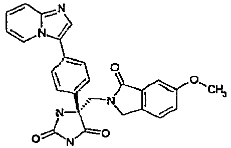 |
| 6009 | 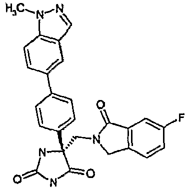 |
| 6010 | 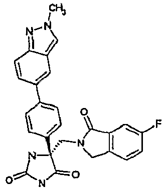 |
| 6011 | 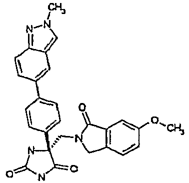 |
| 6012 | 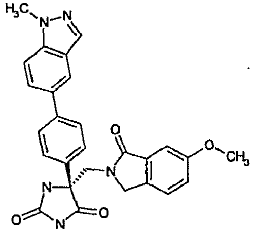 |
| 6013 | 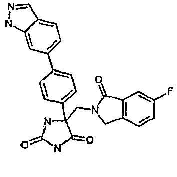 |
| 6014 | 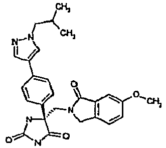 |
| 6015 | 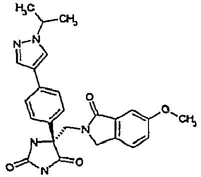 |
| 6016 | 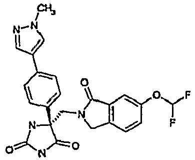 |
| 6017 | 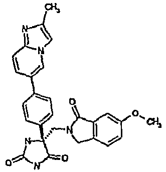 |
| 6018 | 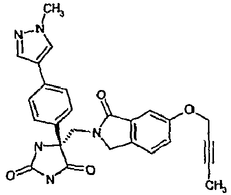 |
| 6019 | 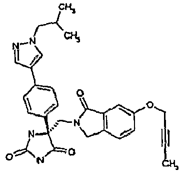 |
| 6020 | 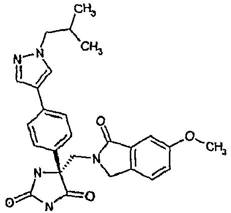 |
| 6021 | 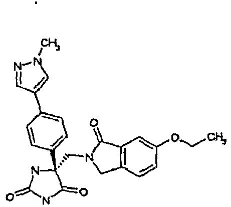 |
| | |
| 6022 | 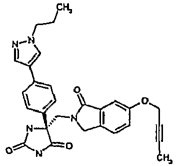 |
| 6023 | 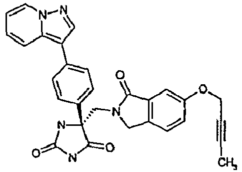 |
| 6024 | 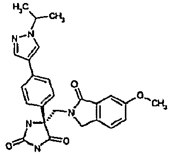 |
| 6025 | 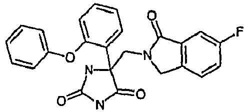 |
| 6026 | 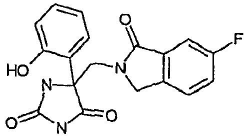 |
| 6027 | 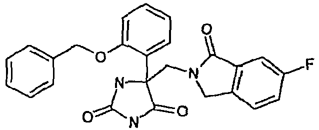 |
| 6028 | 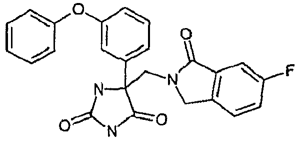 |
| 6029 | 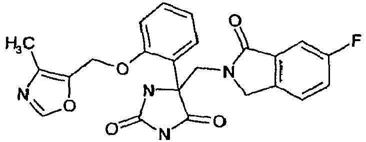 |
| 7000 | 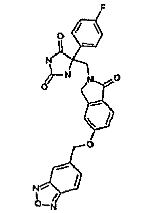 |
| 7001 | 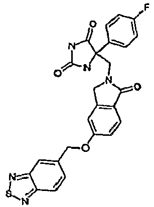 |
| 7002 | 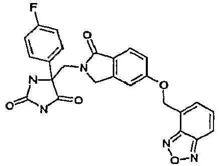 |
| 7003 | 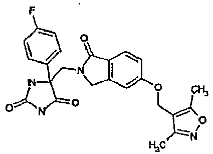 |
| 7004 | 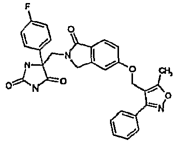 |
| 7005 | 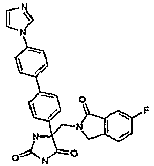 |
| 7006 | 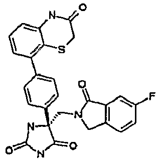 |
| 7007 | 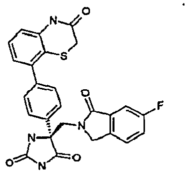 |
| 7008 | 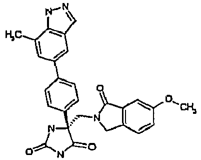 |
| 7009 | 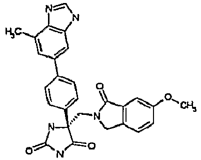 |
| 7010 | 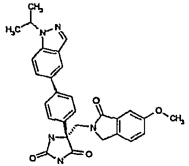 |
| 7011 | 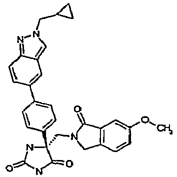 |
| 7012 | 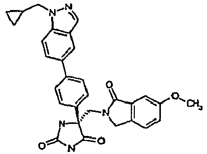 |
| 7013 | 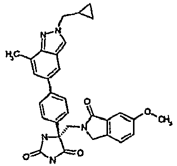 |
| 7014 | 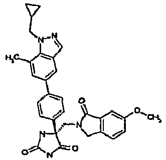 |
| 7015 | 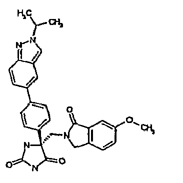 |
| 7016 | 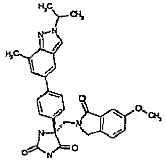 |
| 7017 | 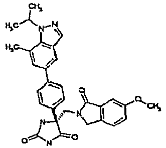 |
| 7018 | 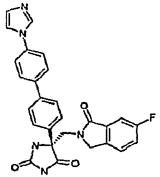 |
| 7019 | 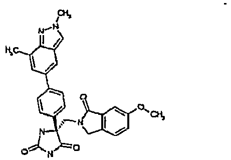 |
| 7020 | 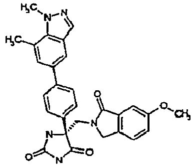 |
| 7021 | 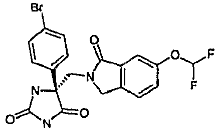 |
| 7022 | 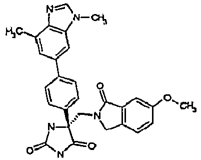 |
| 7023 | 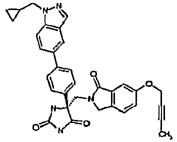 |
| 7024 | 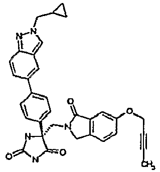 |
| 7025 | 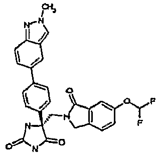 |
| 7026 | 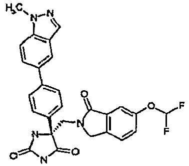 |
| 8001 | 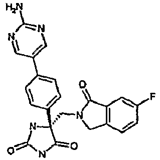 |
| 8002 | 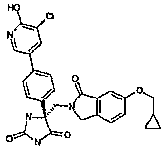 |
| 8003 | 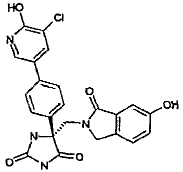 |
| 8004 | 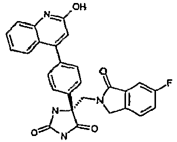 |
| 8005 | 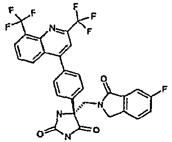 |
| 8006 | 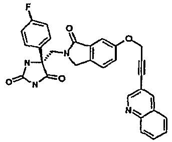 |
| 8007 | 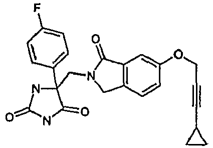 |
| 8008 | 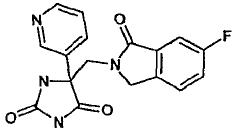 |
| 8009 | 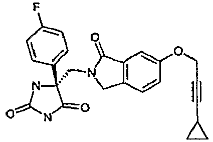 |
| 8010 | 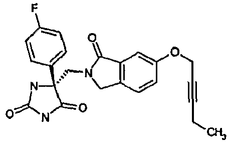 |
| 8011 | 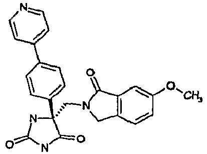 |
| 8012 | 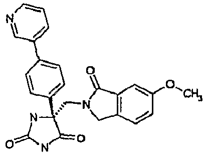 |
| 8013 | 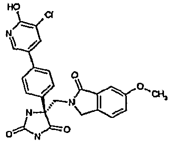 |
| 8014 | 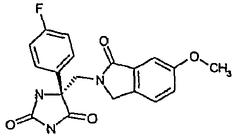 |
| 8015 | 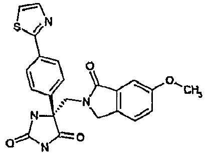 |
| 8016 | 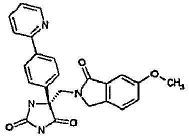 |
| 8017 | 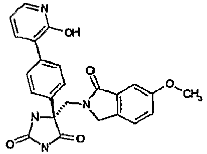 |
| 8018 | 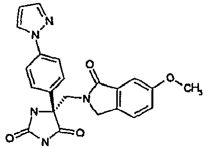 |
| 8019 | 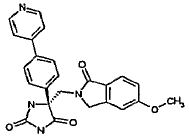 |
| 8020 | 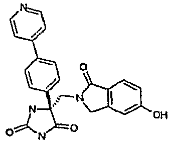 |
| 8021 | 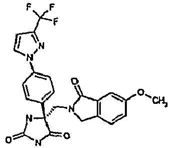 |
| 8022 | 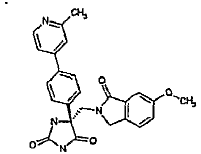 |
| 8023 | 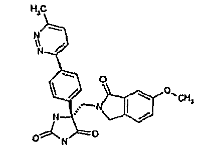 |
| 8024 | 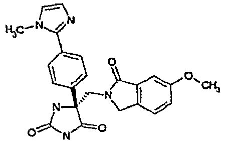 |
| 8025 | 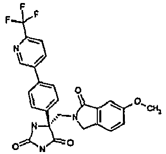 |
| 8027 | 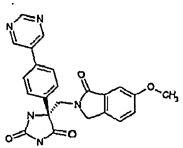 |
| 2021F | 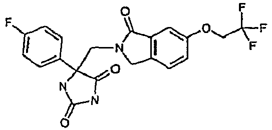 |
| 2023C | 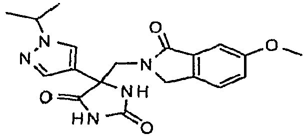 |
| 2024D | 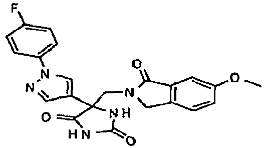 |
| 2022G | 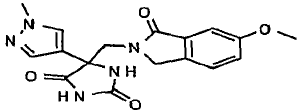 |
| 2025C | 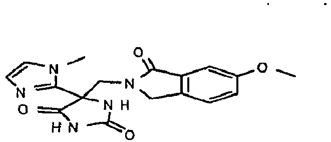 |
| 2028 | 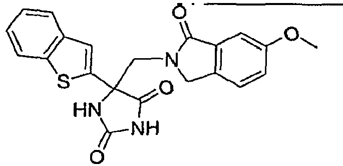 |
| 2026H | 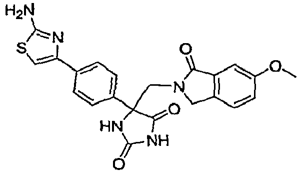 |
| 2030A | 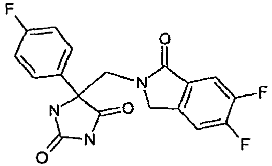 |
| 2030C | 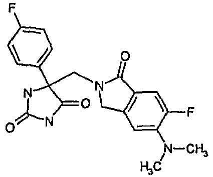 |
| 2030B | 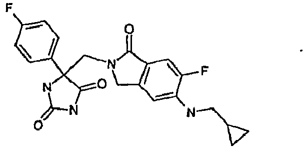 |
| 2031C | 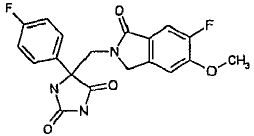 |
| 2031D | 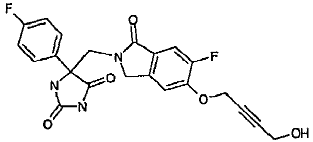 |
| 2030D | 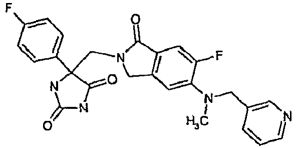 |
| 2031A | 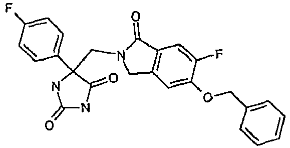 |
| 2031B | 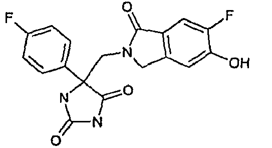 |
| 2032B | 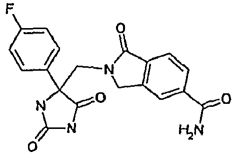 |
| 2031E | 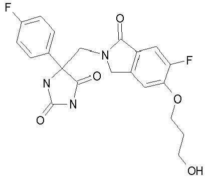 |
| 2031F | 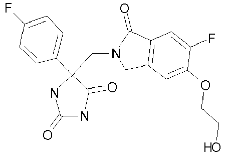 |
| 9200 | 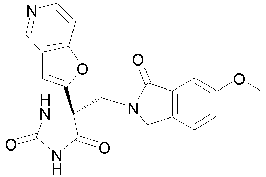 |
or a pharmaceutically acceptable salt or solvate thereof.

2. The compound of claim 1, selected from the group consisting of: 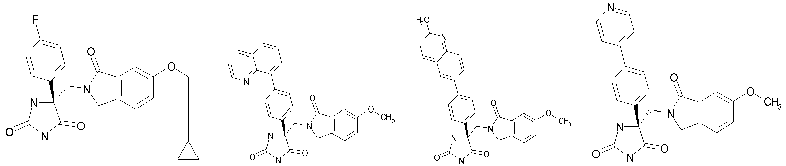 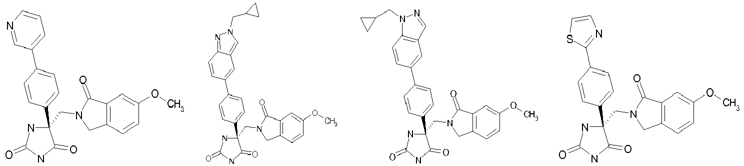 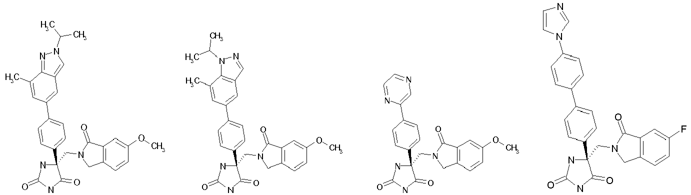 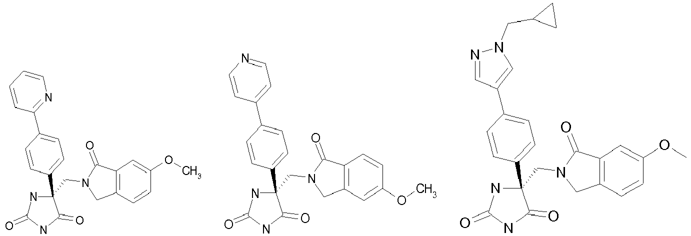 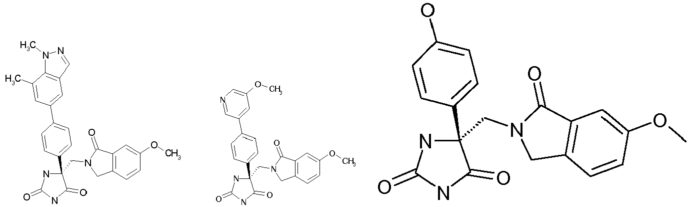 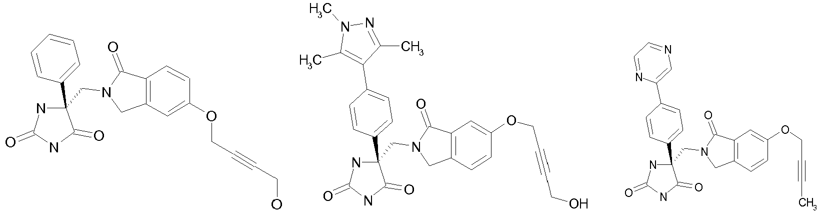 or a pharmaceutically acceptable salt or solvate thereof.

3. The compound of claim 1 which is 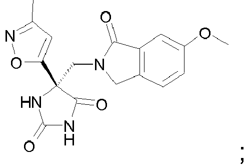 or a pharmaceutically acceptable salt, or solvate thereof.

4. A pharmaceutical composition comprising at least one compound of claim 1, 2 or 3 or a pharmaceutically acceptable salt or solvate thereof, and at least one pharmaceutically acceptable carrier.

5. A compound of claim 1, 2 or 3 or a pharmaceutically acceptable salt or solvate thereof for use in a method of treating the human body by therapy.

6. A compound for use of claim 5 or a pharmaceutically acceptable salt or solvate thereof wherein the therapy is treating a condition or disease selected from rheumatoid arthritis, osteoarthritis, periodontitis, gingivitis, corneal ulceration, solid tumor growth and tumor invasion by secondary metastases, neovascular glaucoma, inflammatory bowel disease, multiple sclerosis and psoriasis.

7. A compound for use of claim 5 or a pharmaceutically acceptable salt or solvate thereof wherein the therapy is treating a condition or disease selected from fever, cardiovascular conditions, hemorrhage, coagulation, cachexia, anorexia, alcoholism, acute phase response, acute infection, shock, graft versus host reaction, autoimmune disease and HIV infection.

8. A compound for use of claim 5 or a pharmaceutically acceptable salt or solvate thereof wherein the therapy is treating a condition or disease selected from septic shock, haemodynamic shock, sepsis syndrome, post ischaemic reperfusion injury, malaria, mycobacterial infection, meningitis, psoriasis, congestive heart failure, fibrotic diseases, cachexia, graft rejection, cancers such as cutaneous T-cell lymphoma, diseases involving angiogenesis, autoimmune diseases, skin inflammatory diseases, inflammatory bowel diseases such as Crohn's disease and colitis, osteo and rheumatoid arthritis, ankylosing spondylitis, psoriatic arthritis, adult Still's disease, ureitis, Wegener's granulomatosis, Behcehe disease, Sjogren's syndrome, sarcoidosis, polymyositis, dermatomyositis, multiple sclerosis, sciatica, complex regional pain syndrome, radiation damage, hyperoxic alveolar injury, periodontal disease, HIV, non-insulin dependent diabetes mellitus, systemic lupus erythematosus, glaucoma, sarcoidosis, idiopathic pulmonary fibrosis, bronchopulmonary dysplasia, retinal disease, scleroderma, osteoporosis, renal ischemia, myocardial infarction, cerebral stroke, cerebral ischemia, nephritis, hepatitis, glomerulonephritis, cryptogenic fibrosing aveolitis, psoriasis, transplant rejection, atopic dermatitis, vasculitis, allergy, seasonal allergic rhinitis, reversible airway obstruction, adult respiratory distress syndrome, asthma, chronic obstructive pulmonary disease (COPD) and bronchitis.

9. A compound of claim 1, 2 or 3 or a pharmaceutically acceptable salt or solvate thereof, in combination with a compound selected from the group consisting of Avonex, Betaseron, Copaxone or other compounds indicated for the treatment of multiple sclerosis.

10. A combination of a compound of claim 1, 2 or 3 or a pharmaceutically acceptable salt or solvate thereof and at least one medicament selected from disease modifying anti-rheumatic drugs (DMARDS), non-steroidal anti-inflammatory drugs (NSAIDs), cycloxygenase-2 selective (COX-2) inhibitors, COX-1 inhibitors, immunosuppressives, biological response modifiers (BRMs), anti-inflammatory agents and H1 antagonists.

11. Use of a compound of claim 1, 2 or 3 or a pharmaceutically acceptable salt of solvate thereof for the manufacturer of a medicament for treating a condition or disease as defined in claim 7, 8 or 9.

## Patentansprüche

1. Eine Verbindung, ausgewählt aus der Gruppe, bestehend aus
| **Verbindung ID** | **Struktur** |
|---|---|
| 3000 | 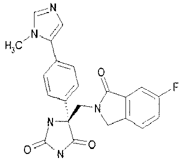 |
| 3001 | 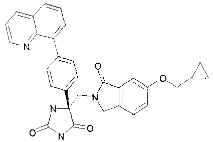 |
| 3002 | 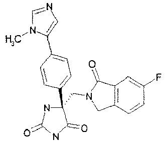 |
| 3003 | 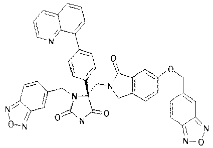 |
| 3004 | 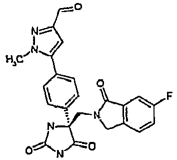 |
| 3005 | 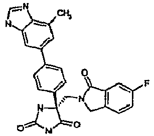 |
| 3006 | 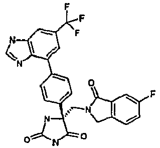 |
| 3007 | 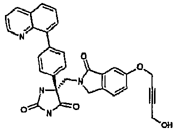 |
| 3008 | 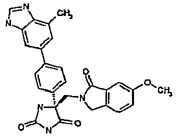 |
| 3009 | 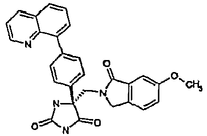 |
| 3010 | 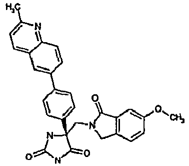 |
| 3011 | 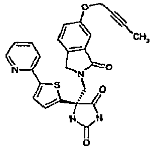 |
| 3012 | 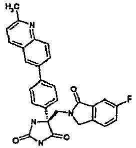 |
| 3013 | 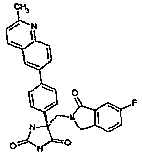 |
| 3014 | 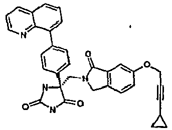 |
| 3015 | 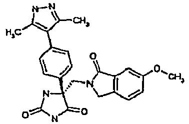 |
| 3016 | 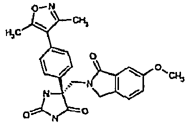 |
| 3017 | 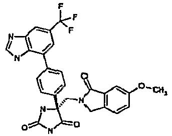 |
| 3018 | 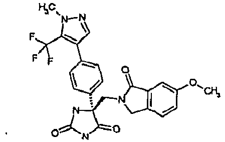 |
| 3019 | 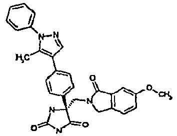 |
| 3020 | 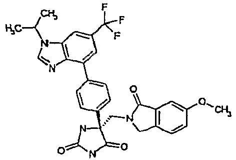 |
| 3021 | 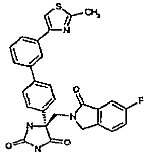 |
| 3022 | 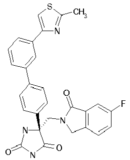 |
| 3024 | 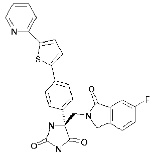 |
| 3025 | 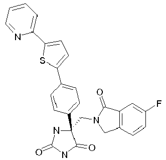 |
| 3026 | 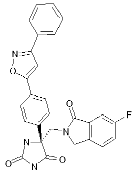 |
| 3027 | 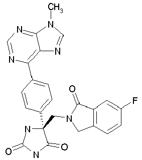 |
| 3028 | 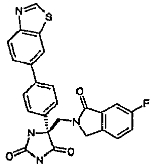 |
| 3029 | 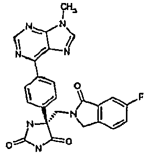 |
| 3030 | 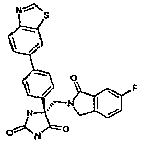 |
| 3031 | 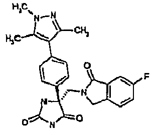 |
| 3032 | 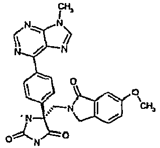 |
| 3033 | 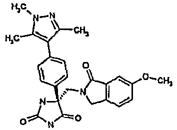 |
| 3034 | 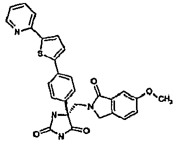 |
| 3035 | 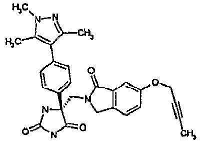 |
| 3003 | 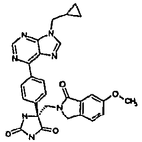 |
| 3037 | 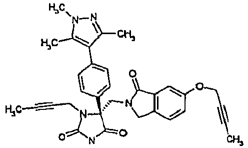 |
| 3038 | 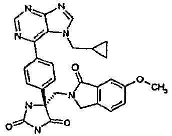 |
| 3039 | 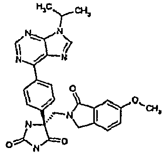 |
| 3040 | 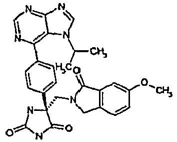 |
| 3041 | 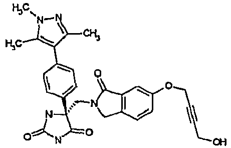 |
| 3042 | 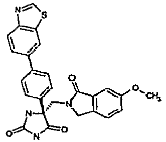 |
| 3043 | 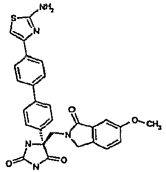 |
| 3044 | 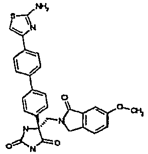 |
| 3045 | 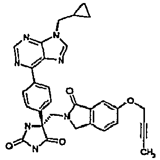 |
| 3046 | 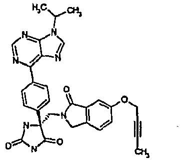 |
| 3047 | 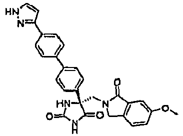 |
| 3048 | 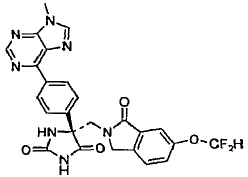 |
| 4001 | 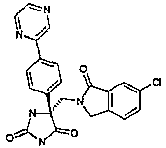 |
| 4002 | 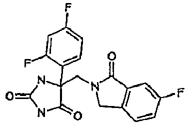 |
| 4003 | 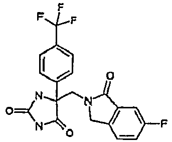 |
| 4004 | 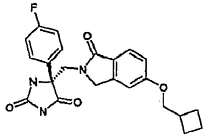 |
| 4005 | 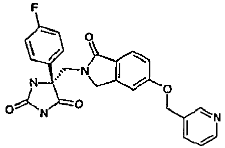 |
| 4006 | 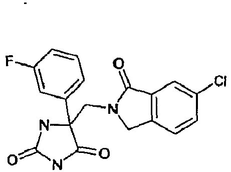 |
| 4007 | 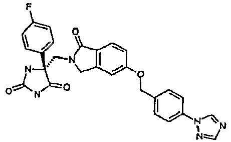 |
| 4008 | 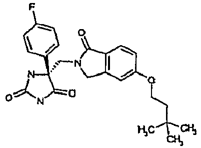 |
| 4009 | 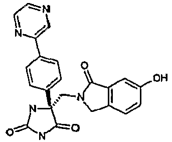 |
| 4010 | 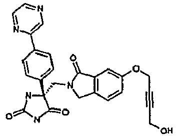 |
| 4011 | 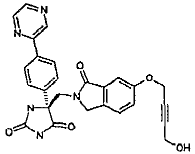 |
| 4012 | 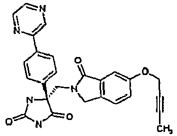 |
| 4013 | 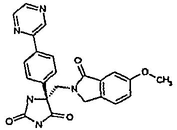 |
| 4014 | 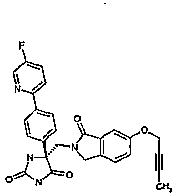 |
| 4015 | 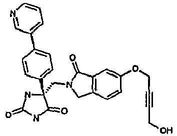 |
| 4016 | 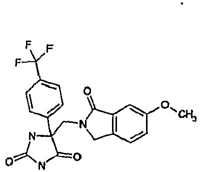 |
| 4017 | 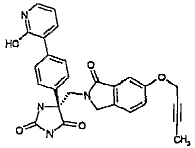 |
| 4018 | 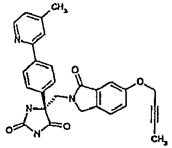 |
| 4019 | 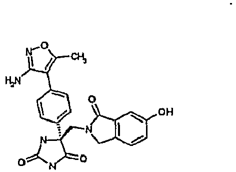 |
| 4020 | 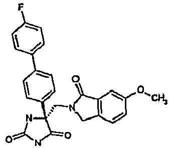 |
| 4021 | 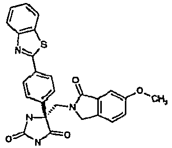 |
| 4022 | 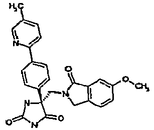 |
| 4023 | 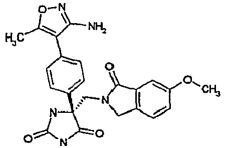 |
| 4024 | 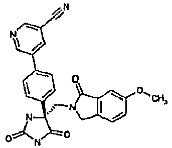 |
| 4025 | 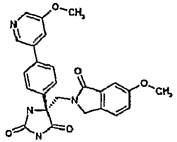 |
| 4026 | 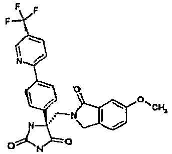 |
| 4027 | 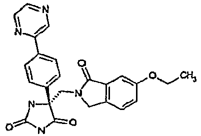 |
| 4028 | 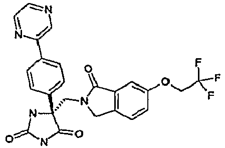 |
| 4029 | 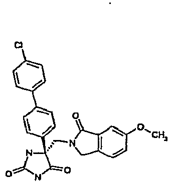 |
| 4030 | 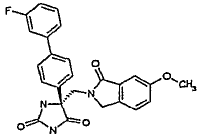 |
| 4031 | 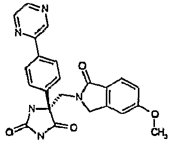 |
| 4032 | 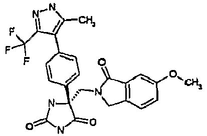 |
| 4033 | 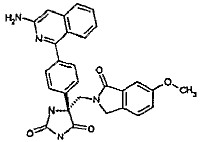 |
| 4034 | 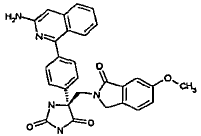 |
| 4035 | 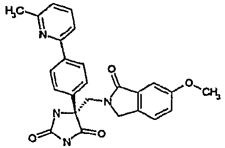 |
| 4036 | 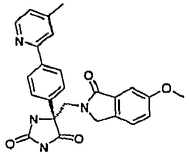 |
| 4037 | 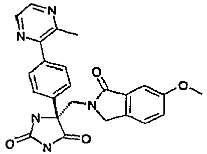 |
| 4038 | 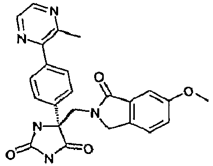 |
| 4039 | 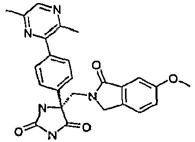 |
| 4040 | 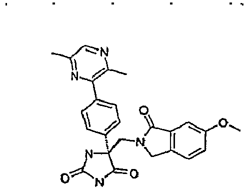 |
| 4041 | 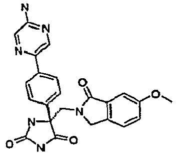 |
| 4042 | 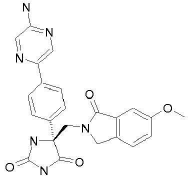 |
| 5000 | 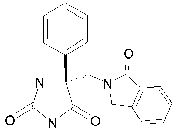 |
| 5001 | 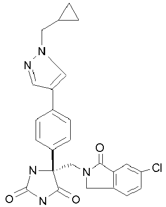 |
| 5003 | 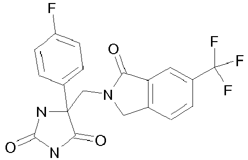 |
| 5005 | 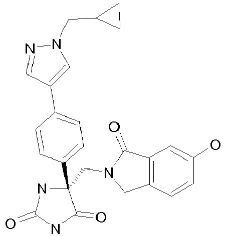 |
| 5006 | 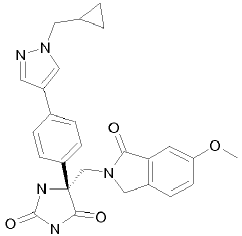 |
| 5007 | 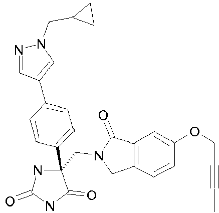 |
| 5009 | 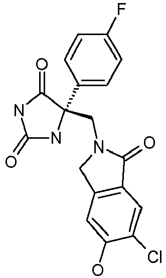 |
| 5010 | 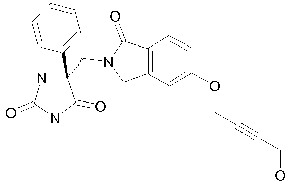 |
| 5011 | 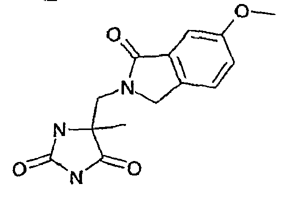 |
| 5012 | 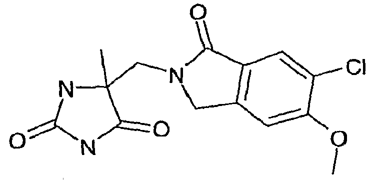 |
| 5013 | 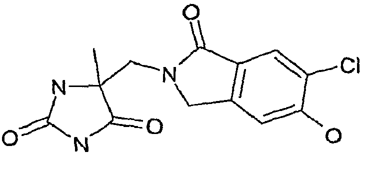 |
| 5014 | 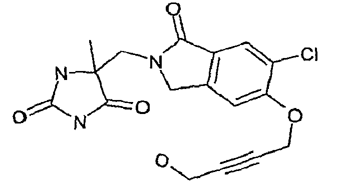 |
| 5015 | 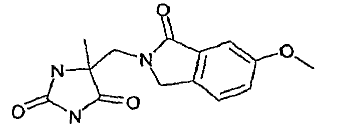 |
| 5016 | 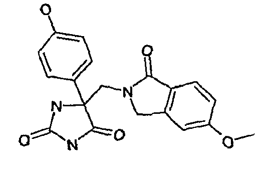 |
| 5017 | 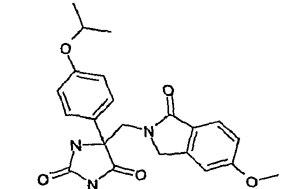 |
| 5018 | 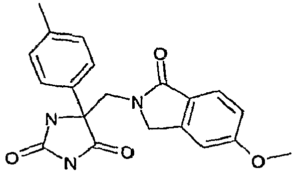 |
| 5019 | 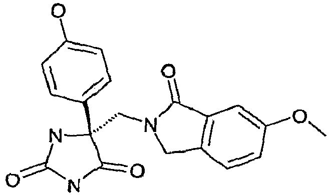 |
| 5020 | 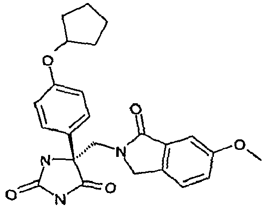 |
| 5021 | 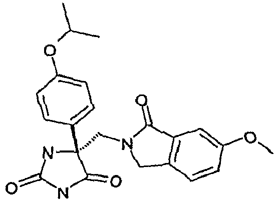 |
| 5022 | 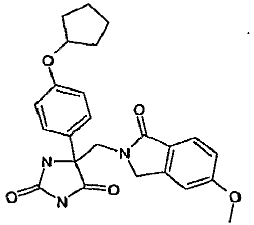 |
| 5023 | 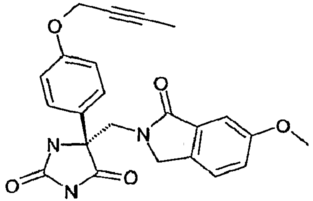 |
| 5024 | 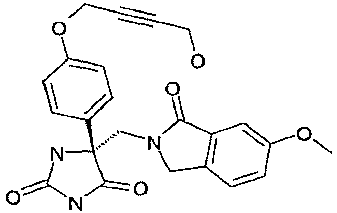 |
| 5025 | 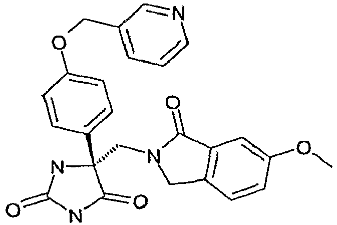 |
| 6000 | 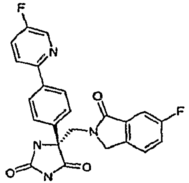 |
| 6001 | 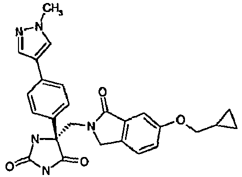 |
| 6002 | 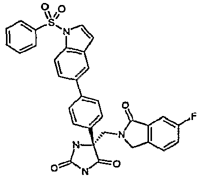 |
| 6003 | 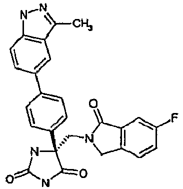 |
| 6004 | 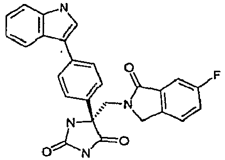 |
| 6005 | 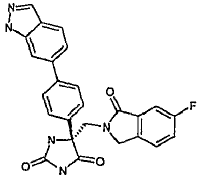 |
| 6006 | 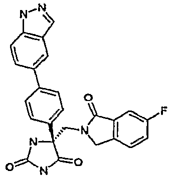 |
| 6007 | 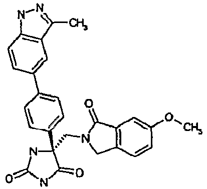 |
| 6008 | 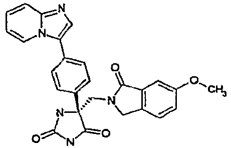 |
| 6009 | 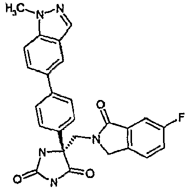 |
| 6010 | 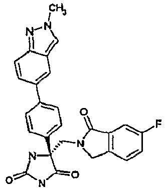 |
| 6011 | 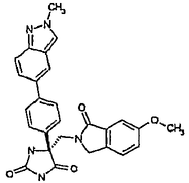 |
| 6012 | 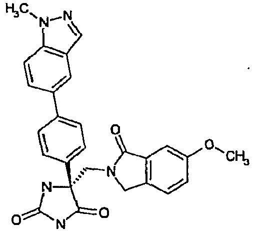 |
| 6013 | 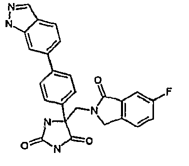 |
| 6014 | 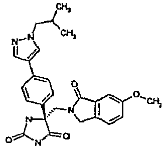 |
| 6015 | 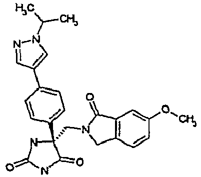 |
| 6016 | 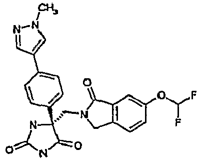 |
| 6017 | 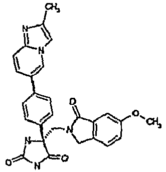 |
| 6018 | 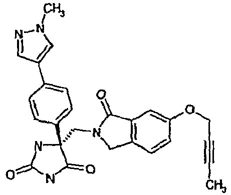 |
| 6019 | 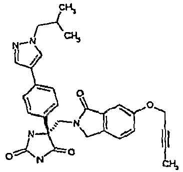 |
| 6020 | 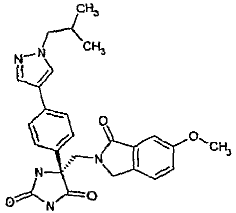 |
| 6021 | 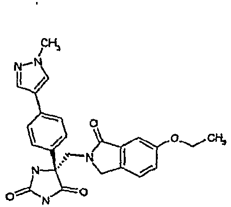 |
| 6022 | 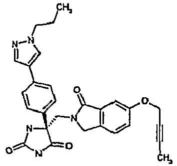 |
| 6023 | 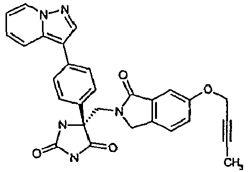 |
| 6024 | 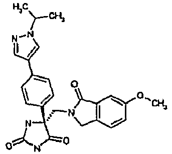 |
| 6025 | 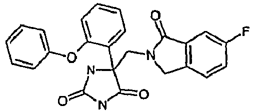 |
| 6026 | 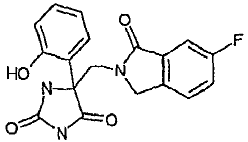 |
| 6027 | 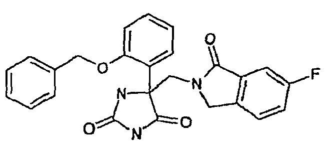 |
| 6028 | 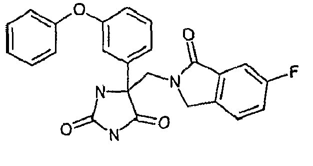 |
| 6029 | 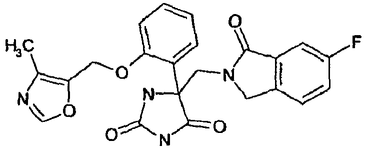 |
| 7000 | 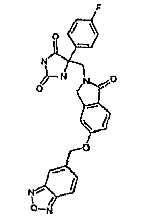 |
| 7001 | 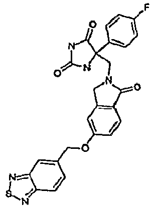 |
| 7002 | 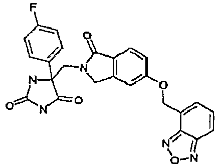 |
| 7003 | 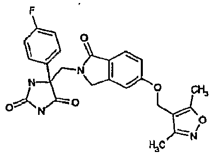 |
| 7004 | 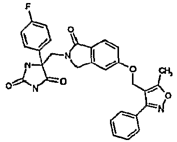 |
| 7005 | 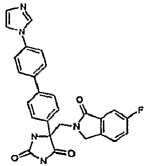 |
| 7006 | 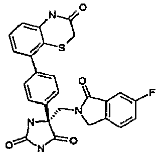 |
| 7007 | 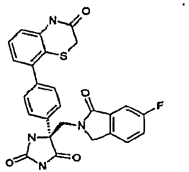 |
| 7008 | 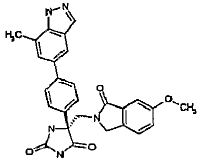 |
| 7009 | 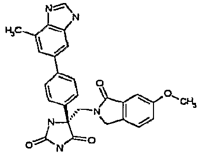 |
| 7010 | 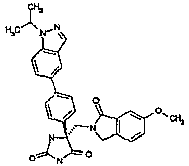 |
| 7011 | 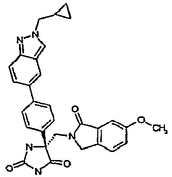 |
| 7012 | 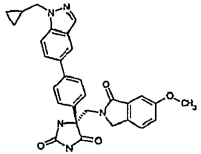 |
| 7013 | 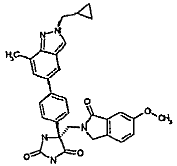 |
| 7014 | 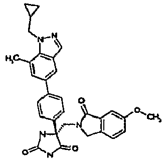 |
| 7015 | 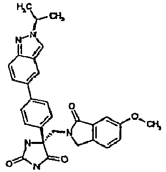 |
| 7016 | 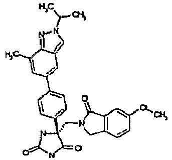 |
| 7017 | 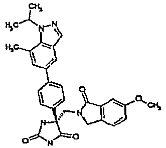 |
| 7018 | 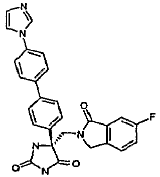 |
| 7019 | 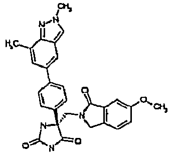 |
| 7020 | 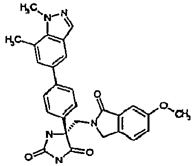 |
| 7021 | 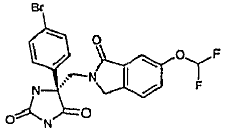 |
| 7022 | 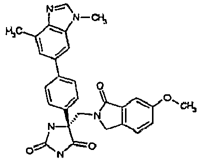 |
| 7023 | 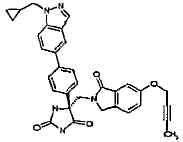 |
| 7024 | 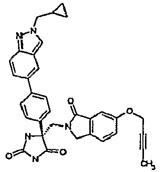 |
| 7025 | 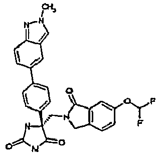 |
| 7026 | 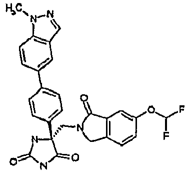 |
| 8001 | 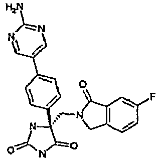 |
| 8002 | 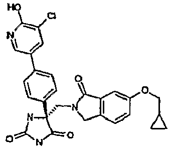 |
| 8003 | 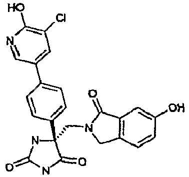 |
| 8004 | 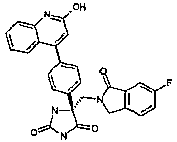 |
| 8005 | 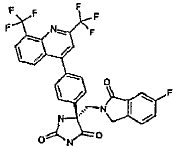 |
| 8006 | 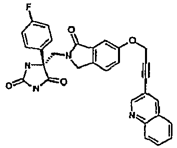 |
| 8007 | 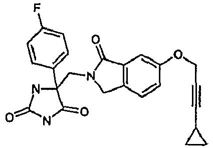 |
| 8008 | 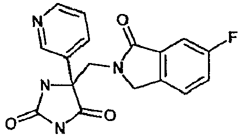 |
| 8009 | 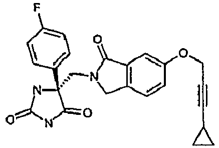 |
| 8010 | 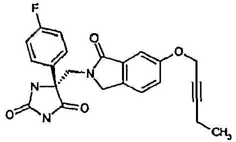 |
| 8011 | 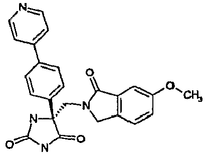 |
| 8012 | 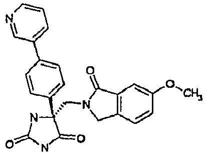 |
| 8013 | 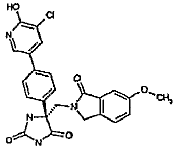 |
| 8014 | 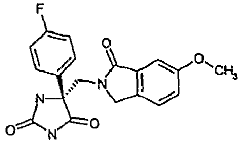 |
| 8015 | 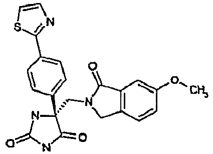 |
| 8016 | 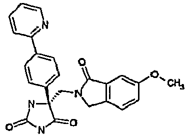 |
| 8017 | 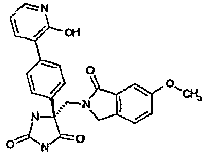 |
| 8018 | 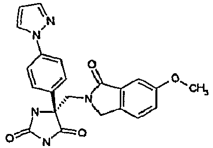 |
| 8019 | 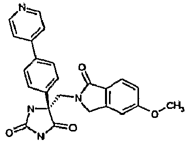 |
| 8020 | 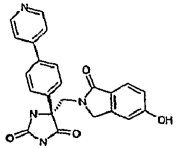 |
| 8021 | 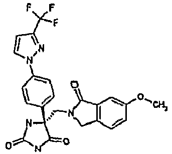 |
| 8022 | 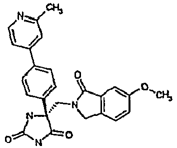 |
| 8023 | 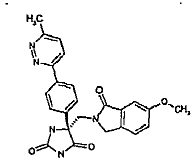 |
| 8024 | 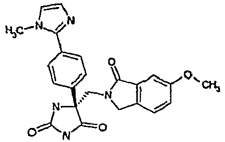 |
| 8025 | 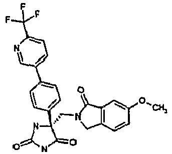 |
| 8027 | 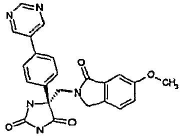 |
| 2021F | 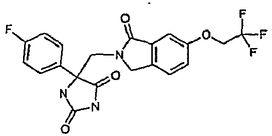 |
| 2023C | 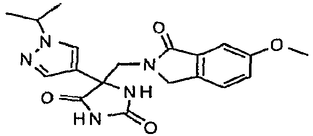 |
| 2024D | 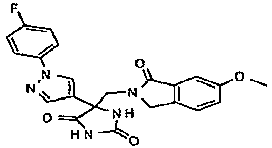 |
| 2022G | 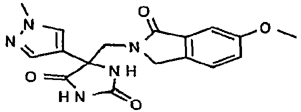 |
| 2025C | 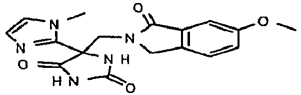 |
| 2028 | 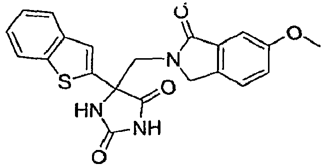 |
| 2026H | 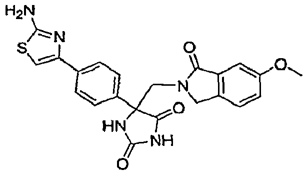 |
| 2030A | 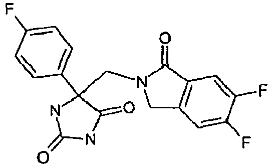 |
| 2030C | 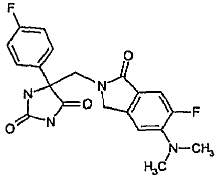 |
| 2030B | 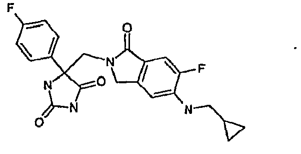 |
| 2031C | 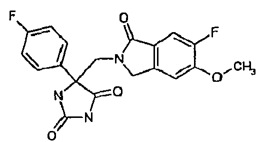 |
| 2031D | 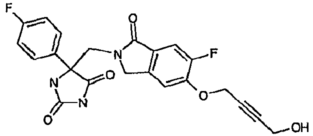 |
| 2030D | 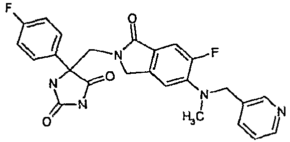 |
| 2031A | 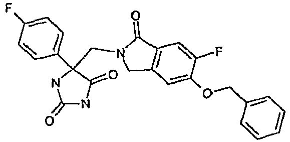 |
| 2031B | 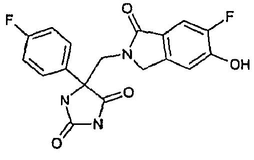 |
| 2032B | 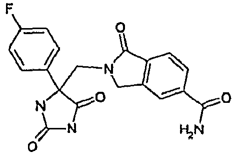 |
| 2031E | 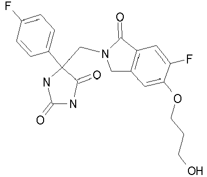 |
| 2031F | 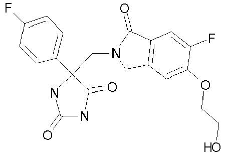 |
| 9200 | 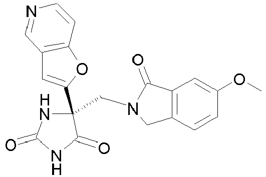 |
oder ein pharmazeutisch annehmbares Salz oder Solvat davon.

2. Die Verbindung nach Anspruch 1, ausgewählt aus der Gruppe, bestehend aus: 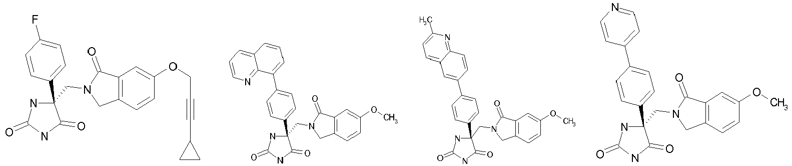 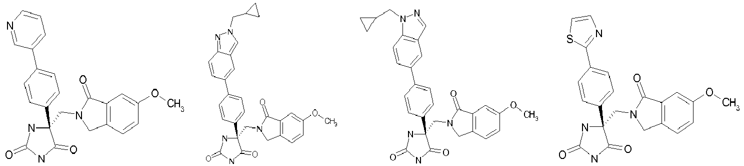 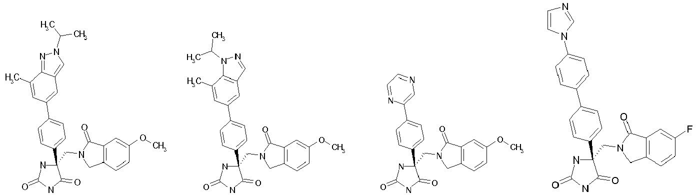 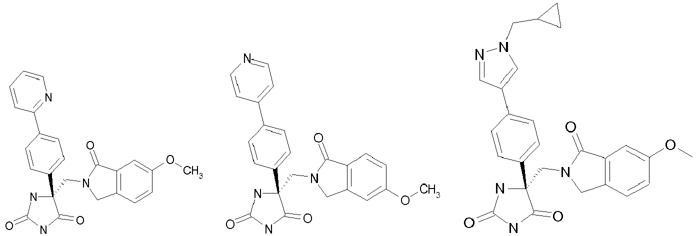 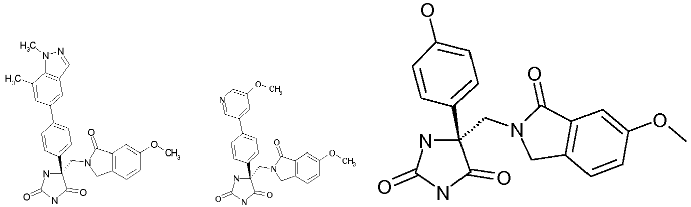 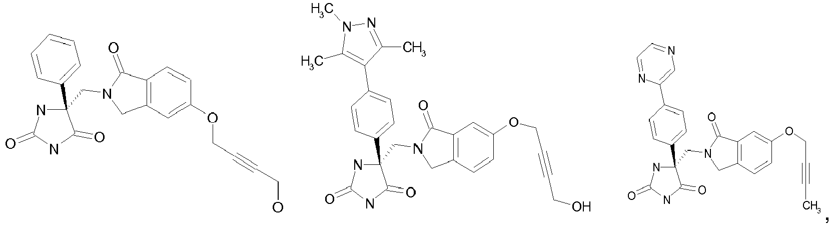 oder ein pharmazeutisch annehmbares Salz oder Solvat davon.

3. Die Verbindung nach Anspruch 1, die 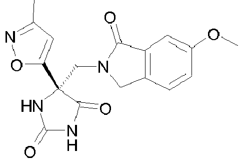 ist, oder ein pharmazeutisch annehmbares Salz oder Solvat davon.

4. Eine pharmazeutische Zusammensetzung, umfassend wenigstens eine Verbindung nach Anspruch 1, 2 oder 3 oder ein pharmazeutisch annehmbares Salz oder Solvat davon und wenigstens einen pharmazeutisch annehmbaren Träger.

5. Eine Verbindung nach Anspruch 1, 2 oder 3 oder ein pharmazeutisch annehmbares Salz oder Solvat davon zur Verwendung bei einem Verfahren zur Behandlung des menschlichen Körpers durch Therapie.

6. Eine Verbindung zur Verwendung nach Anspruch 5 oder ein pharmazeutisch annehmbares Salz oder Solvat davon, wobei die Therapie die Behandlung eines Zustands oder einer Erkrankung, ausgewählt aus Gelenkrheumatismus, Arthrose, Periodontitis, Gingivitis, kornealer Ulzeration, Wachstum solider Tumore und Tumorinvasion durch sekundäre Metastasen, neovaskulärem Glaukom, entzündlicher Darmerkrankung, multipler Sklerose und Psoriasis, ist.

7. Eine Verbindung zur Verwendung nach Anspruch 5 oder ein pharmazeutisch annehmbares Salz oder Solvat davon, wobei die Therapie die Behandlung eines Zustands oder einer Erkrankung, ausgewählt aus Fieber, kardiovaskulären Zuständen, Hämorrhagie, Koagulation, Kachexie, Anorexie, Alkoholsucht, akuter Phasenreaktion, akuter Infektion, Schock, Graft-versus-Host-Reaktion, Autoimmunerkrankung und HIV-Infektion, ist.

8. Eine Verbindung zur Verwendung nach Anspruch 5 oder ein pharmazeutisch annehmbares Salz oder Solvat davon, wobei die Therapie die Behandlung eines Zustands oder einer Erkrankung, ausgewählt aus septischem Schock, hämodynamischem Schock, Sepsis-Syndrom, postischämischer Reperfusionsverletzung, Malaria, mykobakterieller Infektion, Meningitis, Psoriasis, kongestiver Herzinsuffizienz, fibrotischen Erkrankungen, Kachexie, Transplantatabstoßung, Krebserkrankungen wie z.B. kutanem T-Zell-Lymphom, Erkrankungen, die Angiogenese einschließen, Autoimmunerkrankungen, Entzündungserkrankungen der Haut, entzündlichen Darmerkrankungen wie Morbus Crohn und Colitis, Arthrose und Gelenkrheumatismus, Spondylitis ankylosans, psoriatrischer Arthritis, Morbus Still bei Erwachsenen, Ureitis, Wegener-Granulomatose, Morbus Behget, Sjögren-Syndrom, Sarkoidose, Polymyositis, Dermatomyositis, multipler Sklerose, Ischias, komplexem regionalem Schmerzsyndrom, Strahlenschädigung, hyperoxischer Alveolarverletzung, Periodontalerkrankung, HIV, nichtinsulinabhängigem Diabetes mellitus, systemischem Lupus erythematodes, Glaukom, Sarkoidose, idiopathischer Lungenfibrose, bronchopulmonaler Dysplasie, Netzhauterkrankung, Sklerodermie, Osteoporose, renaler Ischämie, Myokardinfarkt, Zerebralinfarkt, zerebraler Ischämie, Nephritis, Hepatitis, Glomerulonephritis, kryptogener fibrosierender Alveolitis, Psoriasis, Transplantatabstoßung, atopischer Dermatitis, Vaskulitis, Allergie, saisonaler allergischer Rhinitis, reversibler Atemwegsobstruktion, Atemnotsyndrom bei Erwachsenen, Asthma, chronisch obstruktiver Lungenerkrankung (COPD) und Bronchitis.

9. Eine Verbindung nach Anspruch 1, 2 oder 3 oder ein pharmazeutisch annehmbares Salz oder Solvat davon in Kombination mit einer Verbindung, ausgewählt aus der Gruppe, bestehend aus Avonex, Betaseron, Copaxon oder anderen Verbindungen, die zur Behandlung von multipler Sklerose indiziert sind.

10. Eine Kombination aus einer Verbindung nach Anspruch 1, 2 oder 3 oder einem pharmazeutisch annehmbaren Salz oder Solvat davon und wenigstens einem Medikament, ausgewählt aus krankheitsmodifizierenden Antirheumatika (DMARDS), nichtsteroidalen Antiphlogistika (NSAIDs), Cyclooxygenase-2-selektiven (COX-2-) Inhibitoren, COX-1-Inhibitoren, Immunsuppressiva, Biological-Response-Modifizierern (BRMs), antiinflammatorischen Mitteln und H1-Antagonisten.

11. Verwendung einer Verbindung nach Anspruch 1, 2 oder 3 oder eines pharmazeutisch annehmbaren Salzes oder Solvats davon zur Herstellung eines Medikaments zur Behandlung eines Zustands oder einer Erkrankung wie in Anspruch 7, 8 oder 9 definiert.

## Revendications

1. Composé choisi dans le groupe constitué par:
| **ID Composé** | **Structure** |
|---|---|
| 3000 | 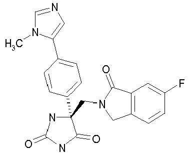 |
| 3001 | 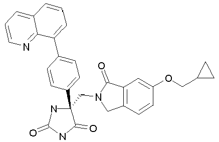 |
| 3002 | 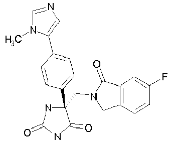 |
| 3003 | 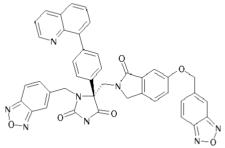 |
| 3004 | 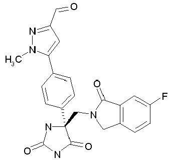 |
| 3005 | 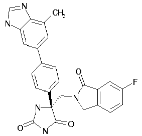 |
| 3006 | 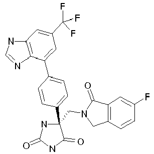 |
| 3007 | 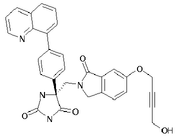 |
| 3008 | 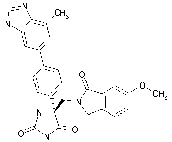 |
| 3009 | 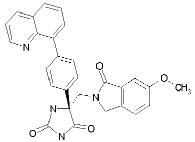 |
| 3010 | 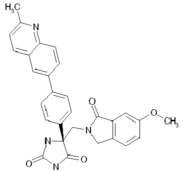 |
| 3011 | 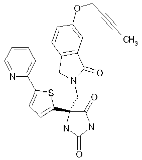 |
| 3012 | 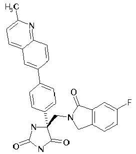 |
| 3013 | 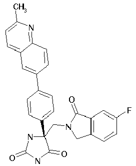 |
| 3014 | 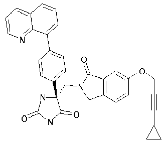 |
| 3015 | 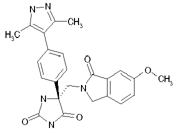 |
| 3016 | 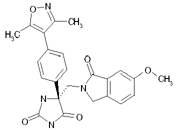 |
| 3017 | 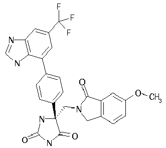 |
| 3018 | 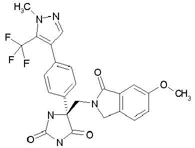 |
| 3019 | 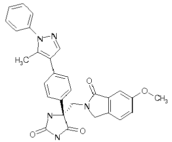 |
| 3020 | 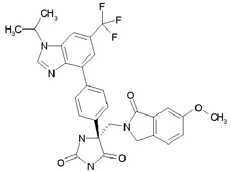 |
| 3021 | 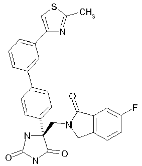 |
| 3022 | 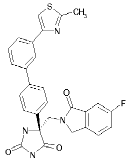 |
| 3024 | 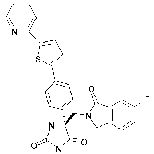 |
| 3025 | 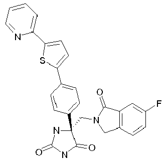 |
| 3026 | 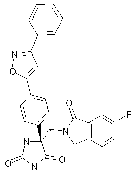 |
| 3027 | 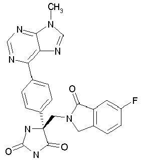 |
| 3028 | 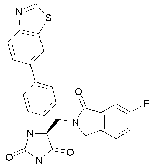 |
| 3029 | 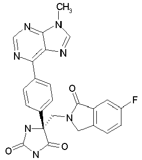 |
| 3030 | 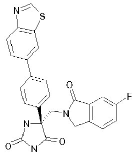 |
| 3031 | 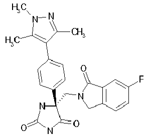 |
| 3032 | 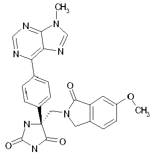 |
| 3033 | 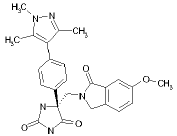 |
| 3034 | 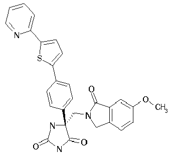 |
| 3035 | 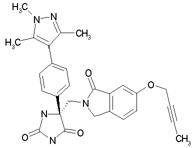 |
| 3003 | 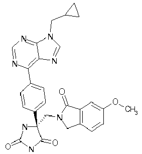 |
| 3037 | 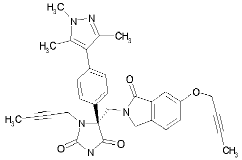 |
| 3038 | 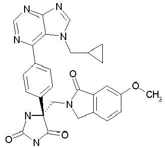 |
| 3039 | 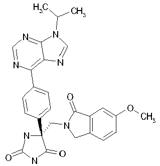 |
| 3040 | 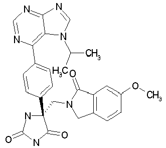 |
| 3041 | 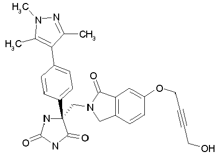 |
| 3042 | 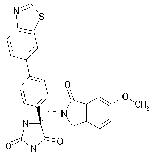 |
| 3043 | 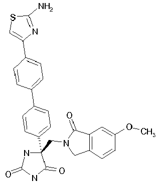 |
| 3044 | 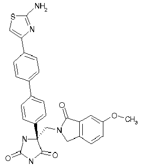 |
| 3045 | 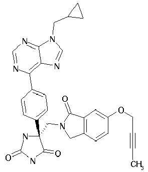 |
| 3046 | 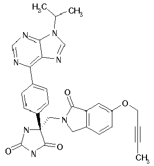 |
| 3047 | 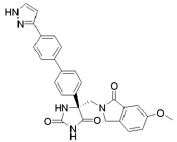 |
| 3048 | 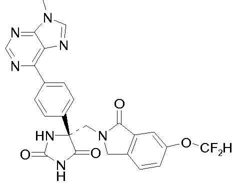 |
| 4001 | 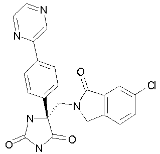 |
| 4002 | 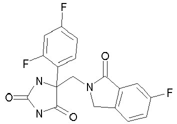 |
| 4003 | 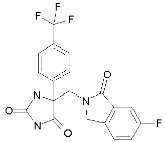 |
| 4004 !! SHAPE \* | 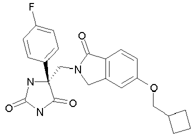 |
| 4005 | 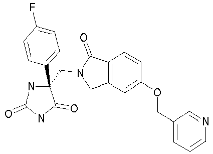 |
| 4006 | 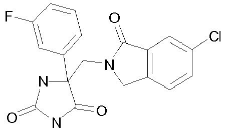 |
| 4007 | 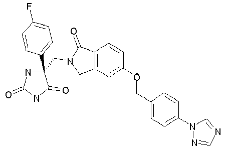 |
| 4008 | 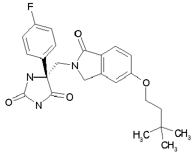 |
| 4009 | 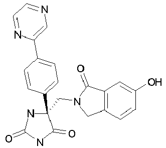 |
| 4010 | 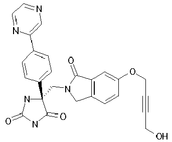 |
| 4011 | 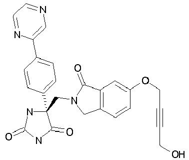 |
| 4012 | 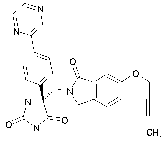 |
| 4013 | 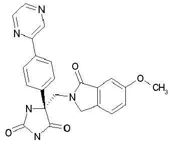 |
| 4014 | 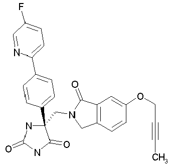 |
| 4015 | 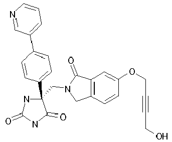 |
| 4016 | 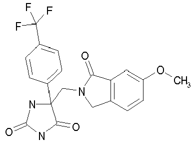 |
| 4017 | 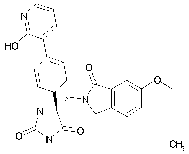 |
| 4018 | 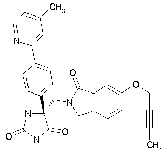 |
| 4019 | 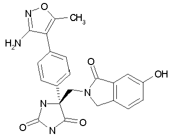 |
| 4020 | 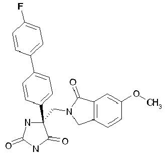 |
| 4021 | 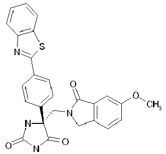 |
| 4022 | 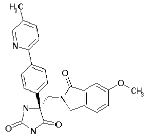 |
| 4023 | 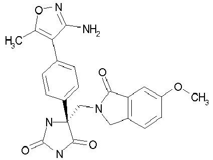 |
| 4024 | 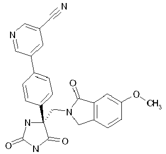 |
| 4025 | 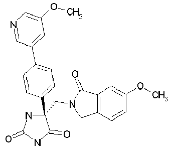 |
| 4026 | 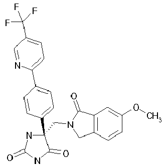 |
| 4027 | 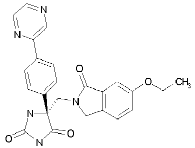 |
| 4028 | 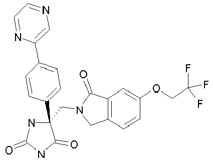 |
| 4029 | 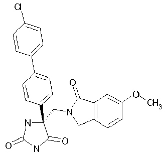 |
| 4030 | 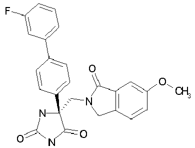 |
| 4031 | 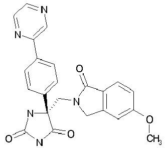 |
| 4032 | 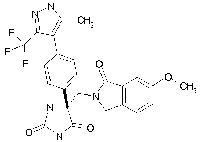 |
| 4033 | 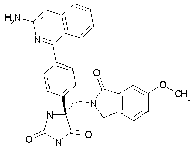 |
| 4034 | 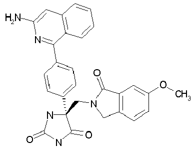 |
| 4035 | 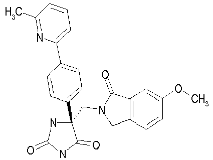 |
| 4036 | 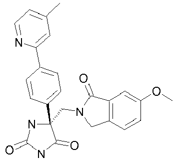 |
| 4037 | 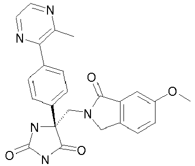 |
| 4038 | 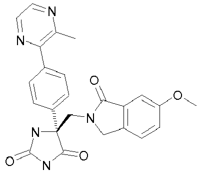 |
| 4039 | 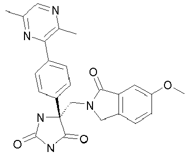 |
| 4040 | 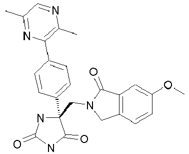 |
| 4041 | 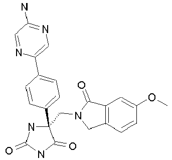 |
| 4042 | 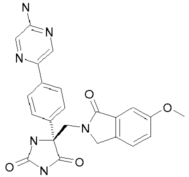 |
| 5000 | 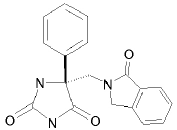 |
| 5001 | 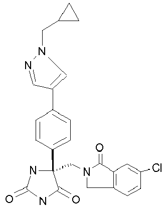 |
| 5003 | 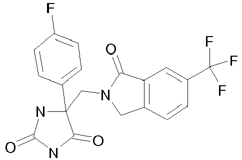 |
| 5005 | 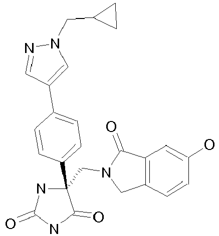 |
| 5006 | 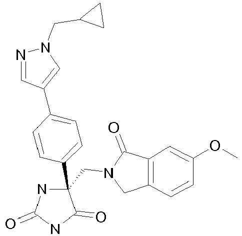 |
| 5007 | 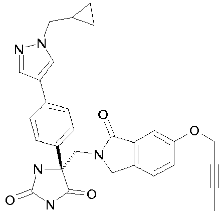 |
| 5009 | 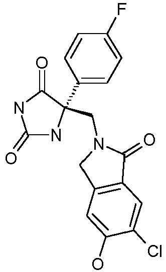 |
| 5010 | 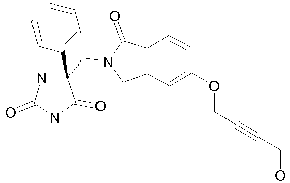 |
| 5011 | 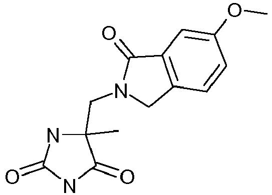 |
| 5012 | 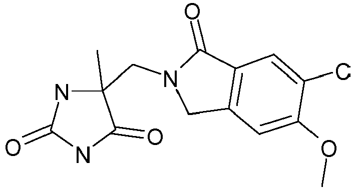 |
| 5013 | 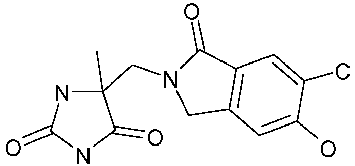 |
| 5014 | 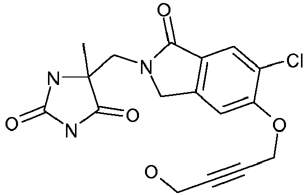 |
| 5015 | 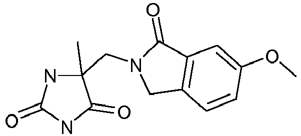 |
| 5016 | 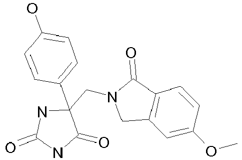 |
| 5017 | 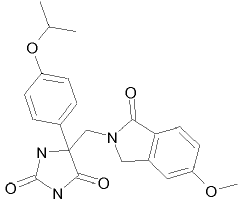 |
| 5018 | 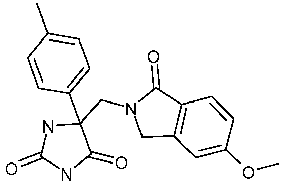 |
| 5019 | 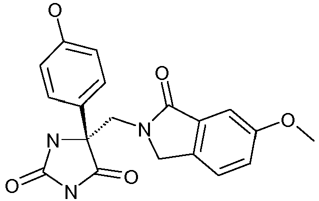 |
| 5020 | 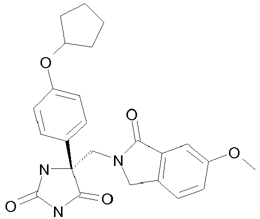 |
| 5021 | 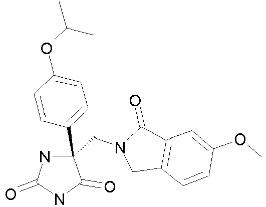 |
| 5022 | 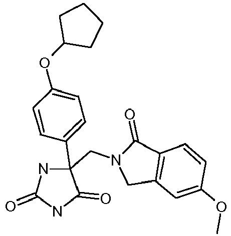 |
| 5023 | 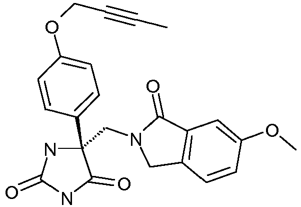 |
| 5024 | 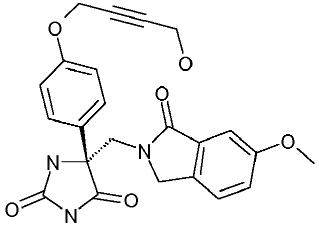 |
| 5025 | 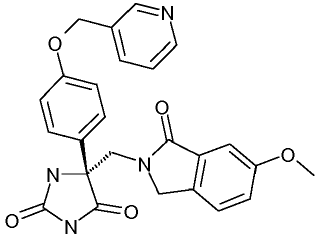 |
| 6000 | 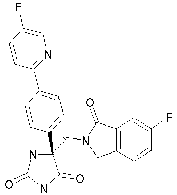 |
| 6001 | 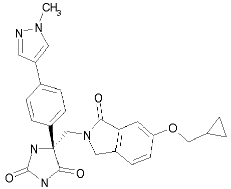 |
| 6002 | 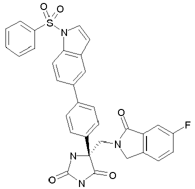 |
| 6003 | 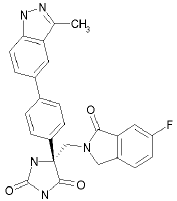 |
| 6004 | 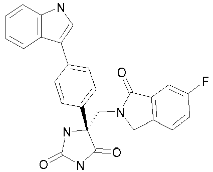 |
| 6005 | 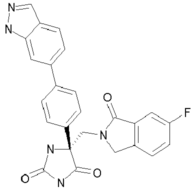 |
| 6006 | 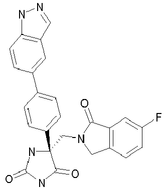 |
| 6007 | 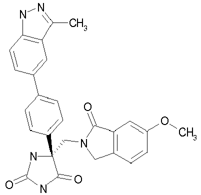 |
| 6008 | 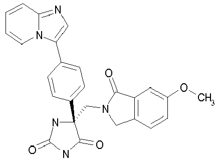 |
| 6009 | 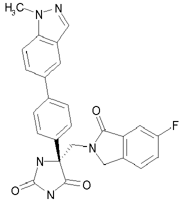 |
| 6010 | 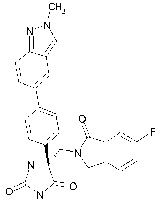 |
| 6011 | 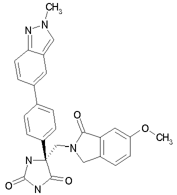 |
| 6012 | 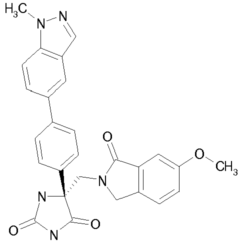 |
| 6013 | 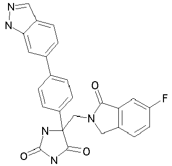 |
| 6014 | 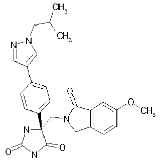 |
| 6015 | 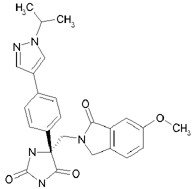 |
| 6016 | 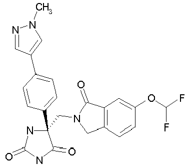 |
| 6017 | 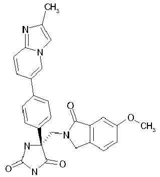 |
| 6018 | 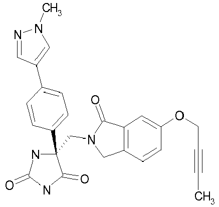 |
| 6019 | 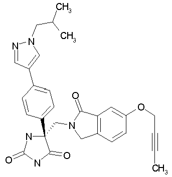 |
| 6020 | 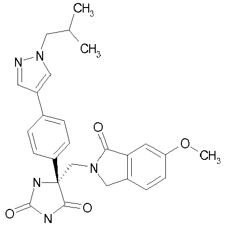 |
| 6021 | 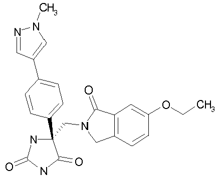 |
| 6022 | 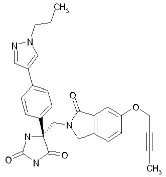 |
| 6023 | 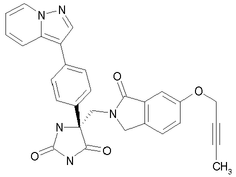 |
| 6024 | 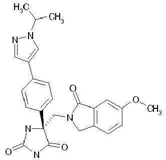 |
| 6025 | 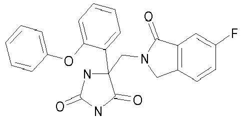 |
| 6026 | 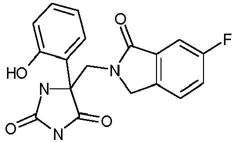 |
| 6027 | 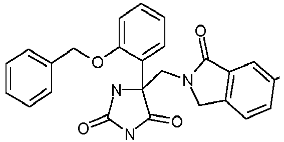 |
| 6028 | 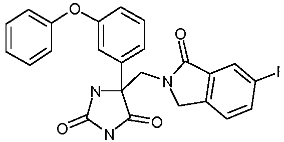 |
| 6029 | 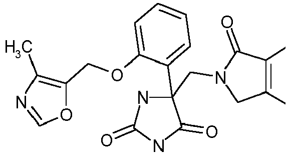 |
| 7000 | 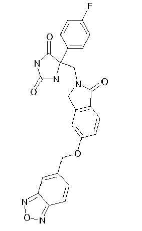 |
| 7001 | 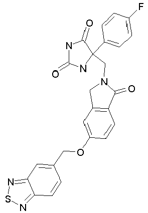 |
| 7002 | 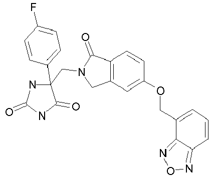 |
| 7003 | 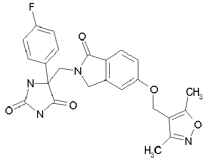 |
| 7004 | 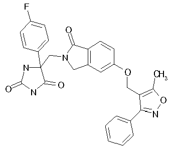 |
| 7005 | 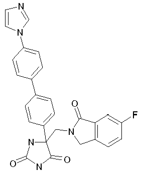 |
| 7006 | 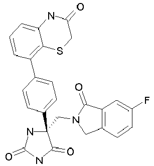 |
| 7007 | 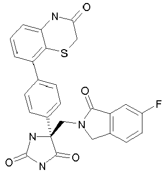 |
| 7008 | 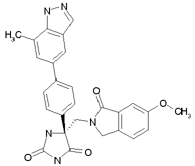 |
| 7009 | 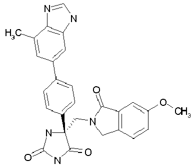 |
| 7010 | 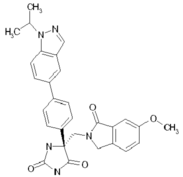 |
| 7011 | 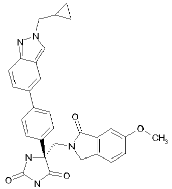 |
| 7012 | 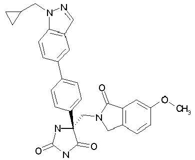 |
| 7013 | 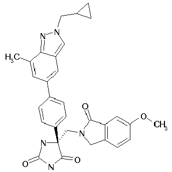 |
| 7014 | 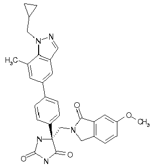 |
| 7015 | 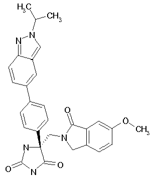 |
| 7016 | 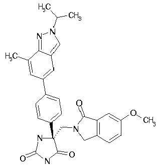 |
| 7017 | 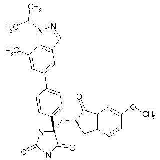 |
| 7018 | 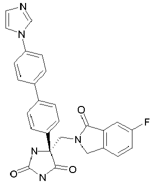 |
| 7019 | 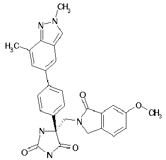 |
| 7020 | 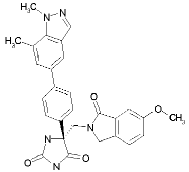 |
| 7021 | 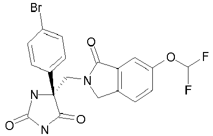 |
| 7022 | 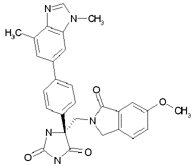 |
| 7023 | 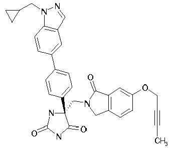 |
| 7024 | 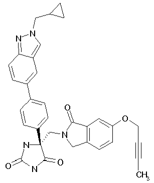 |
| 7025 | 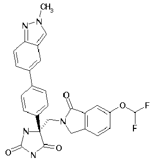 |
| 7026 | 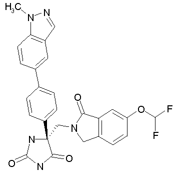 |
| 8001 | 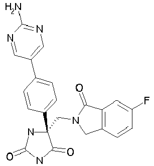 |
| 8002 | 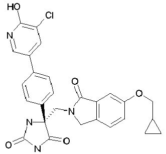 |
| 8003 | 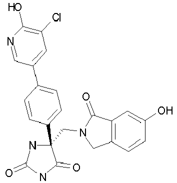 |
| 8004 | 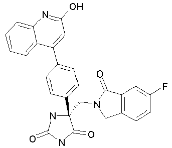 |
| 8005 | 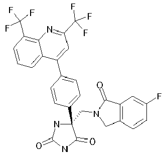 |
| 8006 | 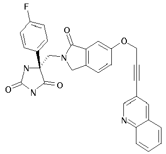 |
| 8007 | 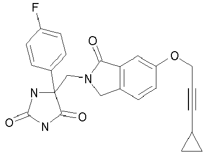 |
| 8008 | 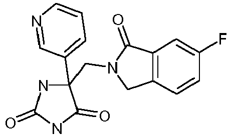 |
| 8009 | 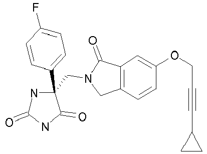 |
| 8010 | 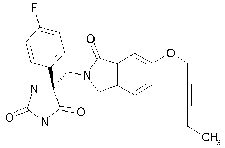 |
| 8011 | 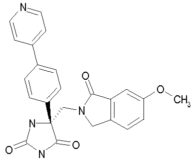 |
| 8012 | 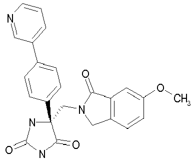 |
| 8013 | 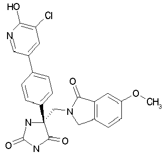 |
| 8014 | 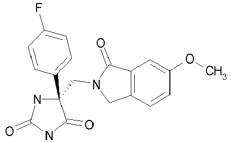 |
| 8015 | 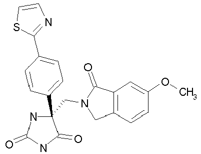 |
| 8016 | 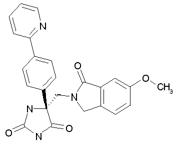 |
| 8017 | 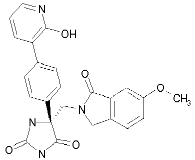 |
| 8018 | 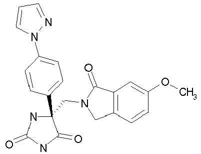 |
| 8019 | 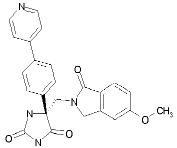 |
| 8020 | 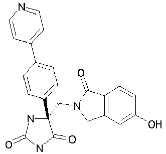 |
| 8021 | 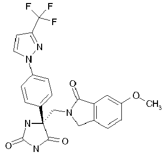 |
| 8022 | 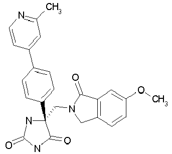 |
| 8023 | 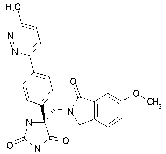 |
| 8024 | 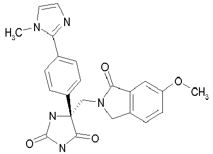 |
| 8025 | 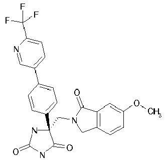 |
| 8027 | 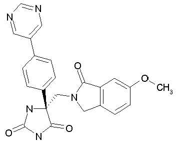 |
| 2021F | 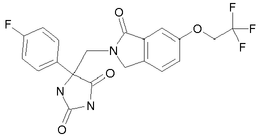 |
| 2023C | 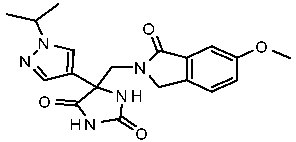 |
| 2024D | 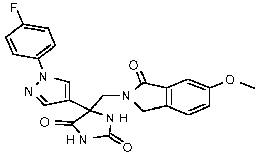 |
| 2022G | 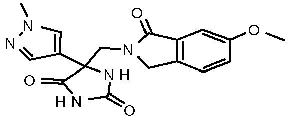 |
| 2025C | 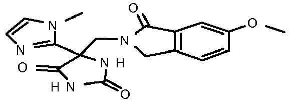 |
| 2028 | 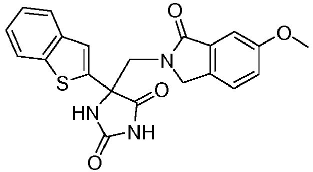 |
| 2026H | 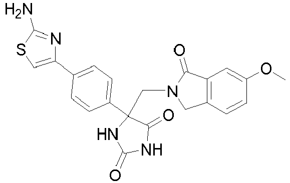 |
| 2030A | 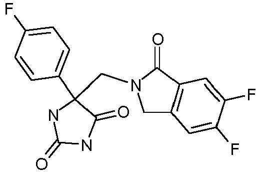 |
| 2030C | 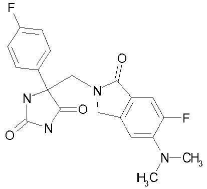 |
| 2030B | 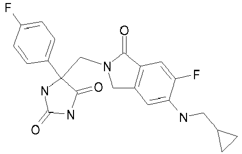 |
| 2031C | 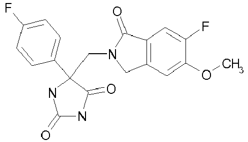 |
| 2031D | 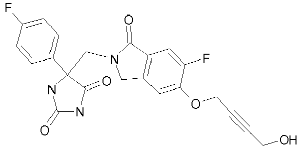 |
| 2030D | 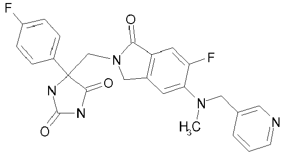 |
| 2031A | 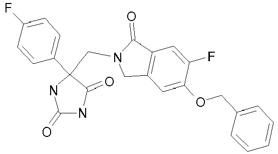 |
| 2031B | 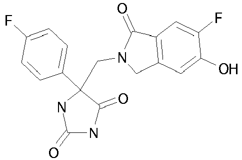 |
| 2032B | 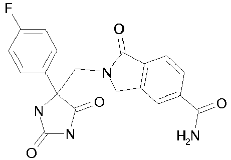 |
| 2031E | 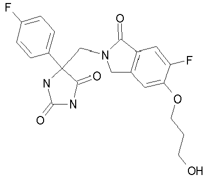 |
| 2031F | 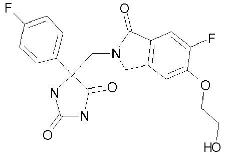 |
| 9200 | 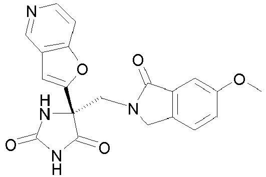 |
ou sel ou solvate pharmaceutiquement acceptable de celui-ci.

2. Composé selon la revendication 1, choisi dans le groupe constitué par: 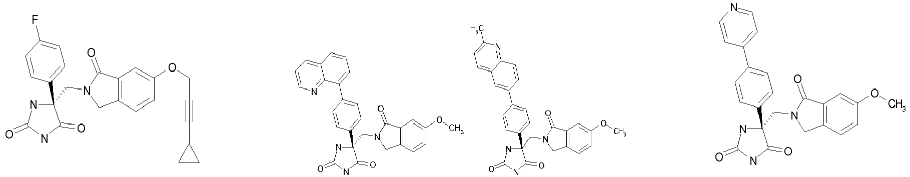 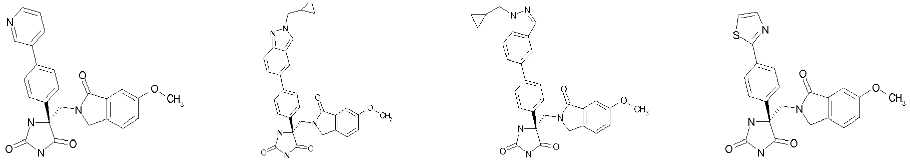 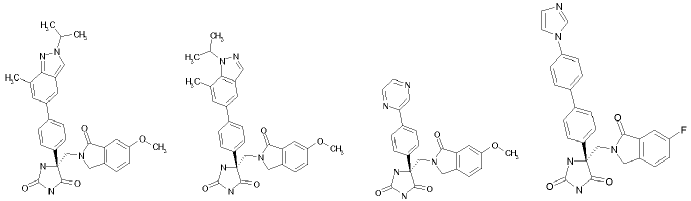 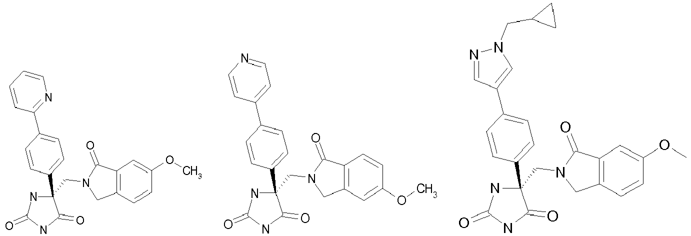 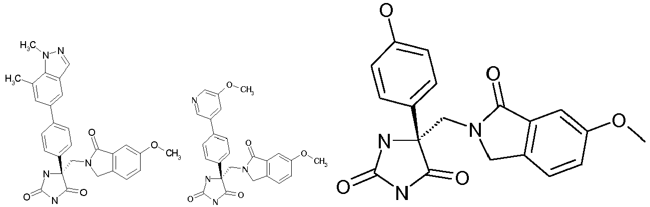 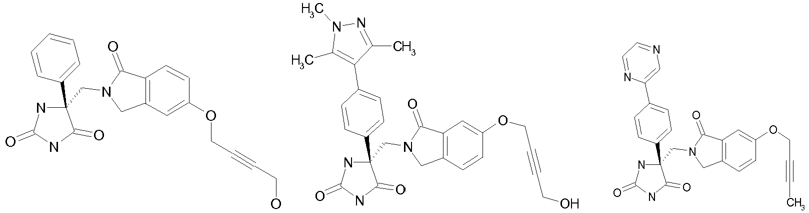 ou sel ou solvate pharmaceutiquement acceptable de celui-ci.

3. Composé selon la revendication 1, qui est 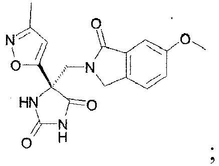 ou sel ou solvate pharmaceutiquement acceptable de celui-ci.

4. Composition pharmaceutique comprenant au moins un composé selon la revendication 1, 2 ou 3 ou un sel ou solvate pharmaceutiquement acceptable de celui-ci, et au moins un véhicule pharmaceutiquement acceptable.

5. Composé selon la revendication 1, 2 ou 3 ou sel ou solvate pharmaceutiquement acceptable de celui-ci, pour l'utilisation dans un procédé de traitement du corps humain par thérapie.

6. Composé pour l'utilisation selon la revendication 5 ou sel ou solvate pharmaceutiquement acceptable de celui-ci, dans lequel la thérapie est le traitement d'une affection ou maladie choisie parmi une polyarthrite rhumatoïde, une ostéoarthrite, une parodontite, une gingivite, une ulcération cornéenne, une croissance de tumeur solide et une invasion tumorale par des métastases secondaires, un glaucome néovasculaire, une maladie intestinale inflammatoire, une sclérose en plaques et un psoriasis.

7. Composé pour l'utilisation selon la revendication 5 ou sel ou solvate pharmaceutiquement acceptable de celui-ci, dans lequel la thérapie est le traitement d'une affection ou maladie choisie parmi une fièvre, des affections cardio-vasculaires, une hémorragie, une coagulation, une cachexie, une anorexie, un alcoolisme, une réponse de phase aiguë, une infection aiguë, un choc, une réaction greffon contre hôte, une maladie auto-immune et une infection par VIH.

8. Composé pour l'utilisation selon la revendication 5 ou sel ou solvate pharmaceutiquement acceptable de celui-ci, dans lequel la thérapie est le traitement d'une affection ou maladie choisie parmi un choc septique, un choc hémodynamique, un syndrome septicémique, une blessure de reperfusion post-ischémique, une malaria, une infection mycobactérienne, une méningite, un psoriasis, une insuffisance cardiaque congestive, des maladies fibrotiques, une cachexie, un rejet de greffon, des cancers tels qu'un lymphome cutané à cellules T, des maladies faisant intervenir l'angiogenèse, des maladies auto-immunes, des maladies inflammatoires cutanées, des maladies intestinales inflammatoires telles qu'une maladie de Crohn et une colite, une ostéoarthrite et une polyarthrite rhumatoïde, une spondylarthrite ankylosante, une arthrite psoriasique, une maladie de Still de l'adulte, une uréite, une granulomatose de Wegener, une maladie de Behcehe, un syndrome de Sjogren, une sarcoïdose, une polymyosite, une dermatomyosite, une sclérose en plaques, une sciatique, un syndrome de douleur régionale complexe, un dommage par rayonnement, une lésion alvéolaire hyperoxique, une maladie parodontale, un VIH, un diabète sucré non insulinodépendant, un lupus érythémateux systémique, un glaucome, une sarcoïdose, une fibrose pulmonaire idiopathique, une dysplasie broncho-pulmonaire, une maladie rétinienne, un sclérodermie, une ostéoporose, une ischémie rénale, un infarctus du myocarde, une attaque cérébrale, une ischémie cérébrale, une néphrite, une hépatite, une glomérulonéphrite, une alvéolite fibrosante cryptogénique, un psoriasis, un rejet de greffon, une dermatite atopique, une vasculite, une allergie, une rhinite allergique saisonnière, une obstruction des voies aériennes réversible, un syndrome de détresse respiratoire de l'adulte, un asthme, une maladie pulmonaire obstructive chronique (COPD) et une bronchite.

9. Composé selon la revendication 1, 2 ou 3 ou sel ou solvate pharmaceutiquement acceptable de celui-ci, en combinaison avec un composé choisi dans le groupe constitué par l'Avonex, le Betaseron, le Copaxone ou d'autres composés indiqués pour le traitement d'une sclérose en plaques.

10. Combinaison d'un composé selon la revendication 1, 2 ou 3 ou d'un sel ou solvate pharmaceutiquement acceptable de celui-ci et d'au moins un médicament choisi parmi les substances médicamenteuses antirhumatismales modifiant la maladie (DMARDs), les substances médicamenteuses anti-inflammatoires non stéroïdiennes (NSAIDs), les inhibiteurs sélectifs vis-à-vis de la cycloxygénase-2 (COX-2), les inhibiteurs de COX-1, les immunosuppresseurs, les modificateurs de réponse biologique (BRMs), les agents anti-inflammatoires et les antagonistes de H1.

11. Utilisation d'un composé selon la revendication 1, 2 ou 3 ou d'un sel ou solvate pharmaceutiquement acceptable de celui-ci pour la fabrication d'un médicament pour traiter une affection ou maladie telle que définie dans la revendication 7, 8 ou 9.
